# EUROPEAN PATENT APPLICATION

(11) **EP 3 735 984 A1**
(43) Date of publication of application: **11.11.2020**
(21) Application number: 20179172.0
(22) Date of filing: 26.05.2017
(51) Int. Cl.: A61K 39/00, C12N 15/86, C07K 14/705, C07K 14/54, C07K 14/715

(54) **NEOEPITOPE VACCINE COMPOSITIONS AND METHODS OF USE THEREOF**

(30) Priority: 27.05.2016 US 201662342752 P
(62) Divisional of application: 17803712.3
(71) Applicant: Etubics Corporation, Seattle WA 98119 (US); Nant Holdings IP, LLC, Culver City CA 90232 (US); NantCell, Inc., Culver City, CA 90232 (US)
(72) Inventor: JONES, Frank R, Seattle, WA 98119 (US); RICE, Adrian, Seattle, WA 98119 (US); SOON-SHIONG, Patrick, Culver City, CA 90232 (US); RABIZADEH, Shahrooz, Culver City, CA 90232 (US); NIAZI, Kayvan, Culver City, CA 90232 (US)
(74) Representative: J A Kemp LLP

(57) **Abstract**

In certain embodiments, methods and compositions are provided for generating immune responses against tumor neo-antigens or neo-epitopes. In particular embodiments there may be provided methods for constructing and producing recombinant adenovirus-based vector vaccines containing nucleic acid sequences encoding tumor neo-antigens and neo-epitopes that allow for vaccinations in individuals with preexisting immunity to adenovirus. In additional embodiments, methods and compositions are provided for the treatment of cancer using immunotherapy based on recombinant adenovirus-based vectors combined with engineered natural killer cells. In some embodiments, the methods and compositions further comprises a nucleic acid encoding for an immunological fusion partner.

SEQUENCE LISTING

<110> ETUBICS CORPORATION

<110> NANT HOLDINGS IP, LLC

<110> NANTCELL, INC.

<120> NEOEPITOPE VACCINE COMPOSITIONS AND METHODS OF USE THEREOF

<130> N415339EP-A

<150> PCT/US2017/034802
<151> 2017-05-26

<150> 62/342,752
<151> 2016-05-27

<160> 112

<170> PatentIn version 3.5

<210> 1
<211> 9
<212> PRT
<213> Artificial Sequence

<220>
<223> Description of Artificial Sequence: Synthetic
      peptide

<400> 1
Gly Glu Thr Val Thr Met Pro Cys Pro
1               5


<210> 2
<211> 9
<212> PRT
<213> Artificial Sequence

<220>
<223> Description of Artificial Sequence: Synthetic
      peptide

<400> 2
Val Gly Pro Val Asn Pro Ser His Arg
1               5


<210> 3
<211> 9
<212> PRT
<213> Artificial Sequence

<220>
<223> Description of Artificial Sequence: Synthetic
      peptide

<400> 3
Gly Leu Gly Ala Gln Cys Ser Glu Ala
1               5

<210> 4
<211> 9
<212> PRT
<213> Artificial Sequence

<220>
<223> Description of Artificial Sequence: Synthetic
      peptide

<400> 4
Arg Lys Leu Thr Thr Glu Leu Thr Ile
1               5


<210> 5
<211> 9
<212> PRT
<213> Artificial Sequence

<220>
<223> Description of Artificial Sequence: Synthetic
      peptide

<400> 5
Pro Glu Arg Arg Lys Leu Thr Thr Glu
1               5


<210> 6
<211> 9
<212> PRT
<213> Artificial Sequence

<220>
<223> Description of Artificial Sequence: Synthetic
      peptide

<400> 6
Met Asp Trp Val Trp Met Asp Thr Thr
1               5


<210> 7
<211> 9
<212> PRT
<213> Artificial Sequence

<220>
<223> Description of Artificial Sequence: Synthetic
      peptide

<400> 7
Val Trp Met Asp Thr Thr Leu Ser Leu
1               5


<210> 8
<211> 9
<212> PRT
<213> Artificial Sequence

<220>

<223> Description of Artificial Sequence: Synthetic
      peptide

<400> 8
Gly Lys Thr Leu Asn Pro Ser Gln Thr
1               5


<210> 9
<211> 9
<212> PRT
<213> Artificial Sequence

<220>
<223> Description of Artificial Sequence: Synthetic
      peptide

<400> 9
Arg Glu Gly Lys Thr Leu Asn Pro Ser
1               5


<210> 10
<211> 9
<212> PRT
<213> Artificial Sequence

<220>
<223> Description of Artificial Sequence: Synthetic
      peptide

<400> 10
Val Arg Asn Ala Thr Ser Tyr Arg Cys
1               5


<210> 11
<211> 9
<212> PRT
<213> Artificial Sequence

<220>
<223> Description of Artificial Sequence: Synthetic
      peptide

<400> 11
Asn Val Thr Val Arg Asn Ala Thr Ser
1               5


<210> 12
<211> 9
<212> PRT
<213> Artificial Sequence

<220>
<223> Description of Artificial Sequence: Synthetic
      peptide

<400> 12
Phe Ala Met Ala Gln Ile Pro Ser Leu
1               5

<210> 13
<211> 9
<212> PRT
<213> Artificial Sequence

<220>
<223> Description of Artificial Sequence: Synthetic
      peptide

<400> 13
Ala Gln Ile Pro Ser Leu Ser Leu Arg
1               5


<210> 14
<211> 15
<212> PRT
<213> Artificial Sequence

<220>
<223> Description of Artificial Sequence: Synthetic
      peptide

<400> 14
Pro Phe Ala Met Ala Gln Ile Pro Ser Leu Ser Leu Arg Ala Val
1               5                   10                  15


<210> 15
<211> 15
<212> PRT
<213> Artificial Sequence

<220>
<223> Description of Artificial Sequence: Synthetic
      peptide

<400> 15
Leu Pro Asn Val Thr Val Arg Asn Ala Thr Ser Tyr Arg Cys Gly
1               5                   10                  15


<210> 16
<211> 15
<212> PRT
<213> Artificial Sequence

<220>
<223> Description of Artificial Sequence: Synthetic
      peptide

<400> 16
Ser Trp Phe Arg Glu Gly Lys Thr Leu Asn Pro Ser Gln Thr Ser
1               5                   10                  15


<210> 17
<211> 15
<212> PRT
<213> Artificial Sequence

<220>

<223> Description of Artificial Sequence: Synthetic
      peptide

<400> 17
Ala Val Met Asp Trp Val Trp Met Asp Thr Thr Leu Ser Leu Ser
1               5                   10                  15


<210> 18
<211> 15
<212> PRT
<213> Artificial Sequence

<220>
<223> Description of Artificial Sequence: Synthetic
      peptide

<400> 18
Leu Gly Pro Glu Arg Arg Lys Leu Thr Thr Glu Leu Thr Ile Ile
1               5                   10                  15


<210> 19
<211> 15
<212> PRT
<213> Artificial Sequence

<220>
<223> Description of Artificial Sequence: Synthetic
      peptide

<400> 19
Asn Asn Gly Leu Gly Ala Gln Cys Ser Glu Ala Val Thr Leu Asn
1               5                   10                  15


<210> 20
<211> 15
<212> PRT
<213> Artificial Sequence

<220>
<223> Description of Artificial Sequence: Synthetic
      peptide

<400> 20
Asn Val Gly Glu Thr Val Thr Met Pro Cys Pro Lys Val Phe Ser
1               5                   10                  15


<210> 21
<211> 9
<212> PRT
<213> Artificial Sequence

<220>
<223> Description of Artificial Sequence: Synthetic
      peptide

<400> 21
Glu Gln Val Trp Gly Met Ala Val Arg
1               5

<210> 22
<211> 15
<212> PRT
<213> Artificial Sequence

<220>
<223> Description of Artificial Sequence: Synthetic
      peptide

<400> 22
Cys Gln Gly Pro Glu Gln Val Trp Gly Met Ala Val Arg Glu Leu
1               5                   10                  15


<210> 23
<211> 45
<212> DNA
<213> Artificial Sequence

<220>
<223> Description of Artificial Sequence: Synthetic
      oligonucleotide

<400> 23
cccttcgcca tggcccagat ccccagcctg agcctgaggg ccgtg                        45


<210> 24
<211> 45
<212> DNA
<213> Artificial Sequence

<220>
<223> Description of Artificial Sequence: Synthetic
      oligonucleotide

<400> 24
ctgcccaacg tgaccgtgag gaacgccacc agctacaggt gcggc                        45


<210> 25
<211> 45
<212> DNA
<213> Artificial Sequence

<220>
<223> Description of Artificial Sequence: Synthetic
      oligonucleotide

<400> 25
agctggttca gggagggcaa gaccctgaac cccagccaga ccagc                        45


<210> 26
<211> 45
<212> DNA
<213> Artificial Sequence

<220>
<223> Description of Artificial Sequence: Synthetic
      oligonucleotide

<400> 26
gccgtgatgg actgggtgtg gatggacacc accctgagcc tgagc                45

<210> 27
<211> 45
<212> DNA
<213> Artificial Sequence

<220>
<223> Description of Artificial Sequence: Synthetic
      oligonucleotide

<400> 27
ctgggccccg agaggaggaa gctgaccacc gagctgacca tcatc                45

<210> 28
<211> 45
<212> DNA
<213> Artificial Sequence

<220>
<223> Description of Artificial Sequence: Synthetic
      oligonucleotide

<400> 28
aacaacggcc tgggcgccca gtgcagcgag gccgtgaccc tgaac                45

<210> 29
<211> 45
<212> DNA
<213> Artificial Sequence

<220>
<223> Description of Artificial Sequence: Synthetic
      oligonucleotide

<400> 29
aacgtgggcg agaccgtgac catgccctgc cccaaggtgt tcagc                45

<210> 30
<211> 45
<212> DNA
<213> Artificial Sequence

<220>
<223> Description of Artificial Sequence: Synthetic
      oligonucleotide

<400> 30
tgccagggcc ccgagcaggt gtggggcatg gccgtgaggg agctg                45

<210> 31
<211> 402
<212> DNA
<213> Artificial Sequence

<220>
<223> Description of Artificial Sequence: Synthetic

polynucleotide

<400> 31
ctcgaggaag cttgccgcca ccatgccatt tgccatggcc cagatcccca gcctgagcct 60

gagagctgtg ctgcctaatg tgaccgtgcg gaacgccacc agctacagat gtggcagctg 120

gttcagagag ggcaagaccc tgaaccccag ccagaccagc gccgtgatgg actgggtgtg 180

gatggacacc accctgtccc tgagcctggg ccccgagaga agaaagctga ccaccgagct 240

gacaatcatc aacaatggcc tgggcgctca gtgtagcgag gccgtgaccc tgaataatgt 300

gggcgagaca gtgaccatgc cctgccccaa ggtgttcagc tgccagggcc ccgaacaagt 360

gtggggaatg gctgtgcgcg agctgtgaga tatcgcggcc gc 402

<210> 32
<211> 591
<212> DNA
<213> Artificial Sequence

<220>
<223> Description of Artificial Sequence: Synthetic
      polynucleotide

<400> 32
ctcgaggaag cttgccgcca ccatgccatt tgccatggcc cagatcccca gcctgagcct 60

gagagctgtg ggaagcggga gtggctcagg ttcaggactg cctaatgtga ccgtgcggaa 120

cgccaccagc tacagatgtg gcggaagtgg gtcaggctcc ggttctggaa gctggttcag 180

agagggcaag accctgaacc ccagccagac cagcgggtca ggaagtggta gcggctccgg 240

ggccgtgatg gactgggtgt ggatggacac caccctgtcc ctgagcggaa gtggatcagg 300

ttccggctct gggctgggcc ccgagagaag aaagctgacc accgagctga caatcatcgg 360

atccgggtct ggcagtggtt caggcaacaa tggcctgggc gctcagtgta gcgaggccgt 420

gaccctgaat ggatcagggt ccggcagcgg tagtggcaat gtgggcgaga cagtgaccat 480

gccctgcccc aaggtgttca gcgggtccgg atctggtagt ggctcaggtt gccagggccc 540

cgaacaagtg tggggaatgg ctgtgcgcga gctgtgagat atcgcggccg c 591

<210> 33
<211> 642
<212> DNA
<213> Artificial Sequence

<220>
<223> Description of Artificial Sequence: Synthetic
      polynucleotide

<400> 33
ctcgaggaag cttgccgcca ccatgcctag cgagagccct ccatttgcca tggcccagat 60

ccccagcctg agcctgagag ctgtgtctgg cgctatggga gcccacagca tccccctgcc 120

taatgtgacc gtgcggaacg ccaccagcta cagatgtggc gtgggacctc ctggccctcc 180

tgcttccctg agctggttca gagagggcaa gaccctgaac cccagccaga ccagcatgag 240

cggcaccctg ctgacagagc tgagagccgt gatggactgg gtgtggatgg acaccaccct 300

gtccctgagc agctggatct gtgtggtgtc cgccacagac ctgggccccg agagaagaaa 360

gctgaccacc gagctgacaa tcatcctgca gggcctggac tacagctgcg aggccaacaa 420

tggcctgggc gctcagtgta gcgaggccgt gaccctgaat ttcaccgtgc ccacctgttg 480

gaggcccgcc aatgtgggcg agacagtgac catgccctgc cccaaggtgt tcagcaactt 540

ctacagcaaa gtgcggggct tcatgtgcca gggccccgaa caagtgtggg gaatggctgt 600

gcgcgagctg aacatgaacc tgctgtgaga tatcgcggcc gc 642

<210> 34
<211> 831
<212> DNA
<213> Artificial Sequence

<220>
<223> Description of Artificial Sequence: Synthetic
      polynucleotide

<400> 34
ctcgaggaag cttgccgcca ccatgcctag cgagagccct ccatttgcca tggcccagat 60

ccccagcctg agcctgagag ctgtgtctgg cgctatggga ggaagcggga gtggctcagg 120

ttcaggagcc cacagcatcc ccctgcctaa tgtgaccgtg cggaacgcca ccagctacag 180

atgtggcgtg ggacctcctg gcggaagtgg gtcaggctcc ggttctggac ctcctgcttc 240

cctgagctgg ttcagagagg gcaagaccct gaaccccagc cagaccagca tgagcggcac 300

cctggggtca ggaagtggta gcggctccgg gctgacagag ctgagagccg tgatggactg 360

ggtgtggatg gacaccaccc tgtccctgag cagctggatc tgtgtgggaa gtggatcagg 420

ttccggctct ggggtgtccg ccacagacct gggccccgag agaagaaagc tgaccaccga 480

gctgacaatc atcctgcagg gcctggacgg atccgggtct ggcagtggtt caggctacag 540

ctgcgaggcc aacaatggcc tgggcgctca gtgtagcgag gccgtgaccc tgaatttcac 600

cgtgcccacc ggatcagggt ccggcagcgg tagtggctgt tggaggcccg ccaatgtggg 660

cgagacagtg accatgccct gccccaaggt gttcagcaac ttctacagca aagggtccgg 720

atctggtagt ggctcaggtg tgcggggctt catgtgccag ggccccgaac aagtgtgggg 780

aatggctgtg cgcgagctga acatgaacct gctgtgagat atcgcggccg c 831

<210> 35
<211> 121
<212> PRT
<213> Artificial Sequence

<220>
<223> Description of Artificial Sequence: Synthetic
      polypeptide

<400> 35
Met Pro Phe Ala Met Ala Gln Ile Pro Ser Leu Ser Leu Arg Ala Val
1               5                   10                  15

Leu Pro Asn Val Thr Val Arg Asn Ala Thr Ser Tyr Arg Cys Gly Ser
            20                  25                  30

Trp Phe Arg Glu Gly Lys Thr Leu Asn Pro Ser Gln Thr Ser Ala Val
        35                  40                  45

Met Asp Trp Val Trp Met Asp Thr Thr Leu Ser Leu Ser Leu Gly Pro
    50                  55                  60

Glu Arg Arg Lys Leu Thr Thr Glu Leu Thr Ile Ile Asn Asn Gly Leu
65                  70                  75                  80

Gly Ala Gln Cys Ser Glu Ala Val Thr Leu Asn Asn Val Gly Glu Thr
                85                  90                  95

Val Thr Met Pro Cys Pro Lys Val Phe Ser Cys Gln Gly Pro Glu Gln
            100                 105                 110

Val Trp Gly Met Ala Val Arg Glu Leu
        115                 120


<210> 36
<211> 184
<212> PRT
<213> Artificial Sequence

<220>
<223> Description of Artificial Sequence: Synthetic
      polypeptide

<400> 36
Met Pro Phe Ala Met Ala Gln Ile Pro Ser Leu Ser Leu Arg Ala Val
1               5                   10                  15

Gly Ser Gly Ser Gly Ser Gly Ser Gly Leu Pro Asn Val Thr Val Arg
            20                  25                  30

Asn Ala Thr Ser Tyr Arg Cys Gly Gly Ser Gly Ser Gly Ser Gly Ser
        35                  40                  45

Gly Ser Trp Phe Arg Glu Gly Lys Thr Leu Asn Pro Ser Gln Thr Ser
    50                  55                  60

Gly Ser Gly Ser Gly Ser Gly Ser Gly Ala Val Met Asp Trp Val Trp
65                  70                  75                  80

Met Asp Thr Thr Leu Ser Leu Ser Gly Ser Gly Ser Gly Ser Gly Ser
                85                  90                  95

Gly Leu Gly Pro Glu Arg Arg Lys Leu Thr Thr Glu Leu Thr Ile Ile
            100                 105                 110

Gly Ser Gly Ser Gly Ser Gly Ser Gly Asn Asn Gly Leu Gly Ala Gln
        115                 120                 125

Cys Ser Glu Ala Val Thr Leu Asn Gly Ser Gly Ser Gly Ser Gly Ser
    130                 135                 140

Gly Asn Val Gly Glu Thr Val Thr Met Pro Cys Pro Lys Val Phe Ser
145                 150                 155                 160

Gly Ser Gly Ser Gly Ser Gly Ser Gly Cys Gln Gly Pro Glu Gln Val
                165                 170                 175

Trp Gly Met Ala Val Arg Glu Leu
            180

<210> 37
<211> 201
<212> PRT
<213> Artificial Sequence

<220>
<223> Description of Artificial Sequence: Synthetic
      polypeptide

<400> 37
Met Pro Ser Glu Ser Pro Pro Phe Ala Met Ala Gln Ile Pro Ser Leu
1               5                   10                  15

Ser Leu Arg Ala Val Ser Gly Ala Met Gly Ala His Ser Ile Pro Leu
            20                  25                  30

Pro Asn Val Thr Val Arg Asn Ala Thr Ser Tyr Arg Cys Gly Val Gly
        35                  40                  45

Pro Pro Gly Pro Pro Ala Ser Leu Ser Trp Phe Arg Glu Gly Lys Thr
    50                  55                  60

Leu Asn Pro Ser Gln Thr Ser Met Ser Gly Thr Leu Leu Thr Glu Leu
65                  70                  75                  80

Arg Ala Val Met Asp Trp Val Trp Met Asp Thr Thr Leu Ser Leu Ser

85                  90                  95

Ser Trp Ile Cys Val Val Ser Ala Thr Asp Leu Gly Pro Glu Arg Arg
            100                 105                 110

Lys Leu Thr Thr Glu Leu Thr Ile Ile Leu Gln Gly Leu Asp Tyr Ser
        115                 120                 125

Cys Glu Ala Asn Asn Gly Leu Gly Ala Gln Cys Ser Glu Ala Val Thr
    130                 135                 140

Leu Asn Phe Thr Val Pro Thr Cys Trp Arg Pro Ala Asn Val Gly Glu
145                 150                 155                 160

Thr Val Thr Met Pro Cys Pro Lys Val Phe Ser Asn Phe Tyr Ser Lys
                165                 170                 175

Val Arg Gly Phe Met Cys Gln Gly Pro Glu Gln Val Trp Gly Met Ala
            180                 185                 190

Val Arg Glu Leu Asn Met Asn Leu Leu
        195                 200

<210> 38
<211> 264
<212> PRT
<213> Artificial Sequence

<220>
<223> Description of Artificial Sequence: Synthetic
      polypeptide

<400> 38
Met Pro Ser Glu Ser Pro Pro Phe Ala Met Ala Gln Ile Pro Ser Leu
1               5                   10                  15

Ser Leu Arg Ala Val Ser Gly Ala Met Gly Gly Ser Gly Ser Gly Ser
            20                  25                  30

Gly Ser Gly Ala His Ser Ile Pro Leu Pro Asn Val Thr Val Arg Asn
        35                  40                  45

Ala Thr Ser Tyr Arg Cys Gly Val Gly Pro Pro Gly Gly Ser Gly Ser
    50                  55                  60

Gly Ser Gly Ser Gly Pro Pro Ala Ser Leu Ser Trp Phe Arg Glu Gly
65                  70                  75                  80

Lys Thr Leu Asn Pro Ser Gln Thr Ser Met Ser Gly Thr Leu Gly Ser
                85                  90                  95

Gly Ser Gly Ser Gly Ser Gly Leu Thr Glu Leu Arg Ala Val Met Asp
            100                 105                 110

Trp Val Trp Met Asp Thr Thr Leu Ser Leu Ser Ser Trp Ile Cys Val
        115                 120                 125

Gly Ser Gly Ser Gly Ser Gly Ser Gly Val Ser Ala Thr Asp Leu Gly
    130                 135                 140

Pro Glu Arg Arg Lys Leu Thr Thr Glu Leu Thr Ile Ile Leu Gln Gly
145                 150                 155                 160

Leu Asp Gly Ser Gly Ser Gly Ser Gly Ser Gly Tyr Ser Cys Glu Ala
                165                 170                 175

Asn Asn Gly Leu Gly Ala Gln Cys Ser Glu Ala Val Thr Leu Asn Phe
            180                 185                 190

Thr Val Pro Thr Gly Ser Gly Ser Gly Ser Gly Ser Gly Cys Trp Arg
        195                 200                 205

Pro Ala Asn Val Gly Glu Thr Val Thr Met Pro Cys Pro Lys Val Phe
    210                 215                 220

Ser Asn Phe Tyr Ser Lys Gly Ser Gly Ser Gly Ser Gly Ser Gly Val
225                 230                 235                 240

Arg Gly Phe Met Cys Gln Gly Pro Glu Gln Val Trp Gly Met Ala Val
                245                 250                 255

Arg Glu Leu Asn Met Asn Leu Leu
            260

<210> 39
<211> 132
<212> PRT
<213> Mycobacterium tuberculosis

<400> 39
Thr Ala Ala Ser Asp Asn Phe Gln Leu Ser Gln Gly Gly Gln Gly Phe
1               5                   10                  15

Ala Ile Pro Ile Gly Gln Ala Met Ala Ile Ala Gly Gln Ile Arg Ser
            20                  25                  30

Gly Gly Gly Ser Pro Thr Val His Ile Gly Pro Thr Ala Phe Leu Gly
        35                  40                  45

Leu Gly Val Val Asp Asn Asn Gly Asn Gly Ala Arg Val Gln Arg Val
    50                  55                  60

Val Gly Ser Ala Pro Ala Ala Ser Leu Gly Ile Ser Thr Gly Asp Val
65                  70                  75                  80

Ile Thr Ala Val Asp Gly Ala Pro Ile Asn Ser Ala Thr Ala Met Ala
                85                  90                  95

Asp Ala Leu Asn Gly His His Pro Gly Asp Val Ile Ser Val Thr Trp
            100                 105                 110

Gln Thr Lys Ser Gly Gly Thr Arg Thr Gly Asn Val Thr Leu Ala Glu
        115                 120                 125

Gly Pro Pro Ala
    130

<210> 40
<211> 230
<212> PRT
<213> Artificial Sequence

<220>
<223> Description of Artificial Sequence: Synthetic
      polypeptide

<400> 40
Met His His His His His His Thr Ala Ala Ser Asp Asn Phe Gln Leu
1               5                   10                  15

Ser Gln Gly Gly Gln Gly Phe Ala Ile Pro Ile Gly Gln Ala Met Ala
            20                  25                  30

Ile Ala Gly Gln Ile Arg Ser Gly Gly Gly Ser Pro Thr Val His Ile
        35                  40                  45

Gly Pro Thr Ala Phe Leu Gly Leu Gly Val Val Asp Asn Asn Gly Asn
    50                  55                  60

Gly Ala Arg Val Gln Arg Val Val Gly Ser Ala Pro Ala Ala Ser Leu
65                  70                  75                  80

Gly Ile Ser Thr Gly Asp Val Ile Thr Ala Val Asp Gly Ala Pro Ile
                85                  90                  95

Asn Ser Ala Thr Ala Met Ala Asp Ala Leu Asn Gly His His Pro Gly
            100                 105                 110

Asp Val Ile Ser Val Thr Trp Gln Thr Lys Ser Gly Gly Thr Arg Thr
        115                 120                 125

Gly Asn Val Thr Leu Ala Glu Gly Pro Pro Ala Glu Phe Asp Asp Asp
    130                 135                 140

Asp Lys Asp Pro Pro Asp Pro His Gln Pro Asp Met Thr Lys Gly Tyr
145                 150                 155                 160

Cys Pro Gly Gly Arg Trp Gly Phe Gly Asp Leu Ala Val Cys Asp Gly
                165                 170                 175

Glu Lys Tyr Pro Asp Gly Ser Phe Trp His Gln Trp Met Gln Thr Trp
            180                 185                 190

Phe Thr Gly Pro Gln Phe Tyr Phe Asp Cys Val Ser Gly Gly Glu Pro
        195                 200                 205

Leu Pro Gly Pro Pro Pro Pro Gly Gly Cys Gly Gly Ala Ile Pro Ser
    210                 215                 220

Glu Gln Pro Asn Ala Pro
225                 230

<210> 41
<211> 578
<212> PRT
<213> Artificial Sequence

<220>
<223> Description of Artificial Sequence: Synthetic
      polypeptide

<400> 41
Met His His His His His His Thr Ala Ala Ser Asp Asn Phe Gln Leu
1               5                   10                  15

Ser Gln Gly Gly Gln Gly Phe Ala Ile Pro Ile Gly Gln Ala Met Ala
            20                  25                  30

Ile Ala Gly Gln Ile Arg Ser Gly Gly Gly Ser Pro Thr Val His Ile
        35                  40                  45

Gly Pro Thr Ala Phe Leu Gly Leu Gly Val Val Asp Asn Asn Gly Asn
    50                  55                  60

Gly Ala Arg Val Gln Arg Val Val Gly Ser Ala Pro Ala Ala Ser Leu
65                  70                  75                  80

Gly Ile Ser Thr Gly Asp Val Ile Thr Ala Val Asp Gly Ala Pro Ile

                85                  90                  95

Asn Ser Ala Thr Ala Met Ala Asp Ala Leu Asn Gly His His Pro Gly
            100                 105                 110

Asp Val Ile Ser Val Thr Trp Gln Thr Lys Ser Gly Gly Thr Arg Thr
        115                 120                 125

Gly Asn Val Thr Leu Ala Glu Gly Pro Pro Ala Glu Phe Pro Leu Val
    130                 135                 140

Pro Arg Gly Ser Pro Met Gly Ser Asp Val Arg Asp Leu Asn Ala Leu
145                 150                 155                 160

Leu Pro Ala Val Pro Ser Leu Gly Gly Gly Gly Gly Cys Ala Leu Pro
                165                 170                 175

Val Ser Gly Ala Ala Gln Trp Ala Pro Val Leu Asp Phe Ala Pro Pro
            180                 185                 190

Gly Ala Ser Ala Tyr Gly Ser Leu Gly Gly Pro Ala Pro Pro Pro Ala
        195                 200                 205

Pro Pro Pro Pro Pro Pro Pro Pro Pro His Ser Phe Ile Lys Gln Glu
    210                 215                 220

Pro Ser Trp Gly Gly Ala Glu Pro His Glu Glu Gln Cys Leu Ser Ala
225                 230                 235                 240

Phe Thr Val His Phe Ser Gly Gln Phe Thr Gly Thr Ala Gly Ala Cys
                245                 250                 255

Arg Tyr Gly Pro Phe Gly Pro Pro Pro Pro Ser Gln Ala Ser Ser Gly
            260                 265                 270

Gln Ala Arg Met Phe Pro Asn Ala Pro Tyr Leu Pro Ser Cys Leu Glu
        275                 280                 285

Ser Gln Pro Ala Ile Arg Asn Gln Gly Tyr Ser Thr Val Thr Phe Asp
    290                 295                 300

Gly Thr Pro Ser Tyr Gly His Thr Pro Ser His His Ala Ala Gln Phe
305                 310                 315                 320

Pro Asn His Ser Phe Lys His Glu Asp Pro Met Gly Gln Gln Gly Ser
                325                 330                 335

Leu Gly Glu Gln Gln Tyr Ser Val Pro Pro Pro Val Tyr Gly Cys His
            340                 345                 350

Thr Pro Thr Asp Ser Cys Thr Gly Ser Gln Ala Leu Leu Leu Arg Thr
        355                 360                 365

Pro Tyr Ser Ser Asp Asn Leu Tyr Gln Met Thr Ser Gln Leu Glu Cys
    370                 375                 380

Met Thr Trp Asn Gln Met Asn Leu Gly Ala Thr Leu Lys Gly His Ser
385                 390                 395                 400

Thr Gly Tyr Glu Ser Asp Asn His Thr Thr Pro Ile Leu Cys Gly Ala
                405                 410                 415

Gln Tyr Arg Ile His Thr His Gly Val Phe Arg Gly Ile Gln Asp Val
            420                 425                 430

Arg Arg Val Pro Gly Val Ala Pro Thr Leu Val Arg Ser Ala Ser Glu
        435                 440                 445

Thr Ser Glu Lys Arg Pro Phe Met Cys Ala Tyr Ser Gly Cys Asn Lys
    450                 455                 460

Arg Tyr Phe Lys Leu Ser His Leu Gln Met His Ser Arg Lys His Thr
465                 470                 475                 480

Gly Glu Lys Pro Tyr Gln Cys Asp Phe Lys Asp Cys Glu Arg Arg Phe
                485                 490                 495

Phe Arg Ser Asp Gln Leu Lys Arg His Gln Arg Arg His Thr Gly Val
            500                 505                 510

Lys Pro Phe Gln Cys Lys Thr Cys Gln Arg Lys Phe Ser Arg Ser Asp
        515                 520                 525

His Leu Lys Thr His Thr Arg Thr His Thr Gly Glu Lys Pro Phe Ser
    530                 535                 540

Cys Arg Trp Pro Ser Cys Gln Lys Lys Phe Ala Arg Ser Asp Glu Leu
545                 550                 555                 560

Val Arg His His Asn Met His Gln Arg Asn Met Thr Lys Leu Gln Leu
                565                 570                 575

Ala Leu

<210> 42
<211> 220
<212> PRT
<213> Artificial Sequence

<220>
<223> Description of Artificial Sequence: Synthetic
      polypeptide

<400> 42

<210> 43
<211> 729
<212> PRT
<213> Artificial Sequence

<220>
<223> Description of Artificial Sequence: Synthetic
      polypeptide

<400> 43
Met His His His His His His Thr Ala Ala Ser Asp Asn Phe Gln Leu
1               5                   10                  15

Ser Gln Gly Gly Gln Gly Phe Ala Ile Pro Ile Gly Gln Ala Met Ala
            20                  25                  30

Ile Ala Gly Gln Ile Arg Ser Gly Gly Gly Ser Pro Thr Val His Ile
        35                  40                  45

Gly Pro Thr Ala Phe Leu Gly Leu Gly Val Val Asp Asn Asn Gly Asn
    50                  55                  60

Gly Ala Arg Val Gln Arg Val Val Gly Ser Ala Pro Ala Ala Ser Leu
65                  70                  75                  80

Gly Ile Ser Thr Gly Asp Val Ile Thr Ala Val Asp Gly Ala Pro Ile
                85                  90                  95

Asn Ser Ala Thr Ala Met Ala Asp Ala Leu Asn Gly His His Pro Gly
            100                 105                 110

Asp Val Ile Ser Val Thr Trp Gln Thr Lys Ser Gly Gly Thr Arg Thr
        115                 120                 125

Gly Asn Val Thr Leu Ala Glu Gly Pro Pro Ala Glu Phe Met Val Asp
    130                 135                 140

Phe Gly Ala Leu Pro Pro Glu Ile Asn Ser Ala Arg Met Tyr Ala Gly
145                 150                 155                 160

Pro Gly Ser Ala Ser Leu Val Ala Ala Ala Gln Met Trp Asp Ser Val
                165                 170                 175

Ala Ser Asp Leu Phe Ser Ala Ala Ser Ala Phe Gln Ser Val Val Trp
            180                 185                 190

Gly Leu Thr Val Gly Ser Trp Ile Gly Ser Ser Ala Gly Leu Met Val

195                 200                 205

Ala Ala Ala Ser Pro Tyr Val Ala Trp Met Ser Val Thr Ala Gly Gln
    210                 215                 220

Ala Glu Leu Thr Ala Ala Gln Val Arg Val Ala Ala Ala Ala Tyr Glu
225                 230                 235                 240

Thr Ala Tyr Gly Leu Thr Val Pro Pro Pro Val Ile Ala Glu Asn Arg
                245                 250                 255

Ala Glu Leu Met Ile Leu Ile Ala Thr Asn Leu Leu Gly Gln Asn Thr
            260                 265                 270

Pro Ala Ile Ala Val Asn Glu Ala Glu Tyr Gly Glu Met Trp Ala Gln
        275                 280                 285

Asp Ala Ala Ala Met Phe Gly Tyr Ala Ala Ala Thr Ala Thr Ala Thr
    290                 295                 300

Ala Thr Leu Leu Pro Phe Glu Glu Ala Pro Glu Met Thr Ser Ala Gly
305                 310                 315                 320

Gly Leu Leu Glu Gln Ala Ala Ala Val Glu Glu Ala Ser Asp Thr Ala
                325                 330                 335

Ala Ala Asn Gln Leu Met Asn Asn Val Pro Gln Ala Leu Gln Gln Leu
            340                 345                 350

Ala Gln Pro Thr Gln Gly Thr Thr Pro Ser Ser Lys Leu Gly Gly Leu
        355                 360                 365

Trp Lys Thr Val Ser Pro His Arg Ser Pro Ile Ser Asn Met Val Ser
    370                 375                 380

Met Ala Asn Asn His Met Ser Met Thr Asn Ser Gly Val Ser Met Thr
385                 390                 395                 400

Asn Thr Leu Ser Ser Met Leu Lys Gly Phe Ala Pro Ala Ala Ala Ala
                405                 410                 415

Gln Ala Val Gln Thr Ala Ala Gln Asn Gly Val Arg Ala Met Ser Ser
            420                 425                 430

Leu Gly Ser Ser Leu Gly Ser Ser Gly Leu Gly Gly Gly Val Ala Ala
        435                 440                 445

Asn Leu Gly Arg Ala Ala Ser Val Gly Ser Leu Ser Val Pro Gln Ala
    450                 455                 460

Trp Ala Ala Ala Asn Gln Ala Val Thr Pro Ala Ala Arg Ala Leu Pro
465                 470                 475                 480

Leu Thr Ser Leu Thr Ser Ala Ala Glu Arg Gly Pro Gly Gln Met Leu
                485                 490                 495

Gly Gly Leu Pro Val Gly Gln Met Gly Ala Arg Ala Gly Gly Gly Leu
            500                 505                 510

Ser Gly Val Leu Arg Val Pro Pro Arg Pro Tyr Val Met Pro His Ser
        515                 520                 525

Pro Ala Ala Gly Asp Ile Ala Pro Pro Ala Leu Ser Gln Asp Arg Phe
    530                 535                 540

Ala Asp Phe Pro Ala Leu Pro Leu Asp Pro Ser Ala Met Val Ala Gln
545                 550                 555                 560

Val Gly Pro Gln Val Val Asn Ile Asn Thr Lys Leu Gly Tyr Asn Asn
                565                 570                 575

Ala Val Gly Ala Gly Thr Gly Ile Val Ile Asp Pro Asn Gly Val Val
            580                 585                 590

Leu Thr Asn Asn His Val Ile Ala Gly Ala Thr Asp Ile Asn Ala Phe
        595                 600                 605

Ser Val Gly Ser Gly Gln Thr Tyr Gly Val Asp Val Val Gly Tyr Asp
    610                 615                 620

Arg Thr Gln Asp Val Ala Val Leu Gln Leu Arg Gly Ala Gly Gly Leu
625                 630                 635                 640

Pro Ser Ala Ala Ile Gly Gly Gly Val Ala Val Gly Glu Pro Val Val
                645                 650                 655

Ala Met Gly Asn Ser Gly Gly Gln Gly Gly Thr Pro Arg Ala Val Pro
            660                 665                 670

Gly Arg Val Val Ala Leu Gly Gln Thr Val Gln Ala Ser Asp Ser Leu
        675                 680                 685

Thr Gly Ala Glu Glu Thr Leu Asn Gly Leu Ile Gln Phe Asp Ala Ala
    690                 695                 700

Ile Gln Pro Gly Asp Ser Gly Gly Pro Val Val Asn Gly Leu Gly Gln
705                 710                 715                 720

Val Val Gly Met Asn Thr Ala Ala Ser
                725

<210> 44
<211> 30
<212> PRT
<213> Artificial Sequence

<220>
<223> Description of Artificial Sequence: Synthetic
      polypeptide

<400> 44
Thr Ala Ala Ser Asp Asn Phe Gln Leu Ser Gln Gly Gly Gln Gly Phe
1               5                   10                  15

Ala Ile Pro Ile Gly Gln Ala Met Ala Ile Ala Gly Gln Ile
            20                  25                  30

<210> 45
<211> 128
<212> PRT
<213> Artificial Sequence

<220>
<223> Description of Artificial Sequence: Synthetic
      polypeptide

<400> 45
Thr Ala Ala Ser Asp Asn Phe Gln Leu Ser Gln Gly Gly Gln Gly Phe
1               5                   10                  15

Ala Ile Pro Ile Gly Gln Ala Met Ala Ile Ala Gly Gln Ile Lys Leu
            20                  25                  30

Pro Thr Val His Ile Gly Pro Thr Ala Phe Leu Gly Leu Gly Val Val
        35                  40                  45

Asp Asn Asn Gly Asn Gly Ala Arg Val Gln Arg Val Val Gly Ser Ala
    50                  55                  60

Pro Ala Ala Ser Leu Gly Ile Ser Thr Gly Asp Val Ile Thr Ala Val
65                  70                  75                  80

Asp Gly Ala Pro Ile Asn Ser Ala Thr Ala Met Ala Asp Ala Leu Asn
                85                  90                  95

Gly His His Pro Gly Asp Val Ile Ser Val Thr Trp Gln Thr Lys Ser
            100                 105                 110

Gly Gly Thr Arg Thr Gly Asn Val Thr Leu Ala Glu Gly Pro Pro Ala
        115                 120                 125

<210> 46
<211> 128
<212> PRT
<213> Mycobacterium tuberculosis

<400> 46
Thr Ala Ala Ser Asp Asn Phe Gln Leu Ser Gln Gly Gly Gln Gly Phe
1               5                   10                  15

Ala Ile Pro Ile Gly Gln Ala Met Ala Ile Ala Gly Gln Ile Arg Ser
            20                  25                  30

Gly Gly Gly Ser Pro Thr Val His Ile Gly Pro Thr Ala Phe Leu Gly
        35                  40                  45

Leu Gly Val Val Asp Asn Asn Gly Asn Gly Ala Arg Val Gln Arg Val
    50                  55                  60

Val Gly Ser Ala Pro Ala Ala Ser Leu Gly Ile Ser Thr Gly Asp Val
65                  70                  75                  80

Ile Thr Ala Val Asp Gly Ala Pro Ile Asn Ser Ala Thr Ala Met Ala
                85                  90                  95

Asp Ala Leu Asn Gly His His Pro Gly Asp Val Ile Ser Val Thr Trp
            100                 105                 110

Gln Thr Lys Ser Gly Gly Thr Arg Thr Gly Asn Val Thr Leu Ala Glu
        115                 120                 125

<210> 47
<211> 355
<212> PRT
<213> Mycobacterium tuberculosis

<400> 47
Met Ser Asn Ser Arg Arg Arg Ser Leu Arg Trp Ser Trp Leu Leu Ser
1               5                   10                  15

Val Leu Ala Ala Val Gly Leu Gly Leu Ala Thr Ala Pro Ala Gln Ala
            20                  25                  30

Ala Pro Pro Ala Leu Ser Gln Asp Arg Phe Ala Asp Phe Pro Ala Leu
        35                  40                  45

Pro Leu Asp Pro Ser Ala Met Val Ala Gln Val Gly Pro Gln Val Val

50                  55                  60

Asn Ile Asn Thr Lys Leu Gly Tyr Asn Asn Ala Val Gly Ala Gly Thr
65                  70                  75                  80

Gly Ile Val Ile Asp Pro Asn Gly Val Val Leu Thr Asn Asn His Val
                85                  90                  95

Ile Ala Gly Ala Thr Asp Ile Asn Ala Phe Ser Val Gly Ser Gly Gln
            100                 105                 110

Thr Tyr Gly Val Asp Val Val Gly Tyr Asp Arg Thr Gln Asp Val Ala
        115                 120                 125

Val Leu Gln Leu Arg Gly Ala Gly Gly Leu Pro Ser Ala Ala Ile Gly
    130                 135                 140

Gly Gly Val Ala Val Gly Glu Pro Val Val Ala Met Gly Asn Ser Gly
145                 150                 155                 160

Gly Gln Gly Gly Thr Pro Arg Ala Val Pro Gly Arg Val Val Ala Leu
                165                 170                 175

Gly Gln Thr Val Gln Ala Ser Asp Ser Leu Thr Gly Ala Glu Glu Thr
            180                 185                 190

Leu Asn Gly Leu Ile Gln Phe Asp Ala Ala Ile Gln Pro Gly Asp Ser
        195                 200                 205

Gly Gly Pro Val Val Asn Gly Leu Gly Gln Val Val Gly Met Asn Thr
    210                 215                 220

Ala Ala Ser Asp Asn Phe Gln Leu Ser Gln Gly Gly Gln Gly Phe Ala
225                 230                 235                 240

Ile Pro Ile Gly Gln Ala Met Ala Ile Ala Gly Gln Ile Arg Ser Gly
                245                 250                 255

Gly Gly Ser Pro Thr Val His Ile Gly Pro Thr Ala Phe Leu Gly Leu
            260                 265                 270

Gly Val Val Asp Asn Asn Gly Asn Gly Ala Arg Val Gln Arg Val Val
        275                 280                 285

Gly Ser Ala Pro Ala Ala Ser Leu Gly Ile Ser Thr Gly Asp Val Ile
    290                 295                 300

Thr Ala Val Asp Gly Ala Pro Ile Asn Ser Ala Thr Ala Met Ala Asp
305                 310                 315                 320

Ala Leu Asn Gly His His Pro Gly Asp Val Ile Ser Val Thr Trp Gln
                325                 330                 335

Thr Lys Ser Gly Gly Thr Arg Thr Gly Asn Val Thr Leu Ala Glu Gly
            340                 345                 350

Pro Pro Ala
        355

<210> 48
<211> 364
<212> PRT
<213> Haemophilus influenzae

<400> 48
Met Lys Leu Lys Thr Leu Ala Leu Ser Leu Leu Ala Ala Gly Val Leu
1               5                   10                  15

Ala Gly Cys Ser Ser His Ser Ser Asn Met Ala Asn Thr Gln Met Lys
            20                  25                  30

Ser Asp Lys Ile Ile Ile Ala His Arg Gly Ala Ser Gly Tyr Leu Pro
        35                  40                  45

Glu His Thr Leu Glu Ser Lys Ala Leu Ala Phe Ala Gln Gln Ala Asp
    50                  55                  60

Tyr Leu Glu Gln Asp Leu Ala Met Thr Lys Asp Gly Arg Leu Val Val
65                  70                  75                  80

Ile His Asp His Phe Leu Asp Gly Leu Thr Asp Val Ala Lys Lys Phe
                85                  90                  95

Pro His Arg His Arg Lys Asp Gly Arg Tyr Tyr Val Ile Asp Phe Thr
            100                 105                 110

Leu Lys Glu Ile Gln Ser Leu Glu Met Thr Glu Asn Phe Glu Thr Lys
        115                 120                 125

Asp Gly Lys Gln Ala Gln Val Tyr Pro Asn Arg Phe Pro Leu Trp Lys
    130                 135                 140

Ser His Phe Arg Ile His Thr Phe Glu Asp Glu Ile Glu Phe Ile Gln
145                 150                 155                 160

Gly Leu Glu Lys Ser Thr Gly Lys Lys Val Gly Ile Tyr Pro Glu Ile

                165                 170                 175

Lys Ala Pro Trp Phe His His Gln Asn Gly Lys Asp Ile Ala Ala Glu
            180                 185                 190

Thr Leu Lys Val Leu Lys Lys Tyr Gly Tyr Asp Lys Lys Thr Asp Met
        195                 200                 205

Val Tyr Leu Gln Thr Phe Asp Phe Asn Glu Leu Lys Arg Ile Lys Thr
    210                 215                 220

Glu Leu Leu Pro Gln Met Gly Met Asp Leu Lys Leu Val Gln Leu Ile
225                 230                 235                 240

Ala Tyr Thr Asp Trp Lys Glu Thr Gln Glu Lys Asp Pro Lys Gly Tyr
                245                 250                 255

Trp Val Asn Tyr Asn Tyr Asp Trp Met Phe Lys Pro Gly Ala Met Ala
            260                 265                 270

Glu Val Val Lys Tyr Ala Asp Gly Val Gly Pro Gly Trp Tyr Met Leu
        275                 280                 285

Val Asn Lys Glu Glu Ser Lys Pro Asp Asn Ile Val Tyr Thr Pro Leu
    290                 295                 300

Val Lys Glu Leu Ala Gln Tyr Asn Val Glu Val His Pro Tyr Thr Val
305                 310                 315                 320

Arg Lys Asp Ala Leu Pro Ala Phe Phe Thr Asp Val Asn Gln Met Tyr
                325                 330                 335

Asp Val Leu Leu Asn Lys Ser Gly Ala Thr Gly Val Phe Thr Asp Phe
            340                 345                 350

Pro Asp Thr Gly Val Glu Phe Leu Lys Gly Ile Lys
        355                 360

<210> 49
<211> 313
<212> PRT
<213> Streptococcus pneumonae

<400> 49
Met Glu Ile Asn Val Ser Lys Leu Arg Thr Asp Leu Pro Gln Val Gly
1               5                   10                  15

Val Gln Pro Tyr Arg Gln Val His Ala His Ser Thr Gly Asn Pro His
            20                  25                  30

Ser Thr Val Gln Asn Glu Ala Asp Tyr His Trp Arg Lys Asp Pro Glu
        35                  40                  45

Leu Gly Phe Phe Ser His Ile Val Gly Asn Gly Cys Ile Met Gln Val
    50                  55                  60

Gly Pro Val Asp Asn Gly Ala Trp Asp Val Gly Gly Gly Trp Asn Ala
65                  70                  75                  80

Glu Thr Tyr Ala Ala Val Glu Leu Ile Glu Ser His Ser Thr Lys Glu
                85                  90                  95

Glu Phe Met Thr Asp Tyr Arg Leu Tyr Ile Glu Leu Leu Arg Asn Leu
            100                 105                 110

Ala Asp Glu Ala Gly Leu Pro Lys Thr Leu Asp Thr Gly Ser Leu Ala
        115                 120                 125

Gly Ile Lys Thr His Glu Tyr Cys Thr Asn Asn Gln Pro Asn Asn His
    130                 135                 140

Ser Asp His Val Asp Pro Tyr Pro Tyr Leu Ala Lys Trp Gly Ile Ser
145                 150                 155                 160

Arg Glu Gln Phe Lys His Asp Ile Glu Asn Gly Leu Thr Ile Glu Thr
                165                 170                 175

Gly Trp Gln Lys Asn Asp Thr Gly Tyr Trp Tyr Val His Ser Asp Gly
            180                 185                 190

Ser Tyr Pro Lys Asp Lys Phe Glu Lys Ile Asn Gly Thr Trp Tyr Tyr
        195                 200                 205

Phe Asp Ser Ser Gly Tyr Met Leu Ala Asp Arg Trp Arg Lys His Thr
    210                 215                 220

Asp Gly Asn Trp Tyr Trp Phe Asp Asn Ser Gly Glu Met Ala Thr Gly
225                 230                 235                 240

Trp Lys Lys Ile Ala Asp Lys Trp Tyr Tyr Phe Asn Glu Glu Gly Ala
                245                 250                 255

Met Lys Thr Gly Trp Val Lys Tyr Lys Asp Thr Trp Tyr Tyr Leu Asp
            260                 265                 270

Ala Lys Glu Gly Ala Met Val Ser Asn Ala Phe Ile Gln Ser Ala Asp

275                 280                 285

Gly Thr Gly Trp Tyr Tyr Leu Lys Pro Asp Gly Thr Leu Ala Asp Arg
    290                 295                 300

Pro Glu Phe Arg Met Ser Gln Met Ala
305                 310

<210> 50
<211> 166
<212> PRT
<213> Homo sapiens

<400> 50
Met Lys Tyr Thr Ser Tyr Ile Leu Ala Phe Gln Leu Cys Ile Val Leu
1               5                   10                  15

Gly Ser Leu Gly Cys Tyr Cys Gln Asp Pro Tyr Val Lys Glu Ala Glu
            20                  25                  30

Asn Leu Lys Lys Tyr Phe Asn Ala Gly His Ser Asp Val Ala Asp Asn
        35                  40                  45

Gly Thr Leu Phe Leu Gly Ile Leu Lys Asn Trp Lys Glu Glu Ser Asp
    50                  55                  60

Arg Lys Ile Met Gln Ser Gln Ile Val Ser Phe Tyr Phe Lys Leu Phe
65                  70                  75                  80

Lys Asn Phe Lys Asp Asp Gln Ser Ile Gln Lys Ser Val Glu Thr Ile
                85                  90                  95

Lys Glu Asp Met Asn Val Lys Phe Phe Asn Ser Asn Lys Lys Lys Arg
            100                 105                 110

Asp Asp Phe Glu Lys Leu Thr Asn Tyr Ser Val Thr Asp Leu Asn Val
        115                 120                 125

Gln Arg Lys Ala Ile His Glu Leu Ile Gln Val Met Ala Glu Leu Ser
    130                 135                 140

Pro Ala Ala Lys Thr Gly Lys Arg Lys Arg Ser Gln Met Leu Phe Arg
145                 150                 155                 160

Gly Arg Arg Ala Ser Gln
                165

<210> 51
<211> 233

<212> PRT
<213> Homo sapiens

<400> 51
Met Ser Thr Glu Ser Met Ile Arg Asp Val Glu Leu Ala Glu Glu Ala
1               5                   10                  15

Leu Pro Lys Lys Thr Gly Gly Pro Gln Gly Ser Arg Arg Cys Leu Phe
            20                  25                  30

Leu Ser Leu Phe Ser Phe Leu Ile Val Ala Gly Ala Thr Thr Leu Phe
        35                  40                  45

Cys Leu Leu His Phe Gly Val Ile Gly Pro Gln Arg Glu Glu Phe Pro
    50                  55                  60

Arg Asp Leu Ser Leu Ile Ser Pro Leu Ala Gln Ala Val Arg Ser Ser
65                  70                  75                  80

Ser Arg Thr Pro Ser Asp Lys Pro Val Ala His Val Val Ala Asn Pro
                85                  90                  95

Gln Ala Glu Gly Gln Leu Gln Trp Leu Asn Arg Arg Ala Asn Ala Leu
            100                 105                 110

Leu Ala Asn Gly Val Glu Leu Arg Asp Asn Gln Leu Val Val Pro Ser
        115                 120                 125

Glu Gly Leu Tyr Leu Ile Tyr Ser Gln Val Leu Phe Lys Gly Gln Gly
    130                 135                 140

Cys Pro Ser Thr His Val Leu Leu Thr His Thr Ile Ser Arg Ile Ala
145                 150                 155                 160

Val Ser Tyr Gln Thr Lys Val Asn Leu Leu Ser Ala Ile Lys Ser Pro
                165                 170                 175

Cys Gln Arg Glu Thr Pro Glu Gly Ala Glu Ala Lys Pro Trp Tyr Glu
            180                 185                 190

Pro Ile Tyr Leu Gly Gly Val Phe Gln Leu Glu Lys Gly Asp Arg Leu
        195                 200                 205

Ser Ala Glu Ile Asn Arg Pro Asp Tyr Leu Asp Phe Ala Glu Ser Gly
    210                 215                 220

Gln Val Tyr Phe Gly Ile Ile Ala Leu
225                 230

<210> 52
<211> 153
<212> PRT
<213> Homo sapiens

<400> 52
Met Tyr Arg Met Gln Leu Leu Ser Cys Ile Ala Leu Ser Leu Ala Leu
1               5                   10                  15

Val Thr Asn Ser Ala Pro Thr Ser Ser Ser Thr Lys Lys Thr Gln Leu
            20                  25                  30

Gln Leu Glu His Leu Leu Leu Asp Leu Gln Met Ile Leu Asn Gly Ile
        35                  40                  45

Asn Asn Tyr Lys Asn Pro Lys Leu Thr Arg Met Leu Thr Phe Lys Phe
    50                  55                  60

Tyr Met Pro Lys Lys Ala Thr Glu Leu Lys His Leu Gln Cys Leu Glu
65                  70                  75                  80

Glu Glu Leu Lys Pro Leu Glu Glu Val Leu Asn Leu Ala Gln Ser Lys
                85                  90                  95

Asn Phe His Leu Arg Pro Arg Asp Leu Ile Ser Asn Ile Asn Val Ile
            100                 105                 110

Val Leu Glu Leu Lys Gly Ser Glu Thr Thr Phe Met Cys Glu Tyr Ala
        115                 120                 125

Asp Glu Thr Ala Thr Ile Val Glu Phe Leu Asn Arg Trp Ile Thr Phe
    130                 135                 140

Cys Gln Ser Ile Ile Ser Thr Leu Thr
145                 150


<210> 53
<211> 99
<212> PRT
<213> Homo sapiens

<400> 53
Met Thr Ser Lys Leu Ala Val Ala Leu Leu Ala Ala Phe Leu Ile Ser
1               5                   10                  15

Ala Ala Leu Cys Glu Gly Ala Val Leu Pro Arg Ser Ala Lys Glu Leu
            20                  25                  30

Arg Cys Gln Cys Ile Lys Thr Tyr Ser Lys Pro Phe His Pro Lys Phe
        35                  40                  45

Ile Lys Glu Leu Arg Val Ile Glu Ser Gly Pro His Cys Ala Asn Thr
    50                  55                  60

Glu Ile Ile Val Lys Leu Ser Asp Gly Arg Glu Leu Cys Leu Asp Pro
65                  70                  75                  80

Lys Glu Asn Trp Val Gln Arg Val Val Glu Lys Phe Leu Lys Arg Ala
                85                  90                  95

Glu Asn Ser

<210> 54
<211> 662
<212> PRT
<213> Homo sapiens

<400> 54

<210> 55
<211> 193
<212> PRT
<213> Homo sapiens

<400> 55
Met Ala Ala Glu Pro Val Glu Asp Asn Cys Ile Asn Phe Val Ala Met
1               5                   10                  15

Lys Phe Ile Asp Asn Thr Leu Tyr Phe Ile Ala Glu Asp Asp Glu Asn
            20                  25                  30

Leu Glu Ser Asp Tyr Phe Gly Lys Leu Glu Ser Lys Leu Ser Val Ile
        35                  40                  45

Arg Asn Leu Asn Asp Gln Val Leu Phe Ile Asp Gln Gly Asn Arg Pro
    50                  55                  60

Leu Phe Glu Asp Met Thr Asp Ser Asp Cys Arg Asp Asn Ala Pro Arg
65                  70                  75                  80

Thr Ile Phe Ile Ile Ser Met Tyr Lys Asp Ser Gln Pro Arg Gly Met
                85                  90                  95

Ala Val Thr Ile Ser Val Lys Cys Glu Lys Ile Ser Thr Leu Ser Cys
            100                 105                 110

Glu Asn Lys Ile Ile Ser Phe Lys Glu Met Asn Pro Pro Asp Asn Ile
        115                 120                 125

Lys Asp Thr Lys Ser Asp Ile Ile Phe Phe Gln Arg Ser Val Pro Gly
    130                 135                 140

His Asp Asn Lys Met Gln Phe Glu Ser Ser Ser Tyr Glu Gly Tyr Phe
145                 150                 155                 160

Leu Ala Cys Glu Lys Glu Arg Asp Leu Phe Lys Leu Ile Leu Lys Lys
                165                 170                 175

Glu Asp Glu Leu Gly Asp Arg Ser Ile Met Phe Thr Val Gln Asn Glu
            180                 185                 190

Asp

<210> 56
<211> 177

<212> PRT
<213> Homo sapiens

<400> 56
Met Phe His Val Ser Phe Arg Tyr Ile Phe Gly Leu Pro Pro Leu Ile
1               5                   10                  15

Leu Val Leu Leu Pro Val Ala Ser Ser Asp Cys Asp Ile Glu Gly Lys
            20                  25                  30

Asp Gly Lys Gln Tyr Glu Ser Val Leu Met Val Ser Ile Asp Gln Leu
        35                  40                  45

Leu Asp Ser Met Lys Glu Ile Gly Ser Asn Cys Leu Asn Asn Glu Phe
    50                  55                  60

Asn Phe Phe Lys Arg His Ile Cys Asp Ala Asn Lys Glu Gly Met Phe
65                  70                  75                  80

Leu Phe Arg Ala Ala Arg Lys Leu Arg Gln Phe Leu Lys Met Asn Ser
                85                  90                  95

Thr Gly Asp Phe Asp Leu His Leu Leu Lys Val Ser Glu Gly Thr Thr
            100                 105                 110

Ile Leu Leu Asn Cys Thr Gly Gln Val Lys Gly Arg Lys Pro Ala Ala
        115                 120                 125

Leu Gly Glu Ala Gln Pro Thr Lys Ser Leu Glu Glu Asn Lys Ser Leu
    130                 135                 140

Lys Glu Gln Lys Lys Leu Asn Asp Leu Cys Phe Leu Lys Arg Leu Leu
145                 150                 155                 160

Gln Glu Ile Lys Thr Cys Trp Asn Lys Ile Leu Met Gly Thr Lys Glu
                165                 170                 175

His



<210> 57
<211> 152
<212> PRT
<213> Homo sapiens

<400> 57
Met Ser Arg Leu Pro Val Leu Leu Leu Leu Gln Leu Leu Val Arg Pro
1               5                   10                  15

Gly Leu Gln Ala Pro Met Thr Gln Thr Thr Ser Leu Lys Thr Ser Trp

20                          25                          30

Val Asn Cys Ser Asn Met Ile Asp Glu Ile Ile Thr His Leu Lys Gln
            35                          40                          45

Pro Pro Leu Pro Leu Leu Asp Phe Asn Asn Leu Asn Gly Glu Asp Gln
    50                          55                          60

Asp Ile Leu Met Glu Asn Asn Leu Arg Arg Pro Asn Leu Glu Ala Phe
65                          70                          75                          80

Asn Arg Ala Val Lys Ser Leu Gln Asn Ala Ser Ala Ile Glu Ser Ile
                        85                          90                          95

Leu Lys Asn Leu Leu Pro Cys Leu Pro Leu Ala Thr Ala Ala Pro Thr
                100                         105                         110

Arg His Pro Ile His Ile Lys Asp Gly Asp Trp Asn Glu Phe Arg Arg
            115                         120                         125

Lys Leu Thr Phe Tyr Leu Lys Thr Leu Glu Asn Ala Gln Ala Gln Gln
    130                         135                         140

Thr Thr Leu Ser Leu Ala Ile Phe
145                         150

<210> 58
<211> 153
<212> PRT
<213> Homo sapiens

<400> 58

<210> 59
<211> 134
<212> PRT
<213> Homo sapiens

<400> 59
Met Arg Met Leu Leu His Leu Ser Leu Leu Ala Leu Gly Ala Ala Tyr
1               5                   10                  15

Val Tyr Ala Ile Pro Thr Glu Ile Pro Thr Ser Ala Leu Val Lys Glu
            20                  25                  30

Thr Leu Ala Leu Leu Ser Thr His Arg Thr Leu Leu Ile Ala Asn Glu
        35                  40                  45

Thr Leu Arg Ile Pro Val Pro Val His Lys Asn His Gln Leu Cys Thr
    50                  55                  60

Glu Glu Ile Phe Gln Gly Ile Gly Thr Leu Glu Ser Gln Thr Val Gln
65                  70                  75                  80

Gly Gly Thr Val Glu Arg Leu Phe Lys Asn Leu Ser Leu Ile Lys Lys
                85                  90                  95

Tyr Ile Asp Gly Gln Lys Lys Lys Cys Gly Glu Glu Arg Arg Arg Val
            100                 105                 110

Asn Gln Phe Leu Asp Tyr Leu Gln Glu Phe Leu Gly Val Met Asn Thr
        115                 120                 125

Glu Trp Ile Ile Glu Ser
    130

<210> 60
<211> 212
<212> PRT

<213> Homo sapiens

<400> 60
Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60

Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu
            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
            180                 185                 190

Leu Ile Leu Arg Ser Phe Lys Glu Phe Leu Gln Ser Ser Leu Arg Ala
        195                 200                 205

Leu Arg Gln Met
    210

<210> 61
<211> 140
<212> PRT
<213> Homo sapiens

<400> 61
Met Val Leu Thr Ser Ala Leu Leu Leu Cys Ser Val Ala Gly Gln Gly
1               5                   10                  15

Cys Pro Thr Leu Ala Gly Ile Leu Asp Ile Asn Phe Leu Ile Asn Lys
            20                  25                  30

Met Gln Glu Asp Pro Ala Ser Lys Cys His Cys Ser Ala Asn Val Thr
        35                  40                  45

Ser Cys Leu Cys Leu Gly Ile Pro Ser Asp Asn Cys Thr Arg Pro Cys
    50                  55                  60

Phe Ser Glu Arg Leu Ser Gln Met Thr Asn Thr Thr Met Gln Thr Arg
65                  70                  75                  80

Tyr Pro Leu Ile Phe Ser Arg Val Lys Lys Ser Val Glu Val Leu Lys
                85                  90                  95

Asn Asn Lys Cys Pro Tyr Phe Ser Cys Glu Gln Pro Cys Asn Gln Thr
            100                 105                 110

Thr Ala Gly Asn Ala Leu Thr Phe Leu Lys Ser Leu Leu Glu Ile Phe
        115                 120                 125

Gln Lys Glu Lys Met Arg Gly Met Arg Gly Lys Ile
    130                 135                 140

<210> 62
<211> 178
<212> PRT
<213> Homo sapiens

<400> 62
Met His Ser Ser Ala Leu Leu Cys Cys Leu Val Leu Leu Thr Gly Val
1               5                   10                  15

Arg Ala Ser Pro Gly Gln Gly Thr Gln Ser Glu Asn Ser Cys Thr His
            20                  25                  30

Phe Pro Gly Asn Leu Pro Asn Met Leu Arg Asp Leu Arg Asp Ala Phe
        35                  40                  45

Ser Arg Val Lys Thr Phe Phe Gln Met Lys Asp Gln Leu Asp Asn Leu
    50                  55                  60

Leu Leu Lys Glu Ser Leu Leu Glu Asp Phe Lys Gly Tyr Leu Gly Cys
65                  70                  75                  80

Gln Ala Leu Ser Glu Met Ile Gln Phe Tyr Leu Glu Glu Val Met Pro
                85                  90                  95

Gln Ala Glu Asn Gln Asp Pro Asp Ile Lys Ala His Val Asn Ser Leu
            100                 105                 110

Gly Glu Asn Leu Lys Thr Leu Arg Leu Arg Leu Arg Arg Cys His Arg
        115                 120                 125

Phe Leu Pro Cys Glu Asn Lys Ser Lys Ala Val Glu Gln Val Lys Asn
    130                 135                 140

Ala Phe Asn Lys Leu Gln Glu Lys Gly Ile Tyr Lys Ala Met Ser Glu
145                 150                 155                 160

Phe Asp Ile Phe Ile Asn Tyr Ile Glu Ala Tyr Met Thr Met Lys Ile
                165                 170                 175

Arg Asn

<210> 63
<211> 145
<212> PRT
<213> Homo sapiens

<400> 63

<210> 64
<211> 136
<212> PRT
<213> Homo sapiens

<400> 64

<210> 65
<211> 656
<212> PRT
<213> Homo sapiens

<400> 65
Met Glu Gly Asp Gly Ser Asp Pro Glu Pro Pro Asp Ala Gly Glu Asp
1               5                   10                  15

Ser Lys Ser Glu Asn Gly Glu Asn Ala Pro Ile Tyr Cys Ile Cys Arg
            20                  25                  30

Lys Pro Asp Ile Asn Cys Phe Met Ile Gly Cys Asp Asn Cys Asn Glu
        35                  40                  45

Trp Phe His Gly Asp Cys Ile Arg Ile Thr Glu Lys Met Ala Lys Ala
    50                  55                  60

Ile Arg Glu Trp Tyr Cys Arg Glu Cys Arg Glu Lys Asp Pro Lys Leu
65                  70                  75                  80

Glu Ile Arg Tyr Arg His Lys Lys Ser Arg Glu Arg Asp Gly Asn Glu
                85                  90                  95

Arg Asp Ser Ser Glu Pro Arg Asp Glu Gly Gly Gly Arg Lys Arg Pro
            100                 105                 110

Val Pro Asp Pro Asn Leu Gln Arg Arg Ala Gly Ser Gly Thr Gly Val
        115                 120                 125

Gly Ala Met Leu Ala Arg Gly Ser Ala Ser Pro His Lys Ser Ser Pro
    130                 135                 140

Gln Pro Leu Val Ala Thr Pro Ser Gln His His Gln Gln Gln Gln Gln
145                 150                 155                 160

Gln Ile Lys Arg Ser Ala Arg Met Cys Gly Glu Cys Glu Ala Cys Arg
                165                 170                 175

Arg Thr Glu Asp Cys Gly His Cys Asp Phe Cys Arg Asp Met Lys Lys
            180                 185                 190

Phe Gly Gly Pro Asn Lys Ile Arg Gln Lys Cys Arg Leu Arg Gln Cys
        195                 200                 205

Gln Leu Arg Ala Arg Glu Ser Tyr Lys Tyr Phe Pro Ser Ser Leu Ser
    210                 215                 220

Pro Val Thr Pro Ser Glu Ser Leu Pro Arg Pro Arg Arg Pro Leu Pro
225                 230                 235                 240

Thr Gln Gln Gln Pro Gln Pro Ser Gln Lys Leu Gly Arg Ile Arg Glu
                245                 250                 255

Asp Glu Gly Ala Val Ala Ser Ser Thr Val Lys Glu Pro Pro Glu Ala
            260                 265                 270

Thr Ala Thr Pro Glu Pro Leu Ser Asp Glu Asp Leu Pro Leu Asp Pro
        275                 280                 285

Asp Leu Tyr Gln Asp Phe Cys Ala Gly Ala Phe Asp Asp Asn Gly Leu
    290                 295                 300

Pro Trp Met Ser Asp Thr Glu Glu Ser Pro Phe Leu Asp Pro Ala Leu
305                 310                 315                 320

Arg Lys Arg Ala Val Lys Val Lys His Val Lys Arg Arg Glu Lys Lys
                325                 330                 335

Ser Glu Lys Lys Lys Glu Glu Arg Tyr Lys Arg His Arg Gln Lys Gln
            340                 345                 350

Lys His Lys Asp Lys Trp Lys His Pro Glu Arg Ala Asp Ala Lys Asp
        355                 360                 365

Pro Ala Ser Leu Pro Gln Cys Leu Gly Pro Gly Cys Val Arg Pro Ala
    370                 375                 380

Gln Pro Ser Ser Lys Tyr Cys Ser Asp Asp Cys Gly Met Lys Leu Ala
385                 390                 395                 400

Ala Asn Arg Ile Tyr Glu Ile Leu Pro Gln Arg Ile Gln Gln Trp Gln
                405                 410                 415

Gln Ser Pro Cys Ile Ala Glu Glu His Gly Lys Lys Leu Leu Glu Arg
            420                 425                 430

Ile Arg Arg Glu Gln Gln Ser Ala Arg Thr Arg Leu Gln Glu Met Glu
        435                 440                 445

Arg Arg Phe His Glu Leu Glu Ala Ile Ile Leu Arg Ala Lys Gln Gln
    450                 455                 460

Ala Val Arg Glu Asp Glu Glu Ser Asn Glu Gly Asp Ser Asp Asp Thr
465                 470                 475                 480

Asp Leu Gln Ile Phe Cys Val Ser Cys Gly His Pro Ile Asn Pro Arg
                485                 490                 495

Val Ala Leu Arg His Met Glu Arg Cys Tyr Ala Lys Tyr Glu Ser Gln
            500                 505                 510

Thr Ser Phe Gly Ser Met Tyr Pro Thr Arg Ile Glu Gly Ala Thr Arg

        515                 520                 525

Leu Phe Cys Asp Val Tyr Asn Pro Gln Ser Lys Thr Tyr Cys Lys Arg
    530                 535                 540

Leu Gln Val Leu Cys Pro Glu His Ser Arg Asp Pro Lys Val Pro Ala
545                 550                 555                 560

Asp Glu Val Cys Gly Cys Pro Leu Val Arg Asp Val Phe Glu Leu Thr
                565                 570                 575

Gly Asp Phe Cys Arg Leu Pro Lys Arg Gln Cys Asn Arg His Tyr Cys
            580                 585                 590

Trp Glu Lys Leu Arg Arg Ala Glu Val Asp Leu Glu Arg Val Arg Val
        595                 600                 605

Trp Tyr Lys Leu Asp Glu Leu Phe Glu Gln Glu Arg Asn Val Arg Thr
    610                 615                 620

Ala Met Thr Asn Arg Ala Gly Leu Leu Ala Leu Met Leu His Gln Thr
625                 630                 635                 640

Ile Gln His Asp Pro Leu Thr Thr Asp Leu Arg Ser Ser Ala Asp Arg
                645                 650                 655

<210> 66
<211> 124
<212> PRT
<213> Vibrio cholerae

<400> 66
Met Ile Lys Leu Lys Phe Gly Val Phe Phe Thr Val Leu Leu Ser Ser
1               5                   10                  15

Ala Tyr Ala His Gly Thr Pro Gln Asn Ile Thr Asp Leu Cys Ala Glu
            20                  25                  30

Tyr His Asn Thr Gln Ile Tyr Thr Leu Asn Asp Lys Ile Phe Ser Tyr
        35                  40                  45

Thr Glu Ser Leu Ala Gly Lys Arg Glu Met Ala Ile Ile Thr Phe Lys
    50                  55                  60

Asn Gly Ala Ile Phe Gln Val Glu Val Pro Gly Ser Gln His Ile Asp
65                  70                  75                  80

Ser Gln Lys Lys Ala Ile Glu Arg Met Lys Asp Thr Leu Arg Ile Ala
                85                  90                  95

Tyr Leu Thr Glu Ala Lys Val Glu Lys Leu Cys Val Trp Asn Asn Lys
            100                 105                 110

Thr Pro His Ala Ile Ala Ala Ile Ser Met Ala Asn
        115                 120

<210> 67
<211> 258
<212> PRT
<213> Vibrio cholerae

<400> 67
Met Val Lys Ile Ile Phe Val Phe Phe Ile Phe Leu Ser Ser Phe Ser
1               5                   10                  15

Tyr Ala Asn Asp Asp Lys Leu Tyr Arg Ala Asp Ser Arg Pro Pro Asp
            20                  25                  30

Glu Ile Lys Gln Ser Gly Gly Leu Met Pro Arg Gly Gln Asn Glu Tyr
        35                  40                  45

Phe Asp Arg Gly Thr Gln Met Asn Ile Asn Leu Tyr Asp His Ala Arg
    50                  55                  60

Gly Thr Gln Thr Gly Phe Val Arg His Asp Asp Gly Tyr Val Ser Thr
65                  70                  75                  80

Ser Ile Ser Leu Arg Ser Ala His Leu Val Gly Gln Thr Ile Leu Ser
                85                  90                  95

Gly His Ser Thr Tyr Tyr Ile Tyr Val Ile Ala Thr Ala Pro Asn Met
            100                 105                 110

Phe Asn Val Asn Asp Val Leu Gly Ala Tyr Ser Pro His Pro Asp Glu
        115                 120                 125

Gln Glu Val Ser Ala Leu Gly Gly Ile Pro Tyr Ser Gln Ile Tyr Gly
    130                 135                 140

Trp Tyr Arg Val His Phe Gly Val Leu Asp Glu Gln Leu His Arg Asn
145                 150                 155                 160

Arg Gly Tyr Arg Asp Arg Tyr Tyr Ser Asn Leu Asp Ile Ala Pro Ala
                165                 170                 175

Ala Asp Gly Tyr Gly Leu Ala Gly Phe Pro Pro Glu His Arg Ala Trp
            180                 185                 190

Arg Glu Glu Pro Trp Ile His His Ala Pro Pro Gly Cys Gly Asn Ala
        195                 200                 205

Pro Arg Ser Ser Met Ser Asn Thr Cys Asp Glu Lys Thr Gln Ser Leu
    210                 215                 220

Gly Val Lys Phe Leu Asp Glu Tyr Gln Ser Lys Val Lys Arg Gln Ile
225                 230                 235                 240

Phe Ser Gly Tyr Gln Ser Asp Ile Asp Thr His Asn Arg Ile Lys Asp
                245                 250                 255

Glu Leu

<210> 68
<211> 124
<212> PRT
<213> Vibrio cholerae

<400> 68
Met Ile Lys Leu Lys Phe Gly Val Phe Phe Thr Val Leu Leu Ser Ser
1               5                   10                  15

Ala Tyr Ala His Gly Thr Pro Gln Asn Ile Thr Asp Leu Cys Ala Glu
            20                  25                  30

Tyr His Asn Thr Gln Ile His Thr Leu Asn Asp Lys Ile Leu Ser Tyr
        35                  40                  45

Thr Glu Ser Leu Ala Gly Asn Arg Glu Met Ala Ile Ile Thr Phe Lys
    50                  55                  60

Asn Gly Ala Thr Phe Gln Val Glu Val Pro Gly Ser Gln His Ile Asp
65                  70                  75                  80

Ser Gln Lys Lys Ala Ile Glu Arg Met Lys Asp Thr Leu Arg Ile Ala
                85                  90                  95

Tyr Leu Thr Glu Ala Lys Val Glu Lys Leu Cys Val Trp Asn Asn Lys
            100                 105                 110

Thr Pro His Ala Ile Ala Ala Ile Ser Met Ala Asn
        115                 120

<210> 69
<211> 18
<212> PRT
<213> Homo sapiens

<400> 69
Asp Pro Asn Ala Pro Lys Arg Pro Pro Ser Ala Phe Phe Leu Phe Cys
1               5                   10                  15

Ser Glu

<210> 70
<211> 96
<212> PRT
<213> Homo sapiens

<400> 70
Met Cys Cys Thr Lys Ser Leu Leu Leu Ala Ala Leu Met Ser Val Leu
1               5                   10                  15

Leu Leu His Leu Cys Gly Glu Ser Glu Ala Ala Ser Asn Phe Asp Cys
            20                  25                  30

Cys Leu Gly Tyr Thr Asp Arg Ile Leu His Pro Lys Phe Ile Val Gly
        35                  40                  45

Phe Thr Arg Gln Leu Ala Asn Glu Gly Cys Asp Ile Asn Ala Ile Ile
    50                  55                  60

Phe His Thr Lys Lys Lys Leu Ser Val Cys Ala Asn Pro Lys Gln Thr
65                  70                  75                  80

Trp Val Lys Tyr Ile Val Arg Leu Leu Ser Lys Lys Val Lys Asn Met
                85                  90                  95

<210> 71
<211> 92
<212> PRT
<213> Homo sapiens

<400> 71
Met Gln Val Ser Thr Ala Ala Leu Ala Val Leu Leu Cys Thr Met Ala
1               5                   10                  15

Leu Cys Asn Gln Phe Ser Ala Ser Leu Ala Ala Asp Thr Pro Thr Ala
            20                  25                  30

Cys Cys Phe Ser Tyr Thr Ser Arg Gln Ile Pro Gln Asn Phe Ile Ala
        35                  40                  45

Asp Tyr Phe Glu Thr Ser Ser Gln Cys Ser Lys Pro Gly Val Ile Phe
    50                  55                  60

Leu Thr Lys Arg Ser Arg Gln Val Cys Ala Asp Pro Ser Glu Glu Trp

65                  70                  75                  80

Val Gln Lys Tyr Val Ser Asp Leu Glu Leu Ser Ala
                85                  90

<210> 72
<211> 144
<212> PRT
<213> Homo sapiens

<400> 72
Met Trp Leu Gln Ser Leu Leu Leu Leu Gly Thr Val Ala Cys Ser Ile
1               5                   10                  15

Ser Ala Pro Ala Arg Ser Pro Ser Pro Ser Thr Gln Pro Trp Glu His
            20                  25                  30

Val Asn Ala Ile Gln Glu Ala Arg Arg Leu Leu Asn Leu Ser Arg Asp
        35                  40                  45

Thr Ala Ala Glu Met Asn Glu Thr Val Glu Val Ile Ser Glu Met Phe
    50                  55                  60

Asp Leu Gln Glu Pro Thr Cys Leu Gln Thr Arg Leu Glu Leu Tyr Lys
65                  70                  75                  80

Gln Gly Leu Arg Gly Ser Leu Thr Lys Leu Lys Gly Pro Leu Thr Met
                85                  90                  95

Met Ala Ser His Tyr Lys Gln His Cys Pro Pro Thr Pro Glu Thr Ser
            100                 105                 110

Cys Ala Thr Gln Ile Ile Thr Phe Glu Ser Phe Lys Glu Asn Leu Lys
        115                 120                 125

Asp Phe Leu Leu Val Ile Pro Phe Asp Cys Trp Glu Pro Val Gln Glu
    130                 135                 140

<210> 73
<211> 204
<212> PRT
<213> Homo sapiens

<400> 73
Met Ala Gly Pro Ala Thr Gln Ser Pro Met Lys Leu Met Ala Leu Gln
1               5                   10                  15

Leu Leu Leu Trp His Ser Ala Leu Trp Thr Val Gln Glu Ala Thr Pro
            20                  25                  30

Leu Gly Pro Ala Ser Ser Leu Pro Gln Ser Phe Leu Leu Lys Cys Leu
        35                  40                  45

Glu Gln Val Arg Lys Ile Gln Gly Asp Gly Ala Ala Leu Gln Glu Lys
    50                  55                  60

Leu Cys Ala Thr Tyr Lys Leu Cys His Pro Glu Glu Leu Val Leu Leu
65                  70                  75                  80

Gly His Ser Leu Gly Ile Pro Trp Ala Pro Leu Ser Ser Cys Pro Ser
                85                  90                  95

Gln Ala Leu Gln Leu Ala Gly Cys Leu Ser Gln Leu His Ser Gly Leu
            100                 105                 110

Phe Leu Tyr Gln Gly Leu Leu Gln Ala Leu Glu Gly Ile Ser Pro Glu
        115                 120                 125

Leu Gly Pro Thr Leu Asp Thr Leu Gln Leu Asp Val Ala Asp Phe Ala
    130                 135                 140

Thr Thr Ile Trp Gln Gln Met Glu Glu Leu Gly Met Ala Pro Ala Leu
145                 150                 155                 160

Gln Pro Thr Gln Gly Ala Met Pro Ala Phe Ala Ser Ala Phe Gln Arg
                165                 170                 175

Arg Ala Gly Gly Val Leu Val Ala Ser His Leu Gln Ser Phe Leu Glu
            180                 185                 190

Val Ser Tyr Arg Val Leu Arg His Leu Ala Gln Pro
        195                 200

<210> 74
<211> 7
<212> PRT
<213> Artificial Sequence

<220>
<223> Description of Artificial Sequence: Synthetic
      peptide

<400> 74
Gln Glu Ile Asn Ser Ser Tyr
1               5

<210> 75
<211> 7
<212> PRT
<213> Artificial Sequence

<220>
<223> Description of Artificial Sequence: Synthetic
      peptide

<400> 75
Ser His Pro Arg Leu Ser Ala
1               5


<210> 76
<211> 7
<212> PRT
<213> Artificial Sequence

<220>
<223> Description of Artificial Sequence: Synthetic
      peptide

<400> 76
Ser Met Pro Asn Pro Met Val
1               5


<210> 77
<211> 7
<212> PRT
<213> Artificial Sequence

<220>
<223> Description of Artificial Sequence: Synthetic
      peptide

<400> 77
Gly Leu Gln Gln Val Leu Leu
1               5


<210> 78
<211> 7
<212> PRT
<213> Artificial Sequence

<220>
<223> Description of Artificial Sequence: Synthetic
      peptide

<400> 78
His Glu Leu Ser Val Leu Leu
1               5


<210> 79
<211> 7
<212> PRT
<213> Artificial Sequence

<220>
<223> Description of Artificial Sequence: Synthetic
      peptide

<400> 79
Tyr Ala Pro Gln Arg Leu Pro
1               5

<210> 80
<211> 7
<212> PRT
<213> Artificial Sequence

<220>
<223> Description of Artificial Sequence: Synthetic
      peptide

<400> 80
Thr Pro Arg Thr Leu Pro Thr
1               5


<210> 81
<211> 7
<212> PRT
<213> Artificial Sequence

<220>
<223> Description of Artificial Sequence: Synthetic
      peptide

<400> 81
Ala Pro Val His Ser Ser Ile
1               5


<210> 82
<211> 7
<212> PRT
<213> Artificial Sequence

<220>
<223> Description of Artificial Sequence: Synthetic
      peptide

<400> 82
Ala Pro Pro His Ala Leu Ser
1               5


<210> 83
<211> 7
<212> PRT
<213> Artificial Sequence

<220>
<223> Description of Artificial Sequence: Synthetic
      peptide

<400> 83
Thr Phe Ser Asn Arg Phe Ile
1               5


<210> 84
<211> 7
<212> PRT
<213> Artificial Sequence

<220>
<223> Description of Artificial Sequence: Synthetic
      peptide

<400> 84
Val Val Pro Thr Pro Pro Tyr
1               5


<210> 85
<211> 7
<212> PRT
<213> Artificial Sequence

<220>
<223> Description of Artificial Sequence: Synthetic
      peptide

<400> 85
Glu Leu Ala Pro Asp Ser Pro
1               5


<210> 86
<211> 69
<212> PRT
<213> Shigella dysenteria 1

<400> 86
Thr Pro Asp Cys Val Thr Gly Lys Val Glu Tyr Thr Lys Tyr Asn Asp
1               5                   10                  15

Asp Asp Thr Phe Thr Val Lys Val Gly Asp Lys Glu Leu Phe Thr Asn
            20                  25                  30

Arg Trp Asn Leu Gln Ser Leu Leu Leu Ser Ala Gln Ile Thr Gly Met
        35                  40                  45

Thr Val Thr Ile Lys Gln Asn Ala Cys His Asn Gly Gly Gly Phe Ser
    50                  55                  60

Glu Val Ile Phe Arg
65


<210> 87
<211> 560
<212> PRT
<213> Corynephage omega

<400> 87
Met Ser Arg Lys Leu Phe Ala Ser Ile Leu Ile Gly Ala Leu Leu Gly
1               5                   10                  15

Ile Gly Ala Pro Pro Ser Ala His Ala Gly Ala Asp Asp Val Val Asp
            20                  25                  30

Ser Ser Lys Ser Phe Val Met Glu Asn Phe Ser Ser Tyr His Gly Thr
        35                  40                  45

Lys Pro Gly Tyr Val Asp Ser Ile Gln Lys Gly Ile Gln Lys Pro Lys
    50                  55                  60

Ser Gly Thr Gln Gly Asn Tyr Asp Asp Asp Trp Lys Gly Phe Tyr Ser
65                  70                  75                  80

Thr Asp Asn Lys Tyr Asp Ala Ala Gly Tyr Ser Val Asp Asn Glu Asn
                85                  90                  95

Pro Leu Ser Gly Lys Ala Gly Gly Val Val Lys Val Thr Tyr Pro Gly
            100                 105                 110

Leu Thr Lys Val Leu Ala Leu Lys Val Asp Asn Ala Glu Thr Ile Lys
        115                 120                 125

Lys Glu Leu Gly Leu Ser Leu Thr Glu Pro Leu Met Glu Gln Val Gly
    130                 135                 140

Thr Glu Glu Phe Ile Lys Arg Phe Gly Asp Gly Ala Ser Arg Val Val
145                 150                 155                 160

Leu Ser Leu Pro Phe Ala Glu Gly Ser Ser Ser Val Glu Tyr Ile Asn
                165                 170                 175

Asn Trp Glu Gln Ala Lys Ala Leu Ser Val Glu Leu Glu Ile Asn Phe
            180                 185                 190

Glu Thr Arg Gly Lys Arg Gly Gln Asp Ala Met Tyr Glu Tyr Met Ala
        195                 200                 205

Gln Ala Cys Ala Gly Asn Arg Val Arg Arg Ser Val Gly Ser Ser Leu
    210                 215                 220

Ser Cys Ile Asn Leu Asp Trp Asp Val Ile Arg Asp Lys Thr Lys Thr
225                 230                 235                 240

Lys Ile Glu Ser Leu Lys Glu His Gly Pro Ile Lys Asn Lys Met Ser
                245                 250                 255

Glu Ser Pro Asn Lys Thr Val Ser Glu Glu Lys Ala Lys Gln Tyr Leu
            260                 265                 270

Glu Glu Phe His Gln Thr Ala Leu Glu His Pro Glu Leu Ser Glu Leu
        275                 280                 285

Lys Thr Val Thr Gly Thr Asn Pro Val Phe Ala Gly Ala Asn Tyr Ala
    290                 295                 300

Ala Trp Ala Val Asn Val Ala Gln Val Ile Asp Ser Glu Thr Ala Asp
305                 310                 315                 320

Asn Leu Glu Lys Thr Thr Ala Ala Leu Ser Ile Leu Pro Gly Ile Gly
                325                 330                 335

Ser Val Met Gly Ile Ala Asp Gly Ala Val His His Asn Thr Glu Glu
            340                 345                 350

Ile Val Ala Gln Ser Ile Ala Leu Ser Ser Leu Met Val Ala Gln Ala
        355                 360                 365

Ile Pro Leu Val Gly Glu Leu Val Asp Ile Gly Phe Ala Ala Tyr Asn
    370                 375                 380

Phe Val Glu Ser Ile Ile Asn Leu Phe Gln Val Val His Asn Ser Tyr
385                 390                 395                 400

Asn Arg Pro Ala Tyr Ser Pro Gly His Lys Thr Gln Pro Phe Leu His
                405                 410                 415

Asp Gly Tyr Ala Val Ser Trp Asn Thr Val Glu Asp Ser Ile Ile Arg
            420                 425                 430

Thr Gly Phe Gln Gly Glu Ser Gly His Asp Ile Lys Ile Thr Ala Glu
        435                 440                 445

Asn Thr Pro Leu Pro Ile Ala Gly Val Leu Leu Pro Thr Ile Pro Gly
    450                 455                 460

Lys Leu Asp Val Asn Lys Ser Lys Thr His Ile Ser Val Asn Gly Arg
465                 470                 475                 480

Lys Ile Arg Met Arg Cys Arg Ala Ile Asp Gly Asp Val Thr Phe Cys
                485                 490                 495

Arg Pro Lys Ser Pro Val Tyr Val Gly Asn Gly Val His Ala Asn Leu
            500                 505                 510

His Val Ala Phe His Arg Ser Ser Ser Glu Lys Ile His Ser Asn Glu
        515                 520                 525

Ile Ser Ser Asp Ser Ile Gly Val Leu Gly Tyr Gln Lys Thr Val Asp
    530                 535                 540

His Thr Lys Val Asn Ser Lys Leu Ser Leu Phe Phe Glu Ile Lys Ser
545                 550                 555                 560

<210> 88
<211> 114
<212> PRT
<213> Artificial Sequence

<220>
<223> Description of Artificial Sequence: Synthetic
      polypeptide

<400> 88
Asn Trp Val Asn Val Ile Ser Asp Leu Lys Lys Ile Glu Asp Leu Ile
1               5                   10                  15

Gln Ser Met His Ile Asp Ala Thr Leu Tyr Thr Glu Ser Asp Val His
            20                  25                  30

Pro Ser Cys Lys Val Thr Ala Met Lys Cys Phe Leu Leu Glu Leu Gln
        35                  40                  45

Val Ile Ser Leu Glu Ser Gly Asp Ala Ser Ile His Asp Thr Val Glu
    50                  55                  60

Asn Leu Ile Ile Leu Ala Asn Asp Ser Leu Ser Ser Asn Gly Asn Val
65                  70                  75                  80

Thr Glu Ser Gly Cys Lys Glu Cys Glu Glu Leu Glu Glu Lys Asn Ile
                85                  90                  95

Lys Glu Phe Leu Gln Ser Phe Val His Ile Val Gln Met Phe Ile Asn
            100                 105                 110

Thr Ser

<210> 89
<211> 297
<212> PRT
<213> Artificial Sequence

<220>
<223> Description of Artificial Sequence: Synthetic
      polypeptide

<400> 89
Ile Thr Cys Pro Pro Pro Met Ser Val Glu His Ala Asp Ile Trp Val
1               5                   10                  15

Lys Ser Tyr Ser Leu Tyr Ser Arg Glu Arg Tyr Ile Cys Asn Ser Gly

20                  25                  30

Phe Lys Arg Lys Ala Gly Thr Ser Ser Leu Thr Glu Cys Val Leu Asn
        35                  40                  45

Lys Ala Thr Asn Val Ala His Trp Thr Thr Pro Ser Leu Lys Cys Ile
    50                  55                  60

Arg Glu Pro Lys Ser Cys Asp Lys Thr His Thr Cys Pro Pro Cys Pro
65                  70                  75                  80

Ala Pro Glu Leu Leu Gly Gly Pro Ser Val Phe Leu Phe Pro Pro Lys
                85                  90                  95

Pro Lys Asp Thr Leu Met Ile Ser Arg Thr Pro Glu Val Thr Cys Val
            100                 105                 110

Val Val Asp Val Ser His Glu Asp Pro Glu Val Lys Phe Asn Trp Tyr
        115                 120                 125

Val Asp Gly Val Glu Val His Asn Ala Lys Thr Lys Pro Arg Glu Glu
    130                 135                 140

Gln Tyr Asn Ser Thr Tyr Arg Val Val Ser Val Leu Thr Val Leu His
145                 150                 155                 160

Gln Asp Trp Leu Asn Gly Lys Glu Tyr Lys Cys Lys Val Ser Asn Lys
                165                 170                 175

Ala Leu Pro Ala Pro Ile Glu Lys Thr Ile Ser Lys Ala Lys Gly Gln
            180                 185                 190

Pro Arg Glu Pro Gln Val Tyr Thr Leu Pro Pro Ser Arg Asp Glu Leu
        195                 200                 205

Thr Lys Asn Gln Val Ser Leu Thr Cys Leu Val Lys Gly Phe Tyr Pro
    210                 215                 220

Ser Asp Ile Ala Val Glu Trp Glu Ser Asn Gly Gln Pro Glu Asn Asn
225                 230                 235                 240

Tyr Lys Thr Thr Pro Pro Val Leu Asp Ser Asp Gly Ser Phe Phe Leu
                245                 250                 255

Tyr Ser Lys Leu Thr Val Asp Lys Ser Arg Trp Gln Gln Gly Asn Val
            260                 265                 270

Phe Ser Cys Ser Val Met His Glu Ala Leu His Asn His Tyr Thr Gln
        275                 280                 285

Lys Ser Leu Ser Leu Ser Pro Gly Lys
    290                 295

<210> 90
<211> 535
<212> PRT
<213> Corynebacterium diphtheriae

<400> 90
Gly Ala Asp Asp Val Val Asp Ser Ser Lys Ser Phe Val Met Glu Asn
1               5                   10                  15

Phe Ser Ser Tyr His Gly Thr Lys Pro Gly Tyr Val Asp Ser Ile Gln
            20                  25                  30

Lys Gly Ile Gln Lys Pro Lys Ser Gly Thr Gln Gly Asn Tyr Asp Asp
        35                  40                  45

Asp Trp Lys Glu Phe Tyr Ser Thr Asp Asn Lys Tyr Asp Ala Ala Gly
    50                  55                  60

Tyr Ser Val Asp Asn Glu Asn Pro Leu Ser Gly Lys Ala Gly Gly Val
65                  70                  75                  80

Val Lys Val Thr Tyr Pro Gly Leu Thr Lys Val Leu Ala Leu Lys Val
                85                  90                  95

Asp Asn Ala Glu Thr Ile Lys Lys Glu Leu Gly Leu Ser Leu Thr Glu
            100                 105                 110

Pro Leu Met Glu Gln Val Gly Thr Glu Glu Phe Ile Lys Arg Phe Gly
        115                 120                 125

Asp Gly Ala Ser Arg Val Val Leu Ser Leu Pro Phe Ala Glu Gly Ser
    130                 135                 140

Ser Ser Val Glu Tyr Ile Asn Asn Trp Glu Gln Ala Lys Ala Leu Ser
145                 150                 155                 160

Val Glu Leu Glu Ile Asn Phe Glu Thr Arg Gly Lys Arg Gly Gln Asp
                165                 170                 175

Ala Met Tyr Glu Tyr Met Ala Gln Ala Cys Ala Gly Asn Arg Val Arg
            180                 185                 190

Arg Ser Val Gly Ser Ser Leu Ser Cys Ile Asn Leu Asp Trp Asp Val

195                 200                 205

Ile Arg Asp Lys Thr Lys Thr Lys Ile Glu Ser Leu Lys Glu His Gly
    210                 215                 220

Pro Ile Lys Asn Lys Met Ser Glu Ser Pro Asn Lys Thr Val Ser Glu
225                 230                 235                 240

Glu Lys Ala Lys Gln Tyr Leu Glu Glu Phe His Gln Thr Ala Leu Glu
                245                 250                 255

His Pro Glu Leu Ser Glu Leu Lys Thr Val Thr Gly Thr Asn Pro Val
            260                 265                 270

Phe Ala Gly Ala Asn Tyr Ala Ala Trp Ala Val Asn Val Ala Gln Val
        275                 280                 285

Ile Asp Ser Glu Thr Ala Asp Asn Leu Glu Lys Thr Thr Ala Ala Leu
    290                 295                 300

Ser Ile Leu Pro Gly Ile Gly Ser Val Met Gly Ile Ala Asp Gly Ala
305                 310                 315                 320

Val His His Asn Thr Glu Glu Ile Val Ala Gln Ser Ile Ala Leu Ser
                325                 330                 335

Ser Leu Met Val Ala Gln Ala Ile Pro Leu Val Gly Glu Leu Val Asp
            340                 345                 350

Ile Gly Phe Ala Ala Tyr Asn Phe Val Glu Ser Ile Ile Asn Leu Phe
        355                 360                 365

Gln Val Val His Asn Ser Tyr Asn Arg Pro Ala Tyr Ser Pro Gly His
    370                 375                 380

Lys Thr Gln Pro Phe Leu His Asp Gly Tyr Ala Val Ser Trp Asn Thr
385                 390                 395                 400

Val Glu Asp Ser Ile Ile Arg Thr Gly Phe Gln Gly Glu Ser Gly His
                405                 410                 415

Asp Ile Lys Ile Thr Ala Glu Asn Thr Pro Leu Pro Ile Ala Gly Val
            420                 425                 430

Leu Leu Pro Thr Ile Pro Gly Lys Leu Asp Val Asn Lys Ser Lys Thr
        435                 440                 445

His Ile Ser Val Asn Gly Arg Lys Ile Arg Met Arg Cys Arg Ala Ile
    450                 455                 460

Asp Gly Asp Val Thr Phe Cys Arg Pro Lys Ser Pro Val Tyr Val Gly
465                 470                 475                 480

Asn Gly Val His Ala Asn Leu His Val Ala Phe His Arg Ser Ser Ser
                485                 490                 495

Glu Lys Ile His Ser Asn Glu Ile Ser Ser Asp Ser Ile Gly Val Leu
            500                 505                 510

Gly Tyr Gln Lys Thr Val Asp His Thr Lys Val Asn Ser Lys Leu Ser
        515                 520                 525

Leu Phe Phe Glu Ile Lys Ser
    530                 535

<210> 91
<211> 11
<212> PRT
<213> Artificial Sequence

<220>
<223> Description of Artificial Sequence: Synthetic
      peptide

<400> 91
Met Ala Val Pro Met Gln Leu Ser Cys Ser Arg
1               5                   10

<210> 92
<211> 4
<212> PRT
<213> Artificial Sequence

<220>
<223> Description of Artificial Sequence: Synthetic
      peptide

<400> 92
Arg Ser Thr Gly
1

<210> 93
<211> 2
<212> PRT
<213> Artificial Sequence

<220>
<223> Description of Artificial Sequence: Synthetic
      peptide

<400> 93
Thr Arg

1


<210> 94
<211> 3
<212> PRT
<213> Artificial Sequence

<220>
<223> Description of Artificial Sequence: Synthetic
      peptide

<400> 94
Arg Ser Gln
1


<210> 95
<211> 5
<212> PRT
<213> Artificial Sequence

<220>
<223> Description of Artificial Sequence: Synthetic
      peptide

<400> 95
Arg Ser Ala Gly Glu
1               5


<210> 96
<211> 2
<212> PRT
<213> Artificial Sequence

<220>
<223> Description of Artificial Sequence: Synthetic
      peptide

<400> 96
Arg Ser
1


<210> 97
<211> 2
<212> PRT
<213> Artificial Sequence

<220>
<223> Description of Artificial Sequence: Synthetic
      peptide

<400> 97
Gly Gly
1


<210> 98
<211> 9
<212> PRT
<213> Artificial Sequence

<220>
<223> Description of Artificial Sequence: Synthetic
      peptide

<400> 98
Gly Ser Gly Gly Ser Gly Gly Ser Gly
1               5

<210> 99
<211> 11
<212> PRT
<213> Artificial Sequence

<220>
<223> Description of Artificial Sequence: Synthetic
      peptide

<400> 99
Gly Gly Ser Gly Gly Ser Gly Gly Ser Gly Gly
1               5                   10

<210> 100
<211> 14
<212> PRT
<213> Artificial Sequence

<220>
<223> Description of Artificial Sequence: Synthetic
      peptide

<400> 100
Gly Gly Ser Gly Gly Ser Gly Gly Ser Gly Gly Ser Gly Gly
1               5                   10

<210> 101
<211> 17
<212> PRT
<213> Artificial Sequence

<220>
<223> Description of Artificial Sequence: Synthetic
      peptide

<400> 101
Gly Gly Ser Gly Gly Ser Gly Gly Ser Gly Gly Ser Gly Gly Ser Gly
1               5                   10                  15

Gly

<210> 102
<211> 20
<212> PRT
<213> Artificial Sequence

<220>
<223> Description of Artificial Sequence: Synthetic

peptide

<400> 102
Gly Gly Ser Gly Gly Ser Gly Gly Ser Gly Gly Ser Gly Gly Ser Gly
1               5                   10                  15

Gly Ser Gly Gly
            20

<210> 103
<211> 23
<212> PRT
<213> Artificial Sequence

<220>
<223> Description of Artificial Sequence: Synthetic
      peptide

<400> 103
Gly Gly Ser Gly Gly Ser Gly Gly Ser Gly Gly Ser Gly Gly Ser Gly
1               5                   10                  15

Gly Ser Gly Gly Ser Gly Gly
            20

<210> 104
<211> 16
<212> PRT
<213> Artificial Sequence

<220>
<223> Description of Artificial Sequence: Synthetic
      peptide

<400> 104
Gly Gly Ser Gly Gly Ser Gly Gly Ser Gly Gly Ser Gly Gly Ser Gly
1               5                   10                  15

<210> 105
<211> 16
<212> PRT
<213> Artificial Sequence

<220>
<223> Description of Artificial Sequence: Synthetic
      peptide

<400> 105
Gly Ser Gly Gly Ser Gly Gly Ser Gly Gly Ser Gly Gly Ser Gly Gly
1               5                   10                  15

<210> 106
<211> 2109
<212> DNA
<213> Homo sapiens

<400> 106

atggagtctc cctcggcccc tccccacaga tggtgcatcc cctggcagag gctcctgctc 60

acagcctcac ttctaacctt ctggaacccg cccaccactg ccaagctcac tattgaatcc 120

acgccgttca atgtcgcaga ggggaaggag gtgcttctac ttgtccacaa tctgccccag 180

catctttttg gctacagctg gtacaaaggt gaaagagtgg atggcaaccg tcaaattata 240

ggatatgtaa taggaactca acaagctacc ccagggcccg catacagtgg tcgagagata 300

atatacccca atgcatccct gctgatccag aacatcatcc agaatgacac aggattctac 360

accctacacg tcataaagtc agatcttgtg aatgaagaag caactggcca gttccgggta 420

tacccggagc tgcccaagcc ctccatctcc agcaacaact ccaaacccgt ggaggacaag 480

gatgctgtgg ccttcacctg tgaacctgag actcaggacg caacctacct gtggtgggta 540

aacaatcaga gcctcccggt cagtcccagg ctgcagctgt ccaatggcaa caggaccctc 600

actctattca atgtcacaag aaatgacaca gcaagctaca aatgtgaaac ccagaaccca 660

gtgagtgcca ggcgcagtga ttcagtcatc ctgaatgtcc tctatggccc ggatgccccc 720

accatttccc ctctaaacac atcttacaga tcaggggaaa atctgaacct ctcctgccac 780

gcagcctcta acccacctgc acagtactct tggtttgtca atgggacttt ccagcaatcc 840

acccaagagc tctttatccc caacatcact gtgaataata gtggatccta tacgtgccaa 900

gcccataact cagacactgg cctcaatagg accacagtca cgacgatcac agtctatgca 960

gagccaccca aacccttcat caccagcaac aactccaacc ccgtggagga tgaggatgct 1020

gtagccttaa cctgtgaacc tgagattcag aacacaacct acctgtggtg ggtaaataat 1080

cagagcctcc cggtcagtcc caggctgcag ctgtccaatg acaacaggac cctcactcta 1140

ctcagtgtca caaggaatga tgtaggaccc tatgagtgtg gaatccagaa cgaattaagt 1200

gttgaccaca gcgacccagt catcctgaat gtcctctatg gcccagacga ccccaccatt 1260

tccccctcat acacctatta ccgtccaggg gtgaacctca gcctctcctg ccatgcagcc 1320

tctaacccac ctgcacagta ttcttggctg attgatggga acatccagca acacacacaa 1380

gagctcttta tctccaacat cactgagaag aacagcggac tctatacctg ccaggccaat 1440

aactcagcca gtggccacag caggactaca gtcaagacaa tcacagtctc tgcggagctg 1500

cccaagccct ccatctccag caacaactcc aaacccgtgg aggacaagga tgctgtggcc 1560

ttcacctgtg aacctgaggc tcagaacaca acctacctgt ggtgggtaaa tggtcagagc 1620

ctcccagtca gtcccaggct gcagctgtcc aatggcaaca ggaccctcac tctattcaat 1680

gtcacaagaa atgacgcaag agcctatgta tgtggaatcc agaactcagt gagtgcaaac 1740

cgcagtgacc cagtcaccct ggatgtcctc tatgggccgg acacccccat catttccccc 1800

ccagactcgt cttacctttc gggagcggac ctcaacctct cctgccactc ggcctctaac 1860

ccatccccgc agtattcttg gcgtatcaat gggataccgc agcaacacac acaagttctc 1920

tttatcgcca aaatcacgcc aaataataac gggacctatg cctgttttgt ctctaacttg 1980

gctactggcc gcaataattc catagtcaag agcatcacag tctctgcatc tggaacttct 2040

cctggtctct cagctggggc cactgtcggc atcatgattg gagtgctggt tggggttgct 2100

ctgatatag 2109

<210> 107
<211> 1428
<212> DNA
<213> Homo sapiens

<400> 107
atgacaccgg gcacccagtc tcctttcttc ctgctgctgc tcctcacagt gcttacagtt 60

gttacgggtt ctggtcatgc aagctctacc ccaggtggag aaaaggagac ttcggctacc 120

cagagaagtt cagtgcccag ctctactgag aagaatgctg tgagtatgac cagcagcgta 180

ctctccagcc acagccccgg ttcaggctcc tccaccactc agggacagga tgtcactctg 240

gccccggcca cggaaccagc ttcaggttca gctgcccttt ggggacagga tgtcacctcg 300

gtcccagtca ccaggccagc cctgggctcc accaccccgc cagcccacga tgtcacctca 360

gccccggaca acaagccagc cccgggctcc accgcccccc cagcccacgg tgtcacctcg 420

tatcttgaca ccaggccggc cccggtttat cttgcccccc cagcccatgg tgtcacctcg 480

gccccggaca acaggcccgc cttgggctcc accgcccctc cagtccacaa tgtcacctcg 540

gcctcaggct ctgcatcagg ctcagcttct actctggtgc acaacggcac ctctgccagg 600

gctaccacaa ccccagccag caagagcact ccattctcaa ttcccagcca ccactctgat 660

actcctacca cccttgccag ccatagcacc aagactgatg ccagtagcac tcaccatagc 720

acggtacctc ctctcacctc ctccaatcac agcacttctc cccagttgtc tactggggtc 780

tctttctttt tcctgtcttt tcacatttca aacctccagt ttaattcctc tctggaagat 840

cccagcaccg actactacca agagctgcag agagacattt ctgaaatgtt tttgcagatt 900

tataaacaag gggtttttct gggcctctcc aatattaagt tcaggccagg atctgtggtg 960

gtacaattga ctctggcctt ccgagaaggt accatcaatg tccacgacgt ggagacacag 1020

ttcaatcagt ataaaacgga agcagcctct cgatataacc tgacgatctc agacgtcagc 1080

gtgagtgatg tgccatttcc tttctctgcc cagtctgggg ctggggtgcc aggctggggc 1140

atcgcgctgc tggtgctggt ctgtgttctg gtttatctgg ccattgtcta tctcattgcc 1200

ttggctgtcg ctcaggttcg ccgaaagaac tacgggcagc tggacatctt tccagcccgg 1260

gataaatacc atcctatgag cgagtacgct ctttaccaca cccatgggcg ctatgtgccc 1320

cctagcagtc ttttccgtag cccctatgag aaggtttctg caggtaatgg tggcagctat 1380

ctctcttaca caaacccagc agtggcagcc gcttctgcca acttgtag 1428

<210> 108
<211> 1233
<212> DNA
<213> Homo sapiens

<400> 108
   atgagctccc ctggcaccga gagcgcggga aagagcctgc agtaccgagt ggaccacctg 60
   ctgagcgccg tggagaatga gctgcaggcg ggcagcgaga agggcgaccc cacagagcgc 120
   gaactgcgcg tgggcctgga ggagagcgag ctgtggctgc gcttcaagga gctcaccaat 180
   gagatgatcg tgaccaagaa cggcaggagg atgtttccgg tgctgaaggt gaacgtgtct 240
   ggcctggacc ccaacgccat gtactccttc ctgctggact tcgtggcggc ggacaaccac 300
   cgctggaagt acgtgaacgg ggaatgggtg ccggggggca agccggagcc gcaggcgccc 360
   agctgcgtct acatccaccc cgactcgccc aacttcgggg cccactggat gaaggctccc 420
   gtctccttca gcaaagtcaa gctcaccaac aagctcaacg gagggggcca gatcatgctg 480
   aactccttgc ataagtatga gcctcgaatc cacatagtga gagttggggg tccacagcgc 540
   atgatcacca gccactgctt ccctgagacc cagttcatag cggtgactgc tagaagtgat 600
   cacaaagaga tgatggagga acccggagac agccagcaac ctgggtactc ccaatggggg 660
   tggcttcttc ctggaaccag caccgtgtgt ccacctgcaa atcctcatcc tcagtttgga 720
   ggtgccctct ccctcccctc cacgcacagc tgtgacaggt acccaaccct gaggagccac 780
   cggtcctcac cctaccccag cccctatgct catcggaaca attctccaac ctattctgac 840
   aactcacctg catgtttatc catgctgcaa tcccatgaca attggtccag ccttggaatg 900
   cctgcccatc ccagcatgct ccccgtgagc cacaatgcca gcccacctac cagctccagt 960
   cagtacccca gcctgtggtc tgtgagcaac ggcgccgtca ccccgggctc ccaggcagca 1020
   gccgtgtcca acgggctggg ggcccagttc ttccggggct cccccgcgca ctacacaccc 1080
   ctcacccatc cggtctcggc gccctcttcc tcgggatccc cactgtacga aggggcggcc 1140
   gcggccacag acatcgtgga cagccagtac gacgccgcag cccaaggccg cctcatagcc 1200
   tcatggacac ctgtgtcgcc accttccatg tga 1233


<210> 109
<211> 1331
<212> PRT
<213> Homo sapiens

<400> 109

<210> 110
<211> 155
<212> PRT
<213> Homo sapiens

<400> 110
Met Thr Pro Gly Lys Thr Ser Leu Val Ser Leu Leu Leu Leu Leu Ser
1               5                   10                  15

Leu Glu Ala Ile Val Lys Ala Gly Ile Thr Ile Pro Arg Asn Pro Gly
            20                  25                  30

Cys Pro Asn Ser Glu Asp Lys Asn Phe Pro Arg Thr Val Met Val Asn
        35                  40                  45

Leu Asn Ile His Asn Arg Asn Thr Asn Thr Asn Pro Lys Arg Ser Ser
    50                  55                  60

Asp Tyr Tyr Asn Arg Ser Thr Ser Pro Trp Asn Leu His Arg Asn Glu
65                  70                  75                  80

Asp Pro Glu Arg Tyr Pro Ser Val Ile Trp Glu Ala Lys Cys Arg His
                85                  90                  95

Leu Gly Cys Ile Asn Ala Asp Gly Asn Val Asp Tyr His Met Asn Ser
            100                 105                 110

Val Pro Ile Gln Gln Glu Ile Leu Val Leu Arg Arg Glu Pro Pro His
        115                 120                 125

Cys Pro Asn Ser Phe Arg Leu Glu Lys Ile Leu Val Ser Val Gly Cys
    130                 135                 140

Thr Cys Val Thr Pro Ile Val His His Val Ala
145                 150                 155

<210> 111
<211> 476
<212> PRT
<213> Homo sapiens

<400> 111
Arg Ala Val Pro Gly Gly Ser Ser Pro Ala Trp Thr Gln Cys Gln Gln
1               5                   10                  15

Leu Ser Gln Lys Leu Cys Thr Leu Ala Trp Ser Ala His Pro Leu Val
            20                  25                  30

Gly His Met Asp Leu Arg Glu Glu Gly Asp Glu Glu Thr Thr Asn Asp
        35                  40                  45

Val Pro His Ile Gln Cys Gly Asp Gly Cys Asp Pro Gln Gly Leu Arg
    50                  55                  60

Asp Asn Ser Gln Phe Cys Leu Gln Arg Ile His Gln Gly Leu Ile Phe
65                  70                  75                  80

Tyr Glu Lys Leu Leu Gly Ser Asp Ile Phe Thr Gly Glu Pro Ser Leu
                85                  90                  95

Leu Pro Asp Ser Pro Val Gly Gln Leu His Ala Ser Leu Leu Gly Leu
            100                 105                 110

Ser Gln Leu Leu Gln Pro Glu Gly His His Trp Glu Thr Gln Gln Ile
        115                 120                 125

Pro Ser Leu Ser Pro Ser Gln Pro Trp Gln Arg Leu Leu Leu Arg Phe
    130                 135                 140

Lys Ile Leu Arg Ser Leu Gln Ala Phe Val Ala Val Ala Ala Arg Val
145                 150                 155                 160

Phe Ala His Gly Ala Ala Thr Leu Ser Pro Ile Trp Glu Leu Lys Lys
                165                 170                 175

Asp Val Tyr Val Val Glu Leu Asp Trp Tyr Pro Asp Ala Pro Gly Glu
            180                 185                 190

Met Val Val Leu Thr Cys Asp Thr Pro Glu Glu Asp Gly Ile Thr Trp
        195                 200                 205

Thr Leu Asp Gln Ser Ser Glu Val Leu Gly Ser Gly Lys Thr Leu Thr
    210                 215                 220

Ile Gln Val Lys Glu Phe Gly Asp Ala Gly Gln Tyr Thr Cys His Lys
225                 230                 235                 240

Gly Gly Glu Val Leu Ser His Ser Leu Leu Leu Leu His Lys Lys Glu
                245                 250                 255

Asp Gly Ile Trp Ser Thr Asp Ile Leu Lys Asp Gln Lys Glu Pro Lys
            260                 265                 270

Asn Lys Thr Phe Leu Arg Cys Glu Ala Lys Asn Tyr Ser Gly Arg Phe
        275                 280                 285

Thr Cys Trp Trp Leu Thr Thr Ile Ser Thr Asp Leu Thr Phe Ser Val
    290                 295                 300

Lys Ser Ser Arg Gly Ser Ser Asp Pro Gln Gly Val Thr Cys Gly Ala
305                 310                 315                 320

Ala Thr Leu Ser Ala Glu Arg Val Arg Gly Asp Asn Lys Glu Tyr Glu
                325                 330                 335

Tyr Ser Val Glu Cys Gln Glu Asp Ser Ala Cys Pro Ala Ala Glu Glu
            340                 345                 350

Ser Leu Pro Ile Glu Val Met Val Asp Ala Val His Lys Leu Lys Tyr
        355                 360                 365

Glu Asn Tyr Thr Ser Ser Phe Phe Ile Arg Asp Ile Ile Lys Pro Asp
    370                 375                 380

Pro Pro Lys Asn Leu Gln Leu Lys Pro Leu Lys Asn Ser Arg Gln Val
385                 390                 395                 400

Glu Val Ser Trp Glu Tyr Pro Asp Thr Trp Ser Thr Pro His Ser Tyr
                405                 410                 415

Phe Ser Leu Thr Phe Cys Val Gln Val Gln Gly Lys Ser Lys Arg Glu
            420                 425                 430

Lys Lys Asp Arg Val Phe Thr Asp Lys Thr Ser Ala Thr Val Ile Cys
        435                 440                 445

Arg Lys Asn Ala Ser Ile Ser Val Arg Ala Gln Asp Arg Tyr Tyr Ser
    450                 455                 460

Ser Ser Trp Ser Glu Trp Ala Ser Val Pro Cys Ser
465                 470                 475

<210> 112
<211> 234
<212> PRT
<213> Homo sapiens

<400> 112
Met Cys Phe Pro Lys Val Leu Ser Asp Asp Met Lys Lys Leu Lys Ala
1               5                   10                  15

Arg Met Val Met Leu Leu Pro Thr Ser Ala Gln Gly Leu Gly Ala Trp
            20                  25                  30

Val Ser Ala Cys Asp Thr Glu Asp Thr Val Gly His Leu Gly Pro Trp
        35                  40                  45

Arg Asp Lys Asp Pro Ala Leu Trp Cys Gln Leu Cys Leu Ser Ser Gln
    50                  55                  60

His Gln Ala Ile Glu Arg Phe Tyr Asp Lys Met Gln Asn Ala Glu Ser
65                  70                  75                  80

Gly Arg Gly Gln Val Met Ser Ser Leu Ala Glu Leu Glu Asp Asp Phe
                85                  90                  95

Lys Glu Gly Tyr Leu Glu Thr Val Ala Ala Tyr Tyr Glu Glu Gln His
            100                 105                 110

Pro Glu Leu Thr Pro Leu Leu Glu Lys Glu Arg Asp Gly Leu Arg Cys
        115                 120                 125

Arg Gly Asn Arg Ser Pro Val Pro Asp Val Glu Asp Pro Ala Thr Glu
    130                 135                 140

Glu Pro Gly Glu Ser Phe Cys Asp Lys Val Met Arg Trp Phe Gln Ala
145                 150                 155                 160

Met Leu Gln Arg Leu Gln Thr Trp Trp His Gly Val Leu Ala Trp Val
                165                 170                 175

Lys Glu Lys Val Val Ala Leu Val His Ala Val Gln Ala Leu Trp Lys
            180                 185                 190

Gln Phe Gln Ser Phe Cys Cys Ser Leu Ser Glu Leu Phe Met Ser Ser
        195                 200                 205

Phe Gln Ser Tyr Gly Ala Pro Arg Gly Asp Lys Glu Glu Leu Thr Pro
    210                 215                 220

Gln Lys Cys Ser Glu Pro Gln Ser Ser Lys
225                 230

## Description

### CROSS REFERENCE

This application claims the benefit of U.S. Provisional Patent Application No. 62/342,752, filed May 27, 2016, the entire contents of which are incorporated by reference herein.

### BACKGROUND

Vaccination against cancer has been limited by the identification of relevant target antigens. Consequently, clinical vaccination trials targeting tumor-associated self-antigens have generally failed to elicit therapeutic immunity in spite of detection of vaccine-induced T-cell responses in blood.

In retrospect, these failures can be explained by the finding that many of these self-antigens are expressed in the thymus, resulting in the deletion of the highly reactive T-cell repertoire and development of suppressive T-regulatory cells. Moreover, circumvention of thymic tolerance by infusion of genetically engineered T cells targeting such antigens was found to be associated with severe toxicity in vital somatic tissues, illustrating the physiological importance of immunological tolerance to many tumor-associated antigens.

Therefore, there remains a need to discover novel compositions and methods for enhanced therapeutic response to complex diseases such as cancer.

### SUMMARY

In various aspects, the present disclosure provides a composition comprising a replication-defective vector, wherein the replication-defective vector comprises a nucleic acid sequence encoding for a tumor neo-antigen; and a nucleic acid sequence encoding for an immunological fusion partner.

In various aspects, the present disclosure provides a composition comprising a replication-defective vector, wherein the replication-defective vector comprises a nucleic acid sequenc encoding for CEA, MUC1-c, Brachyury, or any combination thereof; and a nucleic acid sequence encoding for an immunological fusion partner.

In some aspects, the replication-defective vector is an adenovirus vector. In some aspects, the adenovirus vector is an Ad5 vector. In some aspects, the replication defective vector comprises a deletion in an E2b region, an E1 region, an E3 region, and E4 region, or any combination thereof. In some aspects, the replication defective vector comprises a deletion in an E2b region. In some aspects, the replication-defective vector comprises a deletion of a DNA polymerase, preterminal protein (pTP), or a combination thereof in an E2b region. In further aspects, the replication-defective vector is not a gutted vector. In some aspects, the replication-defective vector comprises a plurality of replication-defective vectors, wherein each replication-defective vector comprises a different tumor neo-antigen-coding sequence. In some aspects, the replication-defective vector comprises at least ten replication-defective vectors, wherein each replication-defective vector comprises a different tumor neo-antigen-coding nucleic acid sequence. In some aspects, the replication-defective vector comprises at least five replication-defective vectors, wherein each replication-defective vector comprises a different tumor neo-antigen-coding nucleic acid sequence.

In other aspects, the immunological fusion partner comprises *Mycobacterium sp., Mycobacterium tuberculosis-derived* Ra12 fragment, protein D derived from a surface protein of gram-negative bacterium *Haemophilus influenzae* B, LYTA, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, CpG-ODN, truncated A subunit coding region derived from bacterial ADP-ribosylating exotoxin, truncated B subunit coding region derived from bacterial ADP-ribosylating exotoxin, Hp91, CCL20, CCL3, GM-CSF, G-CSF, LPS peptide mimic, shiga toxin, diphtheria toxin, IL-15 super agonist, ALT-803, CRM₁₉₇, or any combination thereof. In some aspects, the immunological fusion partner is a fragment or derivative of *Mycobacterium sp., Mycobacterium tuberculosis-derived* Ra12 fragment, protein D derived from a surface protein of gram-negative bacterium *Haemophilus influenzae* B, LYTA, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, CpG-ODN, truncated A subunit coding region derived from bacterial ADP-ribosylating exotoxin, truncated B subunit coding region derived from bacterial ADP-ribosylating exotoxin, Hp91, CCL20, CCL3, GM-CSF, G-CSF, LPS peptide mimic, shiga toxin, diphtheria toxin, IL-15 super agonist, ALT-803, CRM₁₉₇, or any combination thereof. In some aspects, the immunological fusion partner is at least 80%, at least 85%, at least 90%, at least 95%, or at least 90% identical to a sequence of any one of SEQ ID NO: 39 - SEQ ID NO: 90 and SEQ ID NO: 109 -SEQ ID NO: 112.

In further aspects, the replication-defective vector further comprises a nucleic acid sequence encoding for a linker. In some aspects, the linker is from 1 to about 150 nucleic acids long, from about 5 to about 100 nucleic acids long, or from about 10 to about 50 nucleic acids long. In other apsects, the nucleic acid sequence encodes an amino acid residue. In some aspects, the amino acid residues form an amino acid sequence. In some aspects, the amino acid sequence comprises 1 to about 50 amino acid residues, about 5 to about 25 amino acid residues, or less than 10 amino acid residues. In some aspects, the linker is a polyalanine linker, or a polyglycine linker. In some aspects, the linker comprises a mixture of alanines and glycines. In some aspects, the nucleic acid sequence encoding for the linker is between the nucleic acid sequence encoding for the tumor neo-antigen and the nucleic acid sequence encoding for the immunological fusion partner. In some aspects, the linker is any one of SEQ ID NO: 91 - SEQ ID NO: 105.

In further aspects, the replication-defective vector comprises more than one nucleic acid sequences encoding more than one tumor neo-antigens. In some aspects, the composition is a vaccine. In some aspects, the composition comprises a pharmaceutically acceptable carrier. In some aspects, the composition comprises at least ten adenovirus vectors. In additional aspects, the composition comprises at least ten adenovirus vectors.

In some aspects, the tumor neo-antigen comprises a tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, Her 4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPl/mbcr-abl, ETV6/AML, LDLR/FUT, Pml/RARα, TEL/AML1, or any combination thereof. In further aspects, the tumor neo-antigen comprises a tumor neo-epitope. In some aspects, the tumor neo-antigen comprises CEA, MUC1, Brachyury, PSA, PSMA, Her2/neu, Her3, HPV-E6, HPV-E7, or any combination thereof. In some aspects, the tumor neo-antigen is a tumor neo-epitope with an amino acid sequence of any one of SEQ ID NO: 1 - SEQ ID NO: 22, a nucleotide sequence of any one of SEQ ID NO: 23 - SEQ ID NO: 30, or has one of the following mutation: Q678P mutation of gene SLC4A11, D1143N mutation of gene SIGLEC1, A292T mutation of gene SIGLEC14, T2356M mutation of PIEZO2, S1613L mutation of gene FAT4, R268C mutation of gene FCRL1, or V73M mutation of gene VIPR2, or R346W mutation of gene FLRT2.

In some aspects, the replication-defective vector further comprises a nucleic acid sequence encoding a costimulatory molecule. In some aspects, the costimulatory molecule comprises B7, ICAM-1, LFA-3, or any combinations thereof. In some aspects, the composition additionally comprises an engineered natural killer (NK) cell. In some aspects, the engineered NK cell comprises an NK cell that has been modified as essentially lacking the expression of KIR (killer inhibitory receptors), an NK cell that has been modified to express a high affinity CD16 variant, and an NK cell that has been modified to express a CAR (chimeric antigen receptor), or any combinations thereof. In some aspects, the engineered NK cells comprise an NK cell that has been modified as essentially lacking the expression KIR. In some aspects, the engineered NK cells comprise an NK cell that has been modified to express a high affinity CD16 variant. In further aspects, the engineered NK cells comprise an NK cell that has been modified to express a CAR. In some aspects, the CAR is a CAR for a tumor neo-antigen, tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, HER4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPl/mbcr-abl, ETV6/AML, LDLR/FUT, Pml/RARα, or TEL/AML1.

In some aspects, the composition additionally comprises an immunostimulant. In further aspects, the immunostimulant is selected from the group consisting of granulocyte macrophage colony-stimulating factor (GM-CSF), granulocyte-colony stimulating factor (G-CSF), interferon-gamma (IFN-γ), tumor necrosis factor-alpha (TNF-a), interleukin-2 (IL-2), IL-7, IL-4, IL-5, IL-6, IL-10, IL-12, IL-15, IL-16, IL-17, IL-23, and IL-32. In some aspects, the composition additionally comprises a therapeutically effective amount of IL-15 or a replication defective vector comprising a nucleic acid sequence encoding IL-15. In some aspects, the composition additionally comprises a cancer therapy.

In some aspects, the cancer therapy is a dose of chemotherapy, a dose of radiation, an immunotherapy, or any combination thereof. In some aspects, the immunotherapy comprises a therapeutically effective amount of a composition comprising a replication-defective vector comprising a nucleic acid sequence encoding a tumor-associated antigen or a tumor neo-antigen. In some aspects, the combination of the dose of chemotherapy and the dose of radiation is present in the composition at a dose lower than a recommended dose if the dose of chemotherapy and the dose of radiation were present alone. In some aspects, the cancer therapy is an anti-PD-1 antibody pembrolizumab.

In further aspects, the composition additionally comprises a therapeutically effective amount of an immune pathway checkpoint inhibitor. In some aspects, the immune pathway checkpoint inhibitor comprises a therapeutically effective of a composition comprising an antibody that binds to an immune pathway checkpoint ligand. In some aspects, the immune pathway checkpoint inhibitor is an antibody that binds PD-1, PD-L1, CTLA-4, LAG-3, or IDO.

In some aspects, the replication-defective vector further comprises a nucleic acid sequence encoding a reporter. In some aspects, the replication-defective vector comprises a nucleic acid sequence encoding a tumor neo-antigen attached to a nucleic acid sequence encoding a reporter. In further aspects, the replication-defective vector is present at a dose that is greater than or equal to 1x10⁹, 2 x10⁹, 3 x10⁹, 4 x10⁹, 5 x10⁹, 6 x10⁹, 7 x10⁹, 8 x10⁹, 9 x10⁹, 1x10¹⁰, 2 x10¹⁰, 3 x10¹⁰, 4 x10¹⁰, 5 x10¹⁰, 6 x10¹⁰, 7 x10¹⁰, 8 x10¹⁰, 9 x10¹⁰, 1 x10¹¹, 2 x10¹¹, 3 x10¹¹, 4 x10¹¹, 5x10¹¹, 6 x10¹¹, 7 x10¹¹, 8 x10¹¹, 9 x10¹¹, 1x10¹², 1.5 x10¹², 2 x10¹², 3 x10¹², or more virus particles (VPs). In some aspects, the replication-defective vector is present at a dose that is less than or equal to 1x10⁹, 2 x10⁹, 3 x10⁹, 4 x10⁹, 5 x10⁹, 6 x10⁹, 7 x10⁹, 8 x10⁹, 9 x10⁹, 1x10¹⁰, 2 x10¹⁰, 3 x10¹⁰, 4 x10¹⁰, 5 x10¹⁰, 6 x10¹⁰, 7 x10¹⁰, 8 x10¹⁰, 9 x10¹⁰, 1 x10¹¹, 2 x10¹¹, 3 x10¹¹, 4 x10¹¹, 5x10¹¹, 6 x10¹¹, 7 x10¹¹, 8 x10¹¹, 9 x10¹¹, 1x10¹², 1.5 x10¹², 2 x10¹², 3 x10¹², 4 x 10¹², 5 x 10¹², or more virus particles per immunization.

In some aspects, the tumor antigen is specific for a subject.

In some aspects, the tumor neo-antigen is encoded by a nucleotide sequence comprising a tumor-specific single nucleotide variant (SNV). In some aspects, the tumor neo-antigen drives cell-mediated immune response or is determined to drive cell-mediated immune response. In some aspects, the tumor neo-antigen is a peptide of having a size of six to ten amino acids. In some aspects, CEA comprises a sequence that has at least 80%, at least 85%, at least 90%, at least 92%, at least 95%, or at least 99% sequence identity to SEQ ID NO: 106. In some aspects, MUC1-c comprises a sequence that has at least 80%, at least 85%, at least 90%, at least 92%, at least 95%, or at least 99% sequence identity to SEQ ID NO: 107. In some aspects, Brachyury comprises a sequence that has at least 80%, at least 85%, at least 90%, at least 92%, at least 95%, or at least 99% sequence identity to SEQ ID NO: 108.

In various aspects, a composition comprising a cell comprises the composition as described herein. In some aspects, the cell is a dendritic cell (DC).

In various aspects, a method of treating a cancer in a subject in need thereof comprises administering to the subject the composition as described herein.

In various aspects, a method of treating a cancer in a subject in need thereof comprises administering to the subject a pharmaceutical composition, the pharmaceutical composition comprising a replication-defective vector, wherein the replication-defective vector comprises a nucleic acid sequence encoding for a tumor neo-antigen; anda nucleic acid sequence encoding for an immunological fusion partner.

In various aspects, a method of monitoring the status of a subject comprises a) administering to a subject a pharmaceutical composition, the pharmaceutical composition comprising a replication-defective vector, wherein the replication-defective vector comprises a nucleic acid sequence encoding for a tumor neo-antigen of the subject and a nucleic acid sequence encoding for an immunological fusion partner; and b) monitoring the status of the tumor neo-antigen in the subject.

In various aspects, a method of detecting a tumor in a subject comprises: a) administering to the subject a pharmaceutical composition, the pharmaceutical composition comprising a replication-defective vector, wherein the replication-defective vector comprisesa nucleic acid sequence encoding for a tumor neo-antigens and a nucleic acid sequence encoding for an immunological fusion partner; and b) detecting the presence or absence of the tumor neo-antigen in the subject following the administering.

In various aspects, a method of treating a cancer in a subject in need thereof comprises: a) administering to the subject a cancer therapy; b) detecting the presence of a tumor neo-antigen in the subject; c) administering to the subject a pharmaceutical composition, the pharmaceutical composition comprising a replication-defective vector, wherein the replication-defective vector comprises a nucleic acid sequence encoding for a tumor neo-antigen and a nucleic acid sequence encoding for an immunological fusion partner; and d) repeating steps b)-c).

In various aspects, a method of treating a cancer in a subject in need thereof comprises administering to the subject a pharmaceutical composition, the pharmaceutical comprising a population of cells, wherein a cell in the population of cells comprises a replication-defective vector, and wherein the replication-defective vector comprises a nucleic acid sequence encoding for a tumor neo-antigen and a nucleic acid sequence encoding for an immunological fusion partner.

In various aspects, a method of treating a cancer in a subject in need thereof comprises administering to the subject a pharmaceutical composition, the pharmaceutical composition comprising a replication-defective vector, wherein the replication-defective vector comprises a nucleic acid sequence encoding for CEA, MUC1-c, Brachyury, or any combination thereof; and a nucleic acid sequence encoding for an immunological fusion partner.

In various aspects, a method of monitoring the status of a subject comprises: a) administering to a subject a pharmaceutical composition, the pharmaceutical composition comprising a replication-defective vector, wherein the replication-defective vector comprises a nucleic acid sequence encoding for CEA, MUC1-c, Brachyury, or any combination thereof, and a nucleic acid sequence encoding for an immunological fusion partner; and b) monitoring the status of the CEA, MUC1-c, Brachyury, or any combination thereof in the subject.

In various aspects, a method of detecting a tumor in a subject comprises: a) administering to the subject a pharmaceutical composition, the pharmaceutical composition comprising a replication-defective vector, wherein the replication-defective vector comprises a nucleic acid sequence encoding for CEA, MUC1-c, Brachyury, or any combination thereof, and a nucleic acid sequence encoding for an immunological fusion partner; and b) detecting the presence or absence of the CEA, MUC1-c, Brachyury, or any combination thereof in the subject following the administering.

In various aspects, a method of treating a cancer in a subject in need thereof comprises: a) administering to the subject a cancer therapy; b) detecting the presence of CEA, MUC1-c, Brachyury, or any combination thereof in the subject; c) administering to the subject a pharmaceutical composition, the pharmaceutical composition comprising a replication-defective vector, wherein the replication-defective vector comprises a nucleic acid sequence encoding for CEA, MUC1-c, Brachyury, or any combination thereof, and a nucleic acid sequence encoding for an immunological fusion partner; and d) repeating steps b)-c).

In various aspects, a method of treating a cancer in a subject in need thereof comprises administering to the subject a pharmaceutical composition, the pharmaceutical comprising a population of cells, wherein a cell in the population of cells comprises a replication-defective vector, and wherein the replication-defective vector comprises a nucleic acid sequence encoding for CEA, MUC1-c, Brachyury, or any combination thereof, and a nucleic acid sequence encoding for an immunological fusion partner.

In some aspects, the replication-defective vector is an adenovirus vector. In some aspects, the adenovirus vector is an Ad5 vector. In some aspects, the replication defective vector comprises a deletion in an E2b region, an E1 region, an E3 region, and E4 region, or any combination thereof. In some aspects, the replication defective vector comprises a deletion in an E2b region. In some aspects, the replication-defective vector comprises a deletion of a DNA polymerase, preterminal protein (pTP), or a combination thereof in an E2b region. In some aspects, the replication-defective vector is not a gutted vector.

In further aspects, the replication-defective vector comprises a plurality of replication-defective vectors, wherein each replication-defective vector comprises a different tumor neo-antigen-coding sequence. In some aspects, the replication-defective vector comprises at least ten replication-defective vectors, wherein each replication-defective vector comprises a different tumor neo-antigen-coding sequence. In some aspects, the replication-defective vector comprises at least five replication-defective vectors, wherein each replication-defective vector comprises a different tumor neo-antigen-coding sequence.

In some aspects, the immunological fusion partner comprises *Mycobacterium sp., Mycobacterium tuberculosis-derived* Ra12 fragment, protein D derived from a surface protein of gram-negative bacterium *Haemophilus influenzae* B, LYTA, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, CpG-ODN, truncated A subunit coding region derived from bacterial ADP-ribosylating exotoxin, truncated B subunit coding region derived from bacterial ADP-ribosylating exotoxin, Hp91, CCL20, CCL3, GM-CSF, G-CSF, LPS peptide mimic, shiga toxin, diphtheria toxin, IL-15 super agonist, ALT-803, CRM₁₉₇, or any combination thereof. In some aspects, the immunological fusion partner can be a fragment or derivative of *Mycobacterium sp., Mycobacterium tuberculosis-derived* Ra12 fragment, protein D derived from a surface protein of gram-negative bacterium *Haemophilus influenzae* B, LYTA, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, CpG-ODN, truncated A subunit coding region derived from bacterial ADP-ribosylating exotoxin, truncated B subunit coding region derived from bacterial ADP-ribosylating exotoxin, Hp91, CCL20, CCL3, GM-CSF, G-CSF, LPS peptide mimic, shiga toxin, diphtheria toxin, IL-15 super agonist, ALT-803, CRM₁₉₇, or any combination thereof. In some aspects, the immunological fusion partner is at least 80%, at least 85%, at least 90%, at least 95%, or at least 90% identical to a sequence of any one of SEQ ID NO: 39 - SEQ ID NO: 90 and SEQ ID NO: 109 - SEQ ID NO: 112.

In some aspects, the replication-defective vector further comprises a nucleic acid sequence encoding for a linker. In some aspects, the linker is from 1 to about 150 nucleic acids long, from about 5 to about 100 nucleic acids long, or from about 10 to about 50 nucleic acids long.

In some aspects, the nucleic acid sequence encodes an amino acid residue. In some aspects, the amino acid residues form an amino acid sequence. In some aspects, the amino acid sequence comprises 1 to about 50 amino acid residues, about 5 to about 25 amino acid residues, or less than 10 amino acid residues. In some aspects, the linker is a polyalanine linker, a polyglycine linker. In some aspects, the linker comprises a mixture of alanines and glycines. In some aspects, the nucleic acid sequence encoding for the linker is between the nucleic acid sequence encoding for the tumor neo-antigen and the nucleic acid sequence encoding for the immunological fusion partner. In some aspects, the linker is any one of SEQ ID NO: 91 - SEQ ID NO: 105.

In some aspects, the replication-defective vector comprises more than one nucleic acid sequences encoding a tumor neo-antigen.

In some aspects, the pharmaceutical composition is a vaccine. In some aspects, the pharmaceutical composition comprises a pharmaceutically acceptable carrier. In some aspects, the pharmaceutical composition comprises at least ten adenovirus vectors.

The method of any one of claims 66-100, wherein the pharmaceutical composition comprises at least five adenovirus vectors.

In some aspects, the tumor neo-antigen comprises a tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, Her 4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPl/mbcr-abl, ETV6/AML, LDLR/FUT, Pml/RARα, TEL/AML1, or any combination thereof.

In some aspects, the tumor antigen comprises a tumor neo-epitope. In some aspects, the tumor neo-antigen comprises CEA, MUC1, Brachyury, PSA, PSMA, Her2/neu, Her3, HPV-E6, HPV-E7, or any combination thereof. In some aspects, the tumor neo-antigen is a tumor neo-epitope with an amino acid sequence of any one of SEQ ID NO: 1 - SEQ ID NO: 22, a nucleotide sequence of any one of SEQ ID NO: 23 - SEQ ID NO: 30, or has one of the following mutation: Q678P mutation of gene SLC4A11, D1143N mutation of gene SIGLEC1, A292T mutation of gene SIGLEC14, T2356M mutation of PIEZO2, S1613L mutation of gene FAT4, R268C mutation of gene FCRL1, or V73M mutation of gene VIPR2, or R346W mutation of gene FLRT2.

In some aspects, the replication-defective vector further comprises a nucleic acid sequence encoding a costimulatory molecule. In some aspects, the costimulatory molecule comprises B7, ICAM-1, LFA-3, or any combinations thereof.

In some aspects, the method further comprises administering to the subject an additional pharmaceutical composition comprising an engineered natural killer (NK) cell. In some aspects, the engineered NK cell comprises an NK cell that has been modified as essentially lacking the expression of KIR (killer inhibitory receptors), an NK cell that has been modified to express a high affinity CD16 variant, and an NK cell that has been modified to express a CAR (chimeric antigen receptor), or any combinations thereof. In some aspects, the engineered NK cells comprise an NK cell that has been modified as essentially lacking the expression KIR. In some aspects, the engineered NK cells comprise an NK cell that has been modified to express a high affinity CD16 variant. In some aspects, the engineered NK cells comprise an NK cell that has been modified to express a CAR. In further aspects, the CAR is a CAR for a tumor neo-antigen, tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, HER4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPl/mbcr-abl, ETV6/AML, LDLR/FUT, Pml/RARα, or TEL/AML1.

In other aspects, the method further comprises administering to the subject an additional pharmaceutical composition comprising an immunostimulant. In some aspects, the immunostimulant is selected from the group consisting of granulocyte macrophage colony-stimulating factor (GM-CSF), granulocyte-colony stimulating factor (G-CSF), interferon-gamma (IFN-γ), tumor necrosis factor-alpha (TNF-a), interleukin-2 (IL-2), IL-7, IL-4, IL-5, IL-6, IL-10, IL-12, IL-15, IL-16, IL-17, IL-23, IL-32.

In some aspects, the method further compres administering to the subject an additional pharmaceutical composition comprising a therapeutically effective amount of IL-15 or a replication defective vector comprising a nucleic acid sequence encoding IL-15.

In some aspects, the subject has been administered a cancer therapy before the subject has been determined to have the tumor neo-antigen. In some aspects, the cancer therapy is chemotherapy, radiation treatment, an immunotherapy, or any combination thereof. In some aspects, the immunotherapy comprises a therapeutically effective amount of a composition comprising a replication-defective vector comprising a nucleic acid sequence encoding a tumor-associated antigen or a tumor neo-antigen. In some aspects, the combination of chemotherapy and radiation is administered at a dose lower than a recommended dose if the chemotherapy and radiation were administered alone. In some aspects, the cancer therapy is an anti-PD-1 antibody pembrolizumab.

In some aspects, the method further comprises administering to the subject an additional pharmaceutical composition comprising a therapeutically effective amount of an immune pathway checkpoint inhibitor. In some aspects, the immune pathway checkpoint inhibitor comprises a therapeutically effective of a composition comprising an antibody that binds to an immune pathway checkpoint ligand. In some aspects, the immune pathway checkpoint inhibitor is an antibody that binds PD-1, PD-L1, CTLA-4, LAG-3, or IDO.

In some aspects, the replication-defective vector comprises a nucleic acid sequence encoding a reporter. In some aspects, the replication-defective vector comprises a nucleic acid sequence encoding a tumor neo-antigen attached to a nucleic acid sequence encoding a reporter. In some aspects, the replication-defective vector is present at a dose that is greater than or equal to 1x10⁹, 2 x10⁹, 3 x10⁹, 4 x10⁹, 5 x10⁹, 6 x10⁹, 7 x10⁹, 8 x10⁹, 9 x10⁹, 1x10¹⁰, 2 x10¹⁰, 3 x10¹⁰, 4 x10¹⁰, 5 x10¹⁰, 6 x10¹⁰, 7 x10¹⁰, 8 x10¹⁰, 9 x10¹⁰, 1 x10¹¹, 2 x10¹¹, 3 x10¹¹, 4 x10¹¹, 5x10¹¹, 6 x10¹¹, 7 x10¹¹, 8 x10¹¹, 9 x10¹¹, 1x10¹², 1.5 x10¹², 2 x10¹², 3 x10¹², or more virus particles (VPs). In some aspects, the replication-defective vector is present at a dose that is less than or equal to 1x10⁹, 2 x10⁹, 3 x10⁹, 4 x10⁹, 5 x10⁹, 6 x10⁹, 7 x10⁹, 8 x10⁹, 9 x10⁹, 1x10¹⁰, 2 x10¹⁰, 3 x10¹⁰, 4 x10¹⁰, 5 x10¹⁰, 6 x10¹⁰, 7 x10¹⁰, 8 x10¹⁰, 9 x10¹⁰, 1 x10¹¹, 2 x10¹¹, 3 x10¹¹, 4 x10¹¹, 5x10¹¹, 6 x10¹¹, 7 x10¹¹, 8 x10¹¹, 9 x10¹¹, 1x10¹², 1.5 x10¹², 2 x10¹², 3 x10¹², 4 x 10¹², 5 x 10¹², or more virus particles per immunization.

In some aspects, the tumor neo-antigen is specific for a subject. In some aspects, the tumor neo-antigen is encoded by a nucleotide sequence comprising a tumor-specific single nucleotide variant (SNV). In some aspects, the tumor neo-antigen drives cell-mediated immune response or is determined to drive cell-mediated immune response. In some aspects, the tumor neo-antigen is a peptide of having a size of six to ten amino acids. In some aspects, the subject has the tumor neo-antigen before the administering.

In some aspects, the method further comprises determining whether the subject develops a new neo-antigen during or after administration. In some aspects, the tumor neo-antigen has been identified as a result of comparing a genomic profile of a tumor sample of the subject to a reference.

In some aspects, the method further comprises obtaining a genomic profile of a tumor sample of the subject.

In some aspects, the method further comprises comparing a genomic profile of a tumor sample of the subject to a reference to identify tumor-specific mutations. In some aspects, obtaining a genomic profile comprises next-generation sequencing, whole-exosome sequencing, sequencing by synthesis, sequencing by litigation, single-molecule sequencing, nano-technology for single-molecule sequencing, ion semiconductor sequencing or a combination thereof.

In some aspects, the method further comprises identifying tumor neo-antigens from tumor-specific mutations. In some aspects, identifying tumor neo-antigens comprises the use of proteomics.

In some aspects, the method further comprises identifying tumor neo-epitopes.

In some aspects, the method further comprises accessing a database to obtain a previously stored genomic profile of the subject or a reference.

In some aspects, the method further comprises identifying additional tumor neo-antigens in the subject after administering the pharmaceutical composition.

In some aspects, the method further comprises identifying an additional tumor neo-antigen in the subject after administering the pharmaceutical composition, administering an additional pharmaceutical composition, the additional pharmaceutical composition comprising an additional replication-defective vector comprising an additional tumor neo-antigen to the subject.

In some aspects, the method further comprises monitoring the status of tumor neo-antigens in the subject that has been administered the pharmaceutical composition.

In some aspects, the cancer is a pancreatic cancer, colorectal cancer, breast cancer, breast cancer, lung cancer, prostate cancer, gastric cancer, liver cancer, ovarian cancer, cervical cancer, head and neck squamous cell carcinoma or any combinations thereof.

In some aspects, the tumor neo-antigen is more than one tumor neo-antigen present in the subject.

In some aspects, the pharmaceutical composition and the additional pharmaceutical composition are administered at the same time.

In some aspects, the cell is a dendritic cell (DC).

In some aspects, CEA comprises a sequence that has at least 80%, at least 85%, at least 90%, at least 92%, at least 95%, or at least 99% sequence identity to SEQ ID NO: 106. In some aspects, MUC1-c comprises a sequence that has at least 80%, at least 85%, at least 90%, at least 92%, at least 95%, or at least 99% sequence identity to SEQ ID NO: 107. In some aspects, Brachyury comprises a sequence that has at least 80%, at least 85%, at least 90%, at least 92%, at least 95%, or at least 99% sequence identity to SEQ ID NO: 108.

In various aspects, the method of treating a cancer in a subject in need thereof comprises: administering to the subject a first pharmaceutical composition, the pharmaceutical composition comprising a first replication-defective vector, wherein the first replication-defective vector comprises a nucleic acid sequence encoding for CEA, MUC1-c, Brachyury, or any combination thereof; or any composition described herein; and administering to the subject a second pharmaceutical composition, the second pharmaceutical composition comprising a second replication-defective vector, wherein the replication-defective vector comprises a nucleic acid sequence encoding any composition decribed herein.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.
**FIG. 1****.** Non-limiting exemplary embodiment illustrating identification of tumor neo-epitopes

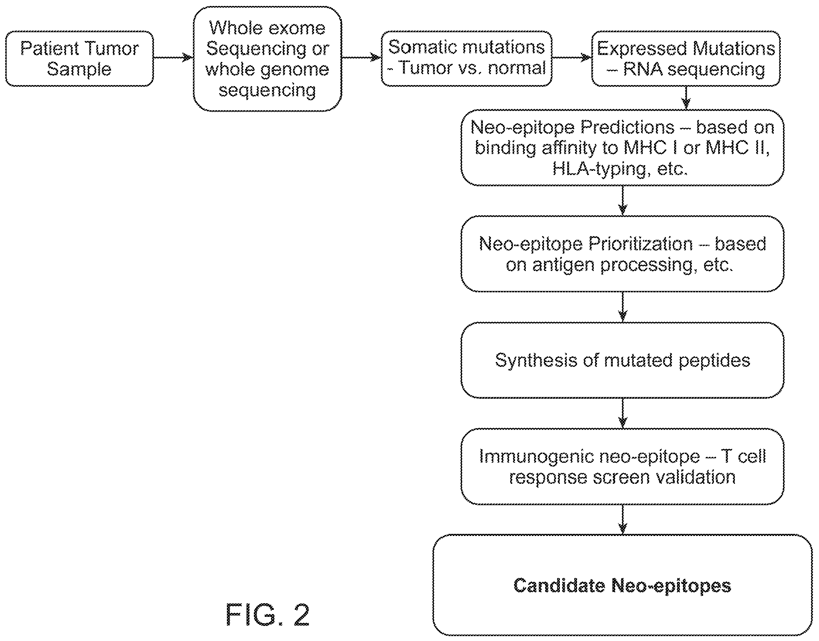

**FIG. 2****.** Non-limiting exemplary embodiment illustrating cancer exome-based identification of tumor neo-epitopes. Tumor material is analyzed for nonsynonymous somatic mutations. RNA sequencing data are used to focus on mutations in expressed genes. Peptide stretches containing any of the identified nonsynonymous mutations are generated *in silico* and are filtered through the use of prediction algorithms or used to identify MHC-associated neo-epitopes in mass spectrometry data. Modeling of the effect of mutations on the resulting peptide-MHC complex is used as an additional filter. Resulting epitope sets are used to identify physiologically occurring neo-epitope-specific T cell responses by MHC multimer-based screens and functional assays within both CD8 and CD4 T cell populations.
**FIG. 3****.** Four exemplary gene constructs designed for insertion of tumor neo-epitopes into Ad5 [E1-, E2b-] platform.
**FIG. 4****.** A549 cells transfected with Ad5[E1-,E2b-]-UCLA-gene 1-hTRICOM: 48 hr transfection and expression of Tricom placed at terminus as reporter genes to verify expression of gene 1 placed before reporter elements.

### DETAILED DESCRIPTION

While the making and using of various embodiments of the present invention are discussed in detail below, it should be appreciated that the present invention provides many applicable inventive concepts that can be embodied in a wide variety of specific contexts. The specific embodiments discussed herein are merely illustrative of specific ways to make and use the invention and do not delimit the scope of the invention.

To facilitate the understanding of certain aspects, a number of terms are defined below. Terms defined herein have meanings as commonly understood by a person of ordinary skill in the areas relevant to the present invention.

Terms such as "a," "an" and "the" are not intended to refer to only a singular entity, but include the general class of which a specific example may be used for illustration. The terminology herein is used to describe specific embodiments of the invention, but their usage does not delimit the invention, except as outlined in the claims.

By "individual," "subject" or "patient" is meant any single subject for which therapy is desired, including but not limited to humans, non-human primates, rodents, dogs, or pigs. Also intended to be included as a subject are any subjects involved in clinical research trials not showing any clinical sign of disease, or subjects involved in epidemiological studies, or subjects used as controls.

As used herein, the term "gene" refers to a functional protein, polypeptide or peptide-encoding unit. As will be understood by those in the art, this functional term includes both genomic sequences, cDNA sequences, or fragments or combinations thereof, as well as gene products, including those that may have been altered by the hand of man. Purified genes, nucleic acids, protein and the like are used to refer to these entities when identified and separated from at least one contaminating nucleic acid or protein with which it is ordinarily associated. The term "allele" or "allelic form" refers to an alternative version of a gene encoding the same functional protein but containing differences in nucleotide sequence relative to another version of the same gene.

As used herein, "nucleic acid" or "nucleic acid molecule" refers to polynucleotides, such as deoxyribonucleic acid (DNA) or ribonucleic acid (RNA), oligonucleotides, fragments generated by the polymerase chain reaction (PCR), and fragments generated by any of ligation, scission, endonuclease action, and exonuclease action. Nucleic acid molecules can be composed of monomers that are naturally-occurring nucleotides (such as DNA and RNA), or analogs of naturally-occurring nucleotides (e.g., α-enantiomeric forms of naturally-occurring nucleotides), or a combination of both. Modified nucleotides can have alterations in sugar moieties and/or in pyrimidine or purine base moieties. Sugar modifications include, for example, replacement of one or more hydroxyl groups with halogens, alkyl groups, amines, and azido groups, or sugars can be functionalized as ethers or esters. Moreover, the entire sugar moiety can be replaced with sterically and electronically similar structures, such as aza-sugars and carbocyclic sugar analogs. Examples of modifications in a base moiety include alkylated purines and pyrimidines, acylated purines or pyrimidines, or other well-known heterocyclic substitutes. Nucleic acid monomers can be linked by phosphodiester bonds or analogs of such linkages. Analogs of phosphodiester linkages include phosphorothioate, phosphorodithioate, phosphoroselenoate, phosphorodiselenoate, phosphoroanilothioate, phosphoranilidate, phosphoramidate, and the like. The term "nucleic acid molecule" also includes so-called "peptide nucleic acids," which comprise naturally-occurring or modified nucleic acid bases attached to a polyamide backbone. Nucleic acids can be either single stranded or double stranded.

As used herein, unless otherwise indicated, the article "a" means one or more unless explicitly otherwise provided for.

As used herein, unless otherwise indicated, terms such as "contain," "containing," "include," "including," and the like mean "comprising."

As used herein, unless otherwise indicated, the term "or" can be conjunctive or disjunctive.

As used herein, unless otherwise indicated, any embodiment can be combined with any other embodiment.

As used herein, unless otherwise indicated, some inventive embodiments herein contemplate numerical ranges. A variety of aspects can be presented in a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range as if explicitly written out. For example, description of a range such as from 1 to 6 should be considered to have specifically disclosed subranges such as from 1 to 3, from 1 to 4, from 1 to 5, from 2 to 4, from 2 to 6, from 3 to 6 etc., as well as individual numbers within that range, for example, 1, 2, 3, 4, 5, and 6. This applies regardless of the breadth of the range. When ranges are present, the ranges include the range endpoints.

The term "adenovirus" or "Ad" refers to a group of non-enveloped DNA viruses from the family Adenoviridae. In addition to human hosts, these viruses can be found in, but are not limited to, avian, bovine, porcine and canine species. Certain aspects may contemplate the use of any adenovirus from any of the four genera of the family Adenoviridae (e.g., Aviadenovirus, Mastadenovirus, Atadenovirus and Siadenovirus) as the basis of an E2b deleted virus vector, or vector containing other deletions as described herein. In addition, several serotypes are found in each species. Ad also pertains to genetic derivatives of any of these viral serotypes, including but not limited to, genetic mutation, deletion or transposition of homologous or heterologous DNA sequences.

A "helper adenovirus" or "helper virus" refers to an Ad that can supply viral functions that a particular host cell cannot (the host may provide Ad gene products such as E1 proteins). This virus is used to supply, in trans, functions (e.g., proteins) that are lacking in a second virus, or helper dependent virus (e.g., a gutted or gutless virus, or a virus deleted for a particular region such as E2b or other region as described herein); the first replication-incompetent virus is said to "help" the second, helper dependent virus thereby permitting the production of the second viral genome in a cell.

The term "Adenovirus5 null (Ad5null)," as used herein, refers to a non-replicating Ad that does not contain any heterologous nucleic acid sequences for expression.

The term "First Generation adenovirus," as used herein, refers to an Ad that has the early region 1 (E1) deleted. In additional cases, the nonessential early region 3 (E3) may also be deleted.

The term "gutted" or "gutless," as used herein, refers to an adenovirus vector that has been deleted of all viral coding regions.

The term "transfection" as used herein refers to the introduction of foreign nucleic acid into eukaryotic cells. Transfection may be accomplished by a variety of means known to the art including calcium phosphate-DNA co-precipitation, DEAE-dextran-mediated transfection, polybrene-mediated transfection, electroporation, microinjection, liposome fusion, lipofection, protoplast fusion, retroviral infection, and biolistics.

The term "stable transfection" or "stably transfected" refers to the introduction and integration of foreign nucleic acid, DNA or RNA, into the genome of the transfected cell. The term "stable transfectant" refers to a cell which has stably integrated foreign DNA into the genomic DNA.

The term "reporter gene" indicates a nucleotide sequence that encodes a reporter molecule (including an enzyme). A "reporter molecule" is detectable in any of a variety of detection systems, including, but not limited to enzyme-based detection assays (e.g., ELISA, as well as enzyme-based histochemical assays), fluorescent, radioactive, and luminescent systems.

In one embodiment, there may be provided the E. coli β-galactosidase gene (available from Pharmacia Biotech, Pistacataway, N.J.), green fluorescent protein (GFP) (commercially available from Clontech, Palo Alto, Calif.), the human placental alkaline phosphatase gene, the chloramphenicol acetyltransferase (CAT) gene as reporter genes; other reporter genes are known to the art and may be employed.

As used herein, the terms "nucleic acid molecule encoding," "DNA sequence encoding," and "DNA encoding" refer to the order or sequence of deoxyribonucleotides along a strand of deoxyribonucleic acid. The order of these deoxyribonucleotides determines the order of amino acids along the polypeptide (protein) chain. The nucleic acid sequence thus codes for the amino acid sequence.

The term "heterologous nucleic acid sequence," as used herein, refers to a nucleotide sequence that is ligated to, or is manipulated to become ligated to, a nucleic acid sequence to which it is not ligated in nature, or to which it is ligated at a different location in nature. Heterologous nucleic acid may include a nucleotide sequence that is naturally found in the cell into which it is introduced or the heterologous nucleic acid may contain some modification relative to the naturally occurring sequence.

The term "transgene" refers to any gene coding region, either natural or heterologous nucleic acid sequences or fused homologous or heterologous nucleic acid sequences, introduced into the cells or genome of a test subject. In certain aspects, transgenes are carried on any viral vector that is used to introduce the transgenes to the cells of the subject.

The term "Second Generation Adenovirus," as used herein, refers to an Ad that has all or parts of the E1, E2, E3, and, in certain embodiments, E4 DNA gene sequences deleted (removed) from the virus.

As used herein, the term "fragment or segment," as applied to a nucleic acid sequence, gene or polypeptide, will ordinarily be at least about 5 contiguous nucleic acid bases (for nucleic acid sequence or gene) or amino acids (for polypeptides), typically at least about 10 contiguous nucleic acid bases or amino acids, more typically at least about 20 contiguous nucleic acid bases or amino acids, usually at least about 30 contiguous nucleic acid bases or amino acids, preferably at least about 40 contiguous nucleic acid bases or amino acids, more preferably at least about 50 contiguous nucleic acid bases or amino acids, and even more preferably at least about 60 to 80 or more contiguous nucleic acid bases or amino acids in length. "Overlapping fragments" as used herein, refer to contiguous nucleic acid or peptide fragments which begin at the amino terminal end of a nucleic acid or protein and end at the carboxy terminal end of the nucleic acid or protein. Each nucleic acid or peptide fragment has at least about one contiguous nucleic acid or amino acid position in common with the next nucleic acid or peptide fragment, more preferably at least about three contiguous nucleic acid bases or amino acid positions in common, most preferably at least about ten contiguous nucleic acid bases amino acid positions in common.

A significant "fragment" in a nucleic acid context is a contiguous segment of at least about 17 nucleotides, generally at least 20 nucleotides, more generally at least 23 nucleotides, ordinarily at least 26 nucleotides, more ordinarily at least 29 nucleotides, often at least 32 nucleotides, more often at least 35 nucleotides, typically at least 38 nucleotides, more typically at least 41 nucleotides, usually at least 44 nucleotides, more usually at least 47 nucleotides, preferably at least 50 nucleotides, more preferably at least 53 nucleotides, and in particularly preferred embodiments will be at least 56 or more nucleotides.

A "vector" is a composition, which can transduce, transfect, transform or infect a cell, thereby causing the cell to express nucleic acids and/or proteins other than those native to the cell, or in a manner not native to the cell. A cell is "transduced" by a nucleic acid when the nucleic acid is translocated into the cell from the extracellular environment. Any method of transferring a nucleic acid into the cell may be used; the term, unless otherwise indicated, does not imply any particular method of delivering a nucleic acid into a cell. A cell is "transformed" by a nucleic acid when the nucleic acid is transduced into the cell and stably replicated. A vector includes a nucleic acid (ordinarily RNA or DNA) to be expressed by the cell. A vector optionally includes materials to aid in achieving entry of the nucleic acid into the cell, such as a viral particle, liposome, protein coating or the like. A "cell transduction vector" is a vector which encodes a nucleic acid capable of stable replication and expression in a cell once the nucleic acid is transduced into the cell.

The term "variant," when used in the context of a polynucleotide sequence, may encompass a polynucleotide sequence related to a wild type gene. This definition may also include, for example, "allelic," "splice," "species," or "polymorphic" variants. A splice variant may have significant identity to a reference molecule, but will generally have a greater or lesser number of polynucleotides due to alternate splicing of exons during mRNA processing. The corresponding polypeptide may possess additional functional domains or an absence of domains. Species variants are polynucleotide sequences that vary from one species to another. Of particular utility in the invention are variants of wild type target genes. Variants may result from at least one mutation in the nucleic acid sequence and may result in altered mRNAs or in polypeptides whose structure or function may or may not be altered. Any given natural or recombinant gene may have none, one, or many allelic forms. Common mutational changes that give rise to variants are generally ascribed to natural deletions, additions, or substitutions of nucleotides. Each of these types of changes may occur alone, or in combination with the others, one or more times in a given sequence.

As used herein, "variant" of polypeptides refers to an amino acid sequence that is altered by one or more amino acid residues. The variant may have "conservative" changes, wherein a substituted amino acid has similar structural or chemical properties (e.g., replacement of leucine with isoleucine). More rarely, a variant may have "nonconservative" changes (e.g., replacement of glycine with tryptophan). Analogous minor variations may also include amino acid deletions or insertions, or both. Guidance in determining which amino acid residues may be substituted, inserted, or deleted without abolishing biological activity may be found using computer programs well known in the art, for example, LASERGENE software (DNASTAR).

The resulting polypeptides generally will have significant amino acid identity relative to each other. A polymorphic variant is a variation in the polynucleotide sequence of a particular gene between individuals of a given species. Polymorphic variants also may encompass "single nucleotide polymorphisms" (SNPs,) or single base mutations in which the polynucleotide sequence varies by one base.

An "antigen" is any substance that reacts specifically with antibodies or T lymphocytes (T cells). An "antigen-binding site" is the part of an immunoglobulin molecule that specifically binds an antigen. Additionally, an antigen-binding site includes any such site on any antigen-binding molecule, including, but not limited to, an MHC molecule or T cell receptor. "Antigen processing" refers to the degradation of an antigen into fragments (e.g., the degradation of a protein into peptides) and the association of one or more of these fragments (e.g., via binding) with MHC molecules for presentation by "antigen-presenting cells" to specific T cells.

"Dendritic cells" (DC) are potent antigen-presenting cells, capable of triggering a robust adaptive immune response in vivo. It has been shown that activated, mature DCs provide the signals required for T cell activation and proliferation. These signals can be categorized into two types. The first type, which gives specificity to the immune response, is mediated through interaction between the T-cell receptor/CD3 ("TCR/CD3") complex and an antigenic peptide presented by a major histocompatibility complex ("MHC" defined above) class I or II protein on the surface of APCs. The second type of signal, called a co-stimulatory signal, is neither antigen-specific nor MHC- restricted, and can lead to a full proliferation response of T cells and induction of T cell effector functions in the presence of the first type of signals. This two-fold signaling can, therefore, result in a vigorous immune response. As noted supra, in most non-avian vertebrates, DCs arise from bone marrow-derived precursors. Immature DCs are found in the peripheral blood and cord blood and in the thymus. Additional immature populations may be present elsewhere. DCs of various stages of maturity are also found in the spleen, lymph nodes, tonsils, and human intestine. Avian DCs may also be found in the bursa of Fabricius, a primary immune organ unique to avians. In a particular embodiment, the dendritic cells are mammalian, preferably human, mouse, or rat.

A "co-stimulatory molecule" encompasses any single molecule or combination of molecules which, when acting together with a peptide MHC complex bound by a T cell receptor on the surface of a T cell, provides a co-stimulatory effect which achieves activation of the T cell that binds the peptide.

"Diagnostic" or "diagnosed" means identifying the presence or nature of a pathologic condition. Diagnostic methods differ in their sensitivity and specificity. The "sensitivity" of a diagnostic assay is the percentage of diseased individuals who test positive (percent of "true positives"). Diseased individuals not detected by the assay are "false negatives." Subjects who are not diseased and who test negative in the assay, are termed "true negatives." The "specificity" of a diagnostic assay is 1 minus the false positive rate, where the "false positive" rate is defined as the proportion of those without the disease who test positive. While a particular diagnostic method may not provide a definitive diagnosis of a condition, it suffices if the method provides a positive indication that aids in diagnosis.

Throughout this application, the term "about" is used to indicate that a value includes the inherent variation of error for the device, the method being employed to determine the value, or the variation that exists among the study subjects.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. As used herein, the phrase "consisting essentially of' limits the scope of a claim to the specified materials or steps and those that do not materially affect the basic and novel characteristic(s) of the claimed invention. As used herein, the phrase "consisting of' excludes any element, step, or ingredient not specified in the claim except for, e.g., impurities ordinarily associated with the element or limitation.

The term "or combinations thereof' as used herein refers to all permutations and combinations of the listed items preceding the term. For example, "A, B, C, or combinations thereof' is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, MB, BBC, AAABCCCC, CBBAAA, CABABB, and so forth. A skilled artisan will understand that typically there is no limit on the number of items or terms in any combination, unless otherwise apparent from the context.

As used herein, words of approximation such as, without limitation, "about," "substantial" or "substantially" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by at least ±1, 2, 3, 4, 5, 6, 7, 10, 12 or 15%.

### I. Tumor Antigens

In certain aspects, methods may comprise administering a pharmaceutical composition comprising nucleic acid sequences encoding one or more tumor neo-antigens to the subject. In certain embodiments, the subject may have been determined to have the tumor neo-antigens before the administering. In other embodiments, methods may comprise administering a pharmaceutical composition comprising a nucleic acid sequence encoding for a tumor neo-antigen and a nucleic acid sequence encoding for an immunological fusion partner. In specific embodiments, replication-defective adenovirus vectors and/or partially E2b-deleted adenovirus vectors, may be used to administer tumor neo-antigens to the subject.

### 1. The Immune System

The immune system recognizes tumor cells based on the basic mechanisms of antigen recognition by T cells. T-cells mature in the thymus, where somatic rearrangement of the T-cell receptor (TCR) locus creates a unique TCR for each T cell. Also within the thymus, self-reactive T cells are deleted through a process called negative-selection or central tolerance. The result is a mature T-cell repertoire with limited reactivity to self but strong reactivity to foreign antigens. The TCR on T cells recognizes antigens as short peptides (called epitopes) bound to the major histocompatibility complex (MHC) on the target cell surface. Only a few peptides from each protein have favorable biochemical characteristics to allow them to be proteolytically cleaved from the parent protein and bound to MHC.

There are two types of MHC molecules encoded by human leukocyte antigen (HLA) genes: MHC class I (MHCI) and MHC class II (MHCII). Almost all nucleated cells express MHCI, which presents epitopes to 'killer' CD8+ T cells, also called cytotoxic T lymphocytes (CTLs). CTL can directly lyse cells that display cognate epitopes on MHCI, and this is thought to be the most important mechanism underlying antitumor immunity.

Professional antigen-presenting cells (APCs) express MHCII, which presents epitopes to CD4+ T-helper cells (Th). Th cells can have multiple antitumor functions such as directly killing tumor cells, augmenting CD8+ T-cell responses, and activating innate antitumor immune cells. Most commonly, T cells recognize antigens derived from pathogens; however, T cells can also recognize tumor antigens, if they are sufficiently different from self-proteins found in healthy tissue.

### 2. Tumor Antigens

Tumor antigens may include tumor-associated antigens (TAAs), cancer-germline/cancer testis antigens (CTAs) and tumor-specific antigens (TSAs).

TAAs include proteins encoded in the normal genome and may be either normal differentiation antigens or aberrantly expressed normal proteins. Overexpressed normal proteins that possess growth/survival-promoting functions, such as Wilms tumor 1 (WT1) or Her2/neu, represent TAAs that directly participate in the oncogenic process. Posttranslational modifications of proteins such as phosphorylation may also lead to formation of TAAs. Additional examples include human epidermal growth factor receptor 2 on breast and ovarian carcinomas, and mouse double minute 2 homolog in multiple cancers. In addition, proteins that are differentially expressed in the tissue of origin of the tumor can give rise to 'differentiation' antigens. For example, melanomas often express the differentiation antigens MART1, gp100, and tyrosinase.

Since TAAs such as differentiation and overexpressed antigens are normal proteins and are also present in healthy tissue, their antigenicity depends on abnormal expression levels or context to circumvent naturally occurring mechanisms of immunological tolerance. Along these lines, TAAs usually have lower T cell receptor (TCR) affinity compared with TSAs or foreign antigens as a result of thymic negative-selection. Moreover, targeting such antigens with immunotherapy brings the risk of autoimmune toxicity.

Another type of tumor antigens includes CTAs, which are normally expressed in testis, fetal ovaries, and trophoblasts, but can also be expressed in cancer cells. Because they are encoded in the normal genome but display highly restricted tissue expression, CTAs have received considerable attention as attractive targets for immunotherapy.

TSAs are antigens that are not encoded in the normal host genome and include oncogenic viral proteins and abnormal proteins that arise as a consequence of somatic mutations (including neo-antigens). Cancers of viral origin express virus-derived proteins that can be recognized by the immune system. For example, the E6 and E7 proteins from human papillomavirus make ideal immunotherapy targets. In addition to oncogenic viral proteins, during cancer initiation and progression, tumor cells acquire protein-altering mutations that are either responsible for transformation (driver mutations) or are a byproduct of the genomic instability that accompanies cellular transformation (passenger mutations). Some of these alterations may result in expression of mutant proteins that are perceived as foreign proteins by the immune system. This class of antigens is likely to be less susceptible to mechanisms of immunological tolerance and therefore may represent more visible targets for immune-mediated tumor control.

### 3. Tumor Neo-Antigens

Tumors develop tens to thousands of coding mutations during the process of tumorigenesis. A small proportion of mutations affect the extracellular domains of cell surface proteins, such as the EGFRvIII mutation, providing unique targets for antibody-based immunotherapies. However, to be recognized by T cells, mutations are processed and presented on MHCI or MHCII, giving rise to so-called neo-antigens.

Neo-antigens can arise when mutations affect either TCR contact residues or anchor residues in peptide epitopes with affinity for MHCI or II. Even a single amino acid substitution can yield an epitope that is sufficiently different from self to mark tumor cells for T-cell-mediated destruction.

By screening tumor-derived cDNA libraries with tumor-reactive T-cell clones, several early studies provided anecdotal examples of immune system recognition of neo-antigens. Such studies demonstrated that neo-antigens can be derived from both driver and passenger genes and are present in many different types of tumors, including melanoma, renal cell carcinoma, oral squamous cell carcinoma, colorectal carcinoma, lung carcinoma, and chronic myelogenous leukemia. Some studies have indicated that neo-antigens can be the predominant class of antigen recognized by TIL. Furthermore, neo-antigen-specific T-cell responses have been associated with complete or partial tumor regression either spontaneously or after therapy.

As therapeutic targets, neo-antigens have several advantages over other classes of tumor antigen. First, neo-antigen-specific T cells are not subject to thymic or peripheral tolerance; therefore, high-affinity T-cell clones are available for immunotherapy. Notably, T cells bearing TCRs with high affinity for their cognate antigens have greater cytotoxic capacity, longer persistence in the tumor environment, and decreased susceptibility to immune suppression. Second, while differentiation and overexpressed antigens are expressed by nontumor tissues, neo-antigens are exclusively expressed by tumor cells, reducing the potential for off-target toxicity. Third, while viral and CT antigens may be expressed in only a limited number of tumors, NGS has revealed that a large proportion of tumors express multiple mutant gene products that could potentially serve as T-cell targets.

For example, useful mutations that results in a protein with a an altered amino acid sequence that is unique to a patient's tumor (e.g., neo-antigens) may include: (1) nonsynonymous mutations leading to different amino acids in the protein; (2) read-through mutations in which a stop codon is modified or deleted, leading to translation of a longer protein with a novel tumor-specific sequence at the C-terminus; (3) splice site mutations that lead to the inclusion of an intron in the mature mRNA and thus a unique tumor-specific protein sequence; (4) chromosomal rearrangements that give rise to a chimeric protein with tumor-specific sequences at the junction of 2 proteins (i.e., gene fusion); (5) frameshift mutations or deletions that lead to a new open reading frame with a novel tumor-specific protein sequence; and the like. Peptides with mutations or mutated polypeptides arising from, for example, splice-site, frameshift, read-through, or gene fusion mutations in tumor cells may be identified by sequencing DNA, RNA or protein in tumor versus normal cells.

Also included herein are personal neo-antigen peptides derived from common tumor driver genes, which may further include previously identified tumor specific mutations. For example, known common tumor driver genes and tumor mutations in common tumor driver genes may be found on the World Wide Web at (www)sanger.ac.uk/cosmic.

### II. Nucleic Acid Assays for Identifying Tumor Mutations

In certain aspects, there are provided nucleic acid assay methods and compositions for identifying tumor mutations and tumor neo-epitopes or neo-antigens. Disclosed herein can be combining nucleic acid assays such as next-generation sequencing of cancer DNA with reverse immunology to identify T cell epitopes from unique tumor antigens and tumor epitopes involved in the control of human cancers. In certain aspects, the initial step is to sequence DNA or RNA from a patient's tumor and normal tissue to identify mutations that create neo-antigens (both missense and neoORFs), particularly in genes expressed in the tumor cell.

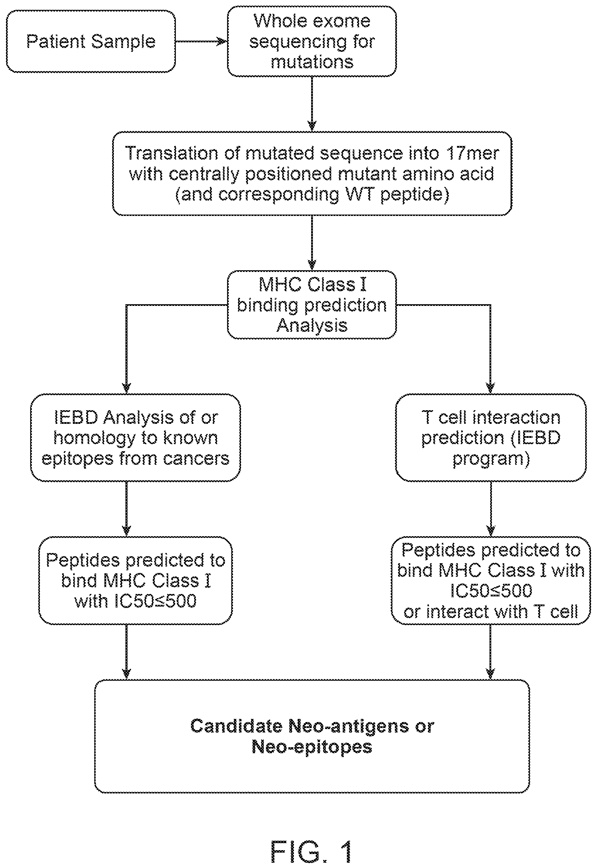

In certain aspects, tumor material can be analyzed for tumor-specific mutations, such as nonsynonymous somatic mutations by sequencing such as whole exome sequencing or whole genome sequencing **(****FIG. 1** **or** **FIG. 2****).** In certain aspects, RNA sequencing data are used to focus on tumor mutations in expressed genes. In certain aspects, peptide stretches containing any of the identified nonsynonymous tumor mutations are generated *in silico* and are filtered through the use of prediction algorithms or used to identify MHC-associated neo-epitopes in mass spectrometry data. In certain aspects, modeling of the effect of tumor mutations on the resulting peptide-MHC complex is used as an additional filter. In certain aspects, MHC-binding neo-epitope sets are used to identify physiologically occurring neo-epitope-specific T cell responses by MHC multimer-based screens and functional assays within both CD8 and CD4 T cell populations.

In one embodiment mutated epitopes are determined by sequencing the genome and/or exome of tumor tissue and healthy tissue from a cancer patient using next generation sequencing technologies. In another embodiment genes that are selected based on their frequency of mutation and ability to act as a neo-antigen are sequenced using next generation sequencing technology.

Next-generation sequencing applies to genome sequencing, genome resequencing, transcriptome profiling (RNA-Seq), DNA-protein interactions (ChIP-sequencing), and epigenome characterization.

Next-generation sequencing can now rapidly reveal the presence of discrete mutations such as coding mutations in individual tumors, most commonly single amino acid changes (e.g., missense mutations) and less frequently novel stretches of amino acids generated by frame-shift insertions/deletions/gene fusions, read-through mutations in stop codons, and translation of improperly spliced introns (e.g., neoORFs).

NeoORFs are valuable as immunogens because the entirety of their sequence is completely novel to the immune system and so are analogous to a viral or bacterial foreign antigen. Thus, neoORFs: (1) are highly specific to the tumor (i.e., there is no expression in any normal cells); (2) can bypass central tolerance, thereby increasing the precursor frequency of neo-antigen-specific CTLs.

Sequencing technology has revealed that each tumor contains multiple, patient-specific mutations that alter the protein coding content of a gene. Such mutations create altered proteins, ranging from single amino acid changes (caused by missense mutations) to addition of long regions of novel amino acid sequence due to frame shifts, read-through of termination codons or translation of intron regions (novel open reading frame mutations; neoORFs). These mutated proteins are valuable targets for the host's immune response to the tumor as, unlike native proteins, they are not subject to the immune-dampening effects of self-tolerance. Therefore, mutated proteins are more likely to be immunogenic and are also more specific for the tumor cells compared to normal cells of the patient.

Advances in sequencing technology have transformed our ability to decode cancer-specific mutations by coupling the sequencing reaction with detection of nucleotide incorporation events for hundreds of millions of genomic fragments in the same instrument run.

In particular, tumor-specific or "somatic" mutations can be identified using massively parallel sequencing (MPS) approaches to compare DNA isolated from tumor versus normal sources.

Similar to DNA-based assays using MPS, RNA from tumors can be analyzed by conversion to cDNA and construction of a library suitable for sequencing.

Since the genome is large (3 billion base pairs) and its analysis complex, the advent of hybrid capture technology has permitted investigators to focus only on the 1% of the genome that comprises the coding exons of known genes, (i.e., the "exome"). Here, probes designed to bind the exon sequences of annotated genes are synthesized, biotinylated, and hybridized in solution with a fragmented whole genome library. The probe-bound library fragments are subsequently captured and isolated using streptavidin-coated magnetic beads. After release from the beads by denaturation, the library fragments are amplified, quantitated, and sequenced.

Exome-capture can be used in a clinical setting, but challenges can include (a) obtaining information in a clinically relevant time frame, (b) the small amounts of DNA/RNA available from a core biopsy procedure, (c) tissue preservation in formalin and paraffin (formalin-fixed paraffin embedded [FFPE]), which promotes the degradation of nucleic acids via backbone crosslinking, and (d) data interpretation. Recent technical innovations have reduced the time for this approach from approximately one week to around two hours for hybrid capture. It is now feasible to generate exome-capture data and produce a list of somatic mutations in about three days. Hybrid capture also enhances the sequencing data quality obtained from tumor RNA (cDNA) sequencing, especially for low yield and/or FFPE-derived samples.

Mutation calling from exome-capture sequencing data is achieved by aligning sequence reads to reference genomes, which serve as the keystone for analyzing the short read lengths (-100 bp) produced by MPS platforms. Once reads are aligned to the genome, variants are identified using several algorithms to interpret different types of mutations, including point mutations (or single nucleotide variants [SNVs]) and focused insertion or deletion variants (indels). Tumor variant calls are then compared with data from a matched normal tissue DNA obtained using a similar capture reagent in order to identify tumor-unique ("somatic") mutations. Subsequent annotation steps convert variations in nucleic acid sequence to changes in amino acid sequence, thereby providing the initial data required to identify and rank order tumor neo-antigens.

One aspect of this process is the ability to predict neo-antigens arising from more "extreme" mutations. In principle, variants that add or delete an amino acid or truncate or extend open reading frames or fusion genes arising from translocations or inversions could be a source of highly antigenic novel epitopes; however, indel variants have, in the past, been difficult to detect with high certainty, even when algorithms are employed that are specifically tuned to detect this type of mutation. However, recent advances now permit the identification of some indels with a fairly high degree of certainty, although indels containing highly repetitive regions remain a difficulty. Structural variants also are difficult to identify, especially from exome-capture data, and hence are not likely to be easily detected unless RNA sequencing (RNA-Seq) data can be evaluated for fusion transcripts (which has a correlative high false-positive rate). In all cases, the use of RNA data from cDNA capture sequencing (cDNA Cap-Seq) or RNA-Seq to identify and/or confirm such variants is critically important.

In certain aspects, a "reference" may be used to correlate and compare the results obtained in the nucleic acid assay methods from a tumor specimen to identify tumor mutations. Typically the "reference" may be obtained on the basis of one or more normal specimens, in particular specimens which are not affected by a cancer disease, either obtained from a patient or one or more different individuals, particularly healthy individuals, in particular individuals of the same species. In further aspects, a "reference" can be determined empirically by testing a sufficiently large number of normal specimens.

It is contemplated that a number of assays could be employed to identify tumor-specific mutations or tumor neo-antigens or epitopes in biological samples. Such assays include, but are not limited to, next-generation sequencing, proteomics, array hybridization, solution hybridization, nucleic amplification, polymerase chain reaction, quantitative PCR, RT-PCR, *in situ* hybridization, Northern hybridization, hybridization protection assay (HPA) (GenProbe), branched DNA (bDNA) assay (Chiron), rolling circle amplification (RCA), single molecule hybridization detection (US Genomics), Invader assay (ThirdWave Technologies), and/or Oligo Ligation Assay (OLA), hybridization, and array analysis.

In certain aspects, there may be provided one or more sequencing methods to identify neo-epitopes for vaccine immunotherapy. Sequencing may be performed on patient-derived samples to identify possible neo-epitopes to target utilizing an adenovirus vector-based vaccine. Sequencing analysis can be combined with genomics, bioinformatics, and immunological approaches to identify mutant tumor associated antigens and epitopes.

Any suitable sequencing method can be used. Particularly, next-generation sequencing, or "NGS" may be used. Next-generation sequencing methods may include all novel high throughput sequencing technologies which, in contrast to the "conventional" sequencing methodology known as Sanger chemistry, read nucleic acid templates randomly in parallel along the entire genome by breaking the entire genome into small pieces.

Such NGS technologies (also known as massively parallel sequencing technologies) are able to deliver nucleic acid sequence information of a whole genome, exome, transcriptome (all transcribed sequences of a genome) or methylome (all methylated sequences of a genome) in very short time periods, e.g., within 1-2 weeks, or within 1-7 days, or within less than 24 hours and allow, in principle, single cell sequencing approaches. Multiple NGS platforms which are commercially available or which are mentioned in the literature can be used as described herein, e.g., those described in detail in Zhang et al. 2011: The impact of next- generation sequencing on genomics. J. Genet Genomics 38 (3), 95-109; or in Voelkerding et al. 2009.

In certain aspects, NGS methods used herein may include the sequencing-by-synthesis approaches developed by Solexa (now part of Illumina Inc., San Diego, California) which is based on reversible dye-terminators and implemented e.g., in the Illumina/Solexa Genome Analyzer™ and in the Illumina HiSeq 2000 Genome Analyzer. In this technology, all four nucleotides are added simultaneously into oligo-primed cluster fragments in flow-cell channels along with DNA polymerase. Bridge amplification extends cluster strands with all four fluorescently labeled nucleotides for sequencing.

In certain aspects, NGS methods used herein may include the sequencing-by-ligation approaches, e.g., implemented in the SOLid™ platform of Applied Biosystems (now Life Technologies Corporation, Carlsbad, California). In this technology, a pool of all possible oligonucleotides of a fixed length is labeled according to the sequenced position. Oligonucleotides are annealed and ligated; the preferential ligation by DNA ligase for matching sequences results in a signal informative of the nucleotide at that position. Before sequencing, the DNA is amplified by emulsion PCR. The resulting beads, each containing only copies of the same DNA molecule, are deposited on a glass slide. As a second example, the Polonator™ G.007 platform of Dover Systems (Salem, New Hampshire) also employs a sequencing-by-ligation approach by using a randomly arrayed, bead-based, emulsion PCR to amplify DNA fragments for parallel sequencing.

In certain aspects, NGS methods used herein may include single-molecule sequencing technologies such as e.g., implemented in the PacBio RS system of Pacific Biosciences (Menlo Park, California) or in the HeliScope™ platform of Helicos Biosciences (Cambridge, Massachusetts). The distinct characteristic of this technology is its ability to sequence single DNA or RNA molecules without amplification, defined as Single-Molecule Real Time (SMRT) DNA sequencing. For example, HeliScope uses a highly sensitive fluorescence detection system to directly detect each nucleotide as it is synthesized. A similar approach based on fluorescence resonance energy transfer (FRET) has been developed from Visigen Biotechnology (Houston, Texas). Other fluorescence-based single-molecule techniques are from U.S. Genomics (GeneEngine™) and Genovoxx (AnyGene™).

In certain aspects, NGS methods used herein may include nano-technologies for single-molecule sequencing in which various nano structures are used which are e.g., arranged on a chip to monitor the movement of a polymerase molecule on a single strand during replication. Non-limiting examples for approaches based on nano-technologies are the GridON™ platform of Oxford Nanopore Technologies (Oxford, UK), the hybridization-assisted nano-pore sequencing (HANS™) platforms developed by Nabsys (Providence, Rhode Island), the proprietary ligase-based DNA sequencing platform with DNA nanoball (DNB) technology called combinatorial probe-anchor ligation (cPAL™), and electron microscopy based technologies for single-molecule sequencing.

In certain aspects, NGS methods used herein may include ion semiconductor sequencing which is based on the detection of hydrogen ions that are released during the polymerization of DNA. For example, Ion Torrent Systems (San Francisco, California) uses a high-density array of micro-machined wells to perform this biochemical process in a massively parallel way. Each well holds a different DNA template. Beneath the wells is an ion-sensitive layer and beneath that a proprietary Ion sensor.

In certain aspects, NGS methods used herein may include several targeted NGS methods for exome sequencing (for review see e.g., Teer and Mullikin 2010: Human Mol Genet 19 (2), Rl 45-51). Many of these methods (described e.g., as genome capture, genome partitioning, genome enrichment etc.) use hybridization techniques and include array-based (e.g., Hodges et al. 2007: Nat. Genet. 39, 1522-1527) and liquid- based (e.g., Choi et al. 2009: Proc. Natl. Acad. Sci USA 106, 19096-19101) hybridization approaches. Commercial kits for DNA sample preparation and subsequent exome capture are also available: for example, Illumina Inc. (San Diego, California) offers the TruSeq™ DNA Sample Preparation Kit and the Exome Enrichment Kit TruSeq™ Exome Enrichment Kit.

Disclosed herein are methods involving the use of a panomics-based test that can utilize whole genome sequencing or RNA sequencing or any combination thereof of a patient's tumor. A panomics-based test that can utilize whole genome sequencing or RNA sequencing can compare a patient's tumor with a patient's normal sample or a reference, to identify molecular alterations in the DNA and RNA of a patient's tumor. In some cases, whole genome based sequencing of DNA and RNA can provide clinical information on a cancer patient's molecular alterations that can result in abnormal proteins. Abnormal proteins can comprise tumor neo-epitopes that can be targeted.

A panomics-based test can entail genomic analysis. In some cases, genomic analysis can select for relevant mutations including single nucleotide variances (SNV), copy number variances, insertions, deletions, rearrangements, or any combination thereof by directly contrasting a tumor genome sequence from a normal genome sequence from each patient and by identifying the patient's mutated genes that are expressed.

Suitable samples can be any patient sample. A suitable sample can undergo DNA or RNA extraction. In some cases, sequencing analysis is performed on Formalin Fixed Paraffin Embedded (FFPE) or fresh frozen samples. In some cases, whole blood can be used. Patient samples can be processed pre-testing. In some cases, a patient can donate a tumor sample. A tumor sample can be a solid tumor sample. A tumor sample can also be a liquid tumor sample. In some cases, a sample can be enriched. Enrichment can comprise, increasing the concentration of proteins unique to tumor cells. Any suitable method can be used for enrichment. In some cases, laser microdissection can be used for sample enrichment. Laser microdissection can measure absolute quantities of relevant proteins for targeted and chemotherapy using mass spectrometry analysis.

Targeted enrichment methods can broadly fall into two categories: PCR-amplicon and hybridization capture approaches. As PCR-based approaches can readily be used routinely in diagnostic laboratories they fit well with existing diagnostic workflows. PCR can be highly specific and has the advantage of generating more uniform coverage than comparative hybridization approaches, provided the concentrations of individual PCR products are adequately normalized before pooling and sequencing.

Different strategies have been used to generate PCR amplified libraries. Some use concatenation of PCR products to generate fragment libraries; shearing PCR concatamers and feeding into shotgun library preparation. A more straightforward protocol that is compatible with long-read sequencing instruments is to incorporate the sequence adaptors into the 5' or 3'-end of the PCR primer enabling pooling of amplicons and direct sequencing. Any targeted enrichment method available in the art can be used herein.

In some cases, Fluidigm, a microfluidics-based method that uses multilayer soft lithography (MSL), can be used herein. A microfluidic circuitry can be fabricated from a soft rubber composite that allows the controlled flow of reagents by using pressure to create tiny valves in the circuitry and reaction chambers for PCR. Fluidigm was originally developed for real-time quantitative PCR and single nucleotide polymorphism (SNP) genotyping applications but more recently the Access Array has been released, allowing retrieval of PCR product for targeted resequencing applications. The current Access Array system is capable of parallel PCR reactions for 48 samples by 48 single-plex assays. An attractive aspect of this platform can be that relatively small quantities of template are required (<50 ng/sample). Assays can also be multiplexed to improve throughput.

Disclosed herein can also be the use of RainStorm (Raindance Technologies). RainStorm involves the generation of microdroplets in an oil emulsion, which then act as miniaturized reaction chambers for PCR. This method can be used for DNA extracted from FFPE tissue. Alternatively, molecular inversion probes (MIP) can be used herein. A MIP is a long oligonucleotide composed of sequence specific primer ends tethered by a universal linker sequence. Target specific primer ends hybridize to complementary DNA flanking the region of interest. Polymerase extension and then ligation results in the circularization of the MIP. Captured regions are then amplified either by rolling circle amplification or by PCR from universal PCR priming sites within the linker region

In some cases, RNA sequencing (RNA-seq) can be utilized. In some cases, a population of RNA (total or fractionated, such as poly (A)+) can be converted to a library of cDNA fragments with adaptors attached to one or both ends. Each molecule, with or without amplification, can then be sequenced in a high-throughput manner to obtain short sequences from one end (single-end sequencing) or both ends (pair-end sequencing).The reads can typically be 30-400 bp, depending on the DNA-sequencing technology used.

Any high-throughput sequencing technology can be used for RNA-Seq, including, for example, Illumina IG, Applied Biosystems SOLiD and Roche 454 Life Science systems. In other cases, a Helicos Biosciences tSMS system can be used for RNA-Seq studies. Following sequencing, resulting reads can either be aligned to a reference genome or reference transcripts, or assembled de novo without the genomic sequence to produce a genome-scale transcription map that consists of both the transcriptional structure and/or level of expression for each gene.

In some cases, tumor-specific or "somatic" mutations can be identified using massively parallel sequencing (MPS) (Simpson AJ, et al. Nat Rev Cancer. 2005;5(8):615-625.) approaches to compare DNA isolated from tumor versus normal sources. Similar to DNA-based assays using MPS, RNA from tumors can be analyzed by conversion to cDNA and construction of a library suitable for sequencing. Probes can be designed to bind exon sequences of annotated genes that can be synthesized, biotinylated, and hybridized in solution with a fragmented whole genome library. The probe-bound library fragments can subsequently be captured and isolated using streptavidin-coated magnetic beads. After release from the beads by denaturation, the library fragments are amplified, quantitated, and sequenced.

Exome-capture can be used in a sequencing method in certain aspects. Mutation calling from exome-capture sequencing data can be achieved by aligning sequence reads to reference genomes, which serve as the keystone for analyzing the short read lengths (-100 bp) produced by MPS platforms. Once reads are aligned to the genome, variants can be identified using several algorithms to interpret different types of mutations, including point mutations (or single nucleotide variants (SNVs)) and focused insertion or deletion variants (indels). Tumor variant calls can then be compared with data from a matched normal tissue DNA obtained using a similar capture reagent in order to identify tumor-unique ("somatic") mutations. Subsequent annotation steps convert variations in nucleic acid sequence to changes in amino acid sequence, thereby providing the initial data required to identify and rank order tumor neo-epitopes. In some cases, the use of RNA data from cDNA capture sequencing (cDNA Cap-Seq) or RNA-Seq to identify and/or confirm neo-epitope variants can be performed.

Comprehensive characterization of somatic variants by single-nucleus whole-genome and whole-exome sequencing has already been demonstrated, with the nuc-seq protocol described achieving a mean coverage breadth of over 90%. The relevant clonal structure can also be obtained using more cost-effective single-cell sequencing protocols targeted at predicted variants. These bioinformatics-intensive approaches are important for creating the necessary edifice for any and all approaches on identification of neo-epitopes.

### III. Identification of Tumor Neo-Epitopes

In one embodiment, the sequencing data derived from determining the presence of mutations in a cancer patient is analyzed to predict personal mutated peptides that can bind to HLA molecules of the individual. In one embodiment, the data is analyzed using a computer. In another embodiment, the sequence data is analyzed for the presence of neo-antigens. In one embodiment, neo-antigens are determined by their affinity to MHC molecules.

Efficiently choosing which particular mutations to utilize as immunogen involves the identification of the patient HLA type and the ability to predict which mutated peptides would efficiently bind to the patient's HLA alleles. Neural network based learning approaches with validated binding and non-binding peptides have advanced the accuracy of prediction algorithms for the major HLA-A and -B alleles. Utilizing advanced algorithms for predicting which missense mutations create strong binding peptides to the patient's cognate MHC molecules, a set of peptides representative of optimal mutated epitopes (both neoORF and missense) for each patient may be identified and prioritized.

In certain aspects, neo-epitope prediction algorithms are used to predict binding of candidate peptides to MHC class I molecules or MHC class II molecules. In humans, the MHC class I antigen presentation pathway is responsible for presenting peptides derived from endogenous cell-intrinsic proteins to CD8 CTLs. Endogenous proteins are processed by the proteasome and the resulting 8-11 amino acid peptides transported into the ER by the transporter associated with antigen processing (TAP), where they are loaded onto newly synthesized class I molecules and the stabilized peptide-MHCI (p-MHCI) complexes are transported to the cell surface. In some cases, a neo-epitope can be presented by MHC class I. For example, a neo-epitope can be a peptide of a 6 to 10 mer.

Disclosed herein, can also be a protocol to identify a tumor-derived neo-epitope using a tool to predict peptide binding to MHC class I. Disclosed herein, can also be a method to identify a tumor-derived neo-epitope using a tool to predict peptide binding to MHC class II.

Multiple tools exist to predict peptide binding to MHCI or MHCII. A comprehensive list of prediction tools is available (available through http://cancerimmunity.org/resources/webtools). In some cases, a peptide binding tool can be selected from a list comprising: PAProC, NetChop, MAPPP, TAPPred, RankPep, MHCBench, HLA Peptide Binding Predictions, PREDEP, nHLAPred-I, ProPred-1, SVMHC, EPIPREDICT, ProPred, NetMHC, NetMHCII, NetMHCpan, SMM, POPI, OptiTope, Mosaic Vaccine Tool Suite, HLABinding, Prediction of Antigenic Determinants, ANTIGENIC, BepiPred, DiscoTope, ElliPro, Antibody Epitope Prediction, CTLPred, NetCTL, MHC-I processing predictions, Epitope Cluster Analysis, Epitope Conservancy Analysis, VaxiJen, or combinations thereof. Other programs such as SYFPEITHI (Rammensee H, et al. Immunogenetics. 1999; 50(3-4):213-219), Rankpep (Reche PA, et al. Hum Immunol. 2002; 63(9):701-709), or BIMAS (Parker KC, et al. J Immunol. 1994;152(1):163-175.) can also be used.

In some cases, the Immune Epitope Database and Analysis Resource (IEDB) (Vita R, et al. Nucleic Acids Res. 2015; 43 (Database issue):D405-D412) can be utilized to identify a suitable tumor neo-antigen. In some cases, these algorithms can predicts peptide binding to different MHC class I variants based on artificial neural networks (ANN), providing predicted IC₅₀ as an output. In some cases, NetMHC (Lundegaard C, et al. Nucleic Acids Res. 2008;36 (Web Server issue):W509-W512.) can be used. Neural network-based approaches can depend on the quality and size of a training set and therefore are likely to be more accurate for the more common alleles. In some cases, programs such as SMM (Peters B, et al. BMC Bioinformatics. 2005; 6:132) and SMMPMBEC (Kim Y, et al. BMC Bioinformatics. 2009; 10:394) can be used. These programs use position-weight matrices to describe statistical preferences from p-MHCI binding data. This approach can suppress noise caused by both experimental error and a limited number of data points present in the training set.

In certain aspects, SNPs are removed from candidate neo-antigens or neo-epitopes. Information about SNPs can be obtained from the Single Nucleotide Polymorphism Database: (dbSNP), which is a free public archive for genetic variation within and across different species developed and hosted by the National Center for Biotechnology Information (NCBI) in collaboration with the National Human Genome Research Institute (NHGRI). Although the name of the database implies a collection of one class of polymorphisms only (i.e., single nucleotide polymorphisms (SNPs)), it in fact contains a range of molecular variation: (1) SNPs, (2) short deletion and insertion polymorphisms (indels/DIPs), (3) microsatellite markers or short tandem repeats (STRs), (4) multinucleotide polymorphisms (MNPs), (5) heterozygous sequences, and (6) named variants. The dbSNP accepts apparently neutral polymorphisms, polymorphisms corresponding to known phenotypes, and regions of no variation. It was created in September 1998 to supplement GenBank, NCBI's collection of publicly available nucleic acid and protein sequences.

In certain aspects, there is provided a proteomic-based method for identifying tumor specific neo-antigens such as direct protein sequencing. Protein sequencing of enzymatic digests using multidimensional MS techniques including tandem mass spectrometry (MS/MS)) can also be used to identify neo-antigens. Such proteomic approaches permit rapid, highly automated analysis. It is further contemplated that high-throughput methods for de novo sequencing of unknown proteins may be used to analyze the proteome of a patient's tumor to identify expressed neo-antigens. For example, meta-shotgun protein sequencing may be used to identify expressed neo-antigens.

Tumor specific neo-antigens may also be identified using MHC multimers to identify neo-antigen-specific T-cell responses. For example, high-throughput analysis of neo-antigen-specific T-cell responses in patient samples may be performed using MHC tetramer-based screening techniques. Such tetramer-based screening techniques may be used for the initial identification of tumor specific neo-antigens, or alternatively as a secondary screening protocol to assess what neo-antigens a patient may have already been exposed to, thereby facilitating the selection of candidate neo-antigens.

Additional filters could be applied to eliminate (1) epitopes predicted to be poorly processed by the immunoproteasome and (2) epitopes with lower binding affinity than the corresponding wild-type sequences. In certain aspects, candidate mutated peptides are synthesized and screened to identify T cell neo-antigens. This approach could be very efficient to identify neo-antigens.

In certain aspects, another approach to identify tumor neo-epitopes is provided by pulsing antigen presenting cells with relatively long synthetic peptides that encompass minimal T cell epitopes. In a recent report, nonsynonymous mutated epitopes were identified in three melanoma lesions by evaluating the response of CD4+ tumor infiltrating lymphocytes (TIL) to autologous B cells that were pulsed with 31 amino-acid long peptides encompassing individual mutations. Use of this approach resulted in the identification of mutated CIRH1A, GART, ASAP1, RND3, TNIK, RPS12, ZC3H18 and LEMD2 T cell epitopes. Furthermore, in a recent report, a peptide screening was carried out based on the combination of two peptide libraries: (1) 15-mer overlapping long-peptides (2) peptides based on MHC-binding prediction. This screening led to the identification of mutated HSDL1-reactive T cells isolated from an ovarian tumor.

In certain aspects, a tandem minigene screening approach can be used to identify tumor neo-epitopes. A tandem minigene construct comprised 6 to 24 minigenes that encoded polypeptides containing a mutated amino acid residue flanked on their N- and C-termini by 12 amino acids. In certain aspects, tandem minigene constructs are synthesized and used to transfect autologous APCs or cell lines co-expressing autologous HLA molecules. Using this approach, mutated KIF2 C and POLA2 epitopes were identified in two melanoma patients. In addition, a mutated ERBB2IP epitope was identified in a patient with cholangiocarcinoma. Recent studies using this approach have led to the identification of mutated antigens express on gastrointestinal, breast and ovarian cancers. Notably, the neo-antigen reactivity could be identified from TILs isolated from patients with cholangiocarcinoma or gastrointestinal cancer, which has a relatively low number of mutations.

In certain aspects, tumor neo-epitopes are identified using an approach combined whole-exome/transcriptome sequencing analysis, MHC binding prediction, as well as mass spectrometric technique to detect peptides eluted from HLA molecules. Interestingly, only a small fraction of predicted high-binding peptides were confirmed by mass spectrometry. The relative small number of mutated peptides identified by mass spectrometry might be due to the sensitivity of the peptide purification and mass spectrometry, but it could also suggest that natural antigen process and presentation in cells could be very inefficient. Among 7 neo-epitopes identified by this approach, mutated Adpgk, Reps1 and Dpagt1 epitopes were confirmed to be immunogenic.

### IV. Tumor Neo-Epitope Prioritization

In certain aspects, methods may be provided for prioritization of tumor neo-epitopes or neo-antigens based on one or more criteria such as MHC binding affinity, epitope abundance, antigen processing and/or presentation. Such identified tumor neo-epitopes or neo-antigens may be used in targeted vaccine or immunotherapy using adenoviral vectors disclosed herein.

In certain aspects, for identifying tumor-derived mutant epitopes, a criterion such as predicted p-MHCI binding affinity can be used to generate an initial prioritized list of candidate neo-epitopes. In some cases, natural immune responses to tumor neo-epitopes are selectively directed to epitopes within a group predicted to have the strongest MHCI binding affinities.

Peptide/MHCI binding can be influenced by two additional parameters - epitope abundance and antigen processing (i.e., protein degradation and peptide transport).

In some aspects, mass spectrometry could potentially provide information on epitope abundance. In further aspects, if a somatic mutation is detected to be present or abundant in RNA sequencing, it may be prioritized as a target neo-antigen. In additional aspects, epitope abundance can be estimated indirectly by quantitating RNA expression levels. In one approach, mutations defined by tumor-to-normal DNA comparisons are subjected to bioinformatic analysis to predict their immunogenicity and the levels of candidate immune stimulatory peptides are estimated by RNA-Seq. RNA evaluation provides information regarding (a) whether the variant is expressed in the RNA and (b) the mutant allele's expression level relative to other genes. In a second approach, cDNA capture can be performed from tumor RNA and compared with normal DNA to provide a list of mutated peptides.

In some cases, due to the error rate of reverse transcriptase and sources of false positivity in variant calling in RNA versus DNA, predicted mutations can be refined further by applying a series of filters to remove known sources of false-positive variants (e.g., low coverage in tumor or normal, or low numbers of variant-containing reads) and to eliminate non-expressed genes and/or alleles. A filter can eliminate genes with low expression. Downstream steps of immunogenicity prediction can then be made for this filtered set of mutant peptides.

Additional algorithms exist to refine epitope predictions including those focused on defining proteasomal cleavage (i.e., NetChop) (Nielsen M, et al. Immunogenetics. 2005;57(1-2):33-41) and/or TAP transport (i.e., NetCTL and NetCTLpan) (Peters B, et al. J Immunol. 2003;171(4):1741-1749).

Disclosed herein can also be methods or tools for predicting MHC class II neo-epitopes. Whereas the MHC class I binding groove is closed at both ends, MHC class II has a peptide-binding groove that is open, leading to considerable variation in both the length of peptides that can bind to MHC class II and the location of the binding core. Multiple MHC class II binding prediction algorithms are available such as TEPITOPE (Hammer J, et al. J Exp Med. 1994;180(6):2353-2358); netMHCII (Nielsen M, et al. BMC Bioinformatics. 2009;10:296); and SMM-align (Nielsen M, et al. BMC Bioinformatics 2007;8:238) that can be used herein.

Software can be used to identify mutant neo-epitopes. In some cases, binding affinity of a potential neo-epitope can be calculated. For example, a NetMHCpan software program can potentially predict mutant neo-epitopes. Any suitable program or algorithm can be used to identify neo-epitopes.

A neo-epitope can have any affinity to an MHC molecule, such as less than 10, 20, 30, 40, 50, 60, 70, 80, 90, 100, 110, 120, 130, 140, 150, 160, 170, 180, 190, 200, 210, 220, 230, 240, 250, 260, 270, 280, 290, 300, 310, 320, 330, 340, 350, 400, 450, 500 nmol/L or any intermediate range or value. In some cases, strong affinity can be a half maximal inhibitory concentration (IC₅₀) < 50 nmol/L. In some cases, moderate affinity can be 50 < IC₅₀ < 150. In some cases, weak affinity can be 150 < IC₅₀ < 500 for HLA. In some cases, IC₅₀ > 500 can be low or no affinity.

In further aspects, methods may be provided herein to identify peptides most likely to generate a robust immune response, for example, by using algorithms (e.g., NetMHC, IEDB) that predict peptides binding to the cleft of patient-specific class I HLA molecules. For example, two studies in human patients with leukemia used this approach to identify CTLs targeting HLA-binding peptides derived from mutated regions of known oncogenes, nucleophosmin in acute myelogenous leukemia, and *BCR-ABL* in chronic myelogenous leukemia.

In addition, to carry out an unbiased search for neo-antigens, whole-exome sequencing may be used, for example, to identify all the leukemia-specific mutations in patients with chronic lymphocytic leukemia in a recent report. Within a subset of patients with identified HLA alleles, methods may be provided to use NetMHC to predict which mutated peptides bind to patient-specific HLAs, validate their binding biochemically, and confirm the presence of CTLs targeting a subset of these neo-antigen-derived peptides.

Furthermore, improved prediction rules (based on additional steps in antigen processing) may further improve the odds. In certain aspects, among the neo-epitopes, neoORFs may be prioritized because they provide long stretches of completely novel protein sequence (which bypass central tolerance and have no counterpart in any normal cell).

In addition, methods may be provided to prioritize targets harboring mutations in genes that are required for tumor cell survival (e.g., "oncogenic drivers;") or that diminish fitness when reduced in expression, as well as those that are present in all cancer cells (i.e., clonal) rather than only a subpopulation (i.e., subclonal).

In certain aspects, proteomics methods such as mass spectroscopy of MHC-eluted peptides are used to identify the neo-epitopes that are **actually presented.** Prediction of presentability by MHC molecules may also use a bioinformatic strategy.

In certain aspects, algorithm-based methods or tools may be used to define a neo-epitope's dissimilarity from "self' antigens, such as the DAI algorithm.

In certain aspects, methods for measuring T-cell response may be provided to further select for tumor neo-antigens. The antitumor activity as seen *in vivo* of tumor neo-antigens may be tested by measuring the T-cell activity measured *in vitro.* Specifically, neo-epitopes that elicit tumor rejection in a CD8-dependent manner may be represented by eliciting CD8 responses that can be measured *in vitro.* Improved high-throughput assays that measure T-cell activation to large numbers of antigens in a highly sensitive and multifactorial manner can help neo-epitope discovery. Assays based on sequencing of T-cell receptors may be one such example.

### V. Tumor Neo-Antigen-Based Cancer Therapy

Disclosed herein can be methods and compositions to identify changes that can be unique to a patient's cancer. In some cases, methods may be useful in selecting a therapeutic intervention specific for a patient's cancer. In some cases, targeting a patient's tumor neo-epitope with a vaccine may be a suitable method. For example, next-generation DNA- or RNA-sequencing technologies may be used for identification of tumor neo-epitopes for therapy. A vaccination method as disclosed herein may be used as well as additional therapies. Non-limiting examples of additional therapies can comprise additional immunotherapy, chemotherapy, radiation, gene therapy, targeted therapy, or a combination thereof. A vaccine method as disclosed herein can also comprise administering a composition comprising a replication-defective vector, wherein the replication-defective vector comprises a nucleic acid sequence encoding for a tumor neo-antigen; and a nucleic acid sequence encoding for an immunological fusion partner.

In some cases, a patient can receive therapy followed by sequencing performed on a patient sample. A patient sample can undergo sequencing **(****FIG.** 1). Sequencing can identify cancer-specific SNVs. A SNV can undergo further examination to determine if an SNV is in an expressed protein. A protein can drive MHC immunity. MHC can be class I or class II. A patient with any MHC type or HLA type can undergo sequencing analysis.

Disclosed herein, is a strategy for treating cancer as a chronic disease. In some cases, a patient can have a cancer sample sequenced. Sequencing can identify tumor neo-epitopes. Target neo-epitopes can be cloned into an adenovirus vector and used as a vaccination. In some cases, a patient's cancer may mutate and new neo-epitopes can be used for a second vaccination regime. Disclosed herein, is a method of identifying and utilizing tumor neo-epitopes to treat a patient cancer as it evolves. In some cases, a cancer may mutate to overcome a treatment. A mutation can generate new tumor neo-epitopes that can be targeted with an adenovirus vector disclosed herein.

As tumors develop, they can evolve numerous subclones, and gene expression between these subclones can vary. In addition, although a majority of subclones share driver mutations responsible for supporting tumor growth and survival; they may have many more passenger mutations (mutations in genes not essential for tumor survival) that are more variable. The uneven ratio of driver to passenger mutations may pose a problem because it will likely force vaccines to target neo-epitopes that may not be uniformly expressed across subclones and may not be essential for survival. Tumor variation becomes more complex when considering variation between metastases, which derive from only a subset of subclones from the primary tumor.

Disclosed herein, is a method for treating a cancer as it evolves. In some cases, a vaccine targeting a neo-epitope is administered. A patient that has been treated with a neo-epitope targeted vaccine can undergo secondary sequencing of a sample to identify new neo-epitopes that may have resulted from a tumor evolving. A tumor can evolve to bypass treatment and continue persisting.

Certain aspects involve a treatment method to vaccinate against an evolving cancer and treat a cancer as a chronic disease. A patient may undergo sequencing of a sample and may undergo additional sequencing of a sample at a different time point. Sequencing of a sample can occur at any time. Sequencing may occur at pre-defined time points. In some cases, time points are determined according to a response to a treatment. In other cases, time points are predetermined. A time point can occur at any time. A time point can be weekly. A time point can be monthly. A time point can be yearly. In some cases, a time point may also be hourly. Sequencing of a sample may be performed in order to identify mutations or neo-epitopes as a cancer evolves. New neo-epitopes may be used for vaccination or a suitable targeted therapy.

Certain aspects relate to methods for producing a vaccine that generates immune responses against various neo-epitopes using an adenovirus vector that allows for multiple vaccinations to generate broadly reactive immune responses against tumor neo-epitopes.

One aspect provides a method of generating an immune response against several tumor neo-epitopes in a subject comprising administering to the subject an adenovirus vector comprising: a) a replication-defective adenovirus vector, wherein the adenovirus vector has a deletion in the E2b region, and b) nucleic acids encoding one or multiple tumor neo-epitopes; and re-administering the adenovirus vector at least once to subject; thereby generating an immune response against tumor neo-epitopes. In some aspects, the adenovirus vector can further comprise a nucleic acid sequence encoding for an immunological fusion partner.

Another aspect provides a method for generating an immune response against several neo-epitopes in a subject, wherein the subject has preexisting immunity to adenovirus, comprising: administering to the subject an adenovirus vector comprising: a) a replication-defective adenovirus vector, wherein the adenovirus vector has a deletion in the E2b region, and b) nucleic acids encoding multiple tumor neo-epitopes; and re-administering the adenovirus vector at least once to the subject; thereby generating an immune response against the tumor neo-epitopes. In some aspects, the adenovirus vector can further comprise a nucleic acid sequence encoding for an immunological fusion partner.

An immune system can shape clonal composition of tumors through a process termed immunoediting. Immunoediting demonstrates the potential of an immune system to mount an antitumor response; it may pose a problem for tumor mutome-targeted vaccines as immunoediting could eradicate tumor cells expressing the most immunogenic neo-epitopes, and therefore best vaccine targets, leaving behind only less immunogenic and less optimal targets to be picked up by sequencing. Furthermore, the outgrowth of immunoedited tumors demonstrates the importance of targeting epitopes covering the entire repertoire of tumor subclones, and not necessarily just dominant neo-epitopes. Disclosed herein, is a method for targeting a tumor that has evolved.

Disclosed herein can be a method to treat cancer. For example, a patient can be treated with a cancer vaccine. In some aspects, the cancer vaccine can comprise a nucleic acid sequence encoding for an immunological fusion partner. A cancer vaccine treatment can be combined with any suitable secondary therapy. A secondary therapy can comprise immunotherapy, radiation, chemotherapy, radio-therapy, or any combination thereof.

Vaccines with mutations selected for *in silico* predicted favorable MHC class II binding and abundant expression confer potent anti-tumor control. In some cases, a comparison of MHC II binding scores of immunogenic and non-immunogenic mutated neo-epitope targets can identify suitable targets. In some cases, CD4 T-cell recognition of mutations may have a less stringent length and sequence requirement for peptides binding to MHC class II molecules as compared to MHC class I epitopes increasing the likelihood that a given mutation is found within a presented peptide. In some cases, MHC class II neo-epitopes may be used. In other cases, a library comprised of vectors targeting neo-epitopes of both MHC class I and MHC class II are administered.

As noted elsewhere herein, expression constructs, particularly adenovirus vectors, may comprise nucleic acid sequences that encode one or more target antigens of interest such as any one or more of the tumor neo-antigens or tumor neo-epitopes against which an immune response is to be generated. For example, target antigens may include, but are not limited to, tumor neo-epitopes or neo-antigens identified on solid or liquid tumors. In some aspects, the expression constructs can further comprise a nucleic acid sequence encoding for an immunological fusion partner.

The expression constructs can be used in a number of vaccine settings for generating an immune response against one or more target antigens as described herein. The adenovirus vectors are of particular importance because they can be used to generate immune responses in subjects who have preexisting immunity to Ad and can be used in vaccination regimens that include multiple rounds of immunization using the adenovirus vectors, regimens not possible using previous generations of adenovirus vectors.

Generally, generating an immune response comprises an induction of a humoral response and/or a cell-mediated response. In certain embodiments, it is desirable to increase an immune response against a target antigen of interest. As such "generating an immune response" or "inducing an immune response" comprises any statistically significant change, e.g., increase in the number of one or more immune cells (T cells, B cells, antigen-presenting cells, dendritic cells, neutrophils, and the like) or in the activity of one or more of these immune cells (CTL activity, HTL activity, cytokine secretion, change in profile of cytokine secretion, etc.).

The skilled artisan would readily appreciate that a number of methods for establishing whether an alteration in the immune response has taken place are available. A variety of methods for detecting alterations in an immune response (e.g., cell numbers, cytokine expression, cell activity) are known in the art and are useful in certain aspects. Illustrative methods are described in Current Protocols in Immunology, Edited by: John E. Coligan, Ada M. Kruisbeek, David H. Margulies, Ethan M. Shevach, Warren Strober (2001 John Wiley & Sons, NY, NY) Ausubel et al. (2001 Current Protocols in Molecular Biology, Greene Publ. Assoc. Inc. & John Wiley & Sons, Inc., NY, NY); Sambrook et al. (1989 Molecular Cloning, Second Ed., Cold Spring Harbor Laboratory, Plainview, NY); Maniatis et al. (1982 Molecular Cloning, Cold Spring Harbor Laboratory, Plainview, NY) and elsewhere. Illustrative methods useful in this context include intracellular cytokine staining (ICS), ELISpot, proliferation assays, cytotoxic T cell assays including chromium release or equivalent assays, and gene expression analysis using any number of polymerase chain reactions (PCR) or RT-PCR based assays.

In certain embodiments, generating an immune response comprises an increase in target antigen-specific CTL activity of about 1.5 to 20, or more fold in a subject administered the adenovirus vectors as compared to a control. In another embodiment, generating an immune response comprises an increase in target-specific CTL activity of about 1.5 to 20, or more fold in a subject administered the adenovirus vectors as compared to a control. In a further embodiment, generating an immune response that comprises an increase in target antigen-specific cell-mediated immunity activity as measured by ELISpot assays measuring cytokine secretion, such as interferon-gamma (IFN-γ), interleukin-2 (IL-2), tumor necrosis factor-alpha (TNF-a), or other cytokines, of about 1.5 to 20, or more fold as compared to a control.

In a further embodiment, generating an immune response comprises an increase in target-specific antibody production of between 1.5 and 5 fold in a subject administered the adenovirus vectors as described herein as compared to an appropriate control. In another embodiment, generating an immune response comprises an increase in target-specific antibody production of about 1.5 to 20, or more fold in a subject administered the adenovirus vector as compared to a control.

Thus, there may be provided methods for generating an immune response against tumor antigens, comprising administering to an individual in need thereof an adenovirus vector comprising: a) a replication-defective adenovirus vector, wherein the adenovirus vector has a deletion in the E2b region, and b) nucleic acids encoding one or more tumor antigens such as tumor neo-epitopes or tumor neo-antigens; and readministering the adenovirus vector at least once to the subject; thereby generating an immune response against the tumor antigens. In some aspects, the adenovirus vector can further comprise a nucleic acid sequence encoding for an immunological fusion partner. In certain embodiments, there may be provided methods wherein the adenovirus vector administered is not a gutted vector.

In a further embodiment, there may be provided methods for generating an immune response against tumor antigens such as tumor neo-epitopes or tumor neo-antigens in a subject, wherein the subject has pre-existing immunity to Ad, by administering to the subject an adenovirus vector comprising: a) a replication-defective adenovirus vector, wherein the adenovirus vector has a deletion in the E2b region, and b) nucleic acids encoding one or more tumor antigens such as tumor neo-epitopes or tumor neo-antigens; and re-administering the adenovirus vector at least once to the subject; thereby generating an immune response against the tumor antigens. In some aspects, the adenovirus vector can further comprise a nucleic acid sequence encoding for an immunological fusion partner.

With regard to preexisting immunity to Ad, this can be determined using methods known in the art, such as antibody-based assays to test for the presence of Ad antibodies. Further, in certain embodiments, the methods may include first determining that a subject has preexisting immunity to Ad then administering the E2b deleted adenovirus vectors as described herein.

One embodiment provides a method of generating an immune response against one or more target antigens in an individual comprising administering to the individual a first adenovirus vector comprising a replication-defective adenovirus vector, wherein the adenovirus vector has a deletion in the E2b region, and a nucleic acid encoding at least one target antigen; administering to the individual a second adenovirus vector comprising a replication-defective adenovirus vector, wherein the adenovirus vector has a deletion in the E2b region, and a nucleic acid encoding at least one target antigen, wherein the at least one target antigen of the second adenovirus vector is the same or different from the at least one target antigen of the first adenovirus vector. In particular embodiments, the target antigen may be a wild-type protein, a fragment, a variant, or a variant fragment thereof. In some embodiments, the target antigen comprises a tumor antigen, a tumor neo-antigen, a tumor neo-epitope, a tumor-associate antigen, a fragment, a variant, or a variant fragment thereof. In some aspects, the adenovirus vector can further comprise a nucleic acid sequence encoding for an immunological fusion partner. In some aspects, the second adenovirus vector can further comprise a nucleic acid sequence encoding for an immunological fusion partner.

Thus, certain embodiments contemplate multiple immunizations with the same E2b deleted adenovirus vector or multiple immunizations with different E2b deleted adenovirus vectors. In each case, the adenovirus vectors may comprise nucleic acid sequences that encode one or more target antigens as described elsewhere herein. In each case, the adenovirus vectors may further comprise a nucleic acid sequence encoding for an immunological fusion partner. In certain embodiments, the methods comprise multiple immunizations with an E2b deleted adenovirus encoding one target antigen, and re-administration of the same adenovirus vector multiple times, thereby inducing an immune response against the target antigen. In some embodiments, the target antigen comprises a tumor antigen, a tumor neo-antigen, a tumor neo-epitope, a tumor-associate antigen, a fragment, a variant, or a variant fragment thereof.

In a further embodiment, the methods comprise immunization with a first adenovirus vector that encodes one or more target antigens, and then administration with a second adenovirus vector that encodes one or more target antigens that may be the same or different from those antigens encoded by the first adenovirus vector. In this regard, one of the encoded target antigens may be different or all of the encoded antigens may be different, or some may be the same and some may be different. Further, in certain embodiments, the methods include administering the first adenovirus vector multiple times and administering the second adenovirus multiple times. In this regard, the methods comprise administering the first adenovirus vector 1, 2, 3, 4, 5, 6, 7, 8, 9, 10, 11, 12, 13, 14, 15, or more times and administering the second adenovirus vector 1, 2, 3, 4, 5, 6, 7, 8, 9, 10, 11, 12, 13, 14, 15, or more times. The order of administration may comprise administering the first adenovirus one or multiple times in a row followed by administering the second adenovirus vector one or multiple times in a row. In certain embodiments, the methods include alternating administration of the first and the second adenovirus vectors as one administration each, two administrations each, three administrations each, and so on. In certain embodiments, the first and the second adenovirus vectors are administered simultaneously. In other embodiments, the first and the second adenovirus vectors are administered sequentially. In some embodiments, the target antigen comprises a tumor antigen, a tumor neo-antigen, a tumor neo-epitope, a tumor-associate antigen, a fragment, a variant, or a variant fragment thereof. In some aspects, the first and/or second adenovirus vector can further comprise a nucleic acid sequence encoding for an immunological fusion partner.

As would be readily understood by the skilled artisan, more than two adenovirus vectors may be used in the methods as described herein. Three, 4, 5, 6, 7, 8, 9, 10, or more different adenovirus vectors may be used in the methods as described herein. In certain embodiments, the methods comprise administering more than one E2b deleted adenovirus vector at a time. In this regard, immune responses against multiple target antigens of interest can be generated by administering multiple different adenovirus vectors simultaneously, each comprising nucleic acid sequences encoding one or more target antigens.

The adenovirus vectors can be used to generate an immune response against a cancer, such as carcinomas or sarcomas (e.g., solid tumors, lymphomas and leukemia). The adenovirus vectors can be used to generate an immune response against a cancer, such as neurologic cancers, melanoma, non-Hodgkin's lymphoma, Hodgkin's disease, leukemia, plasmocytomas, adenomas, gliomas, thymomas, breast cancer, prostate cancer, colorectal cancer, kidney cancer, renal cell carcinoma, uterine cancer, pancreatic cancer, esophageal cancer, lung cancer, ovarian cancer, cervical cancer, testicular cancer, gastric cancer, multiple myeloma, hepatoma, acute lymphoblastic leukemia (ALL), acute myelogenous leukemia (AML), chronic myelogenous leukemia (CML), and chronic lymphocytic leukemia (CLL), or other cancers.

Methods are also provided for treating or ameliorating the symptoms of any of the infectious diseases or cancers as described herein. The methods of treatment comprise administering the adenovirus vectors one or more times to individuals suffering from or at risk from suffering from an infectious disease or cancer as described herein. In some aspects, the adenovirus vector can further comprise a nucleic acid sequence encoding for an immunological fusion partner. As such, certain embodiments provide methods for vaccinating against infectious diseases or cancers in individuals who are at risk of developing such a disease. Individuals at risk may be individuals who may be exposed to an infectious agent at some time or have been previously exposed but do not yet have symptoms of infection or individuals having a genetic predisposition to developing a cancer or being particularly susceptible to an infectious agent. Individuals suffering from an infectious disease or cancer described herein may be determined to express and/or present a target antigen, which may be use to guide the therapies herein. For example, an individual can be found to express and/or present a target antigen and an adenovirus vector encoding the target antigen, a variant, a fragment or a variant fragment thereof may be administered subsequently. In some aspects, the adenovirus vector can further comprise a nucleic acid sequence encoding for an immunological fusion partner.

Certain embodiments contemplate the use of adenovirus vectors for the *in vivo* delivery of nucleic acids encoding a target antigen, or a fragment, a variant, or a variant fragment thereof. In some aspects, the adenovirus vector can further comprise a nucleic acid sequence encoding for an immunological fusion partner. Once injected into a subject, the nucleic acid sequence is expressed resulting in an immune response against the antigen encoded by the sequence. The adenovirus vector vaccine can be administered in an "effective amount," that is, an amount of adenovirus vector that is effective in a selected route or routes of administration to elicit an immune response as described elsewhere herein. An effective amount can induce an immune response effective to facilitate protection or treatment of the host against the target infectious agent or cancer. The amount of vector in each vaccine dose is selected as an amount which induces an immune, immunoprotective or other immunotherapeutic response without significant adverse effects generally associated with typical vaccines. Once vaccinated, subjects may be monitored to determine the efficacy of the vaccine treatment. Monitoring the efficacy of vaccination may be performed by any method known to a person of ordinary skill in the art. In some embodiments, blood or fluid samples may be assayed to detect levels of antibodies. In other embodiments, ELISpot assays may be performed to detect a cell-mediated immune response from circulating blood cells or from lymphoid tissue cells.

In certain embodiments, between 1 and 10 doses may be administered over a 52 week period. In certain embodiments, 6 doses are administered, at intervals of 1, 2, 3 weeks, 1, 2, 3, 4, 5, 6, 7, 8, 9, 11, 12 months or any range or value derivable therefrom, and further booster vaccinations may be given periodically thereafter, at intervals of 1, 2, 3 weeks, 1, 2, 3, 4, 5, 6, 7, 8, 9, 11, 12 months or any range or value derivable therefrom. Alternate protocols may be appropriate for individual patients. As such, 1, 2, 3, 4, 5, 6, 7, 8, 9, 10, 11, 12, 13, 14, 15, 16, 17, 18, 19, 20, or more doses may be administered over a 1 year period or over shorter or longer periods, such as over 35, 40, 45, 50, 55, 60, 65, 70, 75, 80, 85, 90, 95 or 100 week periods. Doses may be administered at 1, 2, 3, 4, 5, or 6 week intervals or longer intervals.

A vaccine can be infused over a period of less than about 4 hours, and more preferably, over a period of less than about 3 hours. For example, the first 25-50 mg could be infused within 30 minutes, preferably even 15 min, and the remainder infused over the next 2-3 hrs. More generally, the dosage of an administered vaccine construct may be administered as one dosage every 2 or 3 weeks, repeated for a total of at least 3 dosages. Or, the construct may be administered twice per week for 4-6 weeks. The dosing schedule can optionally be repeated at other intervals and dosage may be given through various parenteral routes, with appropriate adjustment of the dose and schedule. Compositions as described herein can be administered to a patient in conjunction with (e.g., before, simultaneously, or following) any number of relevant treatment modalities.

A suitable dose is an amount of an adenovirus vector that, when administered as described above, is capable of promoting a target antigen immune response as described elsewhere herein. In certain embodiments, the immune response is at least 10-50% above the basal (i.e., untreated) level. In certain embodiments, the immune response is at least 2, 3, 4, 5, 6, 7, 8, 9, 10, 12, 15, 20, 25, 30, 35, 40, 45, 50, 55, 60, 65, 70, 75, 80, 85, 90, 100, 110, 125, 150, 200, 250, 300, 400, 500 or more over the basal level. Such response can be monitored by measuring the target antigen(s) antibodies in a patient or by vaccine-dependent generation of cytolytic effector cells capable of killing patient tumor or infected cells in vitro, or other methods known in the art for monitoring immune responses. Such vaccines should also be capable of causing an immune response that leads to an improved clinical outcome of the disease in question in vaccinated patients as compared to non-vaccinated patients. In some embodiments, the improved clinical outcome comprises treating disease, reducing the symptoms of a disease, changing the progression of a disease, or extending life.

Any of the compositions provided herein may be administered to an individual. "Individual" may be used interchangeably with "subject" or "patient." An individual may be a mammal, for example a human or animal such as a non-human primate, a rodent, a rabbit, a rat, a mouse, a horse, a donkey, a goat, a cat, a dog, a cow, a pig, or a sheep. In embodiments, the individual is a human. In embodiments, the individual is a fetus, an embryo, or a child. In some cases, the compositions provided herein are administered to a cell *ex vivo.* In some cases, the compositions provided herein are administered to an individual as a method of treating a disease or disorder. In some embodiments, the individual has a genetic disease. In some cases, the individual is at risk of having the disease, such as any of the diseases described herein. In some embodiments, the individual is at increased risk of having a disease or disorder caused by insufficient amount of a protein or insufficient activity of a protein. If an individual is "at an increased risk" of having a disease or disorder, the method involves preventative or prophylactic treatment. For example, an individual can be at an increased risk of having such a disease or disorder because of family history of the disease. Typically, individuals at an increased risk of having such a disease or disorder benefit from prophylactic treatment (e.g., by preventing or delaying the onset or progression of the disease or disorder).

In some cases, a subject does not have a disease. In some cases, the treatment as described herein is administered before onset of a disease. A subject may have undetected disease. A subject may have a low disease burden. A subject may also have a high disease burden. In certain cases, a subject may be administered a treatment as described herein according to a grading scale. A grading scale can be a Gleason classification. A Gleason classification reflects how different tumor tissue is from normal prostate tissue. It uses a scale from 1 to 5. A physician gives a cancer a number based on the patterns and growth of the cancer cells. The lower the number, the more normal the cancer cells look and the lower the grade. The higher the number, the less normal the cancer cells look and the higher the grade. In certain cases, a treatment may be administered to a patient with a low Gleason score. Preferably, a patient with a Gleason score of 3 or below may be administered a treatment as described herein.

Various embodiments relate to compositions and methods for raising an immune response against one or more particular target antigens in selected patient populations. Accordingly, methods and compositions as described herein may target patients with a cancer including but not limited to carcinomas or sarcomas such as neurologic cancers, melanoma, non-Hodgkin's lymphoma, Hodgkin's disease, leukemia, plasmocytomas, adenomas, gliomas, thymomas, breast cancer, prostate cancer, colorectal cancer, kidney cancer, renal cell carcinoma, uterine cancer, pancreatic cancer, esophageal cancer, lung cancer, ovarian cancer, cervical cancer, testicular cancer, gastric cancer, multiple myeloma, hepatoma, acute lymphoblastic leukemia (ALL), acute myelogenous leukemia (AML), chronic myelogenous leukemia (CML), and chronic lymphocytic leukemia (CLL), or other cancers can be targeted for therapy. In some cases, the targeted patient population may be limited to individuals having colorectal adenocarcinoma, metastatic colorectal cancer, advanced MUC1, MUC1c, MUC1n, T, or CEA expressing colorectal cancer, head and neck cancer, liver cancer, breast cancer, lung cancer, bladder cancer, or pancreas cancer. A histologically confirmed diagnosis of a selected cancer, for example colorectal adenocarcinoma, may be used. A particular disease stage or progression may be selected, for example, patients with one or more of a metastatic, recurrent, stage III, or stage IV cancer may be selected for therapy with the methods and compositions as described herein. In some embodiments, patients may be required to have received and, optionally, progressed through other therapies including but not limited to fluoropyrimidine, irinotecan, oxaliplatin, bevacizumab, cetuximab, or panitumumab containing therapies. In some cases, individual's refusal to accept such therapies may allow the patient to be included in a therapy eligible pool with methods and compositions as described herein. In some embodiments, individuals to receive therapy using the methods and compositions as described herein may be required to have an estimated life expectancy of at least, 1, 2, 3, 4, 5, 6, 7, 8, 9, 10, 11, 12, 14, 15, 18, 21, or 24 months. The patient pool to receive a therapy using the methods and compositions as described herein may be limited by age. For example, individuals who are older than 2, 3, 4, 5, 6, 7, 8, 9, 10, 11, 12, 13, 14, 15, 16, 17, 18, 19, 20, 21, 25, 30, 35, 40, 50, 60, or more years old can be eligible for therapy with methods and compositions as described herein. For another example, individuals who are younger than 75, 70, 65, 60, 55, 50, 40, 35, 30, 25, 20, or fewer years old can be eligible for therapy with methods and compositions as described herein.

In some embodiments, patients receiving therapy using the methods and compositions as described herein are limited to individuals with adequate hematologic function, for example with one or more of a WBC count of at least 1000, 1500, 2000, 2500, 3000, 3500, 4000, 4500, 5000 or more per microliter, a hemoglobin level of at least 5, 6, 7, 8, 9, 10, 11, 12, 13, 14 or higher g/dL, a platelet count of at least 50,000; 60,000; 70,000; 75,000; 90,000; 100,000; 110,000; 120,000; 130,000; 140,000; 150,000 or more per microliter; with a PT-INR value of less than or equal to 0.8, 1.0, 1.2, 1.3, 1.4, 1.5, 1.6, 1.8, 2.0, 2.5, 3.0, or higher, a PTT value of less than or equal to 1.2, 1.4, 1.5, 1.6, 1.8, 2.0 X ULN or more. In various embodiments, hematologic function indicator limits are chosen differently for individuals in different gender and age groups, for example 0-5, 5-10, 10-15, 15-18, 18-21, 21-30, 30-40, 40-50, 50-60, 60-70, 70-80 or older than 80.

In some embodiments, patients receiving therapy using the methods and compositions as described herein are limited to individuals with adequate renal and/or hepatic function, for example with one or more of a serum creatinine level of less than or equal to 0.8, 0.9, 1.0, 1.1, 1.2, 1.3, 1.4, 1.5, 1.6, 1.7, 1.8, 1.9, 2.0, 2.1, 2.2 mg/dL or more, a bilirubin level of .8, 0.9, 1.0, 1.1, 1.2, 1.3, 1.4, 1.5, 1.6, 1.7, 1.8, 1.9, 2.0, 2.1, 2.2 mg/dL or more, while allowing a higher limit for Gilbert's syndrome, for example, less than or equal to 1.5, 1.6, 1.8, 1.9, 2.0, 2.1, 2.2, 2.3, or 2.4 mg/dL, an ALT and AST value of less than or equal to less than or equal to 1.5, 2.0, 2.5, 3.0 x upper limit of normal (ULN) or more. In various embodiments, renal or hepatic function indicator limits are chosen differently for individuals in different gender and age groups, for example 0-5, 5-10, 10-15, 15-18, 18-21, 21-30, 30-40, 40-50, 50-60, 60-70, 70-80 or older than 80.

In some embodiments, the K-ras mutation status of individuals who are candidates for a therapy using the methods and compositions as described herein can be determined. Individuals with a preselected K-ras mutational status can be included in an eligible patient pool for therapies using the methods and compositions as described herein.

In various embodiments, patients receiving therapy using the methods and compositions as described herein are limited to individuals without concurrent cytotoxic chemotherapy or radiation therapy, a history of, or current, brain metastases, a history of autoimmune disease, such as but not restricted to, inflammatory bowel disease, systemic lupus erythematosus, ankylosing spondylitis, scleroderma, multiple sclerosis, thyroid disease and vitiligo, serious intercurrent chronic or acute illness, such as cardiac disease (NYHA class III or IV), or hepatic disease, a medical or psychological impediment to probable compliance with the protocol, concurrent (or within the last 5 years) second malignancy other than non-melanoma skin cancer, cervical carcinoma *in situ,* controlled superficial bladder cancer, or other carcinoma *in situ* that has been treated, an active acute or chronic infection including: a urinary tract infection, HIV (e.g., as determined by ELISA and confirmed by Western Blot), and chronic hepatitis, or concurrent steroid therapy (or other immuno-suppressive drugs, such as azathioprine or cyclosporin A). In some cases, patients with at least 3, 4, 5, 6, 7, 8, 9, or 10 weeks of discontinuation of any steroid therapy (except that used as pre-medication for chemotherapy or contrast-enhanced studies) may be included in a pool of eligible individuals for therapy using the methods and compositions as described herein. In some embodiments, patients receiving therapy using the methods and compositions o as described herein include individuals with thyroid disease and vitiligo.

In various embodiments, samples, for example serum or urine samples, from the individuals or candidate individuals for a therapy using the methods and compositions as described herein may be collected. Samples may be collected before, during, and/or after the therapy for example, within 2, 4, 6, 8, 10 weeks prior to the start of the therapy, within 1 week, 10 day, 2 weeks, 3 weeks, 4 weeks, 6 weeks, 8 weeks, or 12 weeks from the start of the therapy, within 2, 4, 6, 8, 10 weeks prior to the start of the therapy, within 1 week, 10 day, 2 weeks, 3 weeks, 4 weeks, 6 weeks, 8 weeks, 9 weeks, or 12 weeks from the start of the therapy, in 1 week, 10 day, 2 weeks, 3 weeks, 4 weeks, 6 weeks, 8 weeks, 9 weeks, or 12 weeks intervals during the therapy, in 1 month, 3 month, 6 month, 1 year, 2 year intervals after the therapy, within 1 month, 3 months, 6 months, 1 year, 2 years, or longer after the therapy, for a duration of 6 months, 1, 2, 3, 4, 5, 6, 7, 8, 9, 10 years or longer. The samples may be tested for any of the hematologic, renal, or hepatic function indicators described herein as well as suitable others known in the art, for example a β-HCG for women with childbearing potential. In that regard, hematologic and biochemical tests, including cell blood counts with differential, PT, INR and PTT, tests measuring Na, K, Cl, CO₂, BUN, creatinine, Ca, total protein, albumin, total bilirubin, alkaline phosphatase, AST, ALT, and glucose are contemplated in certain aspects. In some embodiments, the presence or the amount of HIV antibody, Hepatitis BsAg, or Hepatitis C antibody are determined in a sample from individuals or candidate individuals for a therapy using the methods and compositions described herein.

Biological markers, such as antibodies to target antigens or the neutralizing antibodies to Ad5 vector can be tested in a sample, such as serum, from individuals or candidate individuals for a therapy using the methods and compositions described herein. In some cases, one or more samples, such as a blood sample can be collected and archived from an individuals or candidate individuals for a therapy using the methods and compositions described herein. Collected samples can be assayed for immunologic evaluation. Individuals or candidate individuals for a therapy using the methods and compositions described herein can be evaluated in imaging studies, for example using CT scans or MRI of the chest, abdomen, or pelvis. Imaging studies can be performed before, during, or after therapy using the methods and compositions described herein, during, and/or after the therapy, for example, within 2, 4, 6, 8, 10 weeks prior to the start of the therapy, within 1 week, 10 day, 2 weeks, 3 weeks, 4 weeks, 6 weeks, 8 weeks, or 12 weeks from the start of the therapy, within 2, 4, 6, 8, 10 weeks prior to the start of the therapy, within 1 week, 10 day, 2 weeks, 3 weeks, 4 weeks, 6 weeks, 8 weeks, 9 weeks, or 12 weeks from the start of the therapy, in 1 week, 10 day, 2 week, 3 week, 4 week, 6 week, 8 week, 9 week, or 12 week intervals during the therapy, in 1 month, 3 month, 6 month, 1 year, 2 year intervals after the therapy, within 1 month, 3 months, 6 months, 1 year, 2 years, or longer after the therapy, for a duration of 6 months, 1, 2, 3, 4, 5, 6, 7, 8, 9, 10 years, or longer.

Compositions and methods described herein contemplate various dosage and administration regimens during therapy. Patients may receive one or more replication-defective adenovirus or adenovirus vector, for example Ad5 [E1-, E2B-]-vectors comprising a target antigen that is capable of raising an immune response in an individual against a target antigen described herein. In some aspects, the Ad5 [E1-, E2B-]-vectors can further comprise a nucleic acid sequence encoding for an immunological fusion partner.

In certain embodiments, the replication-defective adenovirus is administered at a dose that suitable for effecting or enhancing such immune response. In some cases, the replication-defective adenovirus is administered at a dose that is greater than or equal to 1x10⁹, 2 x10⁹, 3 x10⁹, 4 x10⁹, 5 x10⁹, 6 x10⁹, 7 x10⁹, 8 x10⁹, 9 x10⁹, 1x10¹⁰, 2 x10¹⁰, 3 x10¹⁰, 4 x10¹⁰, 5 x10¹⁰, 6x10¹⁰, 7 x10¹⁰, 8 x10¹⁰, 9 x10¹⁰, 1x10¹¹, 2 x10¹¹, 3 x10¹¹, 4 x10¹¹, 5x10¹¹, 6 x10¹¹, 7 x10¹¹, 8x10¹¹, 9x10¹¹, 1x10¹², 1.5x10¹², 2 x10¹², 3 x10¹², or more virus particles (VP) per immunization. In some cases, the replication-defective adenovirus is administered at a dose that is less than or equal to 1x10⁹, 2x10⁹, 3 x10⁹, 4 x10⁹, 5 x10⁹, 6 x10⁹, 7 x10⁹, 8 x10⁹, 9 x10⁹, 1x10¹⁰, 2 x10¹⁰, 3 x10¹⁰, 4 x10¹⁰, 5 x10¹⁰, 6 x10¹⁰, 7 x10¹⁰, 8 x10¹⁰, 9 x10¹⁰, 1x10¹¹, 2 x10¹¹, 3 x10¹¹, 4 x10¹¹, 5x10¹¹, 6 x10¹¹, 7 x10¹¹, 8 x10¹¹, 9x10¹¹, 1x10¹², 1.5 x10¹², 2 x10¹², 3 x10¹², or more virus particles per immunization. In various embodiments, a desired dose described herein is administered in a suitable volume of formulation buffer, for example a volume of about 0.1-10 mL, 0.2-8mL, 0.3-7mL, 0.4-6 mL, 0.5-5 mL, 0.6-4 mL, 0.7-3 mL, 0.8-2 mL, 0.9-1.5 mL, 0.95-1.2 mL, or 1.0-1.1 mL. Those of skill in the art appreciate that the volume may fall within any range bounded by any of these values (e.g., about 0.5 mL to about 1.1 mL). Administration of virus particles can be through a variety of suitable paths for delivery, for example it can be by injection (e.g., intracutaneously, intramuscularly, intravenously or subcutaneously), intranasally (e.g., by aspiration), in pill form (e.g., swallowing, suppository for vaginal or rectal delivery. In some embodiments, a subcutaneous delivery may be preferred and can offer greater access to dendritic cells.

Administration of virus particles to an individual may be repeated. Repeated deliveries of virus particles may follow a schedule or alternatively, may be performed on an as needed basis. For example, an individual's immunity against a target antigen, for example a tumor antigen, a tumor neo-antigen, a tumor neo-epitope, a tumor-associate antigen, a fragment, a variant, or a variant fragment thereof, may be tested and replenished as necessary with additional deliveries. In some embodiments, schedules for delivery include administrations of virus particles at regular intervals. Joint delivery regimens may be designed comprising one or more of a period with a schedule and/or a period of need based administration assessed prior to administration. For example, a therapy regimen may include an administration, such as subcutaneous administration once every three weeks then another immunotherapy treatment every three months until removed from therapy for any reason including death. Another example regimen comprises three administrations every three weeks then another set of three immunotherapy treatments every three months.

Another example regimen comprises a first period with a first number of administrations at a first frequency, a second period with a second number of administrations at a second frequency, a third period with a third number of administrations at a third frequency, etc., and optionally one or more periods with undetermined number of administrations on an as needed basis. The number of administrations in each period can be independently selected and can for example be 1, 2, 3, 4, 5, 6, 7, 8, 9, 10, 11, 12, 13, 14, 15, 16, 17, 18, 19, 20, or more. The frequency of the administration in each period can also be independently selected, can for example be about every day, every other day, every third day, twice a week, once a week, once every other week, every three weeks, every month, every six weeks, every other month, every third month, every fourth month, every fifth month, every sixth month, once a year etc. The therapy can take a total period of up to 1, 2, 3, 4, 5, 6, 7, 8, 9, 10, 11, 12, 13, 14, 15, 16, 17, 18, 19, 20, 21, 22, 23, 24, 30, 36 months, or more.

The scheduled interval between immunizations may be modified so that the interval between immunizations is revised by up to a fifth, a fourth, a third, or half of the interval. For example, for a 3-week interval schedule, an immunization may be repeated between 20 and 28 days (3 weeks -1 day to 3 weeks +7 days). For the first 3 immunizations, if the second and/or third immunization is delayed, the subsequent immunizations may be shifted allowing a minimum amount of buffer between immunizations. For example, for a three week interval schedule, if an immunization is delayed, the subsequent immunization may be scheduled to occur no earlier than 17, 18, 19, or 20 days after the previous immunization.

Compositions described herein can be provided in various states, for example, at room temperature, on ice, or frozen. Compositions may be provided in a container of a suitable size, for example a vial of 2 mL vial. In one embodiment, 1 2ml vial with 1.0 mL of extractable vaccine contains 5x10¹¹ total virus particles/mL. Storage conditions including temperature and humidity may vary. For example, compositions for use in therapy may be stored at room temperature, 4 °C, -20 °C, or lower.

In various embodiments, general evaluations are performed on the individuals receiving treatment according to the methods and compositions as described herein. One or more of any tests may be performed as needed or in a scheduled basis, such as on weeks 0, 3, 6, etc. A different set of tests may be performed concurrent with immunization vs. at time points without immunization.

General evaluations may include one or more of medical history, ECOG Performance Score, Karnofsky performance status, and complete physical examination with weight by the attending physician. Any other treatments, medications, biologics, or blood products that the patient is receiving or has received since the last visit may be recorded. Patients may be followed at the clinic for a suitable period, for example approximately 30 minutes, following receipt of vaccine to monitor for any adverse reactions.

In certain embodiments, local and systemic reactogenicity after each dose of vaccine may be assessed daily for a selected time, for example for 3 days (on the day of immunization and 2 days thereafter). Diary cards may be used to report symptoms and a ruler may be used to measure local reactogenicity. Immunization injection sites may be assessed. CT scans or MRI of the chest, abdomen, and pelvis may be performed.

In various embodiments, hematological and biochemical evaluations are performed on the individuals receiving treatment according to the methods and compositions as described herein. One or more of any tests may be performed as needed or in a scheduled basis, such as on weeks 0, 3, 6, etc. A different set of tests may be performed concurrent with immunization vs. at time points without immunization. Hematological and biochemical evaluations may include one or more of blood test for chemistry and hematology, CBC with differential, Na, K, Cl, CO₂, BUN, creatinine, Ca, total protein, albumin, total bilirubin, alkaline phosphatase, AST, ALT, glucose, and ANA.

In various embodiments, biological markers are evaluated on individuals receiving treatment according to the methods and compositions as described herein. One or more of any tests may be performed as needed or in a scheduled basis, such as on weeks 0, 3, 6, etc. A different set of tests may be performed concurrent with immunization vs. at time points without immunization.

Biological marker evaluations may include one or more of measuring antibodies to target antigens or viral vectors described herein, from a serum sample of adequate volume, for example about 5ml Biomarkers may be reviewed if determined and available.

In various embodiments, an immunological assessment is performed on individuals receiving treatment according to the methods and compositions as described herein. One or more of any tests may be performed as needed or in a scheduled basis, such as on weeks 0, 3, 6, etc. A different set of tests may be performed concurrent with immunization vs. at time points without immunization.

Peripheral blood, for example about 90 mL may be drawn prior to each immunization and at a time after at least some of the immunizations, to determine whether there is an effect on the immune response at specific time points during the study and/or after a specific number of immunizations. Immunological assessment may include one or more of assaying peripheral blood mononuclear cells (PBMC) for T-cell responses to target antigens using ELISpot, proliferation assays, multi-parameter flow cytometric analysis, and cytoxicity assays. Serum from each blood draw may be archived and sent and determined.

In various embodiments, a tumor assessment is performed on individuals receiving treatment according to the methods and compositions as described herein. One or more of any tests may be performed as needed or in a scheduled basis, such as prior to treatment, on weeks 0, 3, 6, etc. A different set of tests may be performed concurrent with immunization vs. at time points without immunization. Tumor assessment may include one or more of CT or MRI scans of chest, abdomen, or pelvis performed prior to treatment, at a time after at least some of the immunizations and at approximately every three months following the completion of a selected number, for example 2, 3, or 4, of first treatments and for example until removal from treatment.

Immune responses against a target antigen described herein, such as tumor neo-epitopes or neo-antigens, may be evaluated from a sample, such as a peripheral blood sample of an individual using one or more suitable tests for immune response, such as ELISpot, cytokine flow cytometry, or antibody response. A positive immune response can be determined by measuring a T-cell response. A T-cell response can be considered positive if the mean number of spots adjusted for background in six wells with antigen exceeds the number of spots in six control wells by 10 and the difference between single values of the six wells containing antigen and the six control wells is statistically significant at a level of p≤0.05 using the Student's t-test. Immunogenicity assays may occur prior to each immunization and at scheduled time points during the period of the treatment. For example, a time point for an immunogenicity assay at around week 1, 2, 3, 4, 5, 6, 7, 8, 9, 10, 11, 12, 13, 14, 15, 18, 20 , 24, 30, 36, or 48 of a treatment may be scheduled even without a scheduled immunization at this time. In some cases, an individual may be considered evaluable for immune response if they receive at least a minimum number of immunizations, for example 1, 2, 3, 4, 5, 6, 7, 8, 9, or more immunizations.

In some embodiments, disease progression or clinical response determination is made according to the RECIST 1.1 criteria among patients with measurable/evaluable disease. In some embodiments, therapies using the methods and compositions as described herein affect a Complete Response (CR; disappearance of all target lesions for target lesions or disappearance of all non-target lesions and normalization of tumor marker level for non-target lesions) in an individual receiving the therapy. In some embodiments, therapies using the methods and compositions as described herein affect a Partial Response (PR; at least a 30% decrease in the sum of the LD of target lesions, taking as reference the baseline sum LD for target lesions) in an individual receiving the therapy.

In some embodiments, therapies using the methods and compositions as described herein affect a Stable Disease (SD; neither sufficient shrinkage to qualify for PR nor sufficient increase to qualify for PD, taking as reference the smallest sum LD since the treatment started for target lesions) in an individual receiving the therapy. In some embodiments, therapies using the methods and compositions described herein affect an Incomplete Response/ Stable Disease (SD; persistence of one or more non-target lesion(s) or/and maintenance of tumor marker level above the normal limits for non-target lesions) in an individual receiving the therapy. In some embodiments, therapies using the methods and compositions as described herein affect a Progressive Disease (PD; at least a 20% increase in the sum of the LD of target lesions, taking as reference the smallest sum LD recorded since the treatment started or the appearance of one or more new lesions for target lesions or persistence of one or more non-target lesion(s) or/and maintenance of tumor marker level above the normal limits for non-target lesions) in an individual receiving the therapy.

### VI. Vectors

Certain aspects include transferring into a cell an expression construct comprising one or more nucleic acid sequences encoding one or more tumor neo-epitopes or neo-antigens. In some aspects, the expression construct can further comprise a nucleic acid sequence encoding for an immunological fusion partner. In certain embodiments, transfer of an expression construct into a cell may be accomplished using a viral vector. A viral vector may be used to include those constructs containing viral sequences sufficient to express a recombinant gene construct that has been cloned therein.

Disclosed herein, can be a composition comprising a library of vectors that target a plurality of tumor neo-antigens or neo-epitopes. A library of vectors can target all identified neo-epitopes of a cancer. In some cases, a library of vectors can be used to treat a patient. A library of vectors can more aggressively target a heterogeneous tumor expressing multiple neo-epitopes. In some cases, a library of vectors is at least two vectors. A library of vectors can be more than two vectors. A library of vectors can comprise or can comprise about 1, 2, 3, 4, 5, 6, 7, 8, 9, 10, 11, 12, 13, 14, 15, 16, 17, 18, 19, 20, or more vectors targeting different neo-epitopes. A library of vectors can target driver mutations and passenger mutations simultaneously. In some cases, a library of vectors is designed according to a patient's tumor landscape. In some cases, a vector in the library of vectors can further comprise a nucleic acid sequence encoding for an immunological fusion partner.

In particular embodiments, the viral vector is an adenovirus vector. Adenoviruses are a family of DNA viruses characterized by an icosahedral, non-enveloped capsid containing a linear double-stranded genome. Of the human adenoviruses, none are associated with any neoplastic disease, and only cause relatively mild, self-limiting illness in immunocompetent individuals.

Adenovirus vectors may have low capacity for integration into genomic DNA. Adenovirus vectors may result in highly efficient gene transfer. Additional advantages of adenovirus vectors include that they are efficient at gene delivery to both nondividing and dividing cells and can be produced in large quantities.

In contrast to integrating viruses, the adenoviral infection of host cells may not result in chromosomal integration because adenoviral DNA can replicate in an episomal manner without potential genotoxicity. Also, adenovirus vectors may be structurally stable, and no genome rearrangement has been detected after extensive amplification. Adenovirus is particularly suitable for use as a gene transfer vector because of its mid- sized genome, ease of manipulation, high titer, wide target-cell range and high infectivity.

The first genes expressed by the virus are the E1 genes, which act to initiate high-level gene expression from the other Ad5 gene promoters present in the wild type genome. Viral DNA replication and assembly of progeny virions occur within the nucleus of infected cells, and the entire life cycle takes about 36 hr with an output of approximately 10⁴ virions per cell.

The wild type Ad5 genome is approximately 36 kb, and encodes genes that are divided into early and late viral functions, depending on whether they are expressed before or after DNA replication. The early/late delineation is nearly absolute, since it has been demonstrated that super-infection of cells previously infected with an Ad5 results in lack of late gene expression from the super-infecting virus until after it has replicated its own genome. Without being bound by theory, this is likely due to a replication dependent *cis-*activation of the Ad5 major late promoter (MLP), preventing late gene expression (primarily the Ad5 capsid proteins) until replicated genomes are present to be encapsulated. The composition and methods may take advantage of these features in the development of advanced generation Ad vectors/vaccines.

The adenovirus vector may be replication-defective, or at least conditionally defective. The adenovirus may be of any of the 42 different known serotypes or subgroups A-F and other serotypes or subgroups are envisioned. Adenovirus type 5 of subgroup C may be used in particular embodiments in order to obtain a replication- defective adenovirus vector. This is because Adenovirus type 5 is a human adenovirus about which a great deal of biochemical and genetic information is known, and it has historically been used for most constructions employing adenovirus as a vector.

Adenovirus growth and manipulation is known to those of skill in the art, and exhibits broad host range *in vitro* and *in vivo.* Modified viruses, such as adenoviruses with alteration of the CAR domain, may also be used. Methods for enhancing delivery or evading an immune response, such as liposome encapsulation of the virus, are also envisioned.

The vector may comprise a genetically engineered form of adenovirus, such as an E2 deleted adenoviral vector, or more specifically, an E2b deleted adenoviral vector. The term "E2b deleted," as used herein, refers to a specific DNA sequence that is mutated in such a way so as to prevent expression and/or function of at least one E2b gene product. Thus, in certain embodiments, "E2b deleted" refers to a specific DNA sequence that is deleted (removed) from the Ad genome. E2b deleted or "containing a deletion within the E2b region" refers to a deletion of at least one base pair within the E2b region of the Ad genome. In certain embodiments, more than one base pair is deleted and in further embodiments, at least 20, 30, 40, 50, 60, 70, 80, 90, 100, 110, 120, 130, 140, or 150 base pairs are deleted. In another embodiment, the deletion is of more than 150, 160, 170, 180, 190, 200, 250, or 300 base pairs within the E2b region of the Ad genome. An E2b deletion may be a deletion that prevents expression and/or function of at least one E2b gene product and therefore, encompasses deletions within exons encoding portions of E2b-specific proteins as well as deletions within promoter and leader sequences. In certain embodiments, an E2b deletion is a deletion that prevents expression and/or function of one or both of the DNA polymerase and the preterminal protein of the E2b region. In a further embodiment, "E2b deleted" refers to one or more point mutations in the DNA sequence of this region of an Ad genome such that one or more encoded proteins is non-functional. Such mutations include residues that are replaced with a different residue leading to a change in the amino acid sequence that result in a nonfunctional protein.

As would be understood by the skilled artisan upon reading the present disclosure, other regions of the Ad genome can be deleted. Thus to be "deleted" in a particular region of the Ad genome, as used herein, refers to a specific DNA sequence that is mutated in such a way so as to prevent expression and/or function of at least one gene product encoded by that region. In certain embodiments, to be "deleted" in a particular region refers to a specific DNA sequence that is deleted (removed) from the Ad genome in such a way so as to prevent the expression and/or the function encoded by that region (e.g., E2b functions of DNA polymerase or preterminal protein function). "Deleted" or "containing a deletion" within a particular region refers to a deletion of at least one base pair within that region of the Ad genome.

Thus, in certain embodiments, more than one base pair is deleted and in further embodiments, at least 20, 30, 40, 50, 60, 70, 80, 90, 100, 110, 120, 130, 140, or 150 base pairs are deleted from a particular region. In another embodiment, the deletion is more than 150, 160, 170, 180, 190, 200, 250, or 300 base pairs within a particular region of the Ad genome. These deletions are such that expression and/or function of the gene product encoded by the region is prevented. Thus deletions encompass deletions within exons encoding portions of proteins as well as deletions within promoter and leader sequences. In a further embodiment, "deleted" in a particular region of the Ad genome refers to one or more point mutations in the DNA sequence of this region of an Ad genome such that one or more encoded proteins is non-functional. Such mutations include residues that are replaced with a different residue leading to a change in the amino acid sequence that result in a nonfunctional protein.

In certain embodiments, the adenovirus vectors contemplated for use include E2b deleted adenovirus vectors that have a deletion in the E2b region of the Ad genome and, optionally, the E1 region. In some cases, such vectors do not have any other regions of the Ad genome deleted.

In another embodiment, the adenovirus vectors contemplated for use include E2b deleted adenovirus vectors that have a deletion in the E2b region of the Ad genome and, optionally, deletions in the E1 and E3 regions. In some cases, such vectors have no other regions deleted.

In a further embodiment, the adenovirus vectors contemplated for use include adenovirus vectors that have a deletion in the E2b region of the Ad genome and, optionally, deletions in the E1, E3 and, also optionally, partial or complete removal of the E4 regions. In some cases, such vectors have no other deletions.

In another embodiment, the adenovirus vectors contemplated for use include adenovirus vectors that have a deletion in the E2b region of the Ad genome and, optionally deletions in the E1 and/or E4 regions. In some cases, such vectors contain no other deletions.

In an additional embodiment, the adenovirus vectors contemplated for use include adenovirus vectors that have a deletion in the E2a, E2b and/or E4 regions of the Ad genome. In some cases, such vectors have no other deletions.

In one embodiment, the adenovirus vectors for use herein comprise vectors having the E1 and/or DNA polymerase functions of the E2b region deleted. In some cases, such vectors have no other deletions.

In a further embodiment, the adenovirus vectors for use herein have the E1 and/or the preterminal protein functions of the E2b region deleted. In some cases, such vectors have no other deletions.

In another embodiment, the adenovirus vectors for use herein have the E1, DNA polymerase and/or the preterminal protein functions deleted. In some cases, such vectors have no other deletions. In one particular embodiment, the adenovirus vectors contemplated for use herein are deleted for at least a portion of the E2b region and/or the E1 region.

In some cases, such vectors are not "gutted" adenovirus vectors. In this regard, the vectors may be deleted for both the DNA polymerase and the preterminal protein functions of the E2b region. In an additional embodiment, the adenovirus vectors for use include adenovirus vectors that have a deletion in the E1, E2b and/or 100K regions of the adenovirus genome. In certain embodiments, the adenovirus vector may be a "gutted" adenovirus vector.

In one embodiment, the adenovirus vectors for use herein comprise vectors having the E1, E2b and/or protease functions deleted. In some cases, such vectors have no other deletions.

In a further embodiment, the adenovirus vectors for use herein have the E1 and/or the E2b regions deleted, while the fiber genes have been modified by mutation or other alterations (e.g., to alter Ad tropism). Removal of genes from the E3 or E4 regions may be added to any of the mentioned adenovirus vectors.

The deleted adenovirus vectors can be generated using recombinant techniques known in the art (see e.g., Amalfitano, et al. J. Virol. 1998; 72:926-33; Hodges, et al. J Gene Med 2000; 2:250-59). As would be recognized by the skilled artisan, the adenovirus vectors for use in certain aspects can be successfully grown to high titers using an appropriate packaging cell line that constitutively expresses E2b gene products and products of any of the necessary genes that may have been deleted. In certain embodiments, HEK-293-derived cells that not only constitutively express the E1 and DNA polymerase proteins, but also the Ad-preterminal protein, can be used. In one embodiment, E.C7 cells are used to successfully grow high titer stocks of the adenovirus vectors (see e.g., Amalfitano, et al. J. Virol. 1998; 72:926-33; Hodges, et al. J Gene Med 2000; 2:250-59)

In order to delete critical genes from self-propagating adenovirus vectors, the proteins encoded by the targeted genes may be coexpressed in HEK-293 cells, or similar, along with the E1 proteins. Therefore, only those proteins which are non-toxic when coexpressed constitutively (or toxic proteins inducibly- expressed) can be utilized. Coexpression in HEK-293 cells of the E1 and E4 genes has been demonstrated (utilizing inducible, not constitutive, promoters) (Yeh, et al. J. Virol. 1996; 70:559; Wang et al. Gene Therapy 1995; 2:775; and Gorziglia, et al. J. Virol. 1996; 70:4173). The E1 and protein IX genes (a virion structural protein) have been coexpressed (Caravokyri, et al. J. Virol. 1995; 69: 6627), and coexpression of the E1, E4, and protein IX genes has also been described (Krougliak, et al. Hum. Gene Ther. 1995; 6:1575). The E1 and 100k genes have been successfully expressed in transcomplementing cell lines, as have E1 and protease genes (Oualikene, et al. Hum Gene Ther 2000; 11:1341-53; Hodges, et al. J. Virol 2001; 75:5913-20).

Cell lines coexpressing E1 and E2b gene products for use in growing high titers of E2b deleted Ad particles are described in U.S. Patent No. 6,063,622. The E2b region encodes the viral replication proteins which are absolutely required for Ad genome replication (Doerfler, et al. Chromosoma 1992; 102:S39-S45). Useful cell lines constitutively express the approximately 140 kDa Ad- DNA polymerase and/or the approximately 90 kDa preterminal protein. In particular, cell lines that have high-level, constitutive coexpression of the E1, DNA polymerase, and preterminal proteins, without toxicity (e.g., E.C7), are desirable for use in propagating Ad for use in multiple vaccinations. These cell lines permit the propagation of adenovirus vectors deleted for the E1, DNA polymerase, and preterminal proteins.

The recombinant Ad can be propagated using techniques known in the art. For example, in certain embodiments, tissue culture plates containing E.C7 cells are infected with the adenovirus vector virus stocks at an appropriate MOI (e.g., 5) and incubated at 37.0 °C for 40-96 h. The infected cells are harvested, resuspended in 10 mM Tris-CI (pH 8.0), and sonicated, and the virus is purified by two rounds of cesium chloride density centrifugation. In certain techniques, the virus containing band is desalted over a Sephadex CL-6B column (Pharmacia Biotech, Piscataway, NJ.), sucrose or glycerol is added, and aliquots are stored at -80 °C. In some embodiments, the virus will be placed in a solution designed to enhance its stability, such as A195 (Evans, et al. J Pharm Sci 2004; 93:2458-75). The titer of the stock is measured (e.g., by measurement of the optical density at 260 nm of an aliquot of the virus after SDS lysis). In another embodiment, plasmid DNA, either linear or circular, encompassing the entire recombinant E2b deleted adenovirus vector can be transfected into E.C7, or similar cells, and incubated at 37.0 °C until evidence of viral production is present (e.g., the cytopathic effect). The conditioned media from these cells can then be used to infect more E.C7, or similar cells, to expand the amount of virus produced, before purification. Purification can be accomplished by two rounds of cesium chloride density centrifugation or selective filtration. In certain embodiments, the virus may be purified by column chromatography, using commercially available products (e.g., Adenopure from Puresyn, Inc., Malvem, PA) or custom made chromatographic columns.

In certain embodiments, the recombinant adenovirus vector may comprise enough of the virus to ensure that the cells to be infected are confronted with a certain number of viruses. Thus, there may be provided a stock of recombinant Ad, for example in an RCA-free stock of recombinant Ad. The preparation and analysis of Ad stocks can use any methods available in the art. Viral stocks vary considerably in titer, depending largely on viral genotype and the protocol and cell lines used to prepare them. The viral stocks can have a titer of at least about 10⁶, 10⁷, or 10⁸ pfu/ml, and many such stocks can have higher titers, such as at least about 10⁹, 10¹⁰, 10¹¹, or 10¹² pfu/ml.

Certain aspects contemplate the use of E2b deleted adenovirus vectors, such as those described in U.S. Pat. Nos. 6,063,622; 6,451,596; 6,057,158; 6,083,750; and 8,298,549. The vectors with deletions in the E2b regions in many cases cripple viral protein expression and/or decrease the frequency of generating replication competent Ad (RCA).

Propagation of these E2b deleted adenovirus vectors can be done utilizing cell lines that express the deleted E2b gene products. Certain aspects also provide such packaging cell lines; for example E.C7 (formally called C-7), derived from the HEK-293 cell line.

In further aspects, the E2b gene products, DNA polymerase and preterminal protein, can be constitutively expressed in E.C7, or similar cells along with the E1 gene products. Transfer of gene segments from the Ad genome to the production cell line has immediate benefits: (1) increased carrying capacity; and, (2) a decreased potential of RCA generation, typically requiring two or more independent recombination events to generate RCA. The E1, Ad DNA polymerase and/or preterminal protein expressing cell lines used herein can enable the propagation of adenovirus vectors with a carrying capacity approaching 13 kb, without the need for a contaminating helper virus. In addition, when genes critical to the viral life cycle are deleted (e.g., the E2b genes), a further crippling of Ad to replicate or express other viral gene proteins occurs. This can decrease immune recognition of virally infected cells, and allow for extended durations of foreign transgene expression.

E1, DNA polymerase, and preterminal protein deleted vectors are typically unable to express the respective proteins from the E1 and E2b regions. Further, they may show a lack of expression of most of the viral structural proteins. For example, the major late promoter (MLP) of Ad is responsible for transcription of the late structural proteins L1 through L5. Though the MLP is minimally active prior to Ad genome replication, the highly toxic Ad late genes are primarily transcribed and translated from the MLP only after viral genome replication has occurred. This cis-dependent activation of late gene transcription is a feature of DNA viruses in general, such as in the growth of polyoma and SV-40. The DNA polymerase and preterminal proteins are important for Ad replication (unlike the E4 or protein IX proteins). Their deletion can be extremely detrimental to adenovirus vector late gene expression, and the toxic effects of that expression in cells such as APCs.E1-deleted adenovirus vectors

Certain aspects contemplate the use of E1-deleted adenovirus vectors. First generation, or E1-deleted adenovirus vectors Ad5 [E1-] are constructed such that a transgene replaces only the E1 region of genes. Typically, about 90% of the wild-type Ad5 genome is retained in the vector. Ad5 [E1-] vectors have a decreased ability to replicate and cannot produce infectious virus after infection of cells not expressing the Ad5 E1 genes. The recombinant Ad5 [E1-] vectors are propagated in human cells (typically 293 cells) allowing for Ad5 [E1-] vector replication and packaging. Ad5 [E1-] vectors have a number of positive attributes; one of the most important is their relative ease for scale up and cGMP production. Currently, well over 220 human clinical trials utilize Ad5 [E1-] vectors, with more than two thousand subjects given the virus subcutaneously, intramuscularly, or intravenously.

Additionally, Ad5 vectors do not integrate; their genomes remain episomal. Generally, for vectors that do not integrate into the host genome, the risk for insertional mutagenesis and/or germ-line transmission is extremely low if at all. Conventional Ad5 [E1-] vectors have a carrying capacity that approaches 7kb.

Studies in humans and animals have demonstrated that pre-existing immunity against Ad5 can be an inhibitory factor to commercial use of Ad-based vaccines. The preponderance of humans have antibody against Ad5, the most widely used subtype for human vaccines, with two-thirds of humans studied having lympho-proliferative responses against Ad5. This pre-existing immunity can inhibit immunization or re-immunization using typical Ad5 vaccines and may preclude the immunization of a vaccine against a second antigen, using an Ad5 vector, at a later time. Overcoming the problem of pre-existing anti-vector immunity has been a subject of intense investigation. Investigations using alternative human (non-Ad5 based) Ad5 subtypes or even non-human forms of Ad5 have been examined. Even if these approaches succeed in an initial immunization, subsequent vaccinations may be problematic due to immune responses to the novel Ad5 subtype.

To avoid the Ad5 immunization barrier, and improve upon the limited efficacy of first generation Ad5 [E1-] vectors to induce optimal immune responses, there are provided certain embodiments related to a next generation Ad5 vector based vaccine platform. The next generation Ad5 platform has additional deletions in the E2b region, removing the DNA polymerase and the preterminal protein genes. The Ad5 [E1-, E2b-] platform has an expanded cloning capacity that is sufficient to allow inclusion of many possible genes. Ad5 [E1-, E2b-] vectors have up to about 12 kb gene-carrying capacity as compared to the 7 kb capacity of Ad5 [E1-] vectors, providing space for multiple genes if needed. In some embodiments, an insert of more than 1, 2, 3, 4, 5, 6, 7, 8, 9, 10, or 11 kb is introduced into an Ad5 vector, such as the Ad5 [E1-, E2b-] vector.

Deletion of the E2b region may confer advantageous immune properties on the Ad5 vectors, often eliciting potent immune responses to target transgene antigens, such as tumor neo-epitopes, while minimizing the immune responses to Ad viral proteins.

In various embodiments, Ad5 [E1-, E2b-] vectors may induce a potent CMI, as well as antibodies against the vector expressed target antigens, such as tumor neo-epitopes or neo-epitopes, even in the presence of Ad immunity.

Ad5 [E1-, E2b-] vectors also have reduced adverse reactions as compared to Ad5 [E1-] vectors, in particular the appearance of hepatotoxicity and tissue damage.

Certain aspects of these Ad5 vectors are that expression of Ad late genes is greatly reduced. For example, production of the capsid fiber proteins could be detected *in vivo* for Ad5 [E1-] vectors, while fiber expression was ablated from Ad5 [E1-, E2b-] vector vaccines. The innate immune response to wild type Ad is complex. Proteins deleted from the Ad5 [E1-, E2b-] vectors generally play an important role. Specifically, Ad5 [E1-, E2b-] vectors with deletions of preterminal protein or DNA polymerase display reduced inflammation during the first 24 to 72 hours following injection compared to Ad5 [E1-] vectors. In various embodiments, the lack of Ad5 gene expression renders infected cells invisible to anti-Ad activity and permits infected cells to express the transgene for extended periods of time, which develops immunity to the target.

Various embodiments contemplate increasing the capability for the Ad5 [E1-, E2b-] vectors to transduce dendritic cells, improving antigen specific immune responses in the vaccine by taking advantage of the reduced inflammatory response against Ad5 [E1-, E2b-] vector viral proteins and the resulting evasion of pre-existing Ad immunity.

In some cases, this immune induction may take months. Ad5 [E1-, E2b-] vectors not only are safer than, but appear to be superior to Ad5 [E1-] vectors in regard to induction of antigen specific immune responses, making them much better suitable as a platform to deliver tumor vaccines that can result in a clinical response.

In certain embodiments, methods and compositions are provided by taking advantage of an Ad5 [E1-, E2b-] vector system for developing a therapeutic tumor vaccine that overcomes barriers found with other Ad5 systems and permits the immunization of people who have previously been exposed to Ad5.

E2b deleted vectors may have up to a 13 kb gene-carrying capacity as compared to the 5 to 6 kb capacity of First Generation adenovirus vectors, easily providing space for nucleic acid sequences encoding any of a variety of target antigens, such as tumor neo-epitopes or neo-antigens. In some aspects, the E2b deleted vectors can further provide space for a nucleic acid sequence encoding for an immunological fusion partner.

The E2b deleted adenovirus vectors also have reduced adverse reactions as compared to First Generation adenovirus vectors. E2b deleted vectors have reduced expression of viral genes, and this characteristic leads to extended transgene expression *in vivo.*

Compared to first generation adenovirus vectors, certain embodiments of the Second Generation E2b deleted adenovirus vectors contain additional deletions in the DNA polymerase gene (pol) and deletions of the pre-terminal protein (pTP).

It appears that Ad proteins expressed from adenovirus vectors play an important role. Specifically, the deletions of pre-terminal protein and DNA polymerase in the E2b deleted vectors appear to reduce inflammation during the first 24 to 72 hours following injection, whereas First Generation adenovirus vectors stimulate inflammation during this period.

In addition, it has been reported that the additional replication block created by E2b deletion also leads to a 10,000-fold reduction in expression of Ad late genes, well beyond that afforded by E1, E3 deletions alone. The decreased levels of Ad proteins produced by E2b deleted adenovirus vectors effectively reduce the potential for competitive, undesired, immune responses to Ad antigens, responses that prevent repeated use of the platform in Ad immunized or exposed individuals.

The reduced induction of inflammatory response by second generation E2b deleted vectors results in increased potential for the vectors to express desired vaccine antigens, such as tumor neo-epitopes, during the infection of antigen presenting cells (i.e., dendritic cells), decreasing the potential for antigenic competition, resulting in greater immunization of the vaccine to the desired antigen relative to identical attempts with First Generation adenovirus vectors.

E2b deleted adenovirus vectors provide an improved Ad-based vaccine candidate that is safer, more effective, and more versatile than previously described vaccine candidates using First Generation adenovirus vectors.

Thus, first generation, E1-deleted Adenovirus subtype 5 (Ad5)-based vectors, although promising platforms for use as vaccines, may be impeded in activity by naturally occurring or induced Ad-specific neutralizing antibodies.

Without being bound by theory, Ad5-based vectors with deletions of the E1 and the E2b regions (Ad5 [E1-, E2b-]), the latter encoding the DNA polymerase and the pre-terminal protein, for example by virtue of diminished late phase viral protein expression, may avoid immunological clearance and induce more potent immune responses against the encoded antigen transgene, such as tumor neo-antigens or neo-epitopes, in Ad-immune hosts.

### VII. Target Antigens

In certain aspects, there may be provided expression constructs or vectors comprising nucleic acid sequences that encode one or more target proteins of interest or target antigens, such as tumor epitopes or tumor neo-epitopes. In this regard, there may be provided expression constructs or vectors that may contain nucleic acid encoding at least, at most or about one, two, three, four, five, six, seven, eight, nine, ten, 11, 12, 13, 14, 15, 16, 17, 18, 19, 20, 30, 40, 50, 60, 70, 80, 90, 100, 200, 300, 400, or 500 different target antigens of interest or any number or ranges derived therefrom. The expression constructs or vectors may contain nucleic acid sequences encoding multiple fragments or epitopes from one target protein of interest or may contain one or more fragments or epitopes from numerous different target neo-epitope antigen proteins of interest. In some aspects, the adenovirus vector can further comprise a nucleic acid sequence encoding for an immunological fusion partner.

In particular embodiments, target antigens may be tumor neo-antigens or tumor neo-epitopes. Cancers can acquire tens to hundreds of somatic mutations (termed the "tumor mutome") during their development. Each of these mutations has the potential to generate one or more novel T-cell neo-antigens and "neo-epitopes" uniquely specific to each individual patient's tumor. Because these neo-epitopes are not present in the germline, and are not encountered until after the onset of oncogenesis, repertoires of high-avidity T cells can be capable of recognizing them and may avoid central tolerance and escape deletion in the thymus. In certain aspects, the tumor-specific mutations may be used as an attractive source of antigenic targets for developing patient-specific tumor vaccines.

In some cases a tumor neo-epitope can be 8 to 10 amino acids long. In some cases a neo-epitope is four to ten amino acids long or over 10 amino acids long. A neo-epitope can comprise a length of or can comprise a length of at least, about, or at most 1, 2, 3, 4, 5, 6, 7, 8, 9, 10, 11, 12, 13, 14, 15, 16, 17, 18, 19, 20 amino acids, or any number or ranges derived therefrom. A neo-epitope can be any length of amino acids.

A target neo-antigens or neo-epitope can be derived from a cancer mutation. A cancer mutation can yield multiple target neo-epitopes. For example, a cancer mutation can generate 38 different peptides that could potentially bind to an HLA class I molecule to produce a targetable neo-epitope. In some cases, a peptide may be proteolytically exposed, but not destroyed, be chaperoned into an endoplasmic reticulum, and if capable bind to MHC class I to be delivered to the cell surface for T-cell recognition. In some cases, epitopes are longer and can be processed differently, but also must be exposed and not destroyed, and can have affinity for HLA class II molecules instead of HLA class I.

In additional aspects, a target neo-antigen or neo-epitope can be produced in a tumor cell in response to a tumor therapy. Disclosed herein can be the generation of a pool of previously identified mutant tumor neo-epitopes recognized by patient CD8 T cells in association with improved clinical responses. Neo-epitopes can consist of missense mutations and frameshift mutations representing different human cancer types, including both solid and hematologic tumors. In some cases, a neo-epitope can be any mutation. In some cases, a mutation can be a missense mutation. Additionally, in other cases a mutation can be a frameshift mutation.

In some cases, enrichment for vaccine targets that are processed and presented by antigen-presenting cells and presented on HLA by the tumor is performed. In some cases, possible targets are screened by affinity to HLA. In one embodiment, peptides that can bind to HLA class I or II provide eligible vaccine targets. HLA can be of any class. In some cases, HLA class I is used. In other cases, HLA class II is used.

One possible method for selecting vaccine targets is to choose candidate neo-epitopes based on their predicted affinities for the HLA molecules expressed by a patient. A patient can have any HLA type. In some cases, HLA-binding affinity prediction algorithms (Parker KC, et al. J Immunol 1994; 152:163-75) can be used to identify suitable tumor neo-epitopes.

In certain embodiments, the target antigen such as neo-epitopes may bind to an MHC class I or class II molecule. As used herein, a target antigen is said to "bind to" an MHC class I or class II molecule if such binding is detectable using any assay known in the art. For example, the ability of a polypeptide to bind to MHC class I may be evaluated indirectly by monitoring the ability to promote incorporation of 1251 labeled β2-microglobulin (β2m) into MHC class 1/β2m/peptide heterotrimeric complexes (see Parker, et al. J. Immunol. 752:163, 1994). Alternatively, functional peptide competition assays may be employed.

The target antigens may be a full length protein or may be an immunogenic fragment (e.g., an epitope) thereof. Immunogenic fragments may be identified using available techniques, such as those summarized in Paul, Fundamental Immunology, 3rd ed., 243-247 (Raven Press, 1993) and references cited therein. Representative techniques for identifying immunogenic fragments include screening polypeptides for the ability to react with antigen-specific antisera and/or T-cell lines or clones. An immunogenic fragment of a particular target polypeptide may be a fragment that reacts with such antisera and/or T-cells at a level that is not substantially less than the reactivity of the full-length target polypeptide (e.g., in an ELISA and/or T-cell reactivity assay). In other words, an immunogenic fragment may react within such assays at a level that is similar to or greater than the reactivity of the full-length polypeptide. Such screens may generally be performed using methods available to those of ordinary skill in the art, such as those described in Harlow and Lane, Antibodies: A Laboratory Manual, Cold Spring Harbor Laboratory, 1988.

Target antigens may include but not be limited to antigens of any cancer. In particular embodiments, a neo-epitope can be targeted. A neo-epitope can be targeted with a vaccine. In some cases, an adeno-virus based vaccine can be used. In some cases, a neo-antigen is targeted. A neo-epitope can be comprised within a neo-antigen. A neo-antigen may comprise multiple neo-epitopes.

In certain aspects, targets antigens can be a tumor cell epitope, neo-antigens or neo-epitopes, tumor-associated antigens or epitopes, or a combination thereof. An antigen can be a tumor cell antigen. An epitope can be a tumor cell epitope. A tumor cell epitope may be derived from a wide variety of tumor antigens such as antigens from tumors resulting from mutations, shared tumor specific antigens, differentiation antigens, and antigens overexpressed in tumors.

Tumor neo-epitopes as used herein are tumor-specific epitopes, such as EQVWGMAVR or CQGPEQVWGMAVREL (R346W mutation of FLRT2), GETVTMPCP or NVGETVTMPCPKVFS (V73M mutation of VIPR2), GLGAQCSEA or NNGLGAQCSEAVTLN (R286C mutation of FCRL1), RKLTTELTI, LGPERRKLTTELTII, or PERRKLTTE (S1613L mutation of FAT4), MDWVWMDTT, AVMDWVWMDTTLSLS, or VWMDTTLSL (T2356M mutation of PIEZO2), GKTLNPSQT, SWFREGKTLNPSQTS, or REGKTLNPS (A292T mutation of SIGLEC14), VRNATSYRC, LPNVTVRNATSYRCG, or NVTVRNATS (D1143N mutation of SIGLEC1), FAMAQIPSL, PFAMAQIPSLSLRAV, or AQIPSLSLR (Q678P mutation of SLC4A11). Non-limiting examples of the nucleotide sequences encoding tumor neo-epitopes are shown in the **Table 3** in Example 9.

Tumor-associated antigens may be antigens not normally expressed by the host; they can be mutated, truncated, misfolded, or otherwise abnormal manifestations of molecules normally expressed by the host; they can be identical to molecules normally expressed but expressed at abnormally high levels; or they can be expressed in a context or environment that is abnormal. Tumor-associated antigens may be, for example, proteins or protein fragments, complex carbohydrates, gangliosides, haptens, nucleic acids, other biological molecules or any combinations thereof.

Additional non-limiting examples of target antigens include carcinoembryonic antigen (CEA), folate receptor alpha, WT1, brachyury (TIVS7-2, polymorphism), brachyury (IVS7 T/C polymorphism), T brachyury, T, hTERT, hTRT, iCE, HPV E6, HPV E7, BAGE, DAM-6, -10, GAGE-1, -2, -8, GAGE-3, -4, -5, -6, -7B, NA88-A, NY-ESO-1, MART-1, MC1R, Gp100, PSA, PSMA, PSCA, STEAP, PAP, Tyrosinase, TRP-1, TRP-2, ART-4, CAMEL, Cyp-B, EGFR, Her2/neu, Her3, MUC1, MUC1 (VNTR polymorphism), MUC1-c, MUC1-n, MUC1, MUC2, PRAME, P15, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPI/mbcr-abl, ETV6/AML, LDLR/FUT, Pml/RARα, TEL/AML1, human epidermal growth factor receptor 2 (HER2/neu), human epidermal growth factor receptor 3 (HER3), Human papillomavirus (HPV), Prostate-specific antigen (PSA), alpha-actinin-4, ARTC1, CAR-ABL fusion protein (b3a2), B-RAF, CASP-5, CASP-8, beta-catenin, Cdc27, CDK4, CDKN2A, COA-1, dek-can fusion protein, EFTUD2, Elongation factor 2, ETV6-AML1 fusion protein, FLT3-ITD, FN1, GPNMB, LDLR-fucosyltransferase fusion protein, HLA-A2d, HLA-A1 1d, hsp70-2, KIAAO205, MART2, ME1, neo-PAP, Myosin class I, NFYC, OGT, OS-9, pml-RARalpha fusion protein, PRDX5, PTPRK, K-ras, N-ras, RBAF600, SIRT2, SNRPD1, SYT-SSX1- or -SSX2 fusion protein, TGF-betaRII, triosephosphate isomerase, BAGE-1, GAGE-1, 2, 8, Gage 3, 4, 5, 6, 7, GnTVf, HERV-K-MEL, KK-LC-1, KM-HN-1, LAGE-1, MAGE-A1, MAGE-A2, MAGE-A3, MAGE-A4, MAGE-A6, MAGE-A9, MAGE-A10, MAGE-A12, MAGE-C2, mucink, NA-88, NY-ESO-1/LAGE-2, SAGE, Sp17, SSX-2, SSX-4, TAG-1, TAG-2, TRAG-3, TRP2-INT2g, XAGE-1b, gp100/Pmel17, Kallikrein 4, mammaglobin-A, Melan-A/MART-1, NY-BR-1, OA1, PSA, RAB38/NY-MEL-1, TRP-1/gp75, TRP-2, tyrosinase, adipophilin, AIM-2, ALDH1A1, BCLX (L), BCMA, BING-4, CPSF, cyclin D1, DKK1, ENAH (hMena), EP-CAM, EphA3, EZH2, FGF5, G250/MN/CAIX, HER-2/neu, IL13Ralpha2, intestinal carboxyl esterase, alpha fetoprotein, M-CSFT, MCSP, mdm-2, MMP-2, MUC1, p53, PBF, PRAME, PSMA, RAGE-1, RGS5, RNF43, RU2AS, secernin 1, SOX10, STEAP1, survivin, Telomerase, VEGF, or any combination thereof. In some embodiments, CEA can comprise a sequence that has at least 80%, at least 85%, at least 90%, at least 92%, at least 95%, or at least 99% sequence identity to ATGGAGTCTCCCTCGGCCCCTCCCCACAGATGGTGCATCCCCTGGCAGAGGCTCC TGCTCACAGCCTCACTTCTAACCTTCTGGAACCCGCCCACCACTGCCAAGCTCAC TATTGAATCCACGCCGTTCAATGTCGCAGAGGGGAAGGAGGTGCTTCTACTTGTC CACAATCTGCCCCAGCATCTTTTTGGCTACAGCTGGTACAAAGGTGAAAGAGTGG ATGGCAACCGTCAAATTATAGGATATGTAATAGGAACTCAACAAGCTACCCCAG GGCCCGCATACAGTGGTCGAGAGATAATATACCCCAATGCATCCCTGCTGATCCA GAACATCATCCAGAATGACACAGGATTCTACACCCTACACGTCATAAAGTCAGA TCTTGTGAATGAAGAAGCAACTGGCCAGTTCCGGGTATACCCGGAGCTGCCCAA GCCCTCCATCTCCAGCAACAACTCCAAACCCGTGGAGGACAAGGATGCTGTGGC CTTCACCTGTGAACCTGAGACTCAGGACGCAACCTACCTGTGGTGGGTAAACAAT CAGAGCCTCCCGGTCAGTCCCAGGCTGCAGCTGTCCAATGGCAACAGGACCCTC ACTCTATTCAATGTCACAAGAAATGACACAGCAAGCTACAAATGTGAAACCCAG AACCCAGTGAGTGCCAGGCGCAGTGATTCAGTCATCCTGAATGTCCTCTATGGCC CGGATGCCCCCACCATTTCCCCTCTAAACACATCTTACAGATCAGGGGAAAATCT GAACCTCTCCTGCCACGCAGCCTCTAACCCACCTGCACAGTACTCTTGGTTTGTC AATGGGACTTTCCAGCAATCCACCCAAGAGCTCTTTATCCCCAACATCACTGTGA ATAATAGTGGATCCTATACGTGCCAAGCCCATAACTCAGACACTGGCCTCAATAG GACCACAGTCACGACGATCACAGTCTATGCAGAGCCACCCAAACCCTTCATCAC CAGCAACAACTCCAACCCCGTGGAGGATGAGGATGCTGTAGCCTTAACCTGTGA ACCTGAGATTCAGAACACAACCTACCTGTGGTGGGTAAATAATCAGAGCCTCCC GGTCAGTCCCAGGCTGCAGCTGTCCAATGACAACAGGACCCTCACTCTACTCAGT GTCACAAGGAATGATGTAGGACCCTATGAGTGTGGAATCCAGAACGAATTAAGT GTTGACCACAGCGACCCAGTCATCCTGAATGTCCTCTATGGCCCAGACGACCCCA CCATTTCCCCCTCATACACCTATTACCGTCCAGGGGTGAACCTCAGCCTCTCCTGC CATGCAGCCTCTAACCCACCTGCACAGTATTCTTGGCTGATTGATGGGAACATCC AGCAACACACACAAGAGCTCTTTATCTCCAACATCACTGAGAAGAACAGCGGAC TCTATACCTGCCAGGCCAATAACTCAGCCAGTGGCCACAGCAGGACTACAGTCA AGACAATCACAGTCTCTGCGGAGCTGCCCAAGCCCTCCATCTCCAGCAACAACTC CAAACCCGTGGAGGACAAGGATGCTGTGGCCTTCACCTGTGAACCTGAGGCTCA GAACACAACCTACCTGTGGTGGGTAAATGGTCAGAGCCTCCCAGTCAGTCCCAG GCTGCAGCTGTCCAATGGCAACAGGACCCTCACTCTATTCAATGTCACAAGAAAT GACGCAAGAGCCTATGTATGTGGAATCCAGAACTCAGTGAGTGCAAACCGCAGT GACCCAGTCACCCTGGATGTCCTCTATGGGCCGGACACCCCCATCATTTCCCCCC CAGACTCGTCTTACCTTTCGGGAGCGGACCTCAACCTCTCCTGCCACTCGGCCTC TAACCCATCCCCGCAGTATTCTTGGCGTATCAATGGGATACCGCAGCAACACACA CAAGTTCTCTTTATCGCCAAAATCACGCCAAATAATAACGGGACCTATGCCTGTT TTGTCTCTAACTTGGCTACTGGCCGCAATAATTCCATAGTCAAGAGCATCACAGT CTCTGCATCTGGAACTTCTCCTGGTCTCTCAGCTGGGGCCACTGTCGGCATCATG ATTGGAGTGCTGGTTGGGGTTGCTCTGATATAG (SEQ ID NO: 106), or a fragment or variant thereof. In some embodiments, MUC1-c can comprise a sequence that has at least 80%, at least 85%, at least 90%, at least 92%, at least 95%, or at least 99% sequence identity to ATGACACCGGGCACCCAGTCTCCTTTCTTCCTGCTGCTGCTCCTCACAGTGCTTAC AGTTGTTACGGGTTCTGGTCATGCAAGCTCTACCCCAGGTGGAGAAAAGGAGAC TTCGGCTACCCAGAGAAGTTCAGTGCCCAGCTCTACTGAGAAGAATGCTGTGAGT ATGACCAGCAGCGTACTCTCCAGCCACAGCCCCGGTTCAGGCTCCTCCACCACTC AGGGACAGGATGTCACTCTGGCCCCGGCCACGGAACCAGCTTCAGGTTCAGCTG CCCTTTGGGGACAGGATGTCACCTCGGTCCCAGTCACCAGGCCAGCCCTGGGCTC CACCACCCCGCCAGCCCACGATGTCACCTCAGCCCCGGACAACAAGCCAGCCCC GGGCTCCACCGCCCCCCCAGCCCACGGTGTCACCTCGTATCTTGACACCAGGCCG GCCCCGGTTTATCTTGCCCCCCCAGCCCATGGTGTCACCTCGGCCCCGGACAACA GGCCCGCCTTGGGCTCCACCGCCCCTCCAGTCCACAATGTCACCTCGGCCTCAGG CTCTGCATCAGGCTCAGCTTCTACTCTGGTGCACAACGGCACCTCTGCCAGGGCT ACCACAACCCCAGCCAGCAAGAGCACTCCATTCTCAATTCCCAGCCACCACTCTG ATACTCCTACCACCCTTGCCAGCCATAGCACCAAGACTGATGCCAGTAGCACTCA CCATAGCACGGTACCTCCTCTCACCTCCTCCAATCACAGCACTTCTCCCCAGTTGT CTACTGGGGTCTCTTTCTTTTTCCTGTCTTTTCACATTTCAAACCTCCAGTTTAATT CCTCTCTGGAAGATCCCAGCACCGACTACTACCAAGAGCTGCAGAGAGACATTT CTGAAATGTTTTTGCAGATTTATAAACAAGGGGGTTTTCTGGGCCTCTCCAATATT AAGTTCAGGCCAGGATCTGTGGTGGTACAATTGACTCTGGCCTTCCGAGAAGGTA CCATCAATGTCCACGACGTGGAGACACAGTTCAATCAGTATAAAACGGAAGCAG CCTCTCGATATAACCTGACGATCTCAGACGTCAGCGTGAGTGATGTGCCATTTCC TTTCTCTGCCCAGTCTGGGGCTGGGGTGCCAGGCTGGGGCATCGCGCTGCTGGTG CTGGTCTGTGTTCTGGTTTATCTGGCCATTGTCTATCTCATTGCCTTGGCTGTCGCT CAGGTTCGCCGAAAGAACTACGGGCAGCTGGACATCTTTCCAGCCCGGGATAAA TACCATCCTATGAGCGAGTACGCTCTTTACCACACCCATGGGCGCTATGTGCCCC CTAGCAGTCTTTTCCGTAGCCCCTATGAGAAGGTTTCTGCAGGTAATGGTGGCAG CTATCTCTCTTACACAAACCCAGCAGTGGCAGCCGCTTCTGCCAACTTGTAG(SEQ ID NO: 107), or a fragment or variant thereof. In some embodiments, Brachyury can comprise a sequence that has at least 80%, at least 85%, at least 90%, at least 92%, at least 95%, or at least 99% sequence identity to ATGAGCTCCCCTGGCACCGAGAGCGCGGGAAAGAGCCTGCAGTACCGAGTGGAC CACCTGCTGAGCGCCGTGGAGAATGAGCTGCAGGCGGGCAGCGAGAAGGGCGA CCCCACAGAGCGCGAACTGCGCGTGGGCCTGGAGGAGAGCGAGCTGTGGCTGCG CTTCAAGGAGCTCACCAATGAGATGATCGTGACCAAGAACGGCAGGAGGATGTT TCCGGTGCTGAAGGTGAACGTGTCTGGCCTGGACCCCAACGCCATGTACTCCTTC CTGCTGGACTTCGTGGCGGCGGACAACCACCGCTGGAAGTACGTGAACGGGGAA TGGGTGCCGGGGGGCAAGCCGGAGCCGCAGGCGCCCAGCTGCGTCTACATCCAC CCCGACTCGCCCAACTTCGGGGCCCACTGGATGAAGGCTCCCGTCTCCTTCAGCA AAGTCAAGCTCACCAACAAGCTCAACGGAGGGGGCCAGATCATGCTGAACTCCT TGCATAAGTATGAGCCTCGAATCCACATAGTGAGAGTTGGGGGTCCACAGCGCA TGATCACCAGCCACTGCTTCCCTGAGACCCAGTTCATAGCGGTGACTGCTAGAAG TGATCACAAAGAGATGATGGAGGAACCCGGAGACAGCCAGCAACCTGGGTACTC CCAATGGGGGTGGCTTCTTCCTGGAACCAGCACCGTGTGTCCACCTGCAAATCCT CATCCTCAGTTTGGAGGTGCCCTCTCCCTCCCCTCCACGCACAGCTGTGACAGGT ACCCAACCCTGAGGAGCCACCGGTCCTCACCCTACCCCAGCCCCTATGCTCATCG GAACAATTCTCCAACCTATTCTGACAACTCACCTGCATGTTTATCCATGCTGCAAT CCCATGACAATTGGTCCAGCCTTGGAATGCCTGCCCATCCCAGCATGCTCCCCGT GAGCCACAATGCCAGCCCACCTACCAGCTCCAGTCAGTACCCCAGCCTGTGGTCT GTGAGCAACGGCGCCGTCACCCCGGGCTCCCAGGCAGCAGCCGTGTCCAACGGG CTGGGGGCCCAGTTCTTCCGGGGCTCCCCCGCGCACTACACACCCCTCACCCATC CGGTCTCGGCGCCCTCTTCCTCGGGATCCCCACTGTACGAAGGGGCGGCCGCGGC CACAGACATCGTGGACAGCCAGTACGACGCCGCAGCCCAAGGCCGCCTCATAGC CTCATGGACACCTGTGTCGCCACCTTCCATGTGA (SEQ ID NO: 108), or fragment or variant thereof.

### VIII. Heterologous Nucleic Acids

In some embodiments, vectors, such as adenovirus vectors, may comprise heterologous nucleic acid sequences that encode one or more tumor antigens such as tumor neo-epitopes or tumor neo-antigens, fusions thereof or fragments thereof, which can modulate the immune response. In certain aspects, there may be provided a Second Generation E2b deleted adenovirus vectors that comprise a heterologous nucleic acid sequence encoding one or more tumor antigens such as tumor neo-epitopes or tumor neo-antigens. In some embodiments, vectors, such as adenovirus vectors, may further comprise heterologous nucleic acid sequences that encode one or more immunological fusion partners, which can modulate the immune response. In certain aspects, there may be provided a Second Generation E2b deleted adenovirus vector that further comprises a heterologous nucleic acid sequence encoding one or more an immunological fusion partner.

As such, there may be provided polynucleotides that encode tumor antigens from any source as described further herein, vectors or constructs comprising such polynucleotides and host cells transformed or transfected with such vectors or expression constructs. Furthermore, there may be provided polynucleotides that encode immunological fusion partners from any source as described further herein, vectors or constructs comprising such polynucleotides and host cells transformed or transfected with such vectors or expression constructs.

The terms "nucleic acid" and "polynucleotide" are used essentially interchangeably herein. As will be also recognized by the skilled artisan, polynucleotides used herein may be single-stranded (coding or antisense) or double-stranded, and may be DNA (genomic, cDNA or synthetic) or RNA molecules. RNA molecules may include HnRNA molecules, which contain introns and correspond to a DNA molecule in a one-to-one manner, and mRNA molecules, which do not contain introns. Additional coding or non-coding sequences may, but need not, be present within a polynucleotide as disclosed herein, and a polynucleotide may, but need not, be linked to other molecules and/or support materials. An isolated polynucleotide, as used herein, means that a polynucleotide is substantially away from other coding sequences. For example, an isolated DNA molecule as used herein does not contain large portions of unrelated coding DNA, such as large chromosomal fragments or other functional genes or polypeptide coding regions. Of course, this refers to the DNA molecule as originally isolated, and does not exclude genes or coding regions later added to the segment through recombination in the laboratory.

As will be understood by those skilled in the art, the polynucleotides can include genomic sequences, extra-genomic and plasmid-encoded sequences and smaller engineered gene segments that express, or may be adapted to express target antigens as described herein, fragments of antigens, peptides and the like. Such segments may be naturally isolated, or modified synthetically by the hand of man.

Polynucleotides may comprise a native sequence (i.e., an endogenous sequence that encodes one or more tumor antigens such as tumor neo-epitopes or tumor neo-antigens or a portion thereof) or may comprise a sequence that encodes a variant or derivative of such a sequence. In certain embodiments, the polynucleotide sequences set forth herein encode one or more tumor antigens such as tumor neo-epitopes or tumor neo-antigens. In some embodiments, polynucleotides represent a novel gene sequence that has been optimized for expression in specific cell types (i.e., human cell lines) that may substantially vary from the native nucleotide sequence or variant but encode a similar protein antigen. Polynucleotides may further comprise a native sequence (i.e., an endogenous sequence that encodes one or more immunological fusion partners or a portion thereof) or may comprise a sequence that encodes a variant or derivative of such a sequence. In certain embodiments, the polynucleotide sequences set forth herein encode one or more immunological fusion partners such as those described in this herein. In some embodiments, polynucleotides represent a novel gene sequence that has been optimized for expression in specific cell types (i.e., human cell lines) that may substantially vary from the native nucleotide sequence or variant but encode a similar protein antigen.

In other related embodiments, there may be provided polynucleotide variants having substantial identity to native sequences encoding one or more tumor antigens such as tumor neo-epitopes or tumor neo-antigens, for example those comprising at least 70, 80, 90, 95, 96, 97, 98, 99% sequence identity or any derivable range or value thereof, particularly at least 75% up to 99% or higher, sequence identity compared to a native polynucleotide sequence encoding one or more tumor antigens such as tumor neo-epitopes or tumor neo-antigens using the methods described herein, (e.g., BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like.

Typically, polynucleotide variants will contain one or more substitutions, additions, deletions and/or insertions, particularly such that the immunogenicity of the epitope of the polypeptide encoded by the variant polynucleotide or such that the immunogenicity of the heterologous target protein is not substantially diminished relative to a polypeptide encoded by the native polynucleotide sequence. As described elsewhere herein, in certain aspects, the polynucleotide variants encode a variant of one or more tumor antigens such as tumor neo-epitopes or tumor neo-antigens, or a fragment (e.g., an epitope) thereof wherein the propensity of the variant polypeptide or fragment (e.g., epitope) thereof to react with antigen-specific antisera and/or T-cell lines or clones is not substantially diminished relative to the native polypeptide. The term "variants" should also be understood to encompass homologous genes of xenogenic origin.

In certain aspects, there may be provided polynucleotides that comprise or consist of at least about 5 up to a 1000 or more contiguous nucleotides encoding a polypeptide, including target protein antigens, as described herein, as well as all intermediate lengths there between. It will be readily understood that "intermediate lengths," in this context, means any length between the quoted values, such as 16, 17, 18, 19, etc.; 21, 22, 23, etc.; 30, 31, 32, etc.; 50, 51, 52, 53, etc.; 100, 101, 102, 103, etc.; 150, 151, 152, 153, etc.; including all integers through 200-500; 500-1,000, and the like. A polynucleotide sequence as described herein may be extended at one or both ends by additional nucleotides not found in the native sequence encoding a polypeptide as described herein, such as an epitope or heterologous target protein. This additional sequence may consist of 1 up 20 nucleotides or more, at either end of the disclosed sequence or at both ends of the disclosed sequence.

The polynucleotides or fragments thereof, regardless of the length of the coding sequence itself, may be combined with other DNA sequences, such as promoters, expression control sequences, polyadenylation signals, additional restriction enzyme sites, multiple cloning sites, other coding segments, and the like, such that their overall length may vary considerably. It is therefore contemplated, in some aspects, that a nucleic acid fragment of almost any length may be employed, with the total length being limited by the ease of preparation and use in the intended recombinant DNA protocol. For example, illustrative polynucleotide segments with total lengths of about 1000, 2000, 3000, 4000, 5000, 6000, 7000, 8000, 9000, 10,000, about 500, about 200, about 100, about 50 base pairs in length, and the like, (including all intermediate lengths) are contemplated to be useful in certain aspects.

When comparing polynucleotide sequences, two sequences are said to be "identical" if the sequence of nucleotides in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, Wl), using default parameters. This program embodies several alignment schemes described in the following references: Dayhoff MO (1978) A model of evolutionary change in proteins - Matrices for detecting distant relationships. In Dayhoff MO (ed.) Atlas of Protein Sequence and Structure, National Biomedical Research Foundation, Washington DC Vol. 5, Suppl. 3, pp. 345- 358; Hein J Unified Approach to Alignment and Phylogenes, pp. 626-645 (1990); Methods in Enzymology vol.183, Academic Press, Inc., San Diego, CA; Higgins, et al. PM CABIOS 1989; 5:151-53; Myers EW, et al. CABIOS 1988; 4:11-17; Robinson ED Comb. Theor 1971; 11A 05; Saitou N, et al. Mol. Biol. Evol. 1987; 4:406-25; Sneath PHA and Sokal RR Numerical Taxonomy - the Principles and Practice of Numerical Taxonomy, Freeman Press, San Francisco, CA (1973); Wilbur WJ, et al. Proc. Natl. Acad., Sci. USA 1983 80:726-30).

Alternatively, optimal alignment of sequences for comparison may be conducted by the local identity algorithm of Smith, et al. Add. APL. Math 1981; 2:482, by the identity alignment algorithm of Needleman, et al. Mol. Biol. 1970 48:443, by the search for similarity methods of Pearson and Lipman, Proc. Natl. Acad. Sci. USA 1988; 85:2444, by computerized implementations of these algorithms (GAP, BESTFIT, BLAST, FASTA, and TFASTA in the Wisconsin Genetics Software Package, Genetics Computer Group (GCG), 575 Science Dr., Madison, Wl), or by inspection.

One example of algorithms that are suitable for determining percent sequence identity and sequence similarity are the BLAST and BLAST 2.0 algorithms, which are described in Altschul et al., Nucl. Acids Res. 1977 25:3389-3402, and Altschul et al. J. Mol. Biol. 1990 215:403-10, respectively. BLAST and BLAST 2.0 can be used, for example with the parameters described herein, to determine percent sequence identity for the polynucleotides. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a word length (W) of 11, and expectation (E) of 10, and the BLOSUM62 scoring matrix (see Henikoff, et al. Proc. Natl. Acad. Sci. USA 1989; 89:10915) alignments, (B) of 50, expectation (E) of 10, M=5, N=-4 and a comparison of both strands.

In some aspects, the "percentage of sequence identity" is determined by comparing two optimally aligned sequences over a window of comparison of at least 20 positions, wherein the portion of the polynucleotide sequence in the comparison window may comprise additions or deletions (i.e., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases occurs in both sequences to yield the number of matched positions, dividing the number of matched positions by the total number of positions in the reference sequence (i.e., the window size) and multiplying the results by 100 to yield the percentage of sequence identity.

It will be appreciated by those of ordinary skill in the art that, as a result of the degeneracy of the genetic code, there are many nucleotide sequences that encode a particular antigen of interest, or fragment thereof, as described herein. Some of these polynucleotides bear minimal homology to the nucleotide sequence of any native gene. Nonetheless, polynucleotides that vary due to differences in codon usage are specifically contemplated.

Further, alleles of the genes comprising the polynucleotide sequences provided herein may also be contemplated. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Therefore, in another embodiment, a mutagenesis approach, such as site-specific mutagenesis, is employed for the preparation of variants and/or derivatives of nucleic acid sequences encoding one or more tumor antigens such as tumor neo-epitopes or tumor neo-antigens, or fragments thereof, as described herein. By this approach, specific modifications in a polypeptide sequence can be made through mutagenesis of the underlying polynucleotides that encode them. These techniques provide a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Mutations may be employed in a selected polynucleotide sequence to improve, alter, decrease, modify, or otherwise change the properties of the polynucleotide itself, and/or alter the properties, activity, composition, stability, or primary sequence of the encoded polypeptide.

Polynucleotide segments or fragments encoding the polypeptides may be readily prepared by, for example, directly synthesizing the fragment by chemical means, as is commonly practiced using an automated oligonucleotide synthesizer. Also, fragments may be obtained by application of nucleic acid reproduction technology, such as the PCR™ technology of U. S. Patent 4,683,202, by introducing selected sequences into recombinant vectors for recombinant production, and by other recombinant DNA techniques generally known to those of skill in the art of molecular biology (see for example, Current Protocols in Molecular Biology, John Wiley and Sons, NY, NY).

In order to express a desired tumor antigen such as tumor neo-epitopes or tumor neo-antigens, polypeptide or fragment thereof, or fusion protein comprising any of the above, as described herein, the nucleotide sequences encoding the polypeptide, or functional equivalents, are inserted into an appropriate vector such as a replication-defective adenovirus vector as described herein using recombinant techniques known in the art. The appropriate vector contains the necessary elements for the transcription and translation of the inserted coding sequence and any desired linkers. In order to express a immunological fusion partner such as those described herein, the nucleotide sequences encoding the polypeptide, or functional equivalents, are inserted into an appropriate vector such as a replication-defective adenovirus vector as described herein using recombinant techniques known in the art. The appropriate vector contains the necessary elements for the transcription and translation of the inserted coding sequence and any desired linkers.

Methods that are available to those skilled in the art may be used to construct these vectors containing sequences encoding one or more tumor antigens such as tumor neo-epitopes or tumor neo-antigens and appropriate transcriptional and translational control elements. These methods include *in vitro* recombinant DNA techniques, synthetic techniques, and *in vivo* genetic recombination. Such techniques are described, for example, in Amalfitano, et al. J. Virol. 1998; 72:926-33; Hodges, et al. J Gene Med 2000; 2:250-259; Sambrook J, et al. (1989) Molecular Cloning, A Laboratory Manual, Cold Spring Harbor Press, Plainview, N.Y., and Ausubel FM, et al. (1989) Current Protocols in Molecular Biology, John Wiley & Sons, New York. N.Y. In some aspects, these methods can also be used for constructing vectors further comprising a nucleic acid sequence encoding for an immunological fusion partner.

A variety of vector/host systems may be utilized to contain and produce polynucleotide sequences. These include, but are not limited to, microorganisms such as bacteria transformed with recombinant bacteriophage, plasmid, or cosmid DNA vectors; yeast transformed with yeast vectors; insect cell systems infected with virus vectors (e.g., baculovirus); plant cell systems transformed with virus vectors (e.g., cauliflower mosaic virus, CaMV; tobacco mosaic virus, TMV) or with bacterial vectors (e.g., Ti or pBR322 plasmids); or animal cell systems.

The "control elements" or "regulatory sequences" present in a vector, such as an adenovirus vector, are those non-translated regions of the vector - enhancers, promoters, 5' and 3' untranslated regions - which interact with host cellular proteins to carry out transcription and translation. Such elements may vary in their strength and specificity. Depending on the vector system and host utilized, any number of suitable transcription and translation elements, including constitutive and inducible promoters, may be used. For example, sequences encoding one or more tumor antigens such as tumor neo-epitopes or tumor neo-antigens may be ligated into an Ad transcription/translation complex consisting of the late promoter and tripartite leader sequence. As another example, sequences encoding one or more immunological fusion partners may be ligated into an Ad transcription/translation complex consisting of the late promoter and tripartite leader sequence. Insertion in a non-essential E1 or E3 region of the viral genome may be used to obtain a viable virus that is capable of expressing the polypeptide in infected host cells (Logan J, et al. Proc. Natl. Acad. Sci 1984; 87:3655-59). In addition, transcription enhancers, such as the Rous sarcoma virus (RSV) enhancer, may be used to increase expression in mammalian host cells.

Specific initiation signals may also be used to achieve more efficient translation of sequences encoding one or more tumor antigens such as tumor neo-epitopes or tumor neo-antigens and/or sequences encoding one or more immunological fusion partners. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers that are appropriate for the particular cell system which is used, such as those described in the literature (Scharf D., et al. Results Probl. Cell Differ. 1994; 20:125-62). Specific termination sequences, either for transcription or translation, may also be incorporated in order to achieve efficient translation of the sequence encoding the polypeptide of choice.

A variety of protocols for detecting and measuring the expression of polynucleotide-encoded products (e.g., one or more tumor antigens such as tumor neo-epitopes or tumor neo-antigens), using either polyclonal or monoclonal antibodies specific for the product are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on a given polypeptide may be used in some applications, but a competitive binding assay may also be employed. These and other assays are described, among other places, in Hampton R et al. (1990; Serological Methods, a Laboratory Manual, APS Press, St Paul. Minn.) and Maddox DE, et al. J. Exp. Med. 1983; 758:1211-16).

In certain embodiments, elements that increase the expression of the desired tumor antigens such as tumor neo-epitopes or tumor neo-antigens and/or that increase the expression of the desired immunological fusion partner may be incorporated into the nucleic acid sequence of expression constructs or vectors such as adenovirus vectors described herein. Such elements include internal ribosome binding sites (IRES; Wang, et al. Curr. Top. Microbiol. Immunol 1995; 203:99; Ehrenfeld, et al. Curr. Top. Microbiol. Immunol. 1995; 203:65; Rees, et al. Biotechniques 1996; 20:102; Sugimoto, et al. Biotechnology 1994; 2:694). IRES increase translation efficiency. As well, other sequences may enhance expression. For some genes, sequences especially at the 5' end inhibit transcription and/or translation. These sequences are usually palindromes that can form hairpin structures. Any such sequences in the nucleic acid to be delivered are generally deleted. Expression levels of the transcript or translated product are assayed to confirm or ascertain which sequences affect expression. Transcript levels may be assayed by any known method, including Northern blot hybridization, RNase probe protection and the like. Protein levels may be assayed by any known method, including ELISA.

As would be recognized by a skilled artisan, vectors, such as adenovirus vectors described herein, that comprise heterologous nucleic acid sequences can be generated using recombinant techniques known in the art, such as those described in Maione, et al. Proc Natl Acad Sci USA 2001; 98:5986-91; Maione, et al. Hum Gene Ther 2000 1:859-68; Sandig, et al. Proc Natl Acad Sci USA, 2000; 97:1002-07; Harui, et al. Gene Therapy 2004; 11:1617-26; Parks et al. Proc Natl Acad Sci USA 1996; 93:13565-570; DelloRusso, et al. Proc Natl Acad Sci USA 2002; 99:12979-984; Current Protocols in Molecular Biology, John Wiley and Sons, NY, NY).

### IX. Pharmaceutical Compositions

In certain aspects, there may be provided pharmaceutical compositions that comprise nucleic acid sequences encoding one or more one or more tumor antigens such as tumor neo-epitopes or tumor neo-antigens against which an immune response is to be generated. For example, tumor antigens may include, but are not limited to, tumor neo-antigens identified on solid or liquid tumors. Tumor neo-antigens may be identified by any suitable means. In some aspects, these pharmaceutical compositions further comprise a nucleic acid sequence encoding for an immunological fusion partner.

For example, the adenovirus vector stock described herein may be combined with an appropriate buffer, physiologically acceptable carrier, excipient or the like. In certain embodiments, an appropriate number of adenovirus vector particles are administered in an appropriate buffer, such as, sterile PBS. In certain circumstances, it will be desirable to deliver the adenovirus vector compositions disclosed herein parenterally, intratumorally, intravenously, intramuscularly, or even intraperitoneally.

In certain embodiments, solutions of the pharmaceutical compositions as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. In other embodiments, E2b deleted adenovirus vectors may be delivered in pill form, delivered by swallowing or by suppository.

Illustrative pharmaceutical forms suitable for injectable use include sterile aqueous solutions or dispersions and sterile powders for the extemporaneous preparation of sterile injectable solutions or dispersions (for example, see U. S. Patent 5,466,468). In all cases the form must be sterile and must be fluid to the extent that easy syringability exists. It must be stable under the conditions of manufacture and storage and must be preserved against the contaminating action of microorganisms, such as bacteria, molds and fungi.

The carrier can be a solvent or dispersion medium containing, for example, water, lipids, ethanol, polyol (e.g., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution may be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biology standards.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

Routes and frequency of administration of the therapeutic compositions described herein, as well as dosage, will vary from individual to individual, and from disease to disease, and may be readily established using standard techniques. In general, the pharmaceutical compositions and vaccines may be administered by injection (e.g., intracutaneous, intramuscular, intravenous or subcutaneous), intranasally (e.g., by aspiration), in pill form (e.g., swallowing, suppository for vaginal or rectal delivery). In certain embodiments, between 1 and 3 doses may be administered over a 6 week period and further booster vaccinations may be given periodically thereafter.

For example, a suitable dose is an amount of an adenovirus vector that, when administered as described above, is capable of promoting a target antigen immune response as described elsewhere herein. In certain embodiments, the immune response is at least 10-50% above the basal (i.e., untreated) level. Such response can be monitored by measuring the target antigen antibodies in a patient or by vaccine-dependent generation of cytolytic effector cells capable of killing tumor neo-epitope expressing cells *in vitro,* or other methods known in the art for monitoring immune responses.

In general, an appropriate dosage and treatment regimen provides the adenovirus vectors in an amount sufficient to provide prophylactic benefit. Protective immune responses may generally be evaluated using standard proliferation, cytotoxicity or cytokine assays, which may be performed using samples obtained from a patient before and after immunization (vaccination).

In certain aspects, the actual dosage amount of a composition administered to a patient or subject can be determined by physical and physiological factors such as body weight, severity of condition, the type of disease being treated, previous or concurrent therapeutic interventions, idiopathy of the patient and on the route of administration. The practitioner responsible for administration will, in any event, determine the concentration of active ingredient(s) in a composition and appropriate dose(s) for the individual subject.

While one advantage of compositions and methods described herein is the capability to administer multiple vaccinations with the same adenovirus vectors, particularly in individuals with preexisting immunity to Ad, the adenoviral vaccines described herein may also be administered as part of a prime and boost regimen. A mixed modality priming and booster inoculation scheme may result in an enhanced immune response. Thus, one aspect is a method of priming a subject with a plasmid vaccine, such as a plasmid vector comprising nucleic acid sequences encoding one or more tumor antigens such as tumor neo-epitopes or tumor neo-antigens, by administering the plasmid vaccine at least one time, allowing a predetermined length of time to pass, and then boosting by administering the adenovirus vector described herein. Another aspect is a method of priming a subject with a plasmid vaccine, such as the plasmid vector further comprising nucleic acid sequences encoding one or more immunological fusion partners, by administering the plasmid vaccine at least one time, allowing a predetermined length of time to pass, and then boosting by administering the adenovirus vector described herein.

Multiple primings, e.g., 1-3, may be employed, although more may be used. The length of time between priming and boost may typically vary from about six months to a year, but other time frames may be used.

In certain embodiments, pharmaceutical compositions may comprise, for example, at least about 0.1% of therapeutic agents, such as the expression constructs or vectors used herein as vaccine, a related lipid nanovesicle, or an exosome or nanovesicle loaded with therapeutic agents. In other embodiments, the therapeutic agent may comprise between about 2% to about 75% of the weight of the unit, or between about 25% to about 60%, for example, and any range derivable therein. In other non-limiting examples, a dose may also comprise from about 1 microgram/kg/body weight, about 5 microgram/kg/body weight, about 10 microgram/kg/body weight, about 50 microgram/kg/body weight, about 100 microgram/kg/body weight, about 200 microgram/kg/body weight, about 350 microgram/kg/body weight, about 500 microgram/kg/body weight, about 1 milligram/kg/body weight, about 5 milligram/kg/body weight, about 10 milligram/kg/body weight, about 50 milligram/kg/body weight, about 100 milligram/kg/body weight, about 200 milligram/kg/body weight, about 350 milligram/kg/body weight, about 500 milligram/kg/body weight, to about 1000 mg/kg/body weight or more per administration, and any range derivable therein. In non-limiting examples of a derivable range from the numbers listed herein, a range of about 5 microgram/kg/body weight to about 100 mg/kg/body weight, about 5 microgram/kg/body weight to about 500 milligram/kg/body weight, etc., can be administered.

An effective amount of the pharmaceutical composition is determined based on the intended goal. The term "unit dose" or "dosage" refers to physically discrete units suitable for use in a subject, each unit containing a predetermined-quantity of the pharmaceutical composition calculated to produce the desired responses discussed above in association with its administration, i.e., the appropriate route and treatment regimen. The quantity to be administered, both according to number of treatments and unit dose, depends on the protection or effect desired.

Precise amounts of the pharmaceutical composition also depend on the judgment of the practitioner and are peculiar to each individual. Factors affecting the dose include the physical and clinical state of the patient, the route of administration, the intended goal of treatment (e.g., alleviation of symptoms versus cure) and the potency, stability and toxicity of the particular therapeutic substance.

In certain aspects, compositions comprising a vaccination regime can be administered either alone or together with a pharmaceutically acceptable carrier or excipient, by any routes, and such administration can be carried out in both single and multiple dosages. More particularly, the pharmaceutical composition can be combined with various pharmaceutically acceptable inert carriers in the form of tablets, capsules, lozenges, troches, hand candies, powders, sprays, aqueous suspensions, injectable solutions, elixirs, syrups, and the like. Such carriers include solid diluents or fillers, sterile aqueous media and various non-toxic organic solvents, etc. Moreover, such oral pharmaceutical formulations can be suitably sweetened and/or flavored by means of various agents of the type commonly employed for such purposes. The compositions described throughout can be formulated into a pharmaceutical medicament and be used to treat a human or mammal, in need thereof, diagnosed with a disease, e.g., cancer, or to enhances an immune response.

In certain embodiments, the viral vectors or compositions described herein may be administered in conjunction with one or more immunostimulants, such as an adjuvant. An immunostimulant refers to essentially any substance that enhances or potentiates an immune response (antibody and/or cell-mediated) to an antigen. One type of immunostimulant comprises an adjuvant. Many adjuvants contain a substance designed to protect the antigen from rapid catabolism, such as aluminum hydroxide or mineral oil, and a stimulator of immune responses, such as lipid A, Bortadella pertussis or Mycobacterium tuberculosis derived proteins. Certain adjuvants are commercially available as, for example, Freund's Incomplete Adjuvant and Complete Adjuvant (Difco Laboratories); Merck Adjuvant 65 (Merck and Company, Inc.) AS-2 (SmithKline Beecham); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and/or MIF, and others, like growth factors, may also be used as adjuvants.

Within certain embodiments, the adjuvant composition can be one that induces an immune response predominantly of the Th1 type. High levels of Th1-type cytokines (e.g., IFN-γ, TNFα, IL-2 and IL-12) tend to favor the induction of cell-mediated immune responses to an administered antigen. In contrast, high levels of Th2-type cytokines (e.g., IL-4, IL-5, IL-6 and IL-10) tend to favor the induction of humoral immune responses. Following application of a vaccine as provided herein, a patient may support an immune response that includes Th1- and/or Th2-type responses. Within certain embodiments, in which a response is predominantly Th1-type, the level of Th1-type cytokines will increase to a greater extent than the level of Th2-type cytokines. The levels of these cytokines may be readily assessed using standard assays. Thus, various embodiments relate to therapies raising an immune response against a target antigen, for example tumor neo-antigens or neo-epitopes, using cytokines, e.g., IFN-γ, TNFα, IL-2, IL-8, IL-12, IL-18, IL-7, IL-3, IL-4, IL-5, IL-6, IL-9, IL-10, IL-13 IL-15, IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and/or MIF supplied concurrently with a replication-defective viral vector treatment. In some embodiments, a cytokine or a nucleic acid encoding a cytokine, is administered together with a replication-defective viral vector described herein. In some embodiments, cytokine administration is performed prior or subsequent to viral vector administration. In some embodiments, a replication-defective viral vector capable of raising an immune response against a target antigen, for example tumor neo-antigens or neo-epitopes, further comprises a sequence encoding a cytokine.

Certain illustrative adjuvants for eliciting a predominantly Th1-type response include, for example, a combination of monophosphoryl lipid A, such as 3-de-O-acylated monophosphoryl lipid A, together with an aluminum salt. MPL® adjuvants are commercially available *(see,* e.g., U.S. Pat. Nos. 4,436,727; 4,877,611; 4,866,034 and 4,912,094). CpG-containing oligonucleotides (in which the CpG dinucleotide is unmethylated) also induce a predominantly Th1 response. *(see,* e.g., WO 96/02555, WO 99/33488 and U.S. Pat. Nos. 6,008,200 and 5,856,462). Immunostimulatory DNA sequences can also be used.

Another adjuvant for use in some embodiments comprises a saponin, such as Quil A, or derivatives thereof, including QS21 and QS7 (Aquila Biopharmaceuticals Inc.), Escin; Digitonin; or Gypsophila or Chenopodium quinoa saponins. Other formulations may include more than one saponin in the adjuvant combinations, e.g., combinations of at least two of the following group comprising QS21, QS7, Quil A, β-escin, or digitonin.

In some embodiments, the compositions may be delivered by intranasal sprays, inhalation, and/or other aerosol delivery vehicles. The delivery of drugs using intranasal microparticle resins and lysophosphatidyl-glycerol compounds can be employed (*see,* e.g., U.S. Pat. No. 5,725,871). Likewise, illustrative transmucosal drug delivery in the form of a polytetrafluoroetheylene support matrix can be employed (*see,* e.g., U.S. Pat. No. 5,780,045).

Liposomes, nanocapsules, microparticles, lipid particles, vesicles, and the like, can be used for the introduction of the compositions as described herein into suitable hot cells/organisms. Compositions as described herein may be formulated for delivery either encapsulated in a lipid particle, a liposome, a vesicle, a nanosphere, or a nanoparticle or the like. Alternatively, compositions as described herein can be bound, either covalently or non-covalently, to the surface of such carrier vehicles. Liposomes can be used effectively to introduce genes, various drugs, radiotherapeutic agents, enzymes, viruses, transcription factors, allosteric effectors and the like, into a variety of cultured cell lines and animals. Furthermore, the use of liposomes does not appear to be associated with autoimmune responses or unacceptable toxicity after systemic delivery. In some embodiments, liposomes are formed from phospholipids dispersed in an aqueous medium and spontaneously form multilamellar concentric bilayer vesicles (i.e., multilamellar vesicles (MLVs)).

In some embodiments, there are provided pharmaceutically-acceptable nanocapsule formulations of the compositions or vectors as described herein. Nanocapsules can generally entrap pharmaceutical compositions in a stable and reproducible way. To avoid side effects due to intracellular polymeric overloading, such ultrafine particles (sized around 0.1 µm) may be designed using polymers able to be degraded *in vivo.*

In certain aspects, a pharmaceutical composition comprising a therapeutically effective amount IL-15 or a replication-defective vector comprising a nucleotide sequence encoding IL-15 may be administered to an individual in need thereof, in combination with one or more therapy provided herein, particularly one or more adenoviral vectors comprising nucleic acid sequences encoding one or more tumor neo-antigens or tumor neo-epitopes.

Interleukin 15 (IL-15) is a cytokine with structural similarity to IL-2. Like IL-2, IL-15 binds to and signals through a complex composed of IL-2/IL-15 receptor beta chain (CD122) and the common gamma chain (gamma-C, CD 132). IL-15 is secreted by mononuclear phagocytes (and some other cells) following infection by virus(es). This cytokine induces cell proliferation of natural killer cells; cells of the innate immune system whose principal role is to kill virally infected cells.

IL-15 can enhance the anti-tumor immunity of CD8+ T cells in pre-clinical models. A phase I clinical trial to evaluate the safety, dosing, and anti-tumor efficacy of IL-15 in patients with metastatic melanoma and renal cell carcinoma (kidney cancer) has begun to enroll patients at the National Institutes of Health.

IL-15 disclosed herein may also include mutants of IL-15 that are modified to maintain the function of its native form.

IL -15 is 14-15 kDa glycoprotein encoded by the 34 kb region 4q31 of chromosome 4, and by the central region of chromosome 8 in mice. The human IL-15 gene comprises nine exons (1 - 8 and 4A) and eight introns, four of which (exons 5 through 8) code for the mature protein. Two alternatively spliced transcript variants of this gene encoding the same protein have been reported. The originally identified isoform, with long signal peptide of 48 amino acids (IL-15 LSP) consisted of a 316 bp 5'-untranslated region (UTR), 486 bp coding sequence and the C-terminus 400 bp 3'-UTR region. The other isoform (IL-15 SSP) has a short signal peptide of 21 amino acids encoded by exons 4A and 5. Both isoforms shared 11 amino acids between signal sequences of the N-terminus. Although both isoforms produce the same mature protein, they differ in their cellular trafficking. IL-15 LSP isoform was identified in Golgi apparatus [GC], early endosomes and in the endoplasmic reticulum (ER). It exists in two forms, secreted and membrane-bound particularly on dendritic cells. On the other hand, IL-15 SSP isoform is not secreted and it appears to be restricted to the cytoplasm and nucleus which plays an important role in the regulation of cell cycle.

It has been demonstrated that two isoforms of IL-15 mRNA are generated by alternatively splicing in mice. The isoform which had an alternative exon 5 containing another 3' splicing site, exhibited a high translational efficiency, and the product lack hydrophobic domains in the signal sequence of the N-terminus. This suggests that the protein derived from this isoform is located intracellularly. The other isoform with normal exon 5, which is generated by integral splicing of the alternative exon 5, may be released extracellularly.

Although IL-15 mRNA can be found in many cells and tissues including mast cells, cancer cells or fibroblasts, this cytokine is produce as a mature protein mainly by dendritic cells, monocytes and macrophages. This discrepancy between the wide appearance of IL-15 mRNA and limited production of protein might be explained by the presence of the twelve in humans and five in mice upstream initiating codons, which can repress translation of IL-15 mRNA. Translational inactive mRNA is stored within the cell and can be induced upon specific signal. Expression of IL-15 can be stimulated by cytokine such as GM-CSF, double-strand mRNA, unmethylated CpG oligonucleotides, lipopolysaccharide (LPS) through Toll-like receptors(TLR), interferon gamma (IFN-γ) or after infection of monocytes herpes virus, Mycobacterium tuberculosis and *Candida albicans.*

### X. Natural Killer (NK) Cells

In certain embodiments, native or engineered NK cells may be provided to be administered to a subject in need thereof, in combination with adenoviral vector-based compositions or immunotherapy as described herein.

The immune system is a tapestry of diverse families of immune cells each with its own distinct role in protecting from infections and diseases. Among these immune cells are the natural killer, or NK, cells as the body's first line of defense. NK cells have the innate ability to rapidly seek and destroy abnormal cells, such as cancer or virally-infected cells, without prior exposure or activation by other support molecules. In contrast to adaptive immune cells such as T cells, NK cells have been utilized as a cell-based "off-the-shelf' treatment in phase 1 clinical trials, and have demonstrated tumor killing abilities for cancer.

### 1. aNK Cells

In addition to native NK cells, there may be provided NK cells for administering to a patient that has do not express Killer Inhibitory Receptors (KIR), which diseased cells often exploit to evade the killing function of NK cells. This unique activated NK, or aNK, cell lack these inhibitory receptors while retaining the broad array of activating receptors which enable the selective targeting and killing of diseased cells. aNK cells also carry a larger pay load of granzyme and perforin containing granules, thereby enabling them to deliver a far greater payload of lethal enzymes to multiple targets.

### 2. taNK Cells

Chimeric antigen receptor (CAR) technology is among the most novel cancer therapy approaches currently in development. CARs are proteins that allow immune effector cells to target cancer cells displaying specific surface antigen (target-activated Natural Killer) is a platform in which aNK cells are engineered with one or more CARs to target proteins found on cancers and is then integrated with a wide spectrum of CARs. This strategy has multiple advantages over other CAR approaches using patient or donor sourced effector cells such as autologous T-cells, especially in terms of scalability, quality control and consistency.

Much of the cancer cell killing relies upon ADCC (antibody dependent cell-mediated cytotoxicity) whereupon effector immune cells attach to antibodies, which are in turn bound to the target cancer cell, thereby facilitating killing of the cancer by the effector cell. NK cells are the key effector cell in the body for ADCC and utilize a specialized receptor (CD16) to bind antibodies.

### 3. HaNK Cells

Studies have shown that perhaps only 20% of the human population uniformly expresses the "high-affinity" variant of CD16, which is strongly correlated with more favorable therapeutic outcomes compared to patients with the "low-affinity" CD16. Additionally, many cancer patients have severely weakened immune systems due to chemotherapy, the disease itself or other factors.

In certain aspects, haNK cells are modified to express high-affinity CD16. As such, haNK cells may potentiate the therapeutic efficacy of a broad spectrum of antibodies directed against cancer cells.

### XI. Combination Therapy

The compositions described throughout can be formulated into a pharmaceutical medicament and be used to treat a human or mammal in need thereof or diagnosed with a disease, e.g., cancer. These medicaments can be co-administered with one or more additional vaccines to a human or mammal, or together with one or more conventional cancer therapies or alternative cancer therapies, cytokines such as IL-15 or nucleic acid sequences encoding such cytokines, engineered natural killer cells, or immune pathway checkpoint modulators as described herein.

Conventional cancer therapies include one or more selected from the group of chemical or radiation based treatments and surgery. Chemotherapies include, for example, cisplatin (CDDP), carboplatin, procarbazine, mechlorethamine, cyclophosphamide, camptothecin, ifosfamide, melphalan, chlorambucil, busulfan, nitrosurea, dactinomycin, daunorubicin, doxorubicin, bleomycin, plicomycin, mitomycin, etoposide (VP16), tamoxifen, raloxifene, estrogen receptor binding agents, taxol, gemcitabien, navelbine, farnesyl-protein tansferase inhibitors, transplatinum, 5-fluorouracil, vincristin, vinblastin and methotrexate, or any analog or derivative variant of the foregoing.

Radiation therapy that causes DNA damage and has been used extensively includes what are commonly known as γ-rays, X-rays, and/or the directed delivery of radioisotopes to tumor cells. Other forms of DNA damaging factors are also contemplated such as microwaves and UV-irradiation. It is most likely that all of these factors effect a broad range of damage on DNA, on the precursors of DNA, on the replication and repair of DNA, and on the assembly and maintenance of chromosomes. Dosage ranges for X-rays range from daily doses of 50 to 200 roentgens for prolonged periods of time (3 to 4 wk), to single doses of 2000 to 6000 roentgens. Dosage ranges for radioisotopes vary widely, and depend on the half-life of the isotope, the strength and type of radiation emitted, and the uptake by the neoplastic cells.

The terms "contacted" and "exposed," when applied to a cell, are used herein to describe the process by which a therapeutic construct and a chemotherapeutic or radiotherapeutic agent are delivered to a target cell or are placed in direct juxtaposition with the target cell. To achieve cell killing or stasis, both agents are delivered to a cell in a combined amount effective to kill the cell or prevent it from dividing.

Approximately 60% of persons with cancer will undergo surgery of some type, which includes preventative, diagnostic or staging, curative and palliative surgery. Curative surgery is a cancer treatment that may be used in conjunction with other therapies, such as the treatment described herein, chemotherapy, radiotherapy, hormonal therapy, gene therapy, immunotherapy and/or alternative therapies.

Curative surgery includes resection in which all or part of cancerous tissue is physically removed, excised, and/or destroyed. Tumor resection refers to physical removal of at least part of a tumor. In addition to tumor resection, treatment by surgery includes laser surgery, cryosurgery, electrosurgery, and microscopically controlled surgery (Mohs' surgery). It is further contemplated that treatment methods described herein may be used in conjunction with removal of superficial cancers, precancers, or incidental amounts of normal tissue.

Upon excision of part of all of cancerous cells, tissue, or tumor, a cavity may be formed in the body. Treatment may be accomplished by perfusion, direct injection or local application of the area with an additional anti-cancer therapy. Such treatment may be repeated, for example, every 1, 2, 3, 4, 5, 6, or 7 days, or every 1, 2, 3, 4, and 5 weeks or every 1, 2, 3, 4, 5, 6, 7, 8, 9, 10, 11, or 12 months. These treatments may be of varying dosages as well.

Alternative cancer therapies include any cancer therapy other than surgery, chemotherapy and radiation therapy, such as immunotherapy, gene therapy, hormonal therapy or a combination thereof. Subjects identified with poor prognosis using the present methods may not have favorable response to conventional treatment(s) alone and may be prescribed or administered one or more alternative cancer therapy *per se* or in combination with one or more conventional treatments.

Immunotherapeutics, generally, rely on the use of immune effector cells and molecules to target and destroy cancer cells. The immune effector may be, for example, an antibody specific for some marker on the surface of a tumor cell. The antibody alone may serve as an effector of therapy or it may recruit other cells to actually effect cell killing. The antibody also may be conjugated to a drug or toxin (chemotherapeutic, radionuclide, ricin A chain, cholera toxin, pertussis toxin, *etc*.) and serve merely as a targeting agent. Alternatively, the effector may be a lymphocyte carrying a surface molecule that interacts, either directly or indirectly, with a tumor cell target. Various effector cells include cytotoxic T cells and NK cells.

Gene therapy is the insertion of polynucleotides, including DNA or RNA, into a subject's cells and tissues to treat a disease. Antisense therapy is also a form of gene therapy. A therapeutic polynucleotide may be administered before, after, or at the same time of a first cancer therapy. Delivery of a vector encoding a variety of proteins is contemplated in certain aspects. For example, cellular expression of the exogenous tumor suppressor oncogenes would exert their function to inhibit excessive cellular proliferation, such as p53, p16 and C-CAM.

Additional agents to be used to improve the therapeutic efficacy of treatment include immunomodulatory agents, agents that affect the upregulation of cell surface receptors and GAP junctions, cytostatic and differentiation agents, inhibitors of cell adhesion, or agents that increase the sensitivity of the hyperproliferative cells to apoptotic inducers. Immunomodulatory agents include tumor necrosis factor; interferon alpha, beta, and gamma; IL-2 and other cytokines; F42K and other cytokine analogs; or MIP-1, MIP-1beta, MCP-1, RANTES, and other chemokines. It is further contemplated that the upregulation of cell surface receptors or their ligands such as Fas / Fas ligand, DR4 or DR5 / TRAIL would potentiate the apoptotic inducing abilities by establishment of an autocrine or paracrine effect on hyperproliferative cells. Increases intercellular signaling by elevating the number of GAP junctions would increase the anti-hyperproliferative effects on the neighboring hyperproliferative cell population. In other embodiments, cytostatic or differentiation agents can be used in combination with pharmaceutical compositions described herein to improve the anti-hyperproliferative efficacy of the treatments. Inhibitors of cell adhesion are contemplated to improve the efficacy of pharmaceutical compositions described herein. Examples of cell adhesion inhibitors are focal adhesion kinase (FAKs) inhibitors and Lovastatin. It is further contemplated that other agents that increase the sensitivity of a hyperproliferative cell to apoptosis, such as the antibody c225, could be used in combination with pharmaceutical compositions described herein to improve the treatment efficacy.

Hormonal therapy may also be used in combination with any other cancer therapy previously described. The use of hormones may be employed in the treatment of certain cancers such as breast, prostate, ovarian, or cervical cancer to lower the level or block the effects of certain hormones such as testosterone or estrogen. This treatment is often used in combination with at least one other cancer therapy as a treatment option or to reduce the risk of metastases.

A "Chemotherapeutic agent" or "chemotherapeutic compound" and their grammatical equivalents as used herein, can be a chemical compound useful in the treatment of cancer. The chemotherapeutic cancer agents that can be used in combination with the disclosed T cell include, but are not limited to, mitotic inhibitors (vinca alkaloids). These include vincristine, vinblastine, vindesine and Navelbine™ (vinorelbine, 5'-noranhydroblastine). In yet other embodiments, chemotherapeutic cancer agents include topoisomerase I inhibitors, such as camptothecin compounds. As used herein, "camptothecin compounds" include Camptosar™ (irinotecan HCL), Hycamtin™ (topotecan HCL) and other compounds derived from camptothecin and its analogues. Another category of chemotherapeutic cancer agents that can be used in the methods and compositions disclosed herein are podophyllotoxin derivatives, such as etoposide, teniposide and mitopodozide.

In certain aspects, methods or compositions described herein further encompass the use of other chemotherapeutic cancer agents known as alkylating agents, which alkylate the genetic material in tumor cells. These include without limitation cisplatin, cyclophosphamide, nitrogen mustard, trimethylene thiophosphoramide, carmustine, busulfan, chlorambucil, belustine, uracil mustard, chlomaphazin, and dacarbazine. The disclosure encompasses antimetabolites as chemotherapeutic agents. Examples of these types of agents include cytosine arabinoside, fluorouracil, methotrexate, mercaptopurine, azathioprime, and procarbazine. An additional category of chemotherapeutic cancer agents that may be used in the methods and compositions disclosed herein includes antibiotics. Examples include without limitation doxorubicin, bleomycin, dactinomycin, daunorubicin, mithramycin, mitomycin, mytomycin C, and daunomycin. There are numerous liposomal formulations commercially available for these compounds. In certain aspects, methods or compositions described herein further encompass the use of other chemotherapeutic cancer agents including without limitation anti-tumor antibodies, dacarbazine, azacytidine, amsacrine, melphalan, ifosfamide and mitoxantrone.

The disclosed adenovirus vaccine herein can be administered in combination with other anti-tumor agents, including cytotoxic/antineoplastic agents and anti-angiogenic agents. Cytotoxic/anti-neoplastic agents can be defined as agents who attack and kill cancer cells. Some cytotoxic/anti-neoplastic agents can be alkylating agents, which alkylate the genetic material in tumor cells, e.g., cis-platin, cyclophosphamide, nitrogen mustard, trimethylene thiophosphoramide, carmustine, busulfan, chlorambucil, belustine, uracil mustard, chlomaphazin, and dacabazine. Other cytotoxic/anti-neoplastic agents can be antimetabolites for tumor cells, e.g., cytosine arabinoside, fluorouracil, methotrexate, mercaptopuirine, azathioprime, and procarbazine. Other cytotoxic/anti-neoplastic agents can be antibiotics, e.g., doxorubicin, bleomycin, dactinomycin, daunorubicin, mithramycin, mitomycin, mytomycin C, and daunomycin. There are numerous liposomal formulations commercially available for these compounds. Still other cytotoxic/anti-neoplastic agents can be mitotic inhibitors (vinca alkaloids). These include vincristine, vinblastine and etoposide. Miscellaneous cytotoxic/anti-neoplastic agents include taxol and its derivatives, L-asparaginase, anti-tumor antibodies, dacarbazine, azacytidine, amsacrine, melphalan, VM-26, ifosfamide, mitoxantrone, and vindesine.

Additional formulations comprising population(s) of CAR T cells, T cell receptor engineered T cells, B cell receptor engineered cells, can be administered to a subject in conjunction, before, or after the administration of the pharmaceutical compositions described herein. A therapeutically-effective population of adoptively transferred cells can be administered to subjects when the methods described herein are practiced. In general, formulations are administered that comprise between about 1 x 10⁴ and about 1 x 10¹⁰ CAR T cells, T cell receptor engineered cells, or B cell receptor engineered cells. In some cases, the formulation comprises between about 1 x 10⁵ and about 1 x 10⁹ engineered cells, from about 5 x 10⁵ to about 5 x 10⁸ engineered cells, or from about 1 x 10⁶ to about 1 x 10⁷ engineered cells. However, the number of engineered cells administered to a subject will vary between wide limits, depending upon the location, source, identity, extent and severity of the cancer, the age and condition of the subject to be treated etc. A physician will ultimately determine appropriate dosages to be used.

Anti-angiogenic agents can also be used. Suitable anti-angiogenic agents for use in the disclosed methods and compositions include anti-VEGF antibodies, including humanized and chimeric antibodies, anti-VEGF aptamers and antisense oligonucleotides. Other inhibitors of angiogenesis include angiostatin, endostatin, interferons, interleukin 1 (including α and β) interleukin 12, retinoic acid, and tissue inhibitors of metalloproteinase-1 and -2. (TIMP-1 and -2). Small molecules, including topoisomerases such as razoxane, a topoisomerase II inhibitor with anti-angiogenic activity, can also be used.

In some cases, for example, in the compositions, formulations and methods of treating cancer, the unit dosage of the composition or formulation administered can be 5, 10, 15, 20, 25, 30, 35, 40, 45, 50, 55, 60, 65, 70, 75, 80, 85, 90, 95 or 100 mg. In some cases, the total amount of the composition or formulation administered can be 0.1, 0.2, 0.3, 0.4, 0.5, 0.6, 0.7, 0.8, 0.9, 1, 1.5, 2, 2.5, 3, 3.5, 4, 4.5, 5, 5.5, 6, 6.5, 7, 7.5, 8, 8.5, 9, 9.5, 10, 11, 12, 13, 14, 15, 16, 17, 18, 19, 20, 25, 30, 40, 50, 60, 70, 80, 90, or 100 g.

### XII. Immunological Fusion Partner Antigen Targets

The viral vectors or composition described herein may further comprise nucleic acid sequences that encode proteins, or an "immunological fusion partner," that can increase the immunogenicity of the target antigen such as a target antigen, target epitope, tumor neo-antigen or neo-epitope. In this regard, the protein produced following immunization with the viral vector containing such a protein may be a fusion protein comprising the target antigen of interest fused to a protein that increases the immunogenicity of the target antigen of interest. Furthermore, combination therapy with Ad5[E1-, E2b-] vectors encoding for neo-epitopes or target epitopes and an immunological fusion partner can result in boosting the immune response, such that the combination of both therapeutic moieties acts to synergistically boost the immune response than either the Ad5[E1-, E2b-] vectors encoding for neo-epitopes or target epitopes alone, or the immunological fusion partner alone. For example, combination therapy with Ad5[E1-, E2b-] vectors encoding for neo-epitope antigens and an immunological fusion partner can result in synergistic enhancement of stimulation of antigen-specific effector CD4+ and CD8+ T cells, stimulation of NK cell response directed towards killing infected cells, stimulation of neutrophils or monocyte cell responses directed towards killing infected cells via antibody dependent cell-mediated cytotoxicity (ADCC), antibody dependent cellular phagocytosis (ADCP) mechanisms, or any combination thereof. This synergistic boost can vastly improve survival outcomes after administration to a subject in need thereof. In certain embodiments, combination therapy with Ad5[E1-, E2b-] vectors encoding for neo-epitope antigens and an immunological fusion partner can result in generating an immune response comprises an increase in target antigen-specific CTL activity of about 1.5 to 20, or more fold in a subject administered the adenovirus vectors as compared to a control. In another embodiment, generating an immune response comprises an increase in target-specific CTL activity of about 1.5 to 20, or more fold in a subject administered the Ad5[E1-, E2b-] vectors encoding for neo-epitope antigens and an immunological fusion partner as compared to a control. In a further embodiment, generating an immune response that comprises an increase in target antigen-specific cell-mediated immunity activity as measured by ELISpot assays measuring cytokine secretion, such as interferon-gamma (IFN-γ), interleukin-2 (IL-2), tumor necrosis factor-alpha (TNF-α), or other cytokines, of about 1.5 to 20, or more fold as compared to a control. In a further embodiment, generating an immune response comprises an increase in target-specific antibody production of between 1.5 and 5 fold in a subject administered the Ad5[E1-, E2b-] vectors encoding for neo-epitope antigens and an immunological fusion partner as described herein as compared to an appropriate control. In another embodiment, generating an immune response comprises an increase in target-specific antibody production of about 1.5 to 20, or more fold in a subject administered the adenovirus vector as compared to a control.

As an additional example, combination therapy with Ad5[E1-, E2b-] vectors encoding for target epitope antigens and an immunological fusion partner can result in synergistic enhancement of stimulation of antigen-specific effector CD4+ and CD8+ T cells, stimulation of NK cell response directed towards killing infected cells, stimulation of neutrophils or monocyte cell responses directed towards killing infected cells via antibody dependent cell-mediated cytotoxicity (ADCC), antibody dependent cellular phagocytosis (ADCP) mechanisms, or any combination thereof. This synergistic boost can vastly improve survival outcomes after administration to a subject in need thereof. In certain embodiments, combination therapy with Ad5[E1-, E2b-] vectors encoding for target epitope antigens and an immunological fusion partner can result in generating an immune response comprises an increase in target antigen-specific CTL activity of about 1.5 to 20, or more fold in a subject administered the adenovirus vectors as compared to a control. In another embodiment, generating an immune response comprises an increase in target-specific CTL activity of about 1.5 to 20, or more fold in a subject administered the Ad5[E1-, E2b-] vectors encoding for target epitope antigens and an immunological fusion partner as compared to a control. In a further embodiment, generating an immune response that comprises an increase in target antigen-specific cell-mediated immunity activity as measured by ELISpot assays measuring cytokine secretion, such as interferon-gamma (IFN-γ), interleukin-2 (IL-2), tumor necrosis factor-alpha (TNF-α), or other cytokines, of about 1.5 to 20, or more fold as compared to a control. In a further embodiment, generating an immune response comprises an increase in target-specific antibody production of between 1.5 and 5 fold in a subject administered the adenovirus vectors as described herein as compared to an appropriate control. In another embodiment, generating an immune response comprises an increase in target-specific antibody production of about 1.5 to 20, or more fold in a subject administered the adenovirus vector as compared to a control.

In one embodiment, such an immunological fusion partner is derived from a *Mycobacterium sp*., such as a *Mycobacterium tuberculosis-derived* Ra12 fragment. The immunological fusion partner derived from *Mycobacterium sp.* can be any one of the sequences set forth in SEQ ID NO: 39 - SEQ ID NO: 47. Ra12 compositions and methods for their use in enhancing the expression and/or immunogenicity of heterologous polynucleotide/polypeptide sequences are described in U.S. Patent No. 7,009,042, which is herein incorporated by reference in its entirety. Briefly, Ra12 refers to a polynucleotide region that is a subsequence of a *Mycobacterium tuberculosis* MTB32A nucleic acid. MTB32A is a serine protease of 32 kDa encoded by a gene in virulent and avirulent strains of *M. tuberculosis.* The nucleotide sequence and amino acid sequence of MTB32A have been described *(see,* e.g., U.S. Patent No. 7,009,042; Skeiky et al., Infection and Immun. 67:3998-4007 (1999), incorporated herein by reference in their entirety). C-terminal fragments of the MTB32A coding sequence can be expressed at high levels and remain as soluble polypeptides throughout the purification process. Moreover, Ra12 may enhance the immunogenicity of heterologous immunogenic polypeptides with which it is fused. A Ra12 fusion polypeptide can comprise a 14 kDa C-terminal fragment corresponding to amino acid residues 192 to 323 of MTB32A. Other Ra12 polynucleotides generally can comprise at least about 15, 30, 60, 100, 200, 300, or more nucleotides that encode a portion of a Ra12 polypeptide. Ra12 polynucleotides may comprise a native sequence (i.e., an endogenous sequence that encodes a Ra12 polypeptide or a portion thereof) or may comprise a variant of such a sequence. Ra12 polynucleotide variants may contain one or more substitutions, additions, deletions and/or insertions such that the biological activity of the encoded fusion polypeptide is not substantially diminished, relative to a fusion polypeptide comprising a native Ra12 polypeptide. Variants can have at least about 70%, 80%, or 90% identity, or more, to a polynucleotide sequence that encodes a native Ra12 polypeptide or a portion thereof.

In certain aspects, an immunological fusion partner can be derived from protein D, a surface protein of the gram-negative bacterium *Haemophilus influenzae B.* The immunological fusion partner derived from protein D can be the sequence set forth in SEQ ID NO: 48. In some cases, a protein D derivative comprises approximately the first third of the protein (e.g., the first N-terminal 100-110 amino acids). A protein D derivative may be lipidated. Within certain embodiments, the first 109 residues of a Lipoprotein D fusion partner is included on the N-terminus to provide the polypeptide with additional exogenous T-cell epitopes, which may increase the expression level in E. coli and may function as an expression enhancer. The lipid tail may ensure optimal presentation of the antigen to antigen presenting cells. Other fusion partners can include the non-structural protein from influenza virus, NS1 (hemagglutinin). Typically, the N-terminal 81 amino acids are used, although different fragments that include T-helper epitopes may be used.

In certain aspects, the immunological fusion partner can be the protein known as LYTA, or a portion thereof (particularly a C-terminal portion). The immunological fusion partner derived from LYTA can the sequence set forth in SEQ ID NO: 49. LYTA is derived from *Streptococcus pneumoniae,* which synthesizes an N-acetyl-L-alanine amidase known as amidase LYTA (encoded by the LytA gene). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus can be employed. Within another embodiment, a repeat portion of LYTA may be incorporated into a fusion polypeptide. A repeat portion can, for example, be found in the C-terminal region starting at residue 178. One particular repeat portion incorporates residues 188-305.

In some embodiments, the target antigen is fused to an immunological fusion partner, also referred to herein as an "immunogenic component," comprising a cytokine selected from the group of IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and MIF. The target antigen fusion can produce a protein with substantial identity to one or more of IFN-γ, TNFα IL-2, IL-8, IL-12, IL-18, IL-7, IL-3, IL-4, IL-5, IL-6, IL-9, IL-10, IL-13, IL-15, IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and MIF. The target antigen fusion can encode a nucleic acid encoding a protein with substantial identity to one or more of IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and MIF. In some embodiments, the target antigen fusion further comprises one or more immunological fusion partner, also referred to herein as an "immunogenic components," comprising a cytokine selected from the group of IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and MIF. The sequence of IFN-γ can be, but is not limited to, a sequence as set forth in SEQ ID NO: 50. The sequence of TNFα can be, but is not limited to, a sequence as set forth in SEQ ID NO: 51. The sequence of IL-2 can be, but is not limited to, a sequence as set forth in SEQ ID NO: 52. The sequence of IL-8 can be, but is not limited to, a sequence as set forth in SEQ ID NO: 53. The sequence of IL-12 can be, but is not limited to, a sequence as set forth in SEQ ID NO: 54. The sequence of IL-18 can be, but is not limited to, a sequence as set forth in SEQ ID NO: 55. The sequence of IL-7 can be, but is not limited to, a sequence as set forth in SEQ ID NO: 56. The sequence of IL-3 can be, but is not limited to, a sequence as set forth in SEQ ID NO: 57. The sequence of IL-4 can be, but is not limited to, a sequence as set forth in SEQ ID NO: 58. The sequence of IL-5 can be, but is not limited to, a sequence as set forth in SEQ ID NO: 59. The sequence of IL-6 can be, but is not limited to, a sequence as set forth in SEQ ID NO: 60. The sequence of IL-9 can be, but is not limited to, a sequence as set forth in SEQ ID NO: 61. The sequence of IL-10 can be, but is not limited to, a sequence as set forth in SEQ ID NO: 62. The sequence of IL-13 can be, but is not limited to, a sequence as set forth in SEQ ID NO: 63. The sequence of IL-15 can be, but is not limited to, a sequence as set forth in SEQ ID NO: 64. The sequence of IL-16 can be, but is not limted to, a sequence as set forth in SEQ ID NO: 109. The sequence of IL-17 can be, but is not limited to, a sequence as set forth in SEQ ID NO: 110. The sequence of IL-23 can be, but is not limited to, a sequence as set forth in SEQ ID NO: 111. The sequence of IL-32 can be, but is not limited to, a sequences as set forth in SEQ ID NO: 112.

In some embodiments, the target antigen is fused or linked to an immunological fusion partner, also referred to herein as an "immunogenic component," comprising a cytokine selected from the group of IFN-γ, TNFα IL-2, IL-8, IL-12, IL-18, IL-7, IL-3, IL-4, IL-5, IL-6, IL-9, IL-10, IL-13, IL-15, , IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and MIF. In some embodiments, the target antigen is co-expressed in a cell with an immunological fusion partner, also referred to herein as an "immunogenic component," comprising a cytokine selected from the group of IFN-γ, TNFα IL-2, IL-8, IL-12, IL-18, IL-7, IL-3, IL-4, IL-5, IL-6, IL-9, IL-10, IL-13, IL-15, IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and MIF.

In some embodiments, the target antigen is fused or linked to an immunological fusion partner, comprising CpG ODN (a non-limiting example sequence is shown in SEQ ID NO: 65), cholera toxin (a non-limiting example sequence is shown in SEQ ID NO: 66), a truncated A subunit coding region derived from a bacterial ADP-ribosylating exotoxin (a non-limiting example sequence is shown in (a non-limiting example sequence is shown in SEQ ID NO: 67), a truncated B subunit coding region derived from a bacterial ADP-ribosylating exotoxin (a non-limiting example sequence is shown in SEQ ID NO: 68), Hp91 (a non-limiting example sequence is shown in SEQ ID NO: 69), CCL20 (a non-limiting example sequence is shown in SEQ ID NO: 70), CCL3 (a non-limiting example sequence is shown in SEQ ID NO: 71), GM-CSF (a non-limiting example sequence is shown in SEQ ID NO: 72), G-CSF (a non-limiting example sequence is shown in SEQ ID NO: 73), LPS peptide mimic (non-limiting example sequences are shown in SEQ ID NO: 74 - SEQ ID NO: 85), shiga toxin (a non-limiting example sequence is shown in SEQ ID NO: 86), diphtheria toxin (a non-limiting example sequence is shown in SEQ ID NO: 87), or CRM₁₉₇ (a non-limiting example sequence is shown in SEQ ID NO: 90).

In some embodiments, the target antigen is fused or linked to an immunological fusion partner, comprising an IL-15 superagonist. Interleukin 15 (IL-15) is a naturally occurring inflammatory cytokine secreted after viral infections. Secreted IL-15 can carry out its function by signaling via the its cognate receptor on effector immune cells, and thus, can lead to overall enhancement of effector immune cell activity.

Based on IL-15's broad ability to stimulate and maintain cellular immune responses, it is believed to be a promising immunotherapeutic drug that could potentially cure certain cancers. However, major limitations in clinical development of IL-15 can include low production yields in standard mammalian cell expression systems and short serum half-life. Moreover, the IL-15:IL-15Rα complex, comprising proteins co-expressed by the same cell, rather than the free IL-15 cytokine, can be responsible for stimulating immune effector cells bearing IL-15 βγc receptor.

To contend with these shortcomings, a novel IL-15 superagonist mutant (IL-15N72D) was identified that has increased ability to bind IL-15Rβγc and enhanced biological activity. Addition of either mouse or human IL-15Rα and Fc fusion protein (the Fc region of immunoglobulin) to equal molar concentrations of IL-15N72D can provide a further increase in IL-15 biologic activity, such that IL-15N72D:IL-15Rα/Fc super-agonist complex exhibits a median effective concentration (EC50) for supporting IL-15-dependent cell growth that was greater than10-fold lower than that of free IL-15 cytokine.

In some embodiments, the IL-15 superagonist can be a novel IL-15 superagonist mutant (IL-15N72D). In certain embodiments, addition of either mouse or human IL-15Rα and Fc fusion protein (the Fc region of immunoglobulin) to equal molar concentrations of IL-15N72D can provide a further increase in IL-15 biologic activity, such that IL-15N72D:IL-15Rα/Fc super-agonist complex exhibits a median effective concentration (EC₅₀) for supporting IL-15-dependent cell growth that can be greater than10-fold lower than that of free IL-15 cytokine

Thus, in some embodiments, the present disclosure provides a IL-15N72D:IL-15Rα/Fc super-agonist complex with an EC50 for supporting IL-15-dependent cell growth that is greater than 2-fold lower, greater than 3-fold lower, greater than 4-fold lower, greater than 5-fold lower, greater than 6-fold lower, greater than 7-fold lower, greater than 8-fold lower, greater than 9-fold lower, greater than 10-fold lower, greater than 15-fold lower, greater than 20-fold lower, greater than 25-fold lower, greater than 30-fold lower, greater than 35-fold lower, greater than 40-fold lower, greater than 45-fold lower, greater than 50-fold lower, greater than 55-fold lower, greater than 60-fold lower, greater than 65-fold lower, greater than 70-fold lower, greater than 75-fold lower, greater than 80-fold lower, greater than 85-fold lower, greater than 90-fold lower, greater than 95-fold lower, or greater than 100-fold lower than that of free IL-15 cytokine.

In some embodiments, the IL-15 super agonist is a biologically active protein complex of two IL-15N72D molecules and a dimer of soluble IL-15Rα/Fc fusion protein, also known as ALT-803. The composition of ALT-803 and methods of producing and using ALT-803 are described in U.S. Patent Application Publication 2015/0374790, which is herein incorporated by reference. It is known that a soluble IL-15Rα fragment, containing the so-called "sushi" domain at the N terminus (Su), can bear most of the structural elements responsible for high affinity cytokine binding. A soluble fusion protein can be generated by linking the human IL-15RαSu domain (amino acids 1-65 of the mature human IL-15Rα protein) with the human IgG1 CH2-CH3 region containing the Fc domain (232 amino acids). This IL-15RαSu/IgG1 Fc fusion protein can have the advantages of dimer formation through disulfide bonding via IgG1 domains and ease of purification using standard Protein A affinity chromatography methods.

In some embodiments, ALT-803 can have a soluble complex consisting of 2 protein subunits of a human IL-15 variant associated with high affinity to a dimeric IL-15Rα sushi domain/human IgG1 Fc fusion protein. The IL-15 variant is a 114 amino acid polypeptide comprising the mature human IL-15 cytokine sequence with an Asn to Asp substitution at position 72 of helix C N72D). The human IL-15R sushi domain/human IgG1 Fc fusion protein comprises the sushi domain of the IL-15R subunit (amino acids 1- 65 of the mature human IL-15Rα protein) linked with the human IgG1 CH2-CH3 region containing the Fc domain (232 amino acids). Aside from the N72D substitution, all of the protein sequences are human. Based on the amino acid sequence of the subunits, the calculated molecular weight of the complex comprising two IL-15N72D polypeptides (an example IL-15N72D sequence is shown in SEQ ID NO: 88) and a disulfide linked homodimeric IL- 15RαSu/IgG1 Fc protein (an example IL-15RαSu/Fc domain is shown in SEQ ID NO: 89) is 92.4 kDa. In some embodiments, a recombinant vector encoding for a target antigen and for ALT-803 can have any sequence described herein to encode for the target antigen and can have SEQ ID NO: 88, SEQ ID NO: 88, and SEQ ID NO: 89, in any order, to encode for ALT-803.

Each IL-15N720 polypeptide has a calculated molecular weight of approximately 12.8 kDa and the IL-15RαSu/IgG 1 Fc fusion protein has a calculated molecular weight of approximately 33.4 kDa. Both the IL-15N72D and IL-15RαSu/IgG 1 Fc proteins can be glycosylated resulting in an apparent molecular weight of ALT- 803 of approximately 114 kDa by size exclusion chromatography. The isoelectric point (pI) determined for ALT-803 can range from approximately 5.6 to 6.5. Thus, the fusion protein can be negatively charged at pH 7.

Combination therapy with Ad5[E1-, E2b-] vectors encoding for neo-epitopes and ALT-803 can result in boosting the immune response, such that the combination of both therapeutic moieties acts to synergistically boost the immune response than either therapy alone. For example, combination therapy with Ad5[E1-, E2b-] vectors encoding for neo-epitope antigens and ALT-803 can result in synergistic enhancement of stimulation of antigen-specific effector CD4+ and CD8+ T cells, stimulation of NK cell response directed towards killing infected cells, stimulation of neutrophils or monocyte cell responses directed towards killing infected cells via antibody dependent cell-mediated cytotoxicity (ADCC), or antibody dependent cellular phagocytosis (ADCP) mechanisms. Combination therapy with Ad5[E1-, E2b-] vectors encoding for neo-epitope antigens and ALT-803 can synergistically boost any one of the above responses, or a combination of the above responses, to vastly improve survival outcomes after administration to a subject in need thereof.

Any of the immunogenicity enhancing agents described herein can be fused or linked to a target antigen by expressing the immunogenicity enhancing agents and the target antigen in the same recombinant vector, using any recombinant vector described herein.

Nucleic acid sequences that encode for such immunogenicity enhancing agents can be any one of SEQ ID NO: 39 - SEQ ID NO: 90 and are summarized in **TABLE 1.**

**TABLE 1: Sequences of Immunogenicity Enhancing Agents**

| **SEQ ID NO** | **Sequence** |
|---|---|
| **SEQ ID NO: 39** | TAASDNFQLSQGGQGFAIPIGQAMAIAGQIRSGGGSPTVHIGPTAFL GLGVVDNNGNGARVQRVVGSAPAASLGISTGDVITAVDGAPINSAT AMADALNGHHPGDVISVTWQTKSGGTRTGNVTLAEGPPA |
| **SEQ ID NO: 40** | MHHHHHHTAASDNFQLSQGGQGFAIPIGQAMAIAGQIRSGGGSPTV HIGPTAFLGLGVVDNNGNARVQRVVGSAPAASLGISTGDVITAVD GAPINSATAMADALNGHHPGDVISVTWQTKSGGTRTGNVTLAEGP PAEFDDDDKDPPDPHQPDMTKGYCPGGRWGFGDLAVCDGEKYPD GSFWHQWMQTWFTGPQFYFDCVSGGEPLPGPPPPGGCGGAIPSEQP NAP |
| **SEQ ID NO: 41** | MHHHHHHTAASDNFQLSQGGQGFAIPIGQAMAIAGQIRSGGGSPTV HIGPTAFLGLGVVDNNGNGARVQRVVGSAPAASLGISTGDVITAVD GAPINSATAMADALNGHHPGDVISVTWQTKSGGTRTGNVTLAEGP PAEFPLVPRGSPMGSDVRDLNALLPAVPSLGGGGGCALPVSGAAQ WAPVLDFAPPGASAYGSLGGPAPPAPPPPPPPPPHSFIKQEPSWGGA EPHEEQCLSAFTVHFSGQFTGTAGACRYGPFGPPPPSQASSGQARMF PNAPYLPSCLESQPAIRNQGYSTVTFDGTPSYGHTPSHHAAQFPNHS FKHEDPMGQQGSLGEQQYSVPPPVYGCHTPTDSCTGSQALLLRTPY SSDNLYQMTSQLECMTWNQMNLGATLKGHSTGYESDNHTTPILCG AQYRIHTHGVFRGIQDVRRVPGVAPTLVRSASETSEKRPFMCAYSG CNKRYFKLSHLQMHSRKHTGEKPYQCDFKDCERRFFRSDQLKRHQ RRHTGVKPFQCKTCQRKFSRSDHLKTHTRTHTGEKPFSCRWPSCQK KFARSDELVRHHNMHQRNMTKLQLAL |
| **SEQ ID NO: 42** | |
| **SEQ ID NO: 43** | MHHHHHHTAASDNFQLSQGGQGFAIPIGQAMAIAGQIRSGGGSPTV HIGPTAFLGLGVVDNNGNGARVQRVVGSAPAASLGISTGDVITAVD GAPINSATAMADALNGHHPGDVISVTWQTKSGGTRTGNVTLAEGP PAEFMVDFGALPPEINSARMYAGPGSASLVAAAQMWDSVASDLFS AASAFQSVVWGLTVGSWIGSSAGLMVAAASPYVAWMSVTAGQAE LTAAQVRVAAAAYETAYGLTVPPPVIAENRAELMILIATNLLGQNT PAIAVNEAEYGEMWAQDAAAMFGYAAATATATATLLPFEEAPEMT SAGGLLEQAAAVEEASDTAAANQLMNNVPQALQQLAQPTQGTTPS SKLGGLWKTVSPHRSPISNMVSMANNHMSMTNSGVSMTNTLSSML KGFAPAAAAQAVQTAAQNGVRAMSSLGSSLGSSGLGGGVAANLG RAASVGSLSVPQAWAAANQAVTPAARALPLTSLTSAAERGPGQML GGLPVGQMGARAGGGLSGVLRVPPRPYVMPHSPAAGDIAPPALSQ DRFADFPALPLDPSAMVAQVGPQVVNINTKLGYNNAVGAGTGIVID PNGVVLTNNHVIAGATDINAFSVGSGQTYGVDVVGYDRTQDVAVL QLRGAGGLPSAAIGGGVAVGEPVVAMGNSGGQGGTPRAVPGRVV ALGQTVQASDSLTGAEETLNGLIQFDAAIQPGDSGGPVVNGLGQVV GMNTAAS |
| **SEQ ID NO: 44** | TAASDNFQLSQGGQGFAIPIGQAMAIAGQI |
| **SEQ ID NO: 45** | TAASDNFQLSQGGQGFAIPIGQAMAIAGQIKLPTVHIGPTAFLGLGV VDNNGNGARVQRVVGSAPAASLGISTGDVITAVDGAPINSATAMA DALNGHHPGDVISVTWQTKSGGTRTGNVTLAEGPPA |
| **SEQ ID NO: 46** | TAASDNFQLSQGGQGFAIPIGQAMAIAGQIRSGGGSPTVHIGPTAFL GLGVVDNNGNGARVQRVVGSAPAASLGISTGDVITAVDGAPINSAT AMADALNGHHPGDVISVTWQTKSGGTRTGNVTLAE |
| **SEQ ID NO: 47** | MSNSRRRSLRWSWLLSVLAAVGLGLATAPAQAAPPALSQDRFADF PALPLDPSAMVAQVGPQVVNINTKLGYNNAVGAGTGIVIDPNGVVL TNNHVIAGATDINAFSVGSGQTYGVDVVGYDRTQDVAVLQLRGAG GLPSAAIGGGVAVGEPVVAMGNSGGQGGTPRAVPGRVVALGQTV QASDSLTGAEETLNGLIQFDAAIQPGDSGGPVVNGLGQVVGMNTA ASDNFQLSQGGQGFAIPIGQAMAIAGQIRSGGGSPTVHIGPTAFLGL GVVDNNGNGARVQRVVGSAPAASLGISTGDVITAVDGAPINSATA MADALNGHHPGDVISVTWQTKSGGTRTGNVTLAEGPPA |
| **SEQ ID NO: 48** | MKLKTLALSLLAAGVLAGCSSHSSNMANTQMKSDKIIIAHRGASGY LPEHTLESKALAFAQQADYLEQDLAMTKDGRLVVIHDHFLDGLTD VAKKFPHRHRKDGRYYVIDFTLKEIQSLEMTENFETKDGKQAQVYP NRFPLWKSHFRIHTFEDEIEFIQGLEKSTGKKVGIYPEIKAPWFHHQN GKDIAAETLKVLKKYGYDKKTDMVYLQTFDFNELKRIKTELLPQM GMDLKLVQLIAYTDWKETQEKDPKGYWVNYNYDWMFKPGAMAE VVKYADGVGPGWYMLVNKEESKPDNIVYTPLVKELAQYNVEVHP YTVRKDALPAFFTDVNQMYDVLLNKSGATGVFTDFPDTGVEFLKGI K |
| **SEQ ID NO: 49** | MEINVSKLRTDLPQVGVQPYRQVHAHSTGNPHSTVQNEADYHWRK DPELGFFSHIVGNGCIMQVGPVDNGAWDVGGGWNAETYAAVELIE SHSTKEEFMTDYRLYIELLRNLADEAGLPKTLDTGSLAGIKTHEYCT NNQPNNHSDHVDPYPYLAKWGISREQFKHDIENGLTIETGWQKNDT GYWYVHSDGSYPKDKFEKINGTWYYFDSSGYMLADRWRKHTDGN WYWFDNSGEMATGWKKIADKWYYFNEEGAMKTGWVKYKDTWY YLDAKEGAMVSNAFIQSADGTGWYYLKPDGTLADRPEFRMSQMA |
| **SEQ ID NO: 50** | MKYTSYILAFQLCIVLGSLGCYCQDPYVKEAENLKKYFNAGHSDVA DNGTLFLGILKNWKEESDRKIMQSQIVSFYFKLFKNFKDDQSIQKSV ETIKEDMNVKFFNSNKKKRDDFEKLTNYSVTDLNVQRKAIHELIQV MAELSPAAKTGKRKRSQMLFRGRRASQ |
| **SEQ ID NO: 51** | MSTESMIRDVELAEEALPKKTGGPQGSRRCLFLSLFSFLIVAGATTLF CLLHFGVIGPQREEFPRDLSLISPLAQAVRSSSRTPSDKPVAHVVANP QAEGQLQWLNRRANALLANGVELRDNQLVVPSEGLYLIYSQVLFK GQGCPSTHVLLTHTISRIAVSYQTKVNLLSAIKSPCQRETPEGAEAKP WYEPIYLGGVFQLEKGDRLSAEINRPDYLDFAESGQVYFGIIAL |
| **SEQ ID NO: 52** | MYRMQLLSCIALSLALVTNSAPTSSSTKKTQLQLEHLLLDLQMILNG INNYKNPKLTRMLTFKFYMPKKATELKHLQCLEEELKPLEEVLNLA QSKNFHLRPRDLISNINVIVLELKGSETTFMCEYADETATIVEFLNRW ITFCQSIISTLT |
| **SEQ ID NO: 53** | MTSKLAVALLAAFLISAALCEGAVLPRSAKELRCQCIKTYSKPFHPK FIKELRVIESGPHCANTEIIVKLSDGRELCLDPKENWVQRVVEKFLK RAENS |
| **SEQ ID NO: 54** | |
| **SEQ ID NO: 55** | MAAEPVEDNCINFVAMKFIDNTLYFIAEDDENLESDYFGKLESKLSV IRNLNDQVLFIDQGNRPLFEDMTDSDCRDNAPRTIFIISMYKDSQPRG MAVTISVKCEKISTLSCENKIISFKEMNPPDNIKDTKSDIIFFQRSVPG HDNKMQFESSSYEGYFLACEKERDLFKLILKKEDELGDRSIMFTVQ NED |
| **SEQ ID NO: 56** | MFHVSFRYIFGLPPLILVLLPVASSDCDIEGKDGKQYESVLMVSIDQL LDSMKEIGSNCLNNEFNFFKRHICDANKEGMFLFRAARKLRQFLKM NSTGDFDLHLLKVSEGTTILLNCTGQVKGRKPAALGEAQPTKSLEE NKSLKEQKKLNDLCFLKRLLQEIKTCWNKILMGTKEH |
| **SEQ ID NO: 57** | MSRLPVLLLLQLLVRPGLQAPMTQTTSLKTSWVNCSNMIDEIITHLK QPPLPLLDFNNLNGEDQDILMENNLRRPNLEAFNRAVKSLQNASAIE SILKNLLPCLPLATAAPTRHPIHIKDGDWNEFRRKLTFYLKTLENAQ AQQTTLSLAIF |
| **SEQ ID NO: 58** | |
| **SEQ ID NO: 59** | MRMLLHLSLLALGAAYVYAIPTEIPTSALVKETLALLSTHRTLLIAN ETLRIPVPVHKNHQLCTEEIFQGIGTLESQTVQGGTVERLFKNLSLIK KYIDGQKKKCGEERRRVNQFLDYLQEFLGVMNTEWIIES |
| **SEQ ID NO: 60** | MNSFSTSAFGPVAFSLGLLLVLPAAFPAPVPPGEDSKDVAAPHRQPL TSSERIDKQIRYILDGISALRKETCNKSNMCESSKEALAENNLNLPK MAEKDGCFQSGFNEETCLVKIITGLLEFEVYLEYLQNRFESSEEQAR AVQMSTKVLIQFLQKKAKNLDAITTPDPTTNASLLTKLQAQNQWLQ DMTTHLILRSFKEFLQSSLRALRQM |
| **SEQ ID NO: 61** | MVLTSALLLCSVAGQGCPTLAGILDINFLINKMQEDPASKCHCSAN VTSCLCLGIPSDNCTRPCFSERLSQMTNTTMQTRYPLIFSRVKKSVE VLKNNKCPYFSCEQPCNQTTAGNALTFLKSLLEIFQKEKMRGMRGK I |
| **SEQ ID NO: 62** | MHSSALLCCLVLLTGVRASPGQGTQSENSCTHFPGNLPNMLRDLRD AFSRVKTFFQMKDQLDNLLLKESLLEDFKGYLGCQALSEMIQFYLE EVMPQAENQDPDIKAHVNSLGENLKTLRLRLRRCHRFLPCENKSKA VEQVKNAFNKLQEKGIYKAMSEFDIFINYIEAYMTMKIRN |
| **SEQ ID NO: 63** | |
| **SEQ ID NO: 64** | |
| **SEQ ID NO: 65** | MEGDGSDPEPPDAGEDSKSENGENAPIYCICRKPDINCFMIGCDNCN EWFHGDCIRITEKMAKAIREWYCRECREKDPKLEIRYRHKKSRERD GNERDSSEPRDEGGGRKRPVPDPNLQRRAGSGTGVGAMLARGSAS PHKSSPQPLVATPSQHHQQQQQIKRSARMCGECEACRRTEDCGHC DFCRDMKKFGGPNKIRQKCRLRQCQLRARESYKYFPSSLSPVTPSES LPRPRRPLPTQQQPQPSQKLGRIREDEGAVASSTVKEPPEATATPEPL SDEDLPLDPDLYQDFCAGAFDDNGLPWMSDTEESPFLDPALRKRAV KVKHVKRREKKSEKKKEERYKRHRQKQKHKDKWKHPERADAKD PASLPQCLGPGCVRPAQPSSKYCSDDCGMKLAANRIYEILPQRIQQW QQSPCIAEEHGKKLLERIRREQQSARTRLQEMERRFHELEAIILRAKQ QAVREDEESNEGDSDDTDLQIFCVSCGHPINPRVALRHMERCYAKY ESQTSFGSMYPTRIEGATRLFCDVYNPQSKTYCKRLQVLCPEHSRDP KVPADEVCGCPLVRDVFELTGDFCRLPKRQCNRHYCWEKLRRAEV DLERVRVWYKLDELFEQERNVRTAMTNRAGLLALMLHQTIQHDPL TTDLRSSADR |
| **SEQ ID NO: 66** | MIKLKFGVFFTVLLSSAYAHGTPQNITDLCAEYHNTQIYTLNDKIFS YTESLAGKREMAIITFKNGAIFQVEVPGSQHIDSQKKAIERMKDTLRI AYLTEAKVEKLCVWNNKTPHAIAAISMAN |
| **SEQ ID NO: 67** | MVKIIFVFFIFLSSFSYANDDKLYRADSRPPDEIKQSGGLMPRGQNEY FDRGTQMNINLYDHARGTQTGFVRHDDGYVSTSISLRSAHLVGQTI LSGHSTYYIYVIATAPNMFNVNDVLGAYSPHPDEQEVSALGGIPYSQ IYGWYRVHFGVLDEQLHRNRGYRDRYYSNLDIAPAADGYGLAGFP PEHRAWREEPWIHHAPPGCGNAPRSSMSNTCDEKTQSLGVKFLDEY QSKVKRQIFSGYQSDIDTHNRIKDEL |
| **SEQ ID NO: 68** | MIKLKFGVFFTVLLSSAYAHGTPQNITDLCAEYHNTQIHTLNDKILS YTESLAGNREMAIITFKNGATFQVEVPGSQHIDSQKKAIERMKDTLR IAYLTEAKVEKLCVWNNKTPHAIAAISMAN |
| **SEQ ID NO: 69** | DPNAPKRPPSAFFLFCSE |
| **SEQ ID NO: 70** | MCCTKSLLLAALMSVLLLHLCGESEAASNFDCCLGYTDRILHPKFIV GFTRQLANEGCDINAIIFHTKKKLSVCANPKQTWVKYIVRLLSKKV KNM |
| **SEQ ID NO: 71** | MQVSTAALAVLLCTMALCNQFSASLAADTPTACCFSYTSRQIPQNFI ADYFETSSQCSKPGVIFLTKRSRQVCADPSEEWVQKYVSDLELSA |
| **SEQ ID NO: 72** | MWLQSLLLLGTVACSISAPARSPSPSTQPWEHVNAIQEARRLLNLSR DTAAEMNETVEVISEMFDLQEPTCLQTRLELYKQGLRGSLTKLKGP LTMMASHYKQHCPPTPETSCATQIITFESFKENLKDFLLVIPFDCWEP VQE |
| **SEQ ID NO: 73** | MAGPATQSPMKLMALQLLLWHSALWTVQEATPLGPASSLPQSFLL KCLEQVRKIQGDGAALQEKLCATYKLCHPEELVLLGHSLGIPWAPL SSCPSQALQLAGCLSQLHSGLFLYQGLLQALEGISPELGPTLDTLQL |
| | DVADFATTIWQQMEELGMAPALQPTQGAMPAFASAFQRRAGGVL VASHLQSFLEVSYRVLRHLAQP |
| **SEQ ID NO: 74** | QEINSSY |
| **SEQ ID NO: 75** | SHPRLSA |
| **SEQ ID NO: 76** | SMPNPMV |
| **SEQ ID NO: 77** | GLQQVLL |
| **SEQ ID NO: 78** | HELSVLL |
| **SEQ ID NO: 79** | YAPQRLP |
| **SEQ ID NO: 80** | TPRTLPT |
| **SEQ ID NO: 81** | APVHSSI |
| **SEQ ID NO: 82** | APPHALS |
| **SEQ ID NO: 83** | TFSNRFI |
| **SEQ ID NO: 84** | VVPTPPY |
| **SEQ ID NO: 85** | ELAPDSP |
| **SEQ ID NO: 86** | TPDCVTGKVEYTKYNDDDTFTVKVGDKELFTNRWNLQSLLLSAQIT GMTVTIKQNACHNGGGFSEVIFR |
| **SEQ ID NO: 87** | MSRKLFASILIGALLGIGAPPSAHAGADDVVDSSKSFVMENFSSYHG TKPGYVDSIQKGIQKPKSGTQGNYDDDWKGFYSTDNKYDAAGYSV DNENPLSGKAGGVVKVTYPGLTKVLALKVDNAETIKKELGLSLTEP LMEQVGTEEFIKRFGDGASRVVLSLPFAEGSSSVEYINNWEQAKALS VELEINFETRGKRGQDAMYEYMAQACAGNRVRRSVGSSLSCINLD WDVIRDKTKTKIESLKEHGPIKNKMSESPNKTVSEEKAKQYLEEFH QTALEHPELSELKTVTGTNPVFAGANYAAWAVNVAQVIDSETADN LEKTTAALSILPGIGSVMGIADGAVHHNTEEIVAQSIALSSLMVAQAI PLVGELVDIGFAAYNFVESIINLFQVVHNSYNRPAYSPGHKTQPFLH DGYAVSWNTVEDSIIRTGFQGESGHDIKITAENTPLPIAGVLLPTIPG KLDVNKSKTHISVNGRKIRMRCRAIDGDVTFCRPKSPVYVGNGVHA NLHVAFHRSSSEKIHSNEISSDSIGVLGYQKTVDHTKVNSKLSLFFEI KS |
| **SEQ ID NO: 88** | NWVNVISDLKKIEDLIQSMHIDATLYTESDVHPSCKVTAMKCFLLEL QVISLESGDASIHDTVENLIILANDSLSSNGNVTESGCKECEELEEKNI KEFLQSFVHIVQMFINTS |
| **SEQ ID NO: 89** | ITCPPPMSVEHADIWVKSYSLYSRERYICNSGFKRKAGTSSLTECVL NKATNVAHWTTPSLKCIREPKSCDKTHTCPPCPAPELLGGPSVFLFP PKPKDTLMISRTPEVTCVVVDVSHEDPEVKFNWYVDGVEVHNAKT KPREEQYNSTYRVVSVLTVLHQDWLNGKEYKCKVSNKALPAPIEK TISKAKGQPREPQVYTLPPSRDELTKNQVSLTCLVKGFYPSDIAVEW ESNGQPENNYKTTPPVLDSDGSFFLYSKLTVDKSRWQQGNVFSCSV MHEALHNHYTQKSLSLSPGK |
| **SEQ ID NO: 90** | GADDVVDSSKSFVMENFSSYHGTKPGYVDSIQKGIQKPKSGTQGNY DDDWKEFYSTDNKYDAAGYSVDNENPLSGKAGGVVKVTYPGLTK VLALKVDNAETIKKELGLSLTEPLMEQVGTEEFIKRFGDGASRVVLS LPFAEGSSSVEYINNWEQAKALSVELEINFETRGKRGQDAMYEYMA QACAGNRVRRSVGSSLSCINLDWDVIRDKTKTKIESLKEHGPIKNK MSESPNKTVSEEKAKQYLEEFHQTALEHPELSELKTVTGTNPVFAG ANYAAWAVNVAQVIDSETADNLEKTTAALSILPGIGSVMGIADGAV HHNTEEIVAQSIALSSLMVAQAIPLVGELVDIGFAAYNFVESIINLFQ VVHNSYNRPAYSPGHKTQPFLHDGYAVSWNTVEDSIIRTGFQGESG HDIKITAENTPLPIAGVLLPTIPGKLDVNKSKTHISVNGRKIRMRCRAI DGDVTFCRPKSPVYVGNGVHANLHVAFHRSSSEKIHSNEISSDSIGV LGYQKTVDHTKVNSKLSLFFEIKS |
| **SEQ ID NO: 109** | |
| **SEQ ID NO: 110** | MTPGKTSLVSLLLLLSLEAIVKAGITIPRNPGCPNSEDKNFPRTVMVN LNIHNRNTNTNPKRSSDYYNRSTSPWNLHRNEDPERYPSVIWEAKC RHLGCINADGNVDYHMNSVPIQQEILVLRREPPHCPNSFRLEKILVS VGCTCVTPIVHHVA |
| **SEQ ID NO: 111** | RAVPGGSSPAWTQCQQLSQKLCTLAWSAHPLVGHMDLREEGDEET TNDVPHIQCGDGCDPQGLRDNSQFCLQRIHQGLIFYEKLLGSDIFTG EPSLLPDSPVGQLHASLLGLSQLLQPEGHHWETQQIPSLSPSQPWQR LLLRFKILRSLQAFVAVAARVFAHGAATLSPIWELKKDVYVVELDW YPDAPGEMVVLTCDTPEEDGITWTLDQSSEVLGSGKTLTIQVKEFG DAGQYTCHKGGEVLSHSLLLLHKKEDGIWSTDILKDQKEPKNKTFL RCEAKNYSGRFTCWWLTTISTDLTFSVKSSRGSSDPQGVTCGAATL SAERVRGDNKEYEYSVECQEDSACPAAEESLPIEVMVDAVHKLKYE NYTSSFFIRDIIKPDPPKNLQLKPLKNSRQVEVSWEYPDTWSTPHSYF SLTFCVQVQGKSKREKKDRVFTDKTSATVICRKNASISVRAQDRYY SSSWSEWASVPCS |
| **SEQ ID NO: 112** | MCFPKVLSDDMKKLKARMVMLLPTSAQGLGAWVSACDTEDTVGH LGPWRDKDPALWCQLCLSSQHQAIERFYDKMQNAESGRGQVMSSL AELEDDFKEGYLETVAAYYEEQHPELTPLLEKERDGLRCRGNRSPV PDVEDPATEEPGESFCDKVMRWFQAMLQRLQTWWHGVLAWVKE KVVALVHAVQALWKQFQSFCCSLSELFMSSFQSYGAPRGDKEELTP QKCSEPQSSK |

In some embodiments, the nucleic acid sequences for the target antigen and the immunological fusion partner are not separated by any nucleic acids. In other embodiments, a nucleic acid sequence that encodes for a linker can be inserted between the nucleic acid sequence encoding for any target antigen described herein and the nucleic acid sequence encoding for any immunological fusion partner described herein. Thus, in certain embodiments, the protein produced following immunization with the viral vector containing a target antigen, a linker, and an immunological fusion partner can be a fusion protein comprising the target antigen of interest followed by the linker and ending with the immunological fusion partner, thus linking the target antigen to an immunological fusion partner that increases the immunogenicity of the target antigen of interest via a linker. In some embodiments, the sequence of linker nucleic acids can be from about 1 to about 150 nucleic acids long, from about 5 to about 100 nucleic acids along, or from about 10 to about 50 nucleic acids in length. In some embodiments, the nucleic acid sequences may encode one or more amino acid residues. In some embodiments, the amino acid sequence of the linker can be from about 1 to about 50, or about 5 to about 25 amino acid residues in length. In some embodiments, the sequence of the linker comprises less than 10 amino acids. In some embodiments, the linker can be a polyalanine linker, a polyglycine linker, or a linker with both alanines and glycines.

Nucleic acid sequences that encode for such linkers can be any one of SEQ ID NO: 91 - SEQ ID NO: 105 and are summarized in **TABLE 2.**

**TABLE 2: Sequences of Linkers**

| **SEQ ID NO** | **Sequence** |
|---|---|
| **SEQ ID NO: 91** | MAVPMQLSCSR |
| **SEQ ID NO: 92** | RSTG |
| **SEQ ID NO: 93** | TR |
| **SEQ ID NO: 94** | RSQ |
| **SEQ ID NO: 95** | RSAGE |
| **SEQ ID NO: 96** | RS |
| **SEQ ID NO: 97** | GG |
| **SEQ ID NO: 98** | GSGGSGGSG |
| **SEQ ID NO: 99** | GGSGGSGGSGG |
| **SEQ ID NO: 100** | GGSGGSGGSGGSGG |
| **SEQ ID NO: 101** | GGSGGSGGSGGSGGSGG |
| **SEQ ID NO: 102** | GGSGGSGGSGGSGGSGGSGG |
| **SEQ ID NO: 103** | GGSGGSGGSGGSGGSGGSGGSGG |
| **SEQ ID NO: 104** | GGSGGSGGSGGSGGSG |
| **SEQ ID NO: 105** | GSGGSGGSGGSGGSGG |

### XIII. Costimulatory Molecules

In addition to the use of a recombinant adenovirus-based vector vaccine containing tumor neo-epitopes or neo-antigens, co-stimulatory molecules can be incorporated into said vaccine that will increase immunogenicity.

Initiation of an immune response requires at least two signals for the activation of naive T cells by APCs (Damle, et al. J Immunol 148:1985-92 (1992); Guinan, et al. Blood 84:3261-82 (1994); Hellstrom, et al. Cancer Chemother Pharmacol 38:S40-44 (1996); Hodge, et al. Cancer Res 39:5800-07 (1999)). An antigen specific first signal is delivered through the T cell receptor (TCR) via the peptide/major histocompatability complex (MHC) and causes the T cell to enter the cell cycle. A second, or costimulatory, signal may be delivered for cytokine production and proliferation.

At least three distinct molecules normally found on the surface of professional antigen presenting cells (APCs) have been reported as capable of providing the second signal critical for T cell activation: B7-1 (CD80), ICAM-1 (CD54), and LFA-3 (human CD58) (Damle, et al. J Immunol 148:1985-92 (1992); Guinan, et al. Blood 84: 3261-82 (1994); Wingren, et al. Crit Rev Immunol 15: 235-53 (1995); Parra, et al. Scand. J Immunol 38: 508-14 (1993); Hellstrom, et al. Ann NY Acad Sci 690: 225-30 (1993); Parra, et al. J Immunol 158: 637-42 (1997); Sperling, et al. J Immunol 157: 3909 -17 (1996); Dubey, et al. J Immunol 155: 45-57 (1995); Cavallo, et al. Eur J Immunol 25: 1154 -62 (1995)).

These costimulatory molecules have distinct T cell ligands. B7-1 interacts with the CD28 and CTLA-4 molecules, ICAM-1 interacts with the CD11a/CD18 (LFA-1β2 integrin) complex, and LFA-3 interacts with the CD2 (LFA-2) molecules. Therefore, in a certain embodiment, it would be desirable to have a recombinant adenovirus vector that contains B7-1, ICAM-1, and LFA-3, respectively, that, when combined with a recombinant adenovirus-based vector vaccine containing one or more nucleic acids encoding target antigens such as tumor neo-antigens, will further increase/enhance anti-tumor immune responses directed to specific target antigens.

### XIV. Immune Pathway Checkpoint Modulators

In certain embodiments, immune pathway checkpoint inhibitors are combined with compositions comprising adenoviral vectors disclosed herein. In some cases, T cells unleashed by checkpoint inhibitors recognize patient- and cancer-specific neo-epitopes derived from non-synonymous mutations rather than conserved self-antigens. Whole-exome sequencing of pre- and post-treatment tumor tissue has since revealed a strong association of a clinical response to checkpoint inhibition with the frequency of pre-treatment non-synonymous mutations in human melanoma and non-small cell lung cancer, two types of cancers with a particularly high mutational load.

Disclosed herein can be treatment methods comprising treating an individual in need thereof with an adenoviral-based composition and/or an immune pathway checkpoint inhibitor followed by sequencing of a cancer sample. Sequencing can identify new target neo-epitopes. Sequencing can identify a new neo-epitope to be included in a second adenoviral-based composition such as a secondary vaccine. In some embodiments, a patient is vaccinated against a neo-epitope that can be identified by sequencing. In some embodiments, a patient received an immune pathway checkpoint inhibitor in conjunction with a vaccine or pharmaceutical compositions described herein.

In further embodiments, compositions are administered with one or more immune pathway checkpoint modulators. A balance between activation and inhibitory signals regulates the interaction between T lymphocytes and disease cells, wherein T-cell responses are initiated through antigen recognition by the T-cell receptor (TCR). The inhibitory pathways and signals are referred to as immune pathway checkpoints. In normal circumstances, immune pathway checkpoints play a critical role in control and prevention of autoimmunity and also protect from tissue damage in response to pathogenic infection.

Certain embodiments provide combination immunotherapies comprising viral vector-based vaccines and compositions for modulating immune pathway checkpoint inhibitory pathways for the prevention and/or treatment of cancer and infectious diseases. In some embodiments, modulating is increasing expression or activity of a gene or protein. In some embodiments, modulating is decreasing expression or activity of a gene or protein. In some embodiments, modulating affects a family of genes or proteins.

In general, the immune inhibitory pathways are initiated by ligand-receptor interactions. It is now clear that in diseases, the disease can co-opt immune-checkpoint pathways as mechanism for inducing immune resistance in a subject.

The induction of immune resistance or immune inhibitory pathways in a subject by a given disease can be blocked by molecular compositions such as siRNAs, antisense, small molecules, mimic, a recombinant form of ligand, receptor or protein, or antibodies (which can be an Ig fusion protein) that are known to modulate one or more of the Immune Inhibitory Pathways. For example, preliminary clinical findings with blockers of immune-checkpoint proteins, such as Cytotoxic T-lymphocyte-associated antigen 4 (CTLA4) and programmed cell death protein 1 (PD1) have shown promise for enhancing anti-tumor immunity.

Because diseased cells can express multiple inhibitory ligands, and disease-infiltrating lymphocytes express multiple inhibitory receptors, dual or triple blockade of immune pathway checkpoints proteins may enhance anti-disease immunity. Combination immunotherapies as provide herein can comprise one or more compositions comprising an immune pathway checkpoint modulator that targets one or more of the following immune-checkpoint proteins: PD1, PDL1, PDL2, CD28, CD80, CD86, CTLA4, B7RP1, ICOS, B7RPI, B7-H3 (also known as CD276), B7-H4 (also known as B7-S1, B7x and VCTN1), BTLA (also known as CD272), HVEM, KIR, TCR, LAG3 (also known as CD223), CD137, CD137L, OX40, OX40L, CD27, CD70, CD40, CD40L, TIM3 (also known as HAVcr2), GAL9, A2aR and Adenosine.

In some embodiments, the molecular composition comprises a siRNAs. In some embodiments, the molecular composition comprises a small molecule. In some embodiments, the molecular composition comprises a recombinant form of a ligand. In some embodiments, the molecular composition comprises a recombinant form of a receptor. In some embodiments, the molecular composition comprises an antibody. In some embodiments, the combination therapy comprises more than one molecular composition and/or more than one type of molecular composition. As it will be appreciated by those in the art, future discovered proteins of the immune checkpoint inhibitory pathways are also envisioned to be encompassed by the present disclosure.

In some embodiments, combination immunotherapies comprise molecular compositions for the modulation of CTLA4. In some embodiments, combination immunotherapies comprise molecular compositions for the modulation of PD1. In some embodiments, combination immunotherapies comprise molecular compositions for the modulation of PDL1. In some embodiments, combination immunotherapies comprise molecular compositions for the modulation of LAG3. In some embodiments, combination immunotherapies comprise molecular compositions for the modulation of B7-H3. In some embodiments, combination immunotherapies comprise molecular compositions for the modulation of B7-H4. In some embodiments, combination immunotherapies comprise molecular compositions for the modulation of TIM3. In some embodiments, modulation is an increase or enhancement of expression. In other embodiments, modulation is the decrease of absence of expression.

Two non-limiting exemplary immune pathway checkpoint inhibitors include the cytotoxic T lymphocyte associated antigen-4 (CTLA-4) and the programmed cell death protein-1 (PD1). CTLA-4 can be expressed exclusively on T-cells where it regulates early stages of T-cell activation. CTLA-4 interacts with the co-stimulatory T-cell receptor CD28 which can result in signaling that inhibits T-cell activity. Once TCR antigen recognition occurs, CD28 signaling may enhances TCR signaling, in some cases leading to activated T-cells and CTLA-4 inhibits the signaling activity of CD28. The present disclosure provides immunotherapies as provided herein in combination with anti-CTLA-4 monoclonal antibody for the prevention and/or treatment of cancer and infectious diseases. The present disclosure provides vaccine or immunotherapies as provided herein in combination with CTLA-4 molecular compositions for the prevention and/or treatment of cancer and infectious diseases.

Programmed death cell protein ligand-1 (PDL1) is a member of the B7 family and is distributed in various tissues and cell types. PDL1 can interact with PD1 inhibiting T-cell activation and CTL mediated lysis. Significant expression of PDL1 has been demonstrated on various human tumors and PDL1 expression is one of the key mechanisms in which tumors evade host anti-tumor immune responses. Programmed death-ligand 1 (PDL1) and programmed cell death protein-1 (PD1) interact as immune pathway checkpoints. This interaction can be a major tolerance mechanism which results in the blunting of anti-tumor immune responses and subsequent tumor progression. PD1 is present on activated T cells and PDL1, the primary ligand of PD1, is often expressed on tumor cells and antigen-presenting cells (APC) as well as other cells, including B cells. Significant expression of PDL1 has been demonstrated on various human tumors including HPV-associated head and neck cancers. PDL1 interacts with PD1 on T cells inhibiting T cell activation and cytotoxic T lymphocyte (CTL) mediated lysis. The present disclosure provides immunotherapies as provided herein in combination with anti-PDl or anti-PDLl monoclonal antibody for the prevention and/or treatment of cancer and infectious diseases.

Certain embodiments may provide immunotherapies as provided herein in combination with PD1 or anti-PDLl molecular compositions for the prevention and/or treatment of cancer and infectious diseases. Certain embodiments may provide immunotherapies as provided herein in combination with anti-CTLA-4 and anti-PDl monoclonal antibodies for the prevention and/or treatment of cancer and infectious diseases. Certain embodiments may provide immunotherapies as provided herein in combination with anti-CTLA-4 and PDL1 monoclonal antibodies. Certain embodiments may provide vaccine or immunotherapies as provided herein in combination with anti-CTLA-4, anti-PDl, anti-PDL1 monoclonal antibodies, or a combination thereof, for the treatment of cancer and infectious diseases.

Immune pathway checkpoint molecules can be expressed by T cells. Immune pathway checkpoint molecules can effectively serve as "brakes" to down-modulate or inhibit an immune response. Immune pathway checkpoint molecules include, but are not limited to Programmed Death 1 (PD1 or PD-1, also known as PDCD1 or CD279, accession number: NM_005018), Cytotoxic T-Lymphocyte Antigen 4 (CTLA-4, also known as CD152, GenBank accession number AF414120.1), LAG3 (also known as CD223, accession number: NM_002286.5), Tim3 (also known as hepatitis A virus cellular receptor 2 (HAVCR2), GenBank accession number: JX049979.1), B and T lymphocyte associated (BTLA) (also known as CD272, accession number: NM_181780.3), BY55 (also known as CD160, GenBank accession number: CR541888.1), TIGIT (also known as IVSTM3, accession number: NM_173799), LAIR1 (also known as CD305, GenBank accession number: CR542051.1), SIGLECIO (GenBank accession number: AY358337.1), natural killer cell receptor 2B4 (also known as CD244, accession number: NM_001166664.1), PPP2CA, PPP2CB, PTPN6, PTPN22, CD96, CRTAM, SIGLEC7, SIGLEC9, TNFRSF10B, TNFRSF10A, CASP8, CASP10, CASP3, CASP6, CASP7, FADD, FAS, TGFBRII, TGFRBRI, SMAD2, SMAD3, SMAD4, SMAD10, SKI, SKIL, TGIF1, ILIORA, IL10RB, HMOX2, IL6R, IL6ST, EIF2AK4, CSK, PAG1, SIT1, FOXP3, PRDM1, BATF, GUCY1A2, GUCY1A3, GUCY1B2, GUCY1B3 which directly inhibit immune cells. For example, PD1 can be combined with an adenoviral vector-based composition to treat a patient in need thereof.

Additional immune pathway checkpoints that can be targeted can be adenosine A2a receptor (ADORA), CD276, V-set domain containing T cell activation inhibitor 1 (VTCN1),, indoleamine 2,3-dioxygenase 1 (IDO1), killer cell immunoglobulin-like receptor, three domains, long cytoplasmic tail, 1 (KIR3DL1), V-domain immunoglobulin suppressor of T-cell activation (VISTA), cytokine inducible SH2-containing protein (CISH), hypoxanthine phosphoribosyltransferase 1 (HPRT), adeno-associated virus integration site 1 (AAVS1), or chemokine (C-C motif) receptor 5 (gene/pseudogene) (CCR5), or any combination thereof.

**Table 3,** without being exhaustive, shows exemplary immune pathway checkpoint genes that can be inactivated to improve the efficiency of the adenoviral vector-based composition as described herein. Immune pathway checkpoints gene can be selected from such genes listed in Table 1 and others involved in co-inhibitory receptor function, cell death, cytokine signaling, arginine tryptophan starvation, TCR signaling, Induced T-reg repression, transcription factors controlling exhaustion or anergy, and hypoxia mediated tolerance.

**TABLE 3: Exemplary immune pathway checkpoint genes**

| **Gene Symbol** | **NCBI # (GRCh38.p2)** | **Start** | **Stop** | **Genome location** |
|---|---|---|---|---|
| ADORA2A | 135 | 24423597 | 24442360 | 22q11.23 |
| CD276 | 80381 | 73684281 | 73714518 | 15q23-q24 |
| VTCN1 | 79679 | 117143587 | 117270368 | 1p13.1 |
| BTLA | 151888 | 112463966 | 112499702 | 3q13.2 |
| CTLA4 | 1493 | 203867788 | 203873960 | 2q33 |
| IDO1 | 3620 | 39913809 | 39928790 | 8p12-p11 |
| KIR3DL1 | 3811 | 54816438 | 54830778 | 19q13.4 |
| LAG3 | 3902 | 6772483 | 6778455 | 12p13.32 |
| PDCD1 | 5133 | 241849881 | 241858908 | 2q37.3 |
| HAVCR2 | 84868 | 157085832 | 157109237 | 5q33.3 |
| VISTA | 64115 | 71747556 | 71773580 | 10q22.1 |
| CD244 | 51744 | 160830158 | 160862902 | 1q23.3 |
| CISH | 1154 | 50606454 | 50611831 | 3p21.3 |

The combination of an adenoviral-based composition and an immune pathway checkpoint modulator may result in reduction in infection, progression, or symptoms of a disease in treated patients, as compared to either agent alone. In another embodiment, the combination of an adenoviral-based composition and an immune pathway checkpoint modulator may result in improved overall survival of treated patients, as compared to either agent alone. In some cases, the combination of an adenoviral-based composition and an immune pathway checkpoint modulator may increase the frequency or intensity of disease-specific T cell responses in treated patients as compared to either agent alone.

Certain embodiments may also provide the use of immune pathway checkpoint inhibition to improve performance of an adenoviral vector-based composition. Certain immune pathway checkpoint inhibitors may be administered at the time of an adenoviral vector-based composition. Certain immune pathway checkpoint inhibitors may also be administered after the administration of an adenoviral vector-based composition. Immune pathway checkpoint inhibition may occur simultaneously to an adenoviral vaccine administration. Immune pathway checkpoint inhibition may occur 1, 2, 3, 4, 5, 6, 7, 8, 9, 10, 15, 20, 30, 40, 50, or 60 minutes after vaccination. Immune pathway checkpoint inhibition may also occur 1, 2, 3, 4, 5, 6, 7, 8, 9, 10, 11, 12, 13, 14, 15, 16, 17, 18, 19, 20, 21, 22, 23, or 24 hours after the administration of an adenoviral vector-based composition. In some cases, immune inhibition may occur 1, 2, 3, 4, 5, 6, or 7 days after vaccination. Immune pathway checkpoint inhibition may occur at any time before or after the administration of an adenoviral vector-based composition.

In another aspect, there are provided methods involving a vaccine comprising one or more nucleic acids encoding an antigen and an immune pathway checkpoint modulator. For example, there is provided a method for treating a subject having a condition that would benefit from downregulation of an immune pathway checkpoint protein, PD1 for example, and its natural binding partner(s) on cells of the subject.

An immune pathway checkpoint modulator may be combined with an adenoviral vector-based composition comprising one or more nucleic acids encoding any antigen. For example, an antigen can be a tumor antigen, such as a tumor neo-antigen or tumor neo-epitope, or any antigen described herein.

An immune pathway checkpoint modulator may produce a synergistic effect when combined with an adenoviral vector-based composition, such as a vaccine. An immune pathway checkpoint modulator may also produce a beneficial effect when combined with an adenoviral vector-based composition.

### XV. Cancer Treatment

It is specifically contemplated that compositions comprising adenoviral vectors described herein can be used to evaluate or treat stages of disease, such as between hyperplasia, dysplasia, neoplasia, pre-cancer and cancer, or between a primary tumor and a metastasized tumor.

As used herein, the terms "neoplastic cells" and "neoplasia" may be used interchangeably and refer to cells which exhibit relatively autonomous growth, so that they exhibit an aberrant growth phenotype characterized by a significant loss of control of cell proliferation. Neoplastic cells can be malignant or benign. In particular aspects, a neoplasia includes both dysplasia and cancer. Neoplasms may be benign, pre-malignant (carcinoma *in situ* or dysplasia) or malignant (cancer). Neoplastic cells may form a lump (i.e., a tumor) or not.

The term "dysplasia" may be used when the cellular abnormality is restricted to the originating tissue, as in the case of an early, in-situ neoplasm. Dysplasia may be indicative of an early neoplastic process. The term "cancer" may refer to a malignant neoplasm, including a broad group of various diseases involving unregulated cell growth.

Metastasis, or metastatic disease, may refer to the spread of a cancer from one organ or part to another non-adjacent organ or part. The new occurrences of disease thus generated may be referred to as metastases.

Cancers that may be evaluated or treated by the disclosed methods and compositions include cancer cells from the pancreas, including pancreatic ductal adenocarcinoma (PDAC), cancer cells from the bladder, blood, bone, bone marrow, brain, breast, colon, esophagus, gastrointestine, gum, head, kidney, liver, lung, nasopharynx, neck, ovary, prostate, skin, stomach, testis, tongue, or uterus.

In addition, the cancer may specifically be of the following histological type, though it is not limited to these: neoplasm, malignant; carcinoma; carcinoma, undifferentiated; giant and spindle cell carcinoma; small cell carcinoma; papillary carcinoma; squamous cell carcinoma; lymphoepithelial carcinoma; basal cell carcinoma; pilomatrix carcinoma; transitional cell carcinoma; papillary transitional cell carcinoma; adenocarcinoma; gastrinoma, malignant; cholangiocarcinoma; hepatocellular carcinoma; combined hepatocellular carcinoma and cholangiocarcinoma; trabecular adenocarcinoma; adenoid cystic carcinoma; adenocarcinoma in adenomatous polyp; adenocarcinoma, familial polyposis coli; solid carcinoma; carcinoid tumor, malignant; branchiolo-alveolar adenocarcinoma; papillary adenocarcinoma; chromophobe carcinoma; acidophil carcinoma; oxyphilic adenocarcinoma; basophil carcinoma; clear cell adenocarcinoma; granular cell carcinoma; follicular adenocarcinoma; papillary and follicular adenocarcinoma; nonencapsulating sclerosing carcinoma; adrenal cortical carcinoma; endometroid carcinoma; skin appendage carcinoma; apocrine adenocarcinoma; sebaceous adenocarcinoma; ceruminous adenocarcinoma; mucoepidermoid carcinoma; cystadenocarcinoma; papillary cystadenocarcinoma; papillary serous cystadenocarcinoma; mucinous cystadenocarcinoma; mucinous adenocarcinoma; signet ring cell carcinoma; infiltrating duct carcinoma; medullary carcinoma; lobular carcinoma; inflammatory carcinoma; paget's disease, mammary; acinar cell carcinoma; adenosquamous carcinoma; adenocarcinoma w/squamous metaplasia; thymoma, malignant; ovarian stromal tumor, malignant; thecoma, malignant; granulosa cell tumor, malignant; androblastoma, malignant; sertoli cell carcinoma; leydig cell tumor, malignant; lipid cell tumor, malignant; paraganglioma, malignant; extra-mammary paraganglioma, malignant; pheochromocytoma; glomangiosarcoma; malignant melanoma; amelanotic melanoma; superficial spreading melanoma; malig melanoma in giant pigmented nevus; epithelioid cell melanoma; blue nevus, malignant; sarcoma; fibrosarcoma; fibrous histiocytoma, malignant; myxosarcoma; liposarcoma; leiomyosarcoma; rhabdomyosarcoma; embryonal rhabdomyosarcoma; alveolar rhabdomyosarcoma; stromal sarcoma; mixed tumor, malignant; mullerian mixed tumor; nephroblastoma; hepatoblastoma; carcinosarcoma; mesenchymoma, malignant; brenner tumor, malignant; phyllodes tumor, malignant; synovial sarcoma; mesothelioma, malignant; dysgerminoma; embryonal carcinoma; teratoma, malignant; struma ovarii, malignant; choriocarcinoma; mesonephroma, malignant; hemangiosarcoma; hemangioendothelioma, malignant; kaposi's sarcoma; hemangiopericytoma, malignant; lymphangiosarcoma; osteosarcoma; juxtacortical osteosarcoma; chondrosarcoma; chondroblastoma, malignant; mesenchymal chondrosarcoma; giant cell tumor of bone; ewing's sarcoma; odontogenic tumor, malignant; ameloblastic odontosarcoma; ameloblastoma, malignant; ameloblastic fibrosarcoma; pinealoma, malignant; chordoma; glioma, malignant; ependymoma; astrocytoma; protoplasmic astrocytoma; fibrillary astrocytoma; astroblastoma; glioblastoma; oligodendroglioma; oligodendroblastoma; primitive neuroectodermal; cerebellar sarcoma; ganglioneuroblastoma; neuroblastoma; retinoblastoma; olfactory neurogenic tumor; meningioma, malignant; neurofibrosarcoma; neurilemmoma, malignant; granular cell tumor, malignant; malignant lymphoma; Hodgkin's disease; Hodgkin's lymphoma; paragranuloma; malignant lymphoma, small lymphocytic; malignant lymphoma, large cell, diffuse; malignant lymphoma, follicular; mycosis fungoides; other specified non-Hodgkin's lymphomas; malignant histiocytosis; multiple myeloma; mast cell sarcoma; immunoproliferative small intestinal disease; leukemia; lymphoid leukemia; plasma cell leukemia; erythroleukemia; lymphosarcoma cell leukemia; myeloid leukemia; basophilic leukemia; eosinophilic leukemia; monocytic leukemia; mast cell leukemia; megakaryoblastic leukemia; myeloid sarcoma; and hairy cell leukemia. Moreover, tumor neo-antigens or neo-epitopes can be evaluated in precancers, such as metaplasia, dysplasia, and hyperplasia.

### XVI. Kits

The compositions, immunotherapy or vaccines described herein may be supplied in the form of a kit. The kits of the present disclosure may further comprise instructions regarding the dosage and or administration including treatment regimen information.

In some embodiments, kits comprise the compositions and methods for providing immunotherapy or vaccines described. In some embodiment's kits may further comprise components useful in administering the kit components and instructions on how to prepare the components. In some embodiments, the kit can further comprise software for conducting monitoring patient before and after treatment with appropriate laboratory tests, or communicating results and patient data with medical staff.

The components comprising the kit may be in dry or liquid form. If they are in dry form, the kit may include a solution to solubilize the dried material. The kit may also include transfer factor in liquid or dry form. If the transfer factor is in dry form, the kit will include a solution to solubilize the transfer factor. The kit may also include containers for mixing and preparing the components. The kit may also include instrument for assisting with the administration such for example needles, tubing, applicator, inhalant, syringe, pipette, forceps, measured spoon, eye dropper or any such medically approved delivery vehicle. The kits or drug delivery systems as described herein also will typically include a means for containing compositions of the present disclosure in close confinement for commercial sale and distribution.

### XVII. Tangible Computer-Readable Medium

There may be provided tangible computer-readable medium having computer usable program code executable to perform operations related to identification, classification, and selection of tumor neo-epitopes or tumor neo-antigens.

A processor or processors can be used in performance of the operations driven by the example tangible computer-readable media disclosed herein. Alternatively, the processor or processors can perform those operations under hardware control, or under a combination of hardware and software control. For example, the processor may be a processor specifically configured to carry out one or more those operations, such as an application specific integrated circuit (ASIC) or a field programmable gate array (FPGA). The use of a processor or processors allows for the processing of information (e.g., data) that is not possible without the aid of a processor or processors, or at least not at the speed achievable with a processor or processors.

Some embodiments of the performance of such operations may be achieved within a certain amount of time, such as an amount of time less than what it would take to perform the operations without the use of a computer system, processor, or processors, including no more than one hour, no more than 30 minutes, no more than 15 minutes, no more than 10 minutes, no more than one minute, no more than one second, and no more than every time interval in seconds between one second and one hour.

Some embodiments of the present tangible computer-readable media may be, for example, a CD-ROM, a DVD-ROM, a flash drive, a hard drive, or any other physical storage device. Some embodiments of the present methods may include recording a tangible computer-readable medium with computer-readable code that, when executed by a computer, causes the computer to perform any of the operations discussed herein, including those associated with the present tangible computer-readable media. Recording the tangible computer-readable medium may include, for example, burning data onto a CD-ROM or a DVD-ROM, or otherwise populating a physical storage device with the data.

The various embodiments described above can be combined to provide further embodiments. All of the U.S. patents, U.S. patent application publications, U.S. patent application, foreign patents, foreign patent application and non-patent publications referred to in this specification and/or listed in the Application Data Sheet are incorporated herein by reference, in their entirety to the same extent as if each individual publication, patent, or patent application was specifically and individually indicated to be incorporated by reference.

Aspects of the embodiments can be modified, if necessary to employ concepts of the various patents, application and publications to provide yet further embodiments.

These and other changes can be made to the embodiments in light of the above-detailed description. In general, in the following claims, the terms used should not be construed to limit the claims to the specific embodiments disclosed in the specification and the claims, but should be construed to include all possible embodiments along with the full scope of equivalents to which such claims are entitled. Accordingly, the claims are not limited by the disclosure.

### EXAMPLES

The following examples are included to demonstrate preferred embodiments of the invention. It should be appreciated by those of skill in the art that the techniques disclosed in the examples which follow represent techniques discovered by the inventor to function well in the practice of the invention, and thus can be considered to constitute preferred modes for its practice. However, those of skill in the art should, in light of the present disclosure, appreciate that many changes can be made in the specific embodiments which are disclosed and still obtain a like or similar result without departing from the spirit and scope of the invention.

### EXAMPLE 1

### Construction of Multiple Neo-Epitope Vector (Alkbh6.2, Slit3, and Atxn10.1) for Insertion into Ad5 [E1-, E2b-]

**Construction of Ad5 [E1-, E2b-] Vector:** The approximately 20 kb *Xba-Bam*HI subfragment of pBHG11 (Bett, et al 1994, Microbix, Toronto, Ontario, Canada) was subcloned into pBluescriptKSII+ (Stratagene, La Jolla, Calif.), yielding pAXB. Plasmid pAXB was digested with *Bsp*EI*,* T4 DNA polymerase end filled, and *Bam*HI digested, and the approximately 9.0 kb fragment was isolated. Plasmid pAXB was also digested with *Bsp*HI*,* T4 DNA polymerase end filled, and *Bam*HI digested, and the approximately 13.7 kb fragment was ligated to the previously isolated 9.0 kb fragment, generating pAXB-Δpol.

This subcloning strategy deleted 608 bp (Δpol; Ad5 nucleotides 7274 to 7881) within the amino terminus of the polymerase gene. This deletion also effectively removed open reading frame 9.4 present on the rightward reading strand in this region of the Ad genome. The *Xba-Bam*HI subfragment of pAXB-Δpol was reintroduced into *Xba-Bam*HI-digested pBHG11, to generate pBHG11-Δpol.

**Construction of the Ad5 [E1-, E2b-]-HER2/neu Vaccine:** A neo-epitope transgene flanked by a minimal cytomegalovirus promoter/enhancer element and the SV40 derived poly adenylation signal was subcloned into the shuttle pShuttleCMV, generating the shuttle plasmid pShuttle CMV/Neo-epitope. The shuttle plasmid was linearized with PmeI and homologously recombined (in E.coli bacteria) with the plasmid pAdΔpp to generate pAdCMV/Neo-epitope/Δpp. Ten micrograms of pAdCMV/ Neo-epitope/Δpp linearized with PacI was CaPO₄ cotransfected into Ad E1, polymerase (E2b) and pTP-expressing (E.C7 cells). Sixteen hours after transfection, the cells were harvested and the cell mixture was distributed into nine 24-well tissue culture cluster plates and incubated at 37 °C for 5 to 9 days. Individual wells demonstrating viral cytopathic effects were harvested, and the isolated virus was amplified by repeated infection of greater numbers of E.C7 cells. Isolation of the Ad5 [E1-, E2b-]- Neo-epitope recombinant vector was subsequently confirmed by (1) DNA restriction mapping of the vector genome, (2) confirmation of expression of neo-epitope and (3) multiple functional studies.

In the neo-epitope transgene, multiple individual neo-epitope gene sequences are separated by "self-cleaving" 2A peptide derived from *Porcine teschovirus-1* and *Thosea asigna* virus, respectively (de Felipe P, et al. Traffic 2004; 5(8), 616-26; Holst J, et al. Nature Immunol. 2008; 9:658-66; Kim JH, et al. PloS One, 2011; 6(4), e18556. doi:10.1371/journal.pone.0018556). As the 2A peptides are translated on the ribosome, the peptide bond between the final two residues of the 2A peptide is never formed, resulting in distinctly expressed proteins in one ribosomal pass. The use of two 2A peptide sequences separating the three genes results in near stoichiometric expression of the three proteins.

### EXAMPLE 2

### Multiple Injections of an Ad5 [E1-, E2b-]-Vector Containing Alkbh6.2, Slit3, and Atxn10.1 Generates Cell-Mediated Immune Responses against These Neo-Epitopes

Mice are immunized with mutant peptides Alkbh6.2, Slit3, and Atxn10.1 or an empty vector control two times, two weeks apart. Draining lymph nodes are harvested 7 days after immunization termination. Lymph nodes are cultured *in vitro* and subsequently stimulated with cognate peptide or unstimulated for 20 hours and then analyzed by ELISpot. Spleens are obtained from individual mice two (2) weeks after the last immunization (vaccination) and assessed for CMI employing ELISpot assays for IFN- secreting splenocytes (Gabitzsch ES, et al. Cancer Immunol Immunother. 2010; 59:1131-35; Gabitzsch ES, et al. Cancer Gene Ther. 2011; 18:326-35; Jones FR, et al. Vaccine 2011; 29:7020-26).

### EXAMPLE 3

### Vaccination with Ad5 [E1-, E2b-]-Vector Containing Alkbh6.2, Slit3, and Atxn10.1 Generates Cell-Mediated Immune Responses against These Neo-Epitopes In Vivo

Groups of five (5) mice each are immunized with doses of 1X10⁸, 1X10⁹, or 1X10¹⁰ VP Ad5 [E1-, E2b-]- Alkbh6.2, Slit3, and Atxn10.1 or an empty control vector subcutaneously at two (2) week intervals. One week later, the mice are challenged intradermally with 300,000 live CMS5-FFLuc tumor cells. Tumor growth is monitored using live bioluminescent imaging (Perkin Elmer). Mouse blood is collected, T cells isolated, and stimulated *in vitro* with cognate peptide, stimulated with an irrelevant peptide, or unstimulated. Culturing media is analyzed for IFN-gamma and IL-2 production. Flow cytometry is performed on mouse T cells for intracellular IFN-gamma production.

### EXAMPLE 4

### Pilot Study to Assess the Safety, Feasibility, and Preliminary Efficacy of a Neo-Epitope-Based Personalized Vaccine Approach in Patients with Pancreatic Cancer

A clinical trial employing the Ad5 [E1-, E2b-]-CEA (carcinoembryonic antigen) platform vaccine for immunotherapy in CEA-expressing pancreatic cancer patients is followed by subsequent vaccinations with an Ad5 [E1-, E2b-]-neo-epitope targeted vector. This is a phase I/II study with the primary purpose to determine the safety of immunization with Ad5 [E1-, E2B-]-CEA (6D), in patients with advanced or metastatic CEA-expressing malignancies. The secondary objectives are to evaluate neo-epitope immune responses to the immunizations and to obtain preliminary data on clinical feasibility of generating a neo-epitope targeted vaccine for pancreatic cancer patients previously treated with vaccination.

The study population consists of patients with a histologically confirmed diagnosis of metastatic malignancy that is CEA positive who are previously treated with standard therapy known to have a possible survival benefit or refused such therapy. The study determines the safety of three dosage levels of Ad5 [E1-, E2B-]-CEA (6D) vaccine (phase I component), and the safety and feasibility of generating an Ad5 [E1-, E2B-]-neo-epitope vaccine.

The study drug is Ad5 [E1-, E2B-]-CEA (6D) given by subcutaneous (SQ) injection every 3 weeks for 3 immunizations. Safety is evaluated in each cohort at least 3 weeks after the last patient in the previous cohort has received their first injection. A dosing scheme is considered safe if <33% of patients treated at a dosage level experience DLT (e.g., 0 of 3, ≤1 of 6, ≤3 of 12 or ≤5 of 18 patients).

Following the third week of immunization, patient tumor samples are acquired and processed for sequencing (see **FIG. 1****).** Whole genome sequencing of a patient's matched tumor sample and normal sample pinpoint tumor-specific alterations at the DNA level, RNA sequencing confirms and gives relevance to mutations or SNVs in DNA. Quantitative proteomics is also performed to measure the levels of clinically important proteins of identified SNVs. Putative SNVs will undergo MHC class I binding prediction with a cut off of (IC≤500). A finalized set of SNVs are cloned in frame with the Ad5 [E1-, E2B-] vector to produce a neo-epitope specific Ad5 [E1-, E2B-] vaccine. Patients are subsequently vaccinated by subcutaneous (SQ) injection every 3 weeks for 3 immunizations using the Ad5 [E1-, E2B-]-neo-epitope vector.

### EXAMPLE 5

### A Neo-Epitope-Based Personalized Vaccine Approach in Patients with Pancreatic Cancer

Tumor neo-epitopes are isolated from patients with a pancreatic tumor that have been treated with Ad5 [E1-, E2b-]-CEA (carcinoembryonic antigen) and subsequently with a check point inhibitor (anti-PDL-1). Tumor-specific mutations like SNVs are identified by comparing the genome sequence in a tumor biopsy of the patients with normal skin tissues. SNVs are determined if they are expressed and SNVs are determined if they drive cell-mediated immunity. Among the 70 tumor neo-antigens in the genome, only 10 of them drive cell-mediated immunity. The identified neo-antigens are inserted into a library of Ad5 [E1-, E2b-] vectors for delivery to human pancreatic cancer patients. The identification of new neo-antigens and treatment with newly identified new neo-antigens are repeated in cycles.

### EXAMPLE 6

### Identification of Tumor Neo-Antigens

Candidate tumor neo-epitopes are identified based on the methods illustrated in **FIG.** 1 or **FIG. 2****.**

Tumor-specific mutations in cancer samples are detected using DNA sequencing such as whole exome sequencing (WES) or whole genome sequencing (WGS) and identified through the application of mutation calling algorithms (such as Mutect).

Expressed mutations are identified by RNA sequencing or indirect RNA analysis to provide candidate neo-antigens or neo-epitopes.

Next, candidate neo-antigens or neo-epitopes can be identified from these expressed mutations based on HLA typing or predicted binding affinity to MHC I or MHC II, e.g., using well-validated algorithms (NetMHCpan), and their identification can be refined by experimental validation for peptide-HLA binding and by confirmation of gene expression at the RNA level.

These candidate neo-antigens or neo-epitopes can be subsequently synthesized and tested for their ability to stimulate tumor-specific T-cell responses to demonstrate if they are immunogeneic neo-antigens or neo-epitopes

### EXAMPLE 7

### Identification of Tumor Neo-Antigens in a Liquid Tumor Sample

### Patient samples

Heparinized blood is obtained from patients enrolled on clinical research protocols. Patient peripheral blood mononuclear cells (PBMCs) are isolated by Ficoll/Hypaque density-gradient centrifugation, cryopreserved with 10% dimethylsulfoxide, and stored in vapor-phase liquid nitrogen until time of analysis. HLA typing is performed.

### Whole-exome capture sequencing data for CLL and other cancers

Somatic mutations are detected in CLL by whole-exome capture sequencing or whole-genome sequencing (WGS) with matched sequencing of germ-line DNA or via comparison with a normal reference.

### Prediction of peptides derived from gene mutations with binding to personal HLA alleles

Major histocompatibility complex (MHC)-binding affinity is predicted across all possible 9- and 10-mer peptides generated from each somatic mutation and the corresponding wild-type peptides using NetMHCpan (v2.4). These tiled peptides are analyzed for their binding affinities (IC₅₀ nM) to each class I allele in the patients' HLA profile. An IC₅₀ value of <150 nM is considered a predicted strong binder; between 150 and 500 nM, an intermediate to weak binder; and >500 nM, a nonbinder. Predicted peptides binding to HLA molecules (IC₅₀ <500 nM) by competitive MHC class I allele-binding experiments are empirically confirmed.

### Generation and detection of patient antigen-specific T cells

Autologous dendritic cells (DCs) are generated. For some experiments, CD40L-Tri activated and expanded CD19⁺ B cells are used as antigen-presenting cells (APCs).

To generate peptide-reactive T cells from CLL patients, immunomagnetically selected CD8⁺ T cells (10 million) from pre- and posttransplant PBMCs (CD8⁺ Microbeads; Miltenyi, Auburn, CA) are cultured with autologous peptide pool-pulsed DCs (at a 40:1 ratio). Subsequently, T cells are restimulated weekly (starting on day 7) with peptide-pulsed CD40L-Tri-activated irradiated B cells (at 4:1 ratio) either once more, to detect memory T-cell responses, or thrice more, to detect naive T-cell responses. All stimulations are conducted in complete medium supplemented with 10% fetal bovine serum and 5 to 10 ng/mL interleukin (IL)-7, IL-12, and IL-15 (R&D Systems, Minneapolis, MN). APCs are pulsed with peptide pools (10 µM/peptide/pool for 3 hours). T-cell specificity against peptide pools or autologous tumor is tested by interferon (IFN)-γ ELISPOT or a CD107a degranulation assay 10 days following the last stimulation.

### Statistical considerations

Two-way analysis of variance models are constructed for cytokine secretion measurements and included concentration and mutational status as fixed effects along with an interaction term as appropriate. P values for these models are adjusted for multiple comparisons post hoc (Tukey method). For other comparisons of continuous measures between groups, a Welch t test is used. All other P values reported are 2-sided and considered significant at the .05 level with appropriate adjustment for multiple comparisons. Analyses are performed in SAS, version 9.2.

### EXAMPLE 8

### Identification of Tumor Neo-antigens in a Solid Tumor Sample

Unique antigens are first identified in cell lines derived from primary surgical specimens of patients with CRC. Single cell suspensions from cancer specimens are cultured in standard conditions to obtain "differentiated" cancer cells and, when possible, also in serum-free conditions to support the generation of colon spheres displaying CSC characteristics.

The cDNAs encoding the 20 most frequently mutated candidate cancer genes in CRC are PCR-amplified from eight differentiated CRC cell lines and two parallel CSC cultures and subjected to massively parallel sequencing. Somatic mutations are found in several of the 20 expressed genes in all CRC cells.

The mutations found in the CRC cDNAs are compared with the DNA obtained from healthy cells (PBMCs or LCLs) of the same patients, and the sequencing of the 20 most frequently mutated genes in CRC provided several somatic mutations per tumor, which are a potential source of unique T cell neo-epitopes.

PBMCs are provided to investigate T cell recognition of epitopes derived from the mutated gene products. PBMCs from a patient is stimulated at least twice *in vitro* with pools of synthetic peptides consisting, for each mutated protein, of three 15 aa long peptides spanning the mutated residues and overlapping by 11 residues. This approach is based on the evidence that 15 aa long peptides are naturally processed by APCs into epitopes that are presented by MHC class I and II molecules to autologous T cells, without prior knowledge of the exact HLA allele-specific epitope structure.

CD8+ T cells isolated from the stimulated PBMCs are tested for the recognition of the autologous cancer cells, which expressed HLA-A, B, C and HLA-DR upon IFNγ treatment. These results prove that the induced CD8+ T cells are specific for a naturally processed neo-epitope presented by the patient's specific HLA.

### EXAMPLE 9

### Ad5 [E1-, E2b-] Vector Constructs of Tumor Neo-Epitopes

A selected pool of candidate tumor neo-epitopes was identified in **TABLE 4.**

**TABLE 4: Candidate tumor neo-epitopes**

| **Genes** | **Mutations** | **Selected Neo-epitopes** | **Selected Neo-epitopes** |
|---|---|---|---|
| VIPR2 | V73M | GETVTMPCP (SEQ ID NO:1) | |
| LILRB3 | T187N | VGPVNPSHR (SEQ ID NO:2) | |
| FCRL1 | R286C | GLGAQCSEA (SEQ ID NO:3) | |
| FAT4 | S1613L | RKLTTELTI (SEQ ID NO:4) | PERRKLTTE (SEQ ID NO:5) |
| PIEZO2 | T2356M | MDWVWMDTT (SEQ ID NO:6) | VWMDTTLSL (SEQ ID NO:7) |
| SIGLEC14 | A292T | GKTLNPSQT (SEQ ID NO:8) | REGKTLNPS (SEQ ID NO:9) |
| SIGLEC1 | D1143N | VRNATSYRC (SEQ ID NO:10) | NVTVRNATS (SEQ ID NO:11) |
| SLC4A11 | Q678P | FAMAQIPSL (SEQ ID NO:12) | AQIPSLSLR (SEQ ID NO:13) |

Among the candidate neo-epitopes in **TABLE 4,** a database SNP variant LILRB3 (T187N mutation in VGPVNPSHR, corresponding to Rs71257443, occurring in 28% of the population) was removed.

Next, RNA sequencing (RNA-seq) was performed, and the RNA-seq data was used to prioritize candidate neo-epitopes. Because one gene FLRT2 was detected in RNA sequencing, it is probably well expressed in the tumor sample and should be prioritized as a target.

Extended 15-mer neo-epitopes of these identified candidate tumor neo-epitopes is summarized in **TABLE 5.**

**TABLE 5: Extended neo-epitopes**

| **Gene** | **Protein Change** | **Neo-Epitope** | **Neo-Epitope** | **Extended 15-mer** | **Nucleotide Sequence** |
|---|---|---|---|---|---|
| SLC4A11 | Q678P | FAMAQIPSL | AQIPSLSLR | PFAMAQIPSLSL RAV (SEQ ID NO:14) | CCCTTCGCCATG GCCCAGATCCCC AGCCTGAGCCTG AGGGCCGTG (SEQ ID NO:23) |
| | | (SEQ ID NO:12) | (SEQ ID NO:13) | | |
| SIGLEC1 | D1143N | VRNATSYRC | NVTVRNATS | LPNVTVRNATS YRCG (SEQ ID NO:15) | CTGCCCAACGTG ACCGTGAGGAAC GCCACCAGCTAC AGGTGCGGC (SEQ ID NO:24) |
| | | (SEQ ID NO:10) | (SEQ ID NO:11) | | |
| SIGLEC14 | A292T | GKTLNPSQT | REGKTLNPS | SWFREGKTLNP SQTS (SEQ ID NO:16) | AGCTGGTTCAGG GAGGGCAAGACC CTGAACCCCAGC CAGACCAGC (SEQ ID NO:25) |
| | | (SEQ ID NO:8) | (SEQ ID NO:9) | | |
| PIEZO2 | T2356M | MDWVWMDTT | VWMDTTLSL | AVMDWVWMD TTLSLS (SEQ ID NO:17) | GCCGTGATGGAC TGGGTGTGGATG GACACCACCCTG AGCCTGAGC (SEQ ID NO:26) |
| | | (SEQ ID NO:6) | (SEQ ID NO:7) | | |
| FAT4 | S1613L | RKLTTELTI | PERRKLTTE | LGPERRKLTTEL TII (SEQ ID NO:18) | CTGGGCCCCGAG AGGAGGAAGCTG ACCACCGAGCTG ACCATCATC (SEQ ID NO:27) |
| | | (SEQ ID NO:4) | (SEQ ID NO:5) | | |
| FCRL1 | R286C | GLGAQCSEA | | NNGLGAQCSEA VTLN (SEQ ID NO:19) | AACAACGGCCTG GGCGCCCAGTGC AGCGAGGCCGTG ACCCTGAAC (SEQ ID NO:28) |
| | | (SEQ ID NO:3) | | | |
| VIPR2 | V73M | GETVTMPCP | | NVGETVTMPCP KVFS | AACGTGGGCGAG ACCGTGACCATG CCCTGCCCCAAG |
| | | SEQ ID NO:1) | | | |
| | | | | (SEQ ID NO:20) | GTGTTCAGC (SEQ ID NO:29) |
| | | ( | | | |
| FLRT2 | R346W | EQVWGMAVR | | CQGPEQVWGM AVREL (SEQ ID NO:22) | TGCCAGGCCCC GAGCAGGTGTGG GGCATGGCCGTG AGGGAGCTG (SEQ ID NO:30) |
| | | (SEQ ID NO:21) | | | |

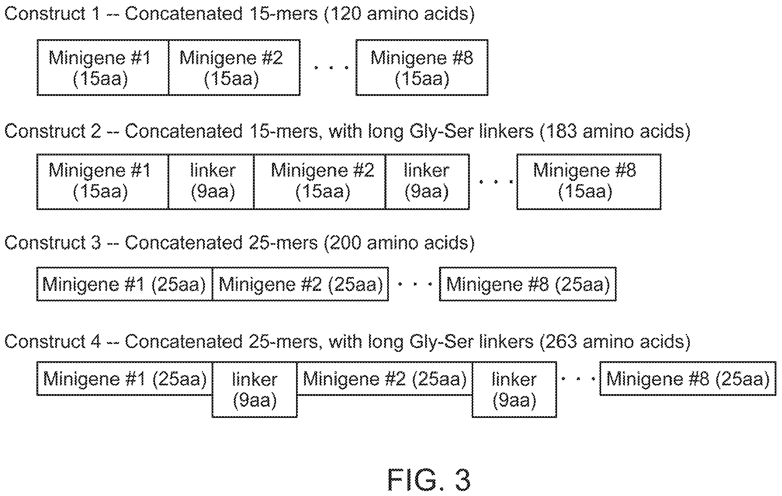

As shown in **FIG. 3****,** four gene constructs were designed for insertion of identified tumor neo-epitopes into Ad5 [E1-, E2b-] vector constructs:
(I) 15-aa minigenes are tumor mutations selected with 7-aa of native sequence on either side. (MHC Class I antigens targeted)
(II) 25-aa minigenes are tumor mutations selected with 12-aa of native sequence on either side. (MHC Class I targeted and MHC Class II antigens available, if present)
(III) 15-aa minigenes as in (I) above with 9-aa linkers between each minigene such that "unnatural" MHC Class I epitopes won't form between adjacent minigenes.
(IV) 25-aa minigenes as in (II) above with 9-aa linkers between each minigene such that "unnatural" MHC Class I epitopes won't form between adjacent minigenes

The nucleotide sequences and proteins sequences of these four gene constructs are represented below:

Gene Construct 1 (SEQ ID NO: 31):

CTCGAGGAAGCTTGCCGCCACCATGCCATTTGCCATGGCCCAGATCCCCAGC
CTGAGCCTGAGAGCTGTGCTGCCTAATGTGACCGTGCGGAACGCCACCAGCTACAG
ATGTGGCAGCTGGTTCAGAGAGGGCAAGACCCTGAACCCCAGCCAGACCAGCGCCG
TGATGGACTGGGTGTGGATGGACACCACCCTGTCCCTGAGCCTGGGCCCCGAGAGA
AGAAAGCTGACCACCGAGCTGACAATCATCAACAATGGCCTGGGCGCTCAGTGTAG
CGAGGCCGTGACCCTGAATAATGTGGGCGAGACAGTGACCATGCCCTGCCCCAAGG
TGTTCAGCTGCCAGGGCCCCGAACAAGTGTGGGGAATGGCTGTGCGCGAGCTGTGA
GATATCGCGGCCGC

Gene Construct 2 (SEQ ID NO: 32):

CTCGAGGAAGCTTGCCGCCACCATGCCATTTGCCATGGCCCAGATCCCCAGC
CTGAGCCTGAGAGCTGTGGGAAGCGGGAGTGGCTCAGGTTCAGGACTGCCTAATGT
GACCGTGCGGAACGCCACCAGCTACAGATGTGGCGGAAGTGGGTCAGGCTCCGGTT
CTGGAAGCTGGTTCAGAGAGGGCAAGACCCTGAACCCCAGCCAGACCAGCGGGTCA
GGAAGTGGTAGCGGCTCCGGGGCCGTGATGGACTGGGTGTGGATGGACACCACCCT
GTCCCTGAGCGGAAGTGGATCAGGTTCCGGCTCTGGGCTGGGCCCCGAGAGAAGAA
AGCTGACCACCGAGCTGACAATCATCGGATCCGGGTCTGGCAGTGGTTCAGGCAAC
AATGGCCTGGGCGCTCAGTGTAGCGAGGCCGTGACCCTGAATGGATCAGGGTCCGG
CAGCGGTAGTGGCAATGTGGGCGAGACAGTGACCATGCCCTGCCCCAAGGTGTTCA
GCGGGTCCGGATCTGGTAGTGGCTCAGGTTGCCAGGGCCCCGAACAAGTGTGGGGA
ATGGCTGTGCGCGAGCTGTGAGATATCGCGGCCGC

Gene Construct 3 (SEQ ID NO: 33):

CTCGAGGAAGCTTGCCGCCACCATGCCTAGCGAGAGCCCTCCATTTGCCATG
GCCCAGATCCCCAGCCTGAGCCTGAGAGCTGTGTCTGGCGCTATGGAGCCCACAG
CATCCCCCTGCCTAATGTGACCGTGCGGAACGCCACCAGCTACAGATGTGGCGTGG
GACCTCCTGGCCCTCCTGCTTCCCTGAGCTGGTTCAGAGAGGGCAAGACCCTGAACC
CCAGCCAGACCAGCATGAGCGGCACCCTGCTGACAGAGCTGAGAGCCGTGATGGAC
TGGGTGTGGATGGACACCACCCTGTCCCTGAGCAGCTGGATCTGTGTGGTGTCCGCC
ACAGACCTGGGCCCCGAGAGAAGAAAGCTGACCACCGAGCTGACAATCATCCTGCA
GGGCCTGGACTACAGCTGCGAGGCCAACAATGGCCTGGGCGCTCAGTGTAGCGAGG
CCGTGACCCTGAATTTCACCGTGCCCACCTGTTGGAGGCCCGCCAATGTGGGCGAGA
CAGTGACCATGCCCTGCCCCAAGGTGTTCAGCAACTTCTACAGCAAAGTGCGGGGCT
TCATGTGCCAGGGCCCCGAACAAGTGTGGGGAATGGCTGTGCGCGAGCTGAACATG
AACCTGCTGTGAGATATCGCGGCCGC

Gene Construct 4 (SEQ ID NO: 34):

CTCGAGGAAGCTTGCCGCCACCATGCCTAGCGAGAGCCCTCCATTTGCCATG
GCCCAGATCCCCAGCCTGAGCCTGAGAGCTGTGTCTGGCGCTATGGGAGGAAGCGG

GAGTGGCTCAGGTTCAGGAGCCCACAGCATCCCCCTGCCTAATGTGACCGTGCGGA
ACGCCACCAGCTACAGATGTGGCGTGGACCTCCTGGCGGAAGTGGGTCAGGCTCC
GGTTCTGGACCTCCTGCTTCCCTGAGCTGGTTCAGAGAGGGCAAGACCCTGAACCCC
AGCCAGACCAGCATGAGCGGCACCCTGGGGTCAGGAAGTGGTAGCGGCTCCGGGCT
GACAGAGCTGAGAGCCGTGATGGACTGGGTGTGGATGGACACCACCCTGTCCCTGA
GCAGCTGGATCTGTGTGGGAAGTGGATCAGGTTCCGGCTCTGGGGTGTCCGCCACAG
ACCTGGGCCCCGAGAGAAGAAAGCTGACCACCGAGCTGACAATCATCCTGCAGGGC
CTGGACGGATCCGGGTCTGGCAGTGGTTCAGGCTACAGCTGCGAGGCCAACAATGG
CCTGGGCGCTCAGTGTAGCGAGGCCGTGACCCTGAATTTCACCGTGCCCACCGGATC
AGGGTCCGGCAGCGGTAGTGGCTGTTGGAGGCCCGCCAATGTGGGCGAGACAGTGA
CCATGCCCTGCCCCAAGGTGTTCAGCAACTTCTACAGCAAAGGGTCCGGATCTGGTA
GTGGCTCAGGTGTGCGGGGCTTCATGTGCCAGGGCCCCGAACAAGTGTGGGGAATG
GCTGTGCGCGAGCTGAACATGAACCTGCTGTGAGATATCGCGGCCGC

Construct 1 protein sequence (SEQ ID NO: 35):

MPFAMAQIPSLSLRAVLPNVTVRNATSYRCGSWFREGKTLNPSQTSAVMDWVW
MDTTLSLSLGPERRKLTTELTIINNGLGAQCSEAVTLNNVGETVTMPCPKVFSCQGPEQV
WGMAVREL

Construct 2 protein sequence (SEQ ID NO: 36):

MPFAMAQIPSLSLRAVGSGSGSGSGLPNVTVRNATSYRCGGSGSGSGSGSWFRE
GKTLNPSQTSGSGSGSGSGAVMDWVWMDTTLSLSGSGSGSGSGLGPERRKLTTELTIIG
SGSGSGSGNNGLGAQCSEAVTLNGSGSGSGSGNVGETVTMPCPKVFSGSGSGSGSGCQ
GPEQVWGMAVREL

Construct 3 protein sequence (SEQ ID NO: 37):

MPSESPPFAMAQIPSLSLRAVSGAMGAHSIPLPNVTVRNATSYRCGVGPPGPPAS
LSWFREGKTLNPSQTSMSGTLLTELRAVMDWVWMDTTLSLSSWICVVSATDLGPERRK
LTTELTIILQGLDYSCEANNGLGAQCSEAVTLNFTVPTCWRPANVGETVTMPCPKVFSN
FYSKVRGFMCQGPEQVWGMAVRELNMNLL

Construct 4 protein sequence (SEQ ID NO: 38):

MPSESPPFAMAQIPSLSLRAVSGAMGGSGSGSGSGAHSIPLPNVTVRNATSYRCG
VGPPGGSGSGSGSGPPASLSWFREGKTLNPSQTSMSGTLGSGSGSGSGLTELRAVMDWV
WMDTTLSLSSWICVGSGSGSGSGVSATDLGPERRKLTTELTIILQGLDGSGSGSGSGYSC
EANNGLGAQCSEAVTLNFTVPTGSGSGSGSGCWRPANVGETVTMPCPKVFSNFYSKGS
GSGSGSGVRGFMCQGPEQVWGMAVRELNMNLL

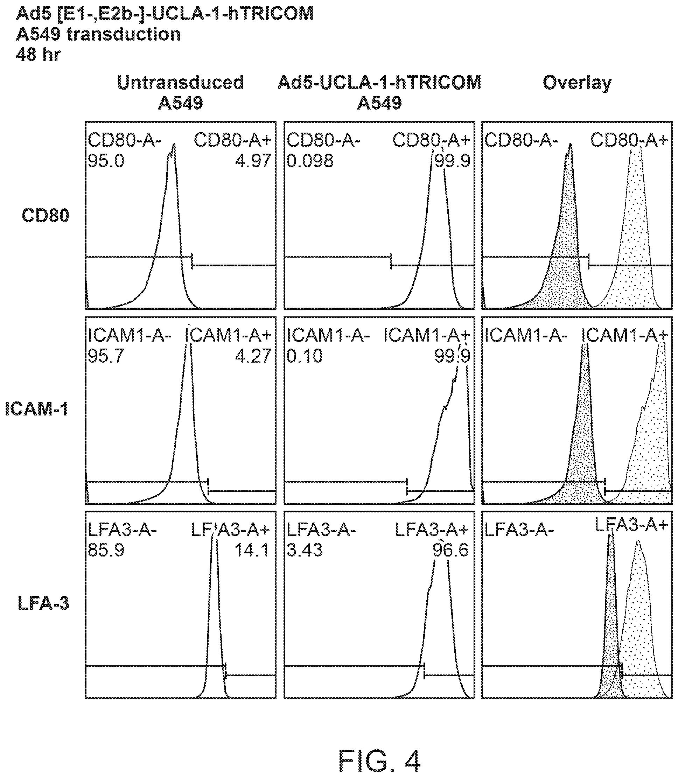

**FIG. 4** shows transfection of human A549 tumor cells with an Ad5 [E1-, E2b-] vector containing the neo-antigen gene 1 sequence with a Tricom reporter element at the end of the gene sequence that could be detected for expression in transfected cells. The rationale here is that if the reporter element is detected then the gene or genes before it must also be expressed.

### EXAMPLE 10

### Treatment of Cancer With an Ad5 [E1-, E2b-] Vector Encoding For a Tumor Neo-Epitope and an Immunological Fusion Partner

This example describes treatment of cancer with an Ad5 [E1-, E2b-] vector encoding for a tumor neo-epitope and an immunological fusion partner. An Ad5 [E1-, E2b-] vector encoding for any one of the tumor neo-epitopes of SEQ ID NO: 23 - SEQ ID NO: 30, any one of the immunological fusion partners of SEQ ID NO: 39 - SEQ ID NO: 90 or any other immunological fusion partner described herein such as IFN-γ, TNFα IL-2, IL-8, IL-12, IL-18, IL-7, IL-3, IL-4, IL-5, IL-6, IL-9, IL-10, IL-13, IL-15, , IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-a, LT-β, CD40 ligand, Fas ligand, CD27 ligand, CD30 ligand, 4-1BBL, Trail, OPG-L, APRIL, LIGHT, TWEAK, BAFF, TGF-β1, and MIF, and optionally, any one of the linkers of SEQ ID NO: 91 - SEQ ID NO: 105 is manufactured using the methods set forth in **EXAMPLE 1.** The Ad5 [E1-, E2b-] vector encoding for the neo-epitope and immunological fusion partner is administered as a therapeutic vaccine to a subject in need thereof. The subject is a mammal, such as a human or a non-human primate. The subject has a condition such any cancer. Establishment of cellular and humoral immunity, driving antibody and cell-mediated responses against the cancer, is induced after administration of the vaccine. The cancer condition is alleviated after administration of the vaccine.

### EXAMPLE 11

### Treatment of Cancer With an Ad5 [E1-, E2b-] Vector Encoding For a Tumor Neo-Epitope and ALT-803 (An Immunological Fusion Partner)

This example describes treatment of cancer with an Ad5 [E1-, E2b-] vector encoding for a tumor neo-epitope and ALT-803 (immunological fusion partner). An Ad5 [E1-, E2b-] vector encoding for any one of the tumor neo-epitopes of SEQ ID NO: 23 - SEQ ID NO: 30, ALT-803, and optionally, any one of the linkers of SEQ ID NO: 91 - SEQ ID NO: 105 is manufactured using the methods set forth in **EXAMPLE 1.** The Ad5 [E1-, E2b-] vector encoding for the neo-epitope and ALT-803 is administered as a therapeutic vaccine to a subject in need thereof. The subject is a mammal, such as a human or a non-human primate. The subject has a condition such as cancer. Establishment of cellular and humoral immunity, driving antibody and cell-mediated responses against the cancer, is induced after administration of the vaccine. The cancer condition is alleviated after administration of the vaccine.

### EXAMPLE 12

### Personalized Treatment of Cancer with an Ad5 [E1-, E2b-] Vector Encoding For a Tumor Neo-Epitope and an Immunological Fusion Partner

Tumor neo-epitopes are isolated from a patient with any tumor that has been treated with Ad5 [E1-, E2b-]-CEA (carcinoembryonic antigen). Tumor-specific mutations like SNVs are identified by comparing the genome sequence in a tumor biopsy of the patients with normal skin tissues. Expression of SNVs is determined, and then these SNVs are assessed for if they drive cell-mediated immunity. If the expressed neo-antigens drive cell-mediated immunity, then they are inserted into a library of Ad5 [E1-, E2b-] vectors with an immunological fusion partner for delivery to human cancer patients. The immunological fusion partner is selected from the immunological fusion partners of SEQ ID NO: 39 - SEQ ID NO: 90, SEQ ID NO: 109 - SEQ ID NO: 112, or any other immunological fusion partner described herein such as IFN-γ, TNFα IL-2, IL-8, IL-12, IL-18, IL-7, IL-3, IL-4, IL-5, IL-6, IL-9, IL-10, IL-13, IL-15, , IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IE-36α,β,λ, IL-36Ra, IL-37, TSLP, LIF, OSM, LT-α, LT-β, CD40 ligand, Fas ligand, CD27 ligand, CD30 ligand, 4-1BBL, Trail, OPG-L, APRIL, LIGHT, TWEAK, BAFF, TGF-β1, and MIF. The identification of new neo-antigens and treatment with newly identified new neo-antigens are repeated in cycles.

### EXAMPLE 13

### Personalized Treatment of Cancer with an Ad5 [E1-, E2b-] Vector Encoding For a Tumor Neo-Epitope and an Immunological Fusion Partner

Tumor neo-epitopes are isolated from a patient with any tumor that has been treated with Ad5 [E1-, E2b-]-CEA (carcinoembryonic antigen)-immunological fusion partner. The immunological fusion partner is selected from the immunological fusion partners of SEQ ID NO: 39 - SEQ ID NO: 90, SEQ ID NO: 109 - SEQ ID NO: 112, or any other immunological fusion partner described herein such as IFN-γ, TNFα IL-2, IL-8, IL-12, IL-18, IL-7, IL-3, IL-4, IL-5, IL-6, IL-9, IL-10, IL-13, IL-15, , IL-16, IL-17, IL-23, IL-32, M-CSF (CSF-1), IFN-α, IFN-β, IL-1α, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-a, LT-β, CD40 ligand, Fas ligand, CD27 ligand, CD30 ligand, 4-1BBL, Trail, OPG-L, APRIL, LIGHT, TWEAK, BAFF, TGF-β1, and MIF. Tumor-specific mutations like SNVs are identified by comparing the genome sequence in a tumor biopsy of the patients with normal skin tissues. Expression of SNVs is determined, and then these SNVs are assessed for if they drive cell-mediated immunity. If the expressed neo-antigens drive cell-mediated immunity, then they are inserted into a library of Ad5 [E1-, E2b-] vectors with an immunological fusion partner for delivery to human cancer patients. The immunological fusion partner is selected from the immunological fusion partners of SEQ ID NO: 39 - SEQ ID NO: 90, SEQ ID NO: 109 - SEQ ID NO: 112, or any other immunological fusion partner described herein such as IFN-γ, TNFα IL-2, IL-8, IL-12, IL-18, IL-7, IL-3, IL-4, IL-5, IL-6, IL-9, IL-10, IL-13, IL-15, , IL-16, IL-17, IL-23, IL-32, M-CSF (CSF-1), IFN-α, IFN-β, IL-la, IL-1β, IL-1RA, IL-11, IL-17A, IL-17F, IL-19, IL-20, IL-21, IL-22, IL-24, IL-25, IL-26, IL-27, IL-28A, B, IL-29, IL-30, IL-31, IL-33, IL-34, IL-35, IL-36α,β,λ, IL-36Ra, IL-37, TSLP, LIF, OSM, LT-a, LT-β, CD40 ligand, Fas ligand, CD27 ligand, CD30 ligand, 4-1BBL, Trail, OPG-L, APRIL, LIGHT, TWEAK, BAFF, TGF-β1, and MIF. The identification of new neo-antigens and treatment with newly identified new neo-antigens are repeated in cycles.

### EXAMPLE 14

### Treatment of Cancer With an Ad5 [E1-, E2b-] Vector Encoding For a Tumor Neo-Epitope and IL-16

This example describes treatment of cancer with an Ad5 [E1-, E2b-] vector encoding for a tumor neo-epitope and IL-16 . An Ad5 [E1-, E2b-] vector encoding for any one of the tumor neo-epitopes of SEQ ID NO: 23 - SEQ ID NO: 30; an immunological fusion partner of SEQ ID NO: 109; and optionally, any one of the linkers of SEQ ID NO: 91 - SEQ ID NO: 105 is manufactured using the methods set forth in **EXAMPLE 1.** The Ad5 [E1**-**, E2b-] vector encoding for the neo-epitope and SEQ ID NO: 109 is administered as a therapeutic vaccine to a subject in need thereof. The subject is a mammal, such as a human or a non-human primate. The subject has a condition such as cancer. Establishment of cellular and humoral immunity, which drives antibody and cell-mediated responses against the cancer, is induced after administration of the vaccine. The cancer condition is alleviated after administration of the vaccine.

### EXAMPLE 15

### Treatment of Cancer With an Ad5 [E1-, E2b-] Vector Encoding For a Tumor Neo-Epitope and IL-17

This example describes treatment of cancer with an Ad5 [E1-, E2b-] vector encoding for a tumor neo-epitope and IL-17. An Ad5 [E1-, E2b-] vector encoding for any one of the tumor neo-epitopes of SEQ ID NO: 23 - SEQ ID NO: 30; an immunological fusion partner of SEQ ID NO: 110; and optionally, any one of the linkers of SEQ ID NO: 91 - SEQ ID NO: 105 is manufactured using the methods set forth in **EXAMPLE 1.** The Ad5 [E1-, E2b-] vector encoding for the neo-epitope and SEQ ID NO: 110 is administered as a therapeutic vaccine to a subject in need thereof. The subject is a mammal, such as a human or a non-human primate. The subject has a condition such as cancer. Establishment of cellular and humoral immunity, which drives antibody and cell-mediated responses against the cancer, is induced after administration of the vaccine. The cancer condition is alleviated after administration of the vaccine.

### EXAMPLE 16

### Treatment of Cancer With an Ad5 [E1-, E2b-] Vector Encoding For a Tumor Neo-Epitope and IL-23

This example describes treatment of cancer with an Ad5 [E1-, E2b-] vector encoding for a tumor neo-epitope and IL-23. An Ad5 [E1-, E2b-] vector encoding for any one of the tumor neo-epitopes of SEQ ID NO: 23 - SEQ ID NO: 30; an immunological fusion partner of SEQ ID NO: 111; and optionally, any one of the linkers of SEQ ID NO: 91 - SEQ ID NO: 105 is manufactured using the methods set forth in **EXAMPLE 1.** The Ad5 [E1-, E2b-] vector encoding for the neo-epitope and SEQ ID NO: 111 is administered as a therapeutic vaccine to a subject in need thereof. The subject is a mammal, such as a human or a non-human primate. The subject has a condition such as cancer. Establishment of cellular and humoral immunity, which drives antibody and cell-mediated responses against the cancer, is induced after administration of the vaccine. The cancer condition is alleviated after administration of the vaccine.

### EXAMPLE 17

### Treatment of Cancer With an Ad5 [E1-, E2b-] Vector Encoding For a Tumor Neo-Epitope and IL-32

This example describes treatment of cancer with an Ad5 [E1-, E2b-] vector encoding for a tumor neo-epitope and IL-32. An Ad5 [E1-, E2b-] vector encoding for any one of the tumor neo-epitopes of SEQ ID NO: 23 - SEQ ID NO: 30; an immunological fusion partner of SEQ ID NO: 112; and optionally, any one of the linkers of SEQ ID NO: 91 - SEQ ID NO: 105 is manufactured using the methods set forth in **EXAMPLE 1.** The Ad5 **[E1-,** E2b-] vector encoding for the neo-epitope and SEQ ID NO: 112 is administered as a therapeutic vaccine to a subject in need thereof. The subject is a mammal, such as a human or a non-human primate. The subject has a condition such as cancer. Establishment of cellular and humoral immunity, which drives antibody and cell-mediated responses against the cancer, is induced after administration of the vaccine. The cancer condition is alleviated after administration of the vaccine.

All of the methods disclosed and claimed herein can be made and executed without undue experimentation in light of the present disclosure. While the compositions and methods of this invention have been described in terms of preferred embodiments, it will be apparent to those of skill in the art that variations may be applied to the methods and in the steps or in the sequence of steps of the method described herein without departing from the concept, spirit and scope of the invention. More specifically, it will be apparent that certain agents which are both chemically and physiologically related may be substituted for the agents described herein while the same or similar results would be achieved. All such similar substitutes and modifications apparent to those skilled in the art are deemed to be within the spirit, scope and concept of the invention as defined by the appended claims.

The invention described herein also relates to the following aspects:
1. A composition comprising a replication-defective vector, wherein the replication-defective vector comprises:
   a nucleic acid sequence encoding for a tumor neo-antigen; and
   a nucleic acid sequence encoding for an immunological fusion partner.
2. A composition comprising a replication-defective vector, wherein the replication-defective vector comprises:
   a nucleic acid sequence encoding for CEA, MUC1-c, Brachyury, or any combination thereof; and
   a nucleic acid sequence encoding for an immunological fusion partner.
3. The composition of any one of aspects 1-2 wherein the replication-defective vector is an adenovirus vector.
4. The composition of any one of aspects 1-3, wherein the adenovirus vector is an Ad5 vector.
5. The composition of any one of aspects 1-4, wherein the replication defective vector comprises a deletion in an E2b region, an E1 region, an E3 region, and E4 region, or any combination thereof.
6. The composition of any one of aspects 1-5, wherein the replication defective vector comprises a deletion in an E2b region.
7. The composition of any one of aspects 1-6, wherein the replication-defective vector comprises a deletion of a DNA polymerase, preterminal protein (pTP), or a combination thereof in an E2b region.
8. The composition of any one of aspects 1-7, wherein the replication-defective vector is not a gutted vector.
9. The composition of any one of aspects 1, or 3-8, wherein the replication-defective vector comprises a plurality of replication-defective vectors, wherein each replication-defective vector comprises a different tumor neo-antigen-coding sequence.
10. The composition of any one of aspects 1, or 3-9, wherein the replication-defective vector comprises at least ten replication-defective vectors, wherein each replication-defective vector comprises a different tumor neo-antigen-coding nucleic acid sequence.
11. The composition of any one of aspects 1, or 3-10, wherein the replication-defective vector comprises at least five replication-defective vectors, wherein each replication-defective vector comprises a different tumor neo-antigen-coding nucleic acid sequence.
12. The composition of any one of aspects 1-11, wherein the immunological fusion partner comprises *Mycobacterium sp., Mycobacterium tuberculosis*-derived Ra12 fragment, protein D derived from a surface protein of gram-negative bacterium *Haemophilus influenzae* B, LYTA, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, CpG-ODN, truncated A subunit coding region derived from bacterial ADP-ribosylating exotoxin, truncated B subunit coding region derived from bacterial ADP-ribosylating exotoxin, Hp91, CCL20, CCL3, GM-CSF, G-CSF, LPS peptide mimic, shiga toxin, diphtheria toxin, IL-15 super agonist, ALT-803, CRM₁₉₇, or any combination thereof.
13. The composition of any one of aspects 1-12, wherein the immunological fusion partner can be a fragment or derivative of *Mycobacterium sp., Mycobacterium tuberculosis*-derived Ra12 fragment, protein D derived from a surface protein of gram-negative bacterium *Haemophilus influenzae* B, LYTA, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, CpG-ODN, truncated A subunit coding region derived from bacterial ADP-ribosylating exotoxin, truncated B subunit coding region derived from bacterial ADP-ribosylating exotoxin, Hp91, CCL20, CCL3, GM-CSF, G-CSF, LPS peptide mimic, shiga toxin, diphtheria toxin, IL-15 super agonist, ALT-803, CRM₁₉₇, or any combination thereof.
14. The composition of any one of aspects 1-13, wherein the immunological fusion partner is at least 80%, at least 85%, at least 90%, at least 95%, or at least 90% identical to a sequence of any one of SEQ ID NO: 39 - SEQ ID NO: 90 or SEQ ID NO: 109 - SEQ ID NO: 112.
15. The composition of any one of aspects 1-14, wherein the replication-defective vector further comprises a nucleic acid sequence encoding for a linker.
16. The composition of aspect 15, wherein the linker is from 1 to about 150 nucleic acids long, from about 5 to about 100 nucleic acids long, or from about 10 to about 50 nucleic acids long.
17. The composition any one of aspects 15-16, wherein the nucleic acid sequence encodes an amino acid residue.
18. The composition of aspect 17, wherein the amino acid residues form an amino acid sequence.
19. The composition of aspect 18, wherein the amino acid sequence comprises 1 to about 50 amino acid residues, about 5 to about 25 amino acid residues, or less than 10 amino acid residues.
20. The composition of any one of aspects 15-19, wherein the linker is a polyalanine linker, or a polyglycine linker.
21. The composition of any one of aspects 15-20, wherein the linker comprises a mixture of alanines and glycines.
22. The composition of any one of aspects 15-22, wherein the nucleic acid sequence encoding for the linker is between the nucleic acid sequence encoding for the tumor neo-antigen and the nucleic acid sequence encoding for the immunological fusion partner.
23. The composition of any one of aspects 15, 18-19, or 21 wherein the linker is any one of SEQ ID NO: 91 - SEQ ID NO: 105.
24. The composition of any one of aspects 1, or 3-23, wherein the replication-defective vector comprises more than one nucleic acid sequences encoding more than one tumor neo-antigens.
25. The composition of any one of aspects 1-24, wherein the composition is a vaccine.
26. The composition of any one of aspects 1-25, wherein the composition comprises a pharmaceutically acceptable carrier.
27. The composition of any one of aspects 1-26, wherein the composition comprises at least ten adenovirus vectors.
28. The composition of any one of aspects 1-27, wherein the composition comprises at least ten adenovirus vectors.
29. The composition of any one of aspects 1, or 3-28, wherein the tumor neo-antigen comprises a tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, Her 4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TP1/mbcr-abl, ETV6/AML, LDLR/FUT, Pml/RARa, TEL/AML1, or any combination thereof.
30. The composition of any one of aspects 1, or 3-29, wherein the tumor neo-antigen comprises a tumor neo-epitope.
31. The composition of any one of aspects 1, or 3-30, wherein the tumor neo-antigen comprises CEA, MUC1, Brachyury, PSA, PSMA, Her2/neu, Her3, HPV-E6, HPV-E7, or any combination thereof.
32. The composition of any one of aspects 1, or 3-31, wherein the tumor neo-antigen is a tumor neo-epitope with an amino acid sequence of any one of SEQ ID NO: 1 - SEQ ID NO: 22, a nucleotide sequence of any one of SEQ ID NO: 23 - SEQ ID NO: 30, or has one of the following mutation: Q678P mutation of gene SLC4A11, D1143N mutation of gene SIGLEC1, A292T mutation of gene SIGLEC14, T2356M mutation of PIEZO2, S1613L mutation of gene FAT4, R268C mutation of gene FCRL1, or V73M mutation of gene VIPR2, or R346W mutation of gene FLRT2.
33. The composition of any one of aspects 1-32, wherein the replication-defective vector further comprises a nucleic acid sequence encoding a costimulatory molecule.
34. The composition of aspect 33, wherein the costimulatory molecule comprises B7, ICAM-1, LFA-3, or any combinations thereof.
35. The composition of any one of aspects 1-34, wherein the composition additionally comprises an engineered natural killer (NK) cell.
36. The composition of aspect 35, wherein the engineered NK cell comprises an NK cell that has been modified as essentially lacking the expression of KIR (killer inhibitory receptors), an NK cell that has been modified to express a high affinity CD16 variant, and an NK cell that has been modified to express a CAR (chimeric antigen receptor), or any combinations thereof.
37. The composition of any one of aspects 35-36, wherein the engineered NK cells comprise an NK cell that has been modified as essentially lacking the expression KIR.
38. The composition of any one of aspects 35-37, wherein the engineered NK cells comprise an NK cell that has been modified to express a high affinity CD16 variant.
39. The composition of any one of aspects 35-38, wherein the engineered NK cells comprise an NK cell that has been modified to express a CAR.
40. The composition of aspect 39, wherein the CAR is a CAR for a tumor neo-antigen, tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, HER4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TP1/mbcr-abl, ETV6/AML, LDLR/FUT, Pml/RARa, or TEL/AML1.
41. The composition of any one of aspects 1-40, wherein the composition additionally comprises an immunostimulant.
42. The composition of aspect 41, wherein the immunostimulant is selected from the group consisting of granulocyte macrophage colony-stimulating factor (GM-CSF), granulocyte-colony stimulating factor (G-CSF), interferon-gamma (IFN-γ), tumor necrosis factor-alpha (TNF-α), interleukin-2 (IL-2), IL-7, IL-4, IL-5, IL-6, IL-10, IL-12, IL-15, IL-16, IL-17, IL-23, and IL-32.
43. The composition of any one of aspects 1-42, wherein the composition additionally comprises a therapeutically effective amount of IL-15 or a replication defective vector comprising a nucleic acid sequence encoding IL-15.
44. The composition of any one of aspects 1-43, wherein the composition additionally comprises a cancer therapy.
45. The composition of aspect 44, wherein the cancer therapy is a dose of chemotherapy, a dose of radiation, an immunotherapy, or any combination thereof.
46. The composition of aspect 45, wherein the immunotherapy comprises a therapeutically effective amount of a composition comprising a replication-defective vector comprising a nucleic acid sequence encoding a tumor-associated antigen or a tumor neo-antigen.
47. The composition of any one of aspects 45-46, wherein the combination of the dose of chemotherapy and the dose of radiation is present in the composition at a dose lower than a recommended dose if the dose of chemotherapy and the dose of radiation were present alone.
48. The composition of any one of aspects 44-47, wherein the cancer therapy is an anti-PD-1 antibody pembrolizumab.
49. The composition of any one of aspects 1-48, wherein the composition additionally comprises a therapeutically effective amount of an immune pathway checkpoint inhibitor.
50. The composition of aspect 49, wherein the immune pathway checkpoint inhibitor comprises a therapeutically effective of a composition comprising an antibody that binds to an immune pathway checkpoint ligand.
51. The composition of any one of aspects 49-50, wherein the immune pathway checkpoint inhibitor is an antibody that binds PD-1, PD-L1, CTLA-4, LAG-3, or IDO.
52. The composition of any one of aspects 1-51, wherein the replication-defective vector further comprises a nucleic acid sequence encoding a reporter.
53. The composition of any one of aspects 1, or 3-52, wherein the replication-defective vector comprises a nucleic acid sequence encoding a tumor neo-antigen attached to a nucleic acid sequence encoding a reporter.
54. The composition of any one of aspects 1-53, wherein the replication-defective vector is present at a dose that is greater than or equal to 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6 x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7 x10¹¹, 8x10¹¹, 9x10¹¹, 1x10¹², 1.5x10¹², 2x10¹², 3x10¹², or more virus particles (VPs).
55. The composition of any one of aspects 1-54, wherein the replication-defective vector is present at a dose that is less than or equal to 1x10⁹, 2 x10⁹, 3 x10⁹, 4 x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6 x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7 x10¹¹, 8x10¹¹, 9x10¹¹, 1x10¹², 1.5x10¹², 2 x10¹², 3x10¹², 4x10¹², 5x10¹², or more virus particles per immunization.
56. The composition of any one of aspects 1-55, wherein the tumor antigen is specific for a subject.
57. The composition of any one of aspects 1, or 3-56, wherein the tumor neo-antigen is encoded by a nucleotide sequence comprising a tumor-specific single nucleotide variant (SNV).
58. The composition of any one of aspects 1, or 3-57, wherein the tumor neo-antigen drives cell-mediated immune response or is determined to drive cell-mediated immune response.
59. The composition of any one of aspects 1, or 3-58, wherein the tumor neo-antigen is a peptide of having a size of six to ten amino acids.
60. The composition of any one of aspects 2-59, wherein CEA comprises a sequence that has at least 80%, at least 85%, at least 90%, at least 92%, at least 95%, or at least 99% sequence identity to SEQ ID NO: 106.
61. The composition of any one of aspects 2-60, wherein MUC1-c comprises a sequence that has at least 80%, at least 85%, at least 90%, at least 92%, at least 95%, or at least 99% sequence identity to SEQ ID NO: 107.
62. The composition of any one of aspects 2-61, wherein Brachyury comprises a sequence that has at least 80%, at least 85%, at least 90%, at least 92%, at least 95%, or at least 99% sequence identity to SEQ ID NO: 108.
63. A composition comprising a cell comprising the composition of any one of aspects 1-62.
64. The composition of aspect 63, wherein the cell is a dendritic cell (DC).
65. A method of treating a cancer in a subject in need thereof, the method comprising administering to the subject the composition of any one of aspects 1-64.
66. A method of treating a cancer in a subject in need thereof, the method comprising administering to the subject a pharmaceutical composition, the pharmaceutical composition comprising a replication-defective vector, wherein the replication-defective vector comprises
   a nucleic acid sequence encoding for a tumor neo-antigen; and
   a nucleic acid sequence encoding for an immunological fusion partner.
67. A method of monitoring the status of a subject, the method comprising:
   a) administering to a subject a pharmaceutical composition, the pharmaceutical composition comprising a replication-defective vector, wherein the replication-defective vector comprises:
      a nucleic acid sequence encoding for a tumor neo-antigen of the subject, and
      a nucleic acid sequence encoding for an immunological fusion partner; and
   b) monitoring the status of the tumor neo-antigen in the subj ect.
68. A method of detecting a tumor in a subject, the method comprising:
   a) administering to the subject a pharmaceutical composition, the pharmaceutical composition comprising a replication-defective vector, wherein the replication-defective vector comprises:
      a nucleic acid sequence encoding for a tumor neo-antigens, and
      a nucleic acid sequence encoding for an immunological fusion partner; and
   b) detecting the presence or absence of the tumor neo-antigen in the subject following the administering.
69. A method of treating a cancer in a subject in need thereof, the method comprising:
   a) administering to the subject a cancer therapy;
   b) detecting the presence of a tumor neo-antigen in the subject;
   c) administering to the subject a pharmaceutical composition, the pharmaceutical composition comprising a replication-defective vector, wherein the replication-defective vector comprises
      a nucleic acid sequence encoding for a tumor neo-antigen and
      a nucleic acid sequence encoding for an immunological fusion partner; and
   d) repeating steps b) - c).
70. A method of treating a cancer in a subject in need thereof, the method comprising administering to the subject a pharmaceutical composition, the pharmaceutical comprising a population of cells, wherein a cell in the population of cells comprises a replication-defective vector, and wherein the replication-defective vector comprises:
   a nucleic acid sequence encoding for a tumor neo-antigen, and
   a nucleic acid sequence encoding for an immunological fusion partner.
71. A method of treating a cancer in a subject in need thereof, the method comprising administering to the subject a pharmaceutical composition, the pharmaceutical composition comprising a replication-defective vector, wherein the replication-defective vector comprises:
   a nucleic acid sequence encoding for CEA, MUC1-c, Brachyury, or any combination thereof; and
   a nucleic acid sequence encoding for an immunological fusion partner.
72. A method of monitoring the status of a subject, the method comprising:
   a) administering to a subject a pharmaceutical composition, the pharmaceutical composition comprising a replication-defective vector, wherein the replication-defective vector comprises:
      a nucleic acid sequence encoding for CEA, MUC1-c, Brachyury, or any combination thereof, and
      a nucleic acid sequence encoding for an immunological fusion partner; and
   b) monitoring the status of the CEA, MUC1-c, Brachyury, or any combination thereof in the subject.
73. A method of detecting a tumor in a subject, the method comprising:
   a) administering to the subject a pharmaceutical composition, the pharmaceutical composition comprising a replication-defective vector, wherein the replication-defective vector comprises:
      a nucleic acid sequence encoding for CEA, MUC1-c, Brachyury, or any combination thereof, and
      a nucleic acid sequence encoding for an immunological fusion partner; and
   b) detecting the presence or absence of the CEA, MUC1-c, Brachyury, or any combination thereof in the subject following the administering.
74. A method of treating a cancer in a subject in need thereof, the method comprising:
   a) administering to the subject a cancer therapy;
   b) detecting the presence of CEA, MUC1-c, Brachyury, or any combination thereof in the subject;
   c) administering to the subject a pharmaceutical composition, the pharmaceutical composition comprising a replication-defective vector, wherein the replication-defective vector comprises:
      a nucleic acid sequence encoding for CEA, MUC1-c, Brachyury, or any combination thereof, and
      a nucleic acid sequence encoding for an immunological fusion partner; and
   d) repeating steps b) - c).
75. A method of treating a cancer in a subject in need thereof, the method comprising administering to the subject a pharmaceutical composition, the pharmaceutical comprising a population of cells, wherein a cell in the population of cells comprises a replication-defective vector, and wherein the replication-defective vector comprises:
   a nucleic acid sequence encoding for CEA, MUC1-c, Brachyury, or any combination thereof, and
   a nucleic acid sequence encoding for an immunological fusion partner.
76. The method of any one of aspects 66-75, wherein the replication-defective vector is an adenovirus vector.
77. The method of any one of aspects 66-76, wherein the adenovirus vector is an Ad5 vector.
78. The method of any one of aspects 66-77, wherein the replication defective vector comprises a deletion in an E2b region, an E1 region, an E3 region, and E4 region, or any combination thereof.
79. The method of any one of aspects 66-78, wherein the replication defective vector comprises a deletion in an E2b region.
80. The method of any one of aspects 66-79, wherein the replication-defective vector comprises a deletion of a DNA polymerase, preterminal protein (pTP), or a combination thereof in an E2b region.
81. The method of any one of aspects 66-80, wherein the replication-defective vector is not a gutted vector.
82. The method of any one of aspects 66-70 or 76-81, wherein the replication-defective vector comprises a plurality of replication-defective vectors, wherein each replication-defective vector comprises a different tumor neo-antigen-coding sequence.
83. The method of any one of aspects 66-70 or 76-82, wherein the replication-defective vector comprises at least ten replication-defective vectors, wherein each replication-defective vector comprises a different tumor neo-antigen-coding sequence.
84. The method of any one of aspects 66-70 or 76-83, wherein the replication-defective vector comprises at least five replication-defective vectors, wherein each replication-defective vector comprises a different tumor neo-antigen-coding sequence.
85. The method of any one of aspects 66-84, wherein the immunological fusion partner comprises *Mycobacterium sp., Mycobacterium tuberculosis-derived* Ra12 fragment, protein D derived from a surface protein of gram-negative bacterium *Haemophilus influenzae* B, LYTA, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, CpG-ODN, truncated A subunit coding region derived from bacterial ADP-ribosylating exotoxin, truncated B subunit coding region derived from bacterial ADP-ribosylating exotoxin, Hp91, CCL20, CCL3, GM-CSF, G-CSF, LPS peptide mimic, shiga toxin, diphtheria toxin, IL-15 super agonist, ALT-803, CRM₁₉₇, or any combination thereof.
86. The method of any one of aspects 66-85, wherein the immunological fusion partner can be a fragment or derivative of *Mycobacterium sp., Mycobacterium tuberculosis*-derived Ra12 fragment, protein D derived from a surface protein of gram-negative bacterium *Haemophilus influenzae* B, LYTA, IFN-γ, TNFα, IL-2, IL-8, IL-12, IL-18, IL-7, IL-3, IL-4, IL-5, IL-6, IL-9, IL-10, IL-13, IL-15, IL-16, IL-17, IL-23, IL-32, CpG-ODN, truncated A subunit coding region derived from bacterial ADP-ribosylating exotoxin, truncated B subunit coding region derived from bacterial ADP-ribosylating exotoxin, Hp91, CCL20, CCL3, GM-CSF, G-CSF, LPS peptide mimic, shiga toxin, diphtheria toxin, IL-15 super agonist, ALT-803, CRM₁₉₇, or any combination thereof.
87. The method of any one of aspects 66-86, wherein the immunological fusion partner is at least 80%, at least 85%, at least 90%, at least 95%, or at least 90% identical to a sequence of any one of SEQ ID NO: 39 - SEQ ID NO: 90 or SEQ ID NO: 109 - SEQ ID NO: 112.
88. The method of any one of aspects 66-87, wherein the replication-defective vector further comprises a nucleic acid sequence encoding for a linker.
89. The method of aspect 88, wherein the linker is from 1 to about 150 nucleic acids long, from about 5 to about 100 nucleic acids long, or from about 10 to about 50 nucleic acids long.
90. The method of any one of aspects 88-89, wherein the nucleic acid sequence encodes an amino acid residue.
91. The method of aspect 90, wherein the amino acid residues form an amino acid sequence.
92. The method of aspect 91, wherein the amino acid sequence comprises 1 to about 50 amino acid residues, about 5 to about 25 amino acid residues, or less than 10 amino acid residues.
93. The method of any one of aspects 88-92, wherein the linker is a polyalanine linker, a polyglycine linker.
94. The method of any one of aspects 88-92, wherein the linker comprises a mixture of alanines and glycines.
95. The method of any one of aspects 88-94, wherein the nucleic acid sequence encoding for the linker is between the nucleic acid sequence encoding for the tumor neo-antigen and the nucleic acid sequence encoding for the immunological fusion partner.
96. The method of any one of aspects 88, 91-92, or 95 wherein the linker is any one of SEQ ID NO: 91 - SEQ ID NO: 105.
97. The method of any one of aspects 66-70 or 76-96, wherein the replication-defective vector comprises more than one nucleic acid sequences encoding a tumor neo-antigen.
98. The method of any one of aspects 66-97, wherein the pharmaceutical composition is a vaccine.
99. The method of any one of aspects 66-98, wherein the pharmaceutical composition comprises a pharmaceutically acceptable carrier.
100. The method of any one of aspects 66-99, wherein the pharmaceutical composition comprises at least ten adenovirus vectors.
101. The method of any one of aspects 66-100, wherein the pharmaceutical composition comprises at least five adenovirus vectors.
102. The method of any one of aspects 66-101, wherein the tumor neo-antigen comprises a tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, Her 4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TP1/mbcr-abl, ETV6/AML, LDLR/FUT, Pml/RARa, TEL/AML1, or any combination thereof.
103. The method of any one of aspects 66-102, wherein the tumor antigen comprises a tumor neo-epitope.
104. The method of any one of aspects 66-103, wherein the tumor neo-antigen comprises CEA, MUC1, Brachyury, PSA, PSMA, Her2/neu, Her3, HPV-E6, HPV-E7, or any combination thereof.
105. The method of any one of aspects 66-104, wherein the tumor neo-antigen is a tumor neo-epitope with an amino acid sequence of any one of SEQ ID NO: 1 - SEQ ID NO: 22, a nucleotide sequence of any one of SEQ ID NO: 23 - SEQ ID NO: 30, or has one of the following mutation: Q678P mutation of gene SLC4A11, D1143N mutation of gene SIGLEC1, A292T mutation of gene SIGLEC14, T2356M mutation of PIEZO2, S1613L mutation of gene FAT4, R268C mutation of gene FCRL1, or V73M mutation of gene VIPR2, or R346W mutation of gene FLRT2.
106. The method of any one of aspects 66-105, wherein the replication-defective vector further comprises a nucleic acid sequence encoding a costimulatory molecule.
107. The method of any one of aspects 66-106, wherein the costimulatory molecule comprises B7, ICAM-1, LFA-3, or any combinations thereof.
108. The method of any one of aspects 66-107, further comprising administering to the subject an additional pharmaceutical composition comprising an engineered natural killer (NK) cell.
109. The method of aspects 108, wherein the engineered NK cell comprises an NK cell that has been modified as essentially lacking the expression of KIR (killer inhibitory receptors), an NK cell that has been modified to express a high affinity CD16 variant, and an NK cell that has been modified to express a CAR (chimeric antigen receptor), or any combinations thereof.
110. The method of any one of aspects 108-109, wherein the engineered NK cells comprise an NK cell that has been modified as essentially lacking the expression KIR.
111. The method of any one of aspects 108-110, wherein the engineered NK cells comprise an NK cell that has been modified to express a high affinity CD16 variant.
112. The method of any one of aspects 108-111, wherein the engineered NK cells comprise an NK cell that has been modified to express a CAR.
113. The method of aspects 112, wherein the CAR is a CAR for a tumor neo-antigen, tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, HER4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TP1/mbcr-abl, ETV6/AML, LDLR/FUT, Pml/RARa, or TEL/AML1.
114. The method of any one of aspects 1-113, wherein the method further comprising administering to the subject an additional pharmaceutical composition comprising an immunostimulant.
115. The method of aspect 114, wherein the immunostimulant is selected from the group consisting of granulocyte macrophage colony-stimulating factor (GM-CSF), granulocyte-colony stimulating factor (G-CSF), interferon-gamma (IFN-γ), tumor necrosis factor-alpha (TNF-α), interleukin-2 (IL-2), IL-7, IL-4, IL-5, IL-6, IL-10, IL-12, IL-15, IL-16, IL-17, IL-23, IL-32.
116. The method of any one of aspects 66-115, further comprising administering to the subject an additional pharmaceutical composition comprising a therapeutically effective amount of IL-15 or a replication defective vector comprising a nucleic acid sequence encoding IL-15.
117. The method of any one of aspects 66-70 or 75-116, wherein the subject has been administered a cancer therapy before the subject has been determined to have the tumor neo-antigen.
118. The method of aspect 117, wherein the cancer therapy is chemotherapy, radiation treatment, an immunotherapy, or any combination thereof.
119. The method of aspects 118, wherein the immunotherapy comprises a therapeutically effective amount of a composition comprising a replication-defective vector comprising a nucleic acid sequence encoding a tumor-associated antigen or a tumor neo-antigen.
120. The method of any one of aspects 118-119, wherein the combination of chemotherapy and radiation is administered at a dose lower than a recommended dose if the chemotherapy and radiation were administered alone.
121. The method of any one of aspects 117-120, wherein the cancer therapy is an anti-PD-1 antibody pembrolizumab.
122. The method of any one of aspects 66-121, further comprising administering to the subject an additional pharmaceutical composition comprising a therapeutically effective amount of an immune pathway checkpoint inhibitor.
123. The method of aspect 122, wherein the immune pathway checkpoint inhibitor comprises a therapeutically effective of a composition comprising an antibody that binds to an immune pathway checkpoint ligand.
124. The method of any one of aspects 122-123, wherein the immune pathway checkpoint inhibitor is an antibody that binds PD-1, PD-L1, CTLA-4, LAG-3, or IDO.
125. The method of any one of aspects 66-124, wherein the replication-defective vector comprises a nucleic acid sequence encoding a reporter.
126. The method of any one of aspects 66-70 or 76-125, wherein the replication-defective vector comprises a nucleic acid sequence encoding a tumor neo-antigen attached to a nucleic acid sequence encoding a reporter.
127. The method of any one of aspects 66-126, wherein the replication-defective vector is present at a dose that is greater than or equal to 1x10⁹, 2x10⁹, 3x10⁹, 4x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6 x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7 x10¹¹, 8x10¹¹, 9x10¹¹, 1x10¹², 1.5x10¹², 2 x10¹², 3x10¹², or more virus particles (VPs).
128. The method of any one of aspects 66-127, wherein the replication-defective vector is present at a dose that is less than or equal to 1x10⁹, 2x10⁹, 3x10⁹, 4 x10⁹, 5x10⁹, 6x10⁹, 7x10⁹, 8x10⁹, 9x10⁹, 1x10¹⁰, 2x10¹⁰, 3x10¹⁰, 4x10¹⁰, 5x10¹⁰, 6 x10¹⁰, 7x10¹⁰, 8x10¹⁰, 9x10¹⁰, 1x10¹¹, 2x10¹¹, 3x10¹¹, 4x10¹¹, 5x10¹¹, 6x10¹¹, 7 x10¹¹, 8x10¹¹, 9x10¹¹, 1x10¹², 1.5x10¹², 2x10¹², 3x10¹², 4x10¹², 5x10¹², or more virus particles per immunization.
129. The method of any one of aspects 66-70 or 77-128, wherein the tumor neo-antigen is specific for a subject.
130. The method of any one of aspects 66-70 or 76-129, wherein the tumor neo-antigen is encoded by a nucleotide sequence comprising a tumor-specific single nucleotide variant (SNV).
131. The method of any one of aspects 66-70 or 76-130, wherein the tumor neo-antigen drives cell-mediated immune response or is determined to drive cell-mediated immune response.
132. The method of any one of aspects 66-70 or 76-131, wherein the tumor neo-antigen is a peptide of having a size of six to ten amino acids.
133. The method of any one of aspects 66-70 or 76-132, wherein the subject has the tumor neo-antigen before the administering.
134. The method of any one of aspects 66-70 or 76-133, further comprising determining whether the subject develops a new neo-antigen during or after administration.
135. The method of any one of aspects 66-70 or 76-134, wherein the tumor neo-antigen has been identified as a result of comparing a genomic profile of a tumor sample of the subject to a reference.
136. The method of any one of aspects 66-135, further comprising obtaining a genomic profile of a tumor sample of the subject.
137. The method of any one of aspects 66-136, further comprising comparing a genomic profile of a tumor sample of the subject to a reference to identify tumor-specific mutations.
138. The method of aspects 136, wherein obtaining a genomic profile comprises next-generation sequencing, whole-exosome sequencing, sequencing by synthesis, sequencing by litigation, single-molecule sequencing, nano-technology for single-molecule sequencing, ion semiconductor sequencing or a combination thereof.
139. The method of 66-138, further comprising identifying tumor neo-antigens from tumor-specific mutations.
140. The method of aspects 139, wherein identifying tumor neo-antigens comprises the use of proteomics.
141. The method of any one of aspects 66-140, further comprising identifying tumor neo-epitopes.
142. The method of any one of aspects 66-141, further comprising accessing a database to obtain a previously stored genomic profile of the subject or a reference.
143. The method of any one of aspects 66-142, further comprising identifying additional tumor neo-antigens in the subject after administering the pharmaceutical composition.
144. The method of any one of aspects 66-143, further comprising identifying an additional tumor neo-antigen in the subject after administering the pharmaceutical composition, administering an additional pharmaceutical composition, the additional pharmaceutical composition comprising an additional replication-defective vector comprising an additional tumor neo-antigens to the subject.
145. The method of any one of aspects 66-70 or 75-144, further comprising monitoring the status of tumor neo-antigens in the subject that has been administered the pharmaceutical composition.
146. The method of any one of aspects 66-145, wherein the cancer is a pancreatic cancer, colorectal cancer, breast cancer, breast cancer, lung cancer, prostate cancer, gastric cancer, liver cancer, ovarian cancer, cervical cancer, head and neck squamous cell carcinoma or any combinations thereof.
147. The method of any one of aspects 66-146, wherein the tumor neo-antigen is more than one tumor neo-antigen present in the subject.
148. The method of any one of aspects 108-113, 114-115, 116-121, or 122-124, wherein the pharmaceutical composition and the additional pharmaceutical composition are administered at the same time.
149. The method of any one of aspects 70-148, wherein the cell is a dendritic cell (DC).
150. The method of any one of aspects 71-149, wherein CEA comprises a sequence that has at least 80%, at least 85%, at least 90%, at least 92%, at least 95%, or at least 99% sequence identity to SEQ ID NO: 106.
151. The composition of any one of aspects 71-150, wherein MUC1-c comprises a sequence that has at least 80%, at least 85%, at least 90%, at least 92%, at least 95%, or at least 99% sequence identity to SEQ ID NO: 107.
152. The composition of any one of aspects 71-151, wherein Brachyury comprises a sequence that has at least 80%, at least 85%, at least 90%, at least 92%, at least 95%, or at least 99% sequence identity to SEQ ID NO: 108.
153. A method of treating a cancer in a subject in need thereof, the method comprising,
   administering to the subject a first pharmaceutical composition, the pharmaceutical composition comprising a first replication-defective vector, wherein the first replication-defective vector comprises:
      a nucleic acid sequence encoding for CEA, MUC1-c, Brachyury, or any combination thereof; or
      the composition of any one of aspects 2-8, 12-23, 25-28, 33-56, or 60-62; and
   administering to the subject a second pharmaceutical composition, the second pharmaceutical composition comprising a second replication-defective vector, wherein the replication-defective vector comprises a nucleic acid sequence encoding composition of any one of aspects 1 or 3-59.

## Claims

1. A composition comprising a replication-defective vector, wherein the replication-defective vector comprises:
a nucleic acid sequence encoding for a tumor neo-antigen; and
a nucleic acid sequence encoding for ALT-803.

2. The composition of claim 1, wherein the replication-defective vector is an adenovirus vector.

3. The composition of any one of claims 1-2, wherein the replication defective vector comprises a deletion in an E2b region, an E1 region, an E3 region, and E4 region, or any combination thereof.

4. The composition of any one of claims 1-3, wherein the replication-defective vector is not a gutted vector.

5. The composition of any one of claims 1-4, wherein the ALT-803 is at least 80%, at least 85%, at least 90%, at least 95%, or at least 99% identical to SEQ ID NO: 88 or SEQ ID NO: 89.

6. The composition of any one of claims 1-5, wherein the replication-defective vector further comprises a nucleic acid sequence encoding for a linker, a costimulatory molecule or a reporter, or wherein the composition further comprises an engineered natural killer (NK) cell, an immunostimulant, a cancer therapy, an immune pathway checkpoint inhibitor, or a combination thereof.

7. The composition of claim 6, wherein the linker is a polyalanine linker, or a polyglycine linker, or comprises a mixture of alanines and glycines, or wherein the linker is any one of SEQ ID NO: 91 - SEQ ID NO: 105.

8. The composition of any one of claims 1-7, wherein the composition comprises at least ten adenovirus vectors.

9. The composition of any one of claims 1-8, wherein the tumor neo-antigen comprises a tumor neo-epitope, WT1, HPV-E6, HPV-E7, p53, MAGE-A1, MAGE-A2, MAGE-A3, MAGE-A4, MAGE-A6, MAGE-A10, MAGE-A12, BAGE, DAM-6, DAM-10, Folate receptor alpha, GAGE-1, GAGE-2, GAGE-8, GAGE-3, GAGE-4, GAGE-5, GAGE-6, GAGE-7B, NA88-A, NY-ESO-1, MART-1, MC1R, Gp100, PSA, PSM, Tyrosinase, TRP-1, TRP-2, ART-4, CAMEL, CEA, Cyp-B, Her1, Her2/neu, Her3, Her 4, BRCA1, Brachyury, Brachyury (TIVS7-2, polymorphism), Brachyury (IVS7 T/C polymorphism), T Brachyury, T, hTERT, hTRT, iCE, MUC1, MUC1 (VNTR polymorphism), MUC1c, MUC1n, MUC2, PRAME, P15, PSCA, PSMA, RU1, RU2, SART-1, SART-3, AFP, β-catenin/m, Caspase-8/m, CDK-4/m, ELF2M, GnT-V, G250, HSP70-2M, HST-2, KIAA0205, MUM-1, MUM-2, MUM-3, Myosin/m, RAGE, SART-2, TRP-2/INT2, 707-AP, Annexin II, CDC27/m, TPl/mbcr-abl, ETV6/AML, LDLR/FUT, Pml/RARa, TEL/AML1, or any combination thereof.

10. The composition of any one of claims 1-9, wherein the tumor neo-antigen is a tumor neo-epitope with an amino acid sequence of any one of SEQ ID NO: 1 - SEQ ID NO: 22, a nucleotide sequence of any one of SEQ ID NO: 23 - SEQ ID NO: 30, or has one of the following mutation: Q678P mutation of gene SLC4A11, D1143N mutation of gene SIGLEC1, A292T mutation of gene SIGLEC14, T2356M mutation of PIEZO2, S1613L mutation of gene FAT4, R268C mutation of gene FCRL1, or V73M mutation of gene VIPR2, or R346W mutation of gene FLRT2.

11. A composition comprising a cell comprising the composition of any one of claims 1-10.

12. The composition of any one of claims 1-11, for use in a method of treating a cancer in a subject in need thereof.

13. A method of detecting a tumor in a subject, the method comprising:
detecting the presence or absence of a tumor neo-antigen in a sample obtained from the subject, wherein a pharmaceutical composition has previously been administered to the subject; the pharmaceutical composition comprising a replication-defective vector comprising:
a nucleic acid sequence encoding for the tumor neo-antigens, and
a nucleic acid sequence encoding for ALT-803.
